(19) Europäisches Patentamt
European Patent Office
Office européen des brevets

(11) EP 3 922 246 A1

(12) EUROPEAN PATENT APPLICATION

(43) Date of publication:
**15.12.2021 Bulletin 2021/50**

(21) Application number: **21158407.3**

(22) Date of filing: **20.03.2015**

(51) Int Cl.:
***A61K 31/4178*** *(2006.01)* ***A61K 31/513*** *(2006.01)*
***A61K 31/55*** *(2006.01)* ***A61K 31/4164*** *(2006.01)*
***A61K 31/167*** *(2006.01)* ***A61P 1/16*** *(2006.01)*
***A61P 13/12*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **21.03.2014 US 201461968829 P**
**15.07.2014 US 201462024713 P**
**10.02.2015 US 201562114304 P**

(62) Document number(s) of the earlier application(s) in accordance with Art. 76 EPC:
**15765327.0 / 3 119 401**

(71) Applicant: **Tobira Therapeutics, Inc.**
**South San Francisco, CA 94080 (US)**

(72) Inventor: **Lefebvre,, Eric**
**South San Francisco, CA California 94080 (US)**

(74) Representative: **J A Kemp LLP**
**80 Turnmill Street**
**London EC1M 5QU (GB)**

Remarks:
•This application was filed on 22-02-2021 as a divisional application to the application mentioned under INID code 62.
•Claims filed after the date of filing of the application / after the date of receipt of the divisional application (Rule 68(4) EPC).

# (54) CENICRIVIROC FOR THE TREATMENT OF FIBROSIS

(57) The present disclosure relates to pharmaceutical compositions containing cenicriviroc, methods for the preparation thereof, and their use in the treatment of inflammation and connective tissue diseases and disorders, especially fibrosis.

EP 3 922 246 A1

## Description

### BACKGROUND

Cenicriviroc (also known as CVC) is the common name of (S,E)-8-(4-(2-Butoxyethoxy)phenyl)-1 -(2-methylpropyl)-N-(4-(((1-propyl-1H-imidazol-5-yl)methyl)sulfinyl)phenyl)-1,2,3,4-tetrahydrobenzo[b]azocine-5-carboxamide. The chemical structure of cenicriviroc mesylate appears in Figure 1. Cenicriviroc binds to and inhibits the activity of the C-C chemokine receptor type 2 (CCR2) and C-C chemokine receptor type 5 (CCR5) receptors (24). These receptors not only play a role in entry of viruses such as Human Immunodeficiency Virus (HIV) into the cell, but also are important for the recruitment of immune cells to sites of injury. Inhibition of this receptor's activity may have an anti-inflammatory effect. More recently, the role that inflammation plays in the development of fibrosis has been examined [30]. It has been shown that C-C chemokine receptor type 2 (CCR2) and CCR5 may play a role in promoting hepatic fibrosis [3, 4, 5, 31 32].

### SUMMARY OF THE INVENTION

In one embodiment, the invention provides a method of treating fibrosis or a fibrotic disease or condition in a subject in need thereof comprising administering to the subject a therapeutically effective amount of cenicriviroc or a salt or solvate thereof. In another embodiment, the fibrosis or fibrotic disease or condition is liver fibrosis or renal fibrosis. In yet a further embodiment, the liver fibrosis is associated with non-alcoholic steatohepatitis (NASH). In yet a further embodiment, the liver fibrosis is associated with non-alcoholic fatty liver disease (NAFLD). In yet a further embodiment, the liver fibrosis is associated with emerging cirrhosis. In another further embodiment, the liver fibrosis comprises non-cirrhotic hepatic fibrosis. In a further embodiment, the subject is infected by human immunodeficiency virus (HIV). In a further embodiment, the cenicriviroc or a salt or solvate thereof is formulated as a pharmaceutical composition comprising cenicriviroc or a salt or solvate thereof and fumaric acid. In a further embodiment, the subject has a disease or condition selected from the group consisting of alcoholic liver disease, HIV and HCV co-infection, HCV infection, type 2 diabetes mellitus (T2DM), metabolic syndrome (MS), and a combination thereof.

In one embodiment, the invention provides a method of treating NASH in a subject in need thereof comprising administering to the subject a therapeutically effective amount of cenicriviroc, or a salt or solvate thereof; wherein the NASH or the livier fibrosis associated with NASH is associated with type 2 diabetes mellitus (T2DM).

In one embodiment, the invention provides a method of treating NASH in a subject in need thereof comprising administering to the subject a therapeutically effective amount of cenicriviroc, or a salt or solvate thereof; wherein the NASH or the livier fibrosis associated with NASHis associated with metabolic syndrome (MS).

In one embodiment, the invention provides a method of treating NASH in a subject in need thereof comprising administering to the subject a therapeutically effective amount of cenicriviroc, or a salt or solvate thereof; wherein liver fibrosis is associated with HIV and HCV co-infection.

In one embodiment, the invention provides a method of treating NASH in a subject in need thereof comprising administering to the subject a therapeutically effective amount of cenicriviroc, or a salt or solvate thereof; wherein liver fibrosis is associated with HCV infection

In one embodiment, the invention provides a method of treatment, wherein the cenicriviroc or a salt or solvate thereof is formulated as an oral composition.

In one embodiment, the invention provides a method of treatment, wherein the cenicriviroc or a salt or solvate thereof is administered once per day or twice per day.

In one embodiment, the invention provides a method of treatment, wherein the cenicriviroc or a salt or solvate thereof is co-administered with one or more additional active agents. In a further embodiment, the one or more additional active agents are one or more antiretroviral agents selected from the group consisting of entry inhibitors, nucleoside reverse transcriptase inhibitors, nucleotide reverse transcriptase inhibitors, non-nucleoside reverse transcriptase inhibitors, protease inhibitors, integrase strand transfer inhibitors, maturation inhibitors, and combinations thereof. In a further embodiment, the one or more additional antiretroviral agents are selected from the group consisting of lamivudine, efavirenz, raltegravir, vivecon, bevirimat, alpha interferon, zidovudine, abacavir, lopinavir, ritonavir, tenofovir, tenofovir disoproxil, tenofovir prodrugs, emtricitabine, elvitegravir, cobicistat, darunavir, atazanavir, rilpivirine, dolutegravir, and a combination thereof.

In a further embodiment, the one or more additional active agents are one or more immune system suppressing agents. In a further embodiment, the one or more additional active agents are selected from the group consisting of cyclosporine, tacrolimus, prednisolone, hydrocortisone, sirolimus, everolimus, azathioprine, mycophenolic acid, methotrexate, basiliximab, daclizumab, rituximab, anti-thymocyte globulin, anti-lymphocyte globulin, and a combination thereof.

In a further embodiment, the one or more additional active agents are one or more anitfibrotic agents including, but not limited to, agents such as N-acetyl-L-cysteins (NAC) as well as angiotensin-converting enzyme (ACE) inhibitors,

AT II antagonists, obeticholic acid (OCA), GFT505, simtuzumab, or a combination thereof.

In one embodiment, the invention provides a method of treatment, comprising detecting a level of one or more biological molecules in the subject treated for fibrosis or the fibrotic disease or condition, and determining a treatment regimen based on an increase or decrease in the level of one or more biological molecules, wherein the biological molecule is selected from the group consisting of lipopolysaccharide (LPS), LPs-binding protein (LBP), 16S rDNA, sCD14, intestinal fatty acid binding protein (I-FABP), zonulin-1, Collagen 1a1 and 3a1, TGF-β, fibronectin-1, and a combination thereof.

In one embodiment, the invention provides a method of treatment, comprising detecting a level of one or biological molecules in the subject treated for fibrosis or the fibrotic disease or condition, wherein an increase or decrease in the level of one or more biological molecules compared to a predetermined standard level is predictive of the treatment efficacy of fibrosis or the fibrotic disease or condition, wherein the biological molecule is selected from the group consisting of lipopolysaccharide (LPS), LPs-binding protein (LBP), 16S rDNA, sCD14, intestinal fatty acid binding protein (I-FABP), zonulin-1, Collagen 1a1 and 3a1, TGF-β, fibronectin-1, and a combination thereof.

In a further embodiment, the one or more biological molecules are measured in a biological sample from a subject treated for fibrosis or the fibrotic disease or condition. In yet a further embodiment, the biological sample is selected from blood, skin, hair follicles, saliva, oral mucous, vaginal mucous, sweat, tears, epithelial tissues, urine, semen, seminal fluid, seminal plasma, prostatic fluid, pre-ejaculatory fluid (Cowper's fluid), excreta, biopsy, ascites, cerebrospinal fluid, lymph, brain, and tissue extract sample or biopsy sample.

BRIEF DESCRIPTION OF THE DRAWINGS

Figure 1 is the chemical formula of cenicriviroc mesylate.

Figure 2 is a graph comparing the absolute bioavailability, in beagle dogs, of cenicriviroc mesylate compounded as an oral solution with that of cenicriviroc mesylate prepared by wet granulation and mixed with various acid solubilizer excipients.

Figure 3 is a graph of the total impurity and degradant content of different cenicriviroc formulations subjected to accelerated stability testing at 40° C and 75% relative humidity when packaged with a desiccant.

Figure 4 is a dynamic vapor sorption isotherm for different cenicriviroc formulations.

Figure 5 shows the absorption of cenicriviroc from different formulations at three pretreatment states in beagle dogs.

Figure 6 shows the beagle dog absolute bioavailability of cenicriviroc and lamivudine in combination tablets.

Figure 7(A-B) shows intracellular HIV DNA levels in the PBMCs of participants in Study 202 at 24 weeks. Scatter plot depicting fold change in intracellular HIV DNA levels between baseline and 24 weeks, separated by treatment group. The lines and error bars represent mean and standard error measurements, respectively. Fold change was calculated using ΔΔCT in HIV/GAPDH multiplexed qPCR reactions, with each patient's baseline sample as a calibrator. A) Full-length HIV DNA (late reverse transcripts), B) strong-stop HIV DNA (early reverse transcripts)

Figure 8 (A-B) the effects of CVC and MVC on R5-tropic viral RNA and p24 in culture fluids. A) Viral load levels in culture fluids of controls or cells treated with CVC or MVC at 4 hrs post-infection. Error bars represent standard deviation. Two independent experiments are represented. B) Mean p24 antigen levels in culture fluids of controls or cells treated with CVC or MVC 4 hrs at post-infection. Error bars represent standard deviation. Two independent experiments are represented.

Figure 9 shows the effects of CVC and MVC on R5-tropic intracellular HIV DNA levels. Mean fold change of intracellular strong-stop DNA levels of CVC or MVC-treated cells compared to a no drug control after 4 hrs. Error bars represent standard deviation. Fold change was calculated using ΔΔCT in HIV/GAPDH multiplexed qPCR reactions, with the no drug control at 4 hrs as a calibrator. Two independent experiments are represented.

Figure 10 shows multiple binding modes of CVC into CCR5. Coordinates of CCR5 were generated from the CCR5 crystal structure bound to Maraviroc in the binding pocket (PDB ID: 4MBS). CVC binding sites were examined after docking of CVC. Docked poses of CVC are displayed as colored thin lines. The seven transmembrane (7TM) a-helices are represented by helices and numbered (1-7) according to the order of amino acid sequences. (A) A top view from the extracellular side of the receptor with three potential binding sites that are circled (site 1 (white), site 2 (black) and site 3 (light pink)). (B) A side view in the CCR5 transmembrane cavity. The extracellular loop 2 (ECL2) is labeled. Secondary structures are represented as cartoon structures. All images were processed using PyMOL software.

Figure 11 shows a comparison of the ligand binding pocket between CCRS/Maraviroc and CCR5/Cenicriviroc. Top view of CCR5 displaying docked poses, colored thin lines, of CVC (left) and MVC, yellow stick, (right) in the ligand binding pocket. CCR5 is shown in a molecular surface representation. Key residues: Tyr37, Trp86, Trp94, Leu104, Tyr108, Phe109, Phe112, Thr177, Ile198, Trp248, Tyr251, Leu255 and Glu283, that are involved in gp120 binding,

are deep in the pocket and colored in red.

Figure 12 shows the study schematic of the evaluation of CVC in mouse UUO model of renal fibrosis. Vehicle control and CVC administered BID; anti-TGF-β1 antibody, compound 1D11 (positive control) administered QD BID, twice daily; CVC, cenicriviroc; ip, intraperitoneal; PBS, phosphate buffered saline; QD, once daily; TGF, transforming growth factor; UUO, unilateral ureter occlusion

Figure 13 shows the change in body weight (Day 5) in each treatment group in mouse UUO model of renal fibrosis.

Figure 14 shows the Collagen Volume Fraction (CVF; % area) score in each treatment group in mouse UUO model of renal fibrosis. Data presented exclude a single outlier from an animal in the CVC 20 mg/kg/day group, which had a CVF value >2 standard deviations higher than any other animal in the group.

Figure 15 shows the mRNA expression from renal cortical tissue of sham-surgery

Figure 16 shows the change in body weight until week 9 in animals treated with Cenicriviroc (low or high dose).

Figure 17A-C shows the change in liver and body weight until week 9 in animals treated with Cenicriviroc (low or high dose). Panel A shows the change in body weight, Panel B shows the change in liver weight, and Panel C shows the change in the liver-to body weight ratio.

Figure 18A-F shows the whole blood and biochemistry of animals treated with Cenicriviroc (low or high dose) at week 9. Panel A shows Whole blood glucose, Panel B shows Plasma ALT, Panel C shows Plasma MCP-1, Panel D shows Plasma MIP-1β, Panel E shows Liver triglyceride, and Panel F shows Liver hydroxyproline.

Figure 19 shows the HE-stained liver sections of animals treated with Cenicriviroc (low or high dose) at week 9.

Figure 20 shows the NAFLD Activity score of animals treated with Cenicriviroc (low or high dose) at week 9.

Figure 21 shows representative photomicrographs of Sirius red-stained liver sections of animals treated with Cenicriviroc (low or high dose) at week 9.

Figure 22 shows representative photomicrographs of F4/80-immunostained liver sections of animals treated with Cenicriviroc (low or high dose) at week 9.

Figure 23 shows the percentages of inflammation area of animals treated with Cenicriviroc (low or high dose) at week 9.

Figure 24 shows representative photomicrographs of F4/80 and CD206 doubleimmunostained liver sections of animals treated with Cenicriviroc (low or high dose) at week 9.

Figure 25 shows the percentages of F4/80 and CD206 double positive cells of F4/80 positive cells of animals treated with Cenicriviroc (low or high dose) at week 9.

Figure 26 shows the representative photomicrographs of F4/80 and CD16/32 doubleimmunostained liver sections of animals treated with Cenicriviroc (low or high dose) at week 9.

Figure 27 shows the percentages of F4/80 and CD16/32 double positive cells of F4/80 positive cells of animals treated with Cenicriviroc (low or high dose) at week 9.

Figure 28 shows the M1/M2 ratio of animals treated with Cenicriviroc (low or high dose) at week 9.

Figure 29 shows representative photomicrographs of oil red-stained liver sections of animals treated with Cenicriviroc (low or high dose) at week 9.

Figure 30 shows the percentages of fat deposition area of animals treated with Cenicriviroc (low or high dose) at week 9.

Figure 31 shows representative photomicrographs of TUNEL-positive cells in livers of animals treated with Cenicriviroc (low or high dose) at week 9.

Figure 32 shows percentages of TUNEL-positive cells of animals treated with Cenicriviroc (low or high dose) at week 9.

Figure 33 shows quantitative RT-PCR of animals treated with Cenicriviroc (low or high dose) at week 9. The levels of TNF-a, MCP-1, Collagen Type 1, and TIMP-1 were measured.

Figure 34A-F shows raw data for quantitative RT-PCR of animals treated with Cenicriviroc (low or high dose) at week 9. Panel A shows the levels of 36B4, Panel B shows the levels of TNF-α, Panel C shows the levels of TIMP-1, Panel D shows the levels of collagen type 1, Panel E shows the levels of 36B4, and Panel f shows the levels of MCP-1.

Figure 35 shows the body weight changes of animals treated with Cenicriviroc (low or high dose) from 6 to 18 weeks.

Figure 36 shows the survival curve of animals treated with Cenicriviroc (low or high dose) from 6 to 18 weeks.

Figure 37A-C shows the body weight and liver weight at of animals treated with Cenicriviroc (low or high dose) at week 18. Panel A shows Body weight, Panel B shows Liver weight, and Panel C shows Liver-to-body weight ratio.

Figure 38A-C shows macroscopic appearance of livers of animals treated with Cenicriviroc (low or high dose) at week 18. Panel A shows the livers of animals treated with vehicle only, Panel B shows the livers of animals treated with low-dose Cenicriviroc, and Panel C shows the livers of animals treated with high-dose Cenicriviroc.

Figure 39 shows the number of visible tumor nodules of animals treated with Cenicriviroc (low or high dose) at week 18.

Figure 40 shows the maximum diameter of visible tumor nodules of animals treated with Cenicriviroc (low or high dose) at week 18.

Figure 41 shows representative photomicrographs of HE-stained liver sections of animals treated with Cenicriviroc

(low or high dose) at week 18.

Figure 42 shows representative photomicrographs of GS-immunostained liver sections of animals treated with Cenicriviroc (low or high dose) at week 18.

Figure 43 shows representative photomicrographs of CD31-immunostained liver sections of animals treated with Cenicriviroc (low or high dose) at week 18.

Figure 44 shows percentages of CD31-positive area of animals treated with Cenicriviroc (low or high dose) at week 18.

Figure 45 shows the median Changes in HIV-1 RNA Levels from Baseline by Cohort and Study Day - Study 201.

Figure 46 Proportion of Subjects With HIV-1 RNA <50 Copies/mL Over Time up to Week 48 - Snapshot Algorithm - ITT - Study 202.

Figure 47 shows the LS mean changes from baseline in sCD14 levels (106 pg/mL) over time up to Week 48 - ITT.

Figure 48 shows the CVC (Pooled Data)- and EFV-treated subjects grouped according to APRI and FIB-4 fibrosis index scores at baseline, Week 24, and Week 48.

Figure 49 shows the scatter plot of change from baseline APRI versus change from baseline sCD14 - Week 48 (ITT).

Figure 50 shows a scatter plot of change from baseline FIB-4 versus change from baseline sCD14 - Week 48 (ITT).

Figure 51 shows mean changes from baseline in creatine phosphokinase (CPK) over time up to Week 48 - Safety Population.

Figure 52 shows a dot density display of CPK elevations by severity grading vs. $c_{avg}$ (ng/mL) - Week 48.

Figure 53 shows a dot density display of ALT elevations by severity grading versus $c_{avg}$ (ng/mL) - Week 48.

Figure 54 shows a dot density display of AST elevations by severity grading versus $c_{avg}$ (ng/mL) - Week 48.

Figure 55 shows a dot density display of bilirubin elevations by severity grading versus $c_{avg}$ (ng/mL) - Week 48.

Figure 56 shows the mean changes from baseline in fasting total cholesterol, calculated LDL cholesterol, HDL cholesterol and triglycerides over time (mg/dL) up to Week 48

## DETAILED DESCRIPTION

It should be understood that singular forms such as "a," "an," and "the" are used throughout this application for convenience, however, except where context or an explicit statement indicates otherwise, the singular forms are intended to include the plural. Further, it should be understood that every journal article, patent, patent application, publication, and the like that is mentioned herein is hereby incorporated by reference in its entirety and for all purposes. All numerical ranges should be understood to include each and every numerical point within the numerical range, and should be interpreted as reciting each and every numerical point individually. The endpoints of all ranges directed to the same component or property are inclusive, and intended to be independently combinable.

*Definitions:*

Except for the terms discussed below, all of the terms used in this Application are intended to have the meanings that one of skill in the art at the time of the invention would ascribe to them.

"About" includes all values having substantially the same effect, or providing substantially the same result, as the reference value. Thus, the range encompassed by the term "about" will vary depending on context in which the term is used, for instance the parameter that the reference value is associated with. Thus, depending on context, "about" can mean, for example, ±15%, ±10%, ±5%, ±4%, ±3%, ±2%, ±1%, or ± less than 1%. Importantly, all recitations of a reference value preceded by the term "about" are intended to also be a recitation of the reference value alone. Notwithstanding the preceding, in this application the term "about" has a special meaning with regard to pharmacokinetic parameters, such as area under the curve (including AUC, $AUC_t$, and $AUC_\infty$) $C_{max}$, $T_{max}$, and the like. When used in relationship to a value for a pharmacokinetic parameter, the term "about" means from 80% to 125% of the reference parameter.

"Cenicriviroc" refers to the chemical compound (*S*)-8-[4-(2-Butoxyethoxy)phenyl]-1-isobutyl-*N*-(4-([(1-propyl-1*H*-imidazol-5-yl)methyl]sulfinyl)phenyl)-1,2,3,4-tetrahydro-1-benzazocine-5-carboxamide (structure shown below). Details of the composition of matter of cenicriviroc are disclosed in US Patent Application Publication No. 2012/0232028 which is hereby incorporated by reference in its entirety for all purposes. Details of related formulations are disclosed in US Application No. 61/823,766 which is hereby incorporated by reference in its entirety for all purposes.

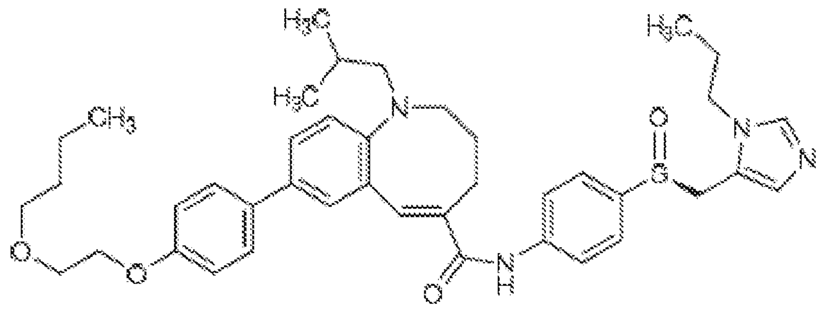

"Compound of the present invention" or "the present compound" refers to cenicriviroc or a salt or solvate thereof.

"Substantially similar" means a composition or formulation that resembles the reference composition or formulation to a great degree in both the identities and amounts of the composition or formulation.

"Pharmaceutically acceptable" refers to a material or method that can be used in medicine or pharmacy, including for veterinary purposes, for example, in administration to a subject.

"Salt" and "pharmaceutically acceptable salt" includes both acid and base addition salts. "Acid addition salt" refers to those salts that retain the biological effectiveness and properties of the free bases, which are not biologically or otherwise undesirable, and which are formed with inorganic acids and organic acids. "Base addition salt" refers to those salts that retain the biological effectiveness and properties of the free acids, which are not biologically or otherwise undesirable, and which are prepared from addition of an inorganic base or an organic base to the free acid. Examples of pharmaceutically acceptable salts include, but are not limited to, mineral or organic acid addition salts of basic residues such as amines; alkali or organic addition salts of acidic residues; and the like, or a combination comprising one or more of the foregoing salts. The pharmaceutically acceptable salts include salts and the quaternary ammonium salts of the active agent. For example, acid salts include those derived from inorganic acids such as hydrochloric, hydrobromic, sulfuric, sulfamic, phosphoric, nitric and the like; other acceptable inorganic salts include metal salts such as sodium salt, potassium salt, cesium salt, and the like; and alkaline earth metal salts, such as calcium salt, magnesium salt, and the like, or a combination comprising one or more of the foregoing salts. Pharmaceutically acceptable organic salts includes salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, mesylic, esylic, besylic, sulfanilic, 2-acetoxybenzoic, fumaric, toluenesulfonic, methanesulfonic, ethane disulfonic, oxalic, isethionic, $HOOC-(CH_2)_n-COOH$ where n is 0-4, and the like; organic amine salts such as triethylamine salt, pyridine salt, picoline salt, ethanolamine salt, triethanolamine salt, dicyclohexylamine salt, N,N'-dibenzylethylenediamine salt, and the like; and amino acid salts such as arginate, asparginate, glutamate, and the like; or a combination comprising one or more of the foregoing salts.

In one embodiment, the acid addition salt of cenicriviroc is cenicriviroc mesylate, e.g., (*S*)-8-[4-(2-Butoxyethoxy)phenyl]-1-isobutyl-*N*-(4-{[(1-propyl-1*H*-imidazol-5-yl)methyl]sulfinyl}phenyl)-1,2,3,4-tetrahydro-1-benzazocine-5-carboxamide monomethanesulfonoate. In one embodiment, the cenicriviroc mesylate is a crystalline material, such as a pale greenish-yellow crystalline powder. In one embodiment, the cenicriviroc mesylate is freely soluble in glacial acetic acid, methanol, benzyl alcohol, dimethylsulfoxide, and N,N-dimethylformamide; soluble in pyridine and acetic anhydride; and sparingly soluble in 99.5% ethanol; slightly soluble in acetonitrile, 1-octanol, and tetrahydrofuran; and practically insoluble in ethyl acetate and diethylether. In one embodiment, the cenicriviroc mesylate is freely soluble in aqueous solution from pH 1 to 2; sparingly soluble at pH 3 and practically insoluble from pH 4 to 13 and in water.

"Solvate" means a complex formed by solvation (the combination of solvent molecules with molecules or ions of the active agent of the present invention), or an aggregate that consists of a solute ion or molecule (the active agent of the present invention) with one or more solvent molecules. In the present invention, the preferred solvate is hydrate.

"Pharmaceutical composition" refers to a formulation of a compound of the disclosure and a medium generally accepted in the art for the delivery of the biologically active compound to mammals, *e.g.,* humans. Such a medium includes all pharmaceutically acceptable carriers, diluents or excipients therefor.

"Treating" includes ameliorating, mitigating, and reducing the instances of a disease or condition, or the symptoms of a disease or condition.

"Administering" includes any mode of administration, such as oral, subcutaneous, sublingual, transmucosal, parenteral, intravenous, intra-arterial, buccal, sublingual, topical, vaginal, rectal, ophthalmic, otic, nasal, inhaled, and transdermal. "Administering" can also include prescribing or filling a prescription for a dosage form comprising a particular compound. "Administering" can also include providing directions to carry out a method involving a particular compound or a dosage form comprising the compound.

"Therapeutically effective amount" means the amount of an active substance that, when administered to a subject for treating a disease, disorder, or other undesirable medical condition, is sufficient to have a beneficial effect

with respect to that disease, disorder, or condition. The therapeutically effective amount will vary depending on the chemical identity and formulation form of the active substance, the disease or condition and its severity, and the age, weight, and other relevant characteristics of the patient to be treated. Determining the therapeutically effective amount of a given active substance is within the ordinary skill of the art and typically requires no more than routine experimentation.

*Fibrosis:*

Fibrosis is the formation of excess fibrous connective tissue in an organ or tissue in a reparative or reactive process. This can be a reactive, benign, or pathological state. The deposition of connective tissue in the organ and/or tissue can obliterate the architecture and function of the underlying organ or tissue. Fibrosis is this pathological state of excess deposition of fibrous tissue, as well as the process of connective tissue deposition in healing.

Fibrosis is similar to the process of scarring, in that both involve stimulated cells laying down connective tissue, including collagen and glycosaminoglycans. Cytokines which mediate many immune and inflammatory reactions play a role in the development of fibrosis. Hepatocyte damage resulting from factors such as fat accumulation, viral agents, excessive alcohol consumption, hepatoxins, inevitably triggers an inflammatory immune response. The increased production of cytokines and chemokines in the liver leads to recruitment of pro-inflammatory monocytes (precursor cells) that subsequently mature into pro-inflammatory macrophages. Pro-inflammatory macrophages are pro-fibrogenic in nature and ultimately lead to the activation of hepatic stellate cells (HSCs) that are primarily responsible for the deposition of extracellular matrix (ECM).

Infiltration of various immune cell populations, resulting in inflammation, is a central pathogenic feature following acute- and chronic liver injury. Chronic liver inflammation leads to continuous hepatocyte injury which can lead to fibrosis, cirrhosis, ESLD, and HCC. Interactions between intra-hepatic immune cells lead to increased activation and migration of Kupffer cells and HSCs and are critical events for developing liver fibrosis. Additionally, there is increasing evidence of the role of CCR2 and CCR5 in the pathogenesis of liver fibrosis [1-7,9, 31]. These members of the C-C chemokine family are expressed by pro-fibrogenic cells including pro-inflammatory monocytes and macrophages, Kupffer cells, and HSCs [1-4]. CCR2 signaling plays an important role in the pathogenesis of renal fibrosis through regulation of bone marrow-derived fibroblasts [8]. CCR2- and CCRS-positive monocytes as well as CCR5-positive T lymphocytes are attracted by locally released MCP-1 and RANTES, and can contribute to chronic interstitial inflammation in the kidney [10, 11]. In rodents, CVC has high distribution in the liver, mesenteric lymph node, and intestine also described as the gut-liver axis. Disruption of the intestinal microbiota and its downstream effects on the gut-liver axis both play an important role in metabolic disorders such as obesity, non-alcoholic fatty liver disease (NAFLD) and non-alcoholic steatohepatitis (NASH) [16,23].

Table 1 lists chemokines expressed by liver cells [30].

| Cell type | Chemokine |
|---|---|
| Hepatocytes | MCP-1 (CCL2) [38], MIP-1α (CCL3) [74], RANTES (CCL5) [16,74], MIP-3β (CCL19) [75], SLC (CCL21) [75], Mig (CXCL9) [64], IP-10 (CXCL10) [64], CXCL16 [76], LEC (CCL16) [77], IL-8 (CXCL8) [78] and Eotaxin (CCL11) [41] |
| Stellate cells | MCP-1 (CCL2) [52,60], ,IP-1α (CCL3) [60], MIP-1β (CCL4) [60], $CX_3CL1$ [59], KC (CXCL1) [60], MIP-2 (CXCL2) [60], IP-10 (CXCL10) [60] and SLC (CCL21) [70] |
| Kupffer cells | MCP1 (CCL2) [52,38,60,79], MIP-1α (CCL3) [80] and MIP-3α (CCL20) [56] |
| Liver endothelial cells | MCP-1 (CCL2) [52], IL-8 (CXCL8) [81,76], CXCL16 [75], Mig (CXCL9) [69], IP-10 (CXCL10) [69], CXCL16 [65], $CX_3CL1$ [82], SLC (CCL21) [83], Eotaxin (CCL11) [41] and TECK (CCL25) [73] |
| *Summarizes selected experimental data from humans and mice/rats regarding the expression of chemokines by different resident hepatic cell populations upon activation or following liver Injury.<br>IP: Interferon-Inducible protein; KC: Kupffer cell; LEC: Liver-expressed chemokine; MCP: Monocyte chemoattractant protein; MIP: Macrophage Inflammatory protein; SLC: Secondary lymphoid-organ chemokine; TECK: Thymus-expressed chemokine | |

The activation of Hepatic stellate cells (HSCs) plays an important role in the pathogenesis of hepatic fibrosis.

Following liver injury, hepatic stellate cells (HSCs) become activated and express a combination of matrix metalloproteinases (MMPs) and their specific tissue inhibitors (TIMPs) [32]. In the early phases of liver injury, HSCs transiently express MMP-3, MMP-13, and uroplasminogen activator (uPA) and exhibit a matrix-degrading phenotype. Degradation of the extracellular matrix does not appear to be CCR2 or CCR5 dependent.

Activated HSCs can amplify the inflammatory response by inducing infiltration of monoand polymorphonuclear leucocytes. Infiltrating monocytes and macrophages participate in the development of fibrosis via several mechanisms, including increased secretion of cytokines and generation of oxidative stress-related products. Activated HSCs can express CCR2 and CCR5 and produce chemokines that include MCP-1, MIP-1$\alpha$, MIP-1$\beta$ and RANTES. CCR2 promotes HSC chemotaxis and the development of hepatic fibrosis. In human liver diseases, increased MCP-1 is associated with macrophage recruitment and severity of hepatic fibrosis and primary biliary cirrhosis. CCR5 stimulates HSC migration and proliferation.

In the later stages of liver injury and HSC activation, the pattern changes and the cells express a combination of MMPs that have the ability to degrade normal liver matrix, while inhibiting degradation of the fibrillar collagens that accumulate in liver fibrosis. This pattern is characterized by the combination of pro-MMP-2 and membrane type 1 (MT1)-MMP expression, which drive pericellular generation of active MMP-2 and local degradation of normal liver matrix. In addition there is a marked increase in expression of TIMP-1 leading to a more global inhibition of degradation of fibrillar liver collagens by interstitial collagenases (MMP-1/MMP-13). In liver injury associated with chronic alcoholic liver disease, the production of TNF-$\alpha$, IL-1, IL-6, as well as the chemokine IL-8/CXCL8 is increased. TNF-$\alpha$ is also an important mediator of non-alcoholic fatty liver disease. These pathways play a significant role in the progression of liver fibrosis. Inhibiting the activation of HSCs and accelerating the clearance of activated HSCs may be effective strategies for resolution of hepatic fibrosis.

Chemokine families play important regulatory roles in inflammation. Members of this family include, but are not limited to CXC receptors and ligands including but not limited to CXCR1, CXCR2, CXCR3, CXCR4, CXCR5, CXCR6, CXCR7, CXCR8, CXCR9, CXCR10, CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, and CXCL17; the CC chemokines and receptors including but not limited to CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCR1, CCR2, CCR3, CCR4, CCR5, CCR6, CCR7, CCR8, CCR, and CCR10; the C chemokines including but not limited to XCL1, XCL2, and XCR1; and the CX3C chemokines including but not limited to CS3CL1 and CX3CR1. These molecules may be upregulated in fibrotic organs or tissues. In further embodiments, these molecules may be downregulated in fibrotic organs or tissues. In further embodiments, the molecules in the signaling pathways of these chemokines may be upregulated in fibrotic organs or tissues. In further embodiments, the molecules in the signaling pathways of these chemokines may be downregulated in fibrotic organs or tissues.

Fibrosis can occur in many tissues within the body including but not limited to, the lungs, liver, bone marrow, joints, skin, digestive tract, lymph nodes, blood vessels, or heart and typically is a result of inflammation or damage. Non-limiting examples include Pulmonary fibrosis, Idiopathic pulmonary fibrosis, Cystic fibrosis, Cirrhosis, Endomyocardial fibrosis, myocardial infarction, Atrial Fibrosis, Mediastinal fibrosis, Myelofibrosis, Retroperitoneal fibrosis, Progressive massive fibrosis, complications from pneumoconiosis, Nephrogenic systemic fibrosis, Crohn's Disease, Keloid, Scleroderma/systemic sclerosis, Arthrofibrosis, Peyronie's disease, Dupuytren's contracture, fibrosis associated with atherosclerosis, lymph node fibrosis, and adhesive capsulitis.

*Embodiments of Therapeutic Utilities:*

The present invention provides methods of treating fibrosis. Anti-fibrotic effects of CVC in animal studies were observed when CVC treatment was initiated at the onset of liver injury (TAA) or soon after (TAA; HFD) but not once cirrhosis was established (TAA). This suggests that antifibrotic effects of CVC may be more pronounced in populations with established liver fibrosis and at significant risk of disease progression. These include: Non-alcoholic hepatosteatosis (NASH) associated with type 2 diabetes mellitus (T2DM) and metabolic syndrome (MS); HIV and HCV co-infection, or HCV infection.

*NASH*

The compositions of the invention may be used to treat liver fibrosis resulting from Nonalcoholic Steatohepatitis (NASH), a common liver disease that affects 2 to 5 percent of Americans. Although liver damage due to NASH has some of the characteristics of alcoholic liver disease, it occurs in people who drink little or no alcohol. The major feature in NASH is fat in the liver, along with inflammation and hepatocyte damage (ballooning). NASH can be severe and can lead to cirrhosis, in which the liver is permanently damaged and scarred and no longer able to work properly.Nonalcoholic fatty liver disease (NAFLD) is a common, often "silent", liver disease associated with obesity related disorders, such as

type-2 diabetes and metabolic syndrome, occurring in people who drink little or no alcohol and is characterized by the accumulation of fat in the liver with no other apparent causes. [32-43] At the beginning of the NAFLD spectrum is simple steatosis, which is characterized by a build-up of fat within the liver. Liver steatosis without inflammation is usually benign and slow or non-progressive. NASH is a more advanced and severe subtype of NAFLD where steatosis is complicated by liver-cell injury and inflammation, with or without fibrosis.

The rising prevalence of obesity-related disorders has contributed to a rapid increase in the prevalence of NASH. Approximately 10% to 20% of subjects with NAFLD will progress to NASH [44].

NAFLD is the most common cause of chronic liver disease. [45] Most US studies report a 10% to 35% prevalence rate of NAFLD; however, these rates vary with the study population and the method of diagnosis. [46] Since approximately one-third of the US population is considered obese, the prevalence of NAFLD in the US population is likely to be about 30%.[46] One study has found that NAFLD affects approximately 27% to 34% of Americans, or an estimated 86 to 108 million patients.[44] NAFLD is not unique to the US. Reports from the rest of the world, including Brazil, China, India, Israel, Italy, Japan, Korea, Sri Lanka, and Taiwan, suggest that the prevalence rate ranges from 6% to 35% (median of 20%). [46] A study by the Gastroenterological Society of Australia/Australian Liver Association has found that NAFLD affects an estimated 5.5 million Australians, including 40% of all adults aged $\geq$ 50 years. [47] An Australian study of severely obese patients found that 25% of these patients had NASH. [48]

Liver biopsy is required to make a definitive diagnosis of NASH. In a US study of middleaged individuals, the prevalence of histologically confirmed NASH was 12.2%.[49] Current estimates place NASH prevalence at approximately 9 to 15 million in the US (3% to 5% of the US population), with similar prevalence in the EU and China.[46, 50] The prevalence of NASH in the obese population ranges from 10% to 56% (median of 33%). [46] In an autopsy series of lean individuals from Canada, the prevalence of steatohepatitis and fibrosis was 3% and 7%, respectively.[46] The prevalence of NASH is also increasing in developing regions, which has been attributed to people in these regions starting to adopt a more sedentary lifestyle and westernized diet [51] consisting of processed food with high fat and sugar/fructose content.[52]

NASH is a serious chronic liver disease defined by the presence of hepatic steatosis and inflammation with hepatocyte injury, with or without fibrosis. [34] Chronic liver inflammation is a precursor to fibrosis, which can progress to cirrhosis, end-stage liver disease and hepatocellular carcinoma. In addition to insulin resistance, altered lipid storage and metabolism, accumulation of cholesterol within the liver, oxidative stress resulting in increased hepatic injury, and bacterial translocation[34,53-56] secondary to disruption of gut microbiota (associated with high fructose-containing diet) have all been implicated as important co-factors contributing to progression of NASH.[57-60] Due to the growing epidemic of obesity and diabetes, NASH is projected to become the most common cause of advanced liver disease and the most common indication for liver transplantation.[46, 61-63] The burden of NASH, combined with a lack of any approved therapeutic interventions, represents an unmet medical need.

In further embodiments, liver fibrosis is associated with emerging cirrhosis. In some embodiments, the cirrhosis is associated with alcohol damage. In further embodiments, the cirrhosis is associated with a hepatitis infection, including but not limited to hepatitis B and hepatitis C infections, primary biliary cirrhosis (PBC), primary sclerosing cholangitis, or fatty liver disease. In some embodiments, the present invention provides for methods of treating subjects at risk of developing liver fibrosis or cirrhosis.

In another embodiment, the fibrosis comprises non-cirrhotic hepatic fibrosis. In another further embodiment, the subject is infected by human immunodeficiency virus (HIV). In yet a further embodiment, the subject is infected with a hepatitis virus, including but not limited to HCV (hepatitis C virus). In further embodiment, the subject has diabetes. In a further embodiment, the subject has type 2 diabetes. In a further embodiment, the subject has type 1 diabetes. In a further embodiment, the subject has metabolic syndrome (MS). In further embodiments, the subject has one or more of these diseases or disorders. In a further embodiment, the subject is at risk of developing one or more of these diseases. In a further embodiment, the subject has insulin resistance. In further embodiments, the subject has increased blood glucose concentrations, high blood pressure, elevated cholesterol levels, elevated triglyceride levels, or is obese. In a further embodiment, the subject has Polycystic ovary syndrome.

In one embodiment, the invention provides a method of treatment, wherein the cenicriviroc or a salt or solvate thereof is coadministered with one or more additional active agents. In a further embodiment, the one or more additional active agents are one or more antiretroviral agents selected from entry inhibitors, nucleoside reverse transcriptase inhibitors, nucleotide reverse transcriptase inhibitors, non-nucleoside reverse transcriptase inhibitors, protease inhibitors, integrase strand transfer inhibitors, maturation inhibitors, and combinations thereof. In a further embodiment, the one or more additional antiretroviral agents are selected from the group consisting of lamivudine, efavirenz, raltegravir, vivecon, bevirimat, alpha interferon, zidovudine, abacavir, lopinavir, ritonavir, tenofovir, tenofovir disoproxil, tenofovir prodrugs, emtricitabine, elvitegravir, cobicistat, darunavir, atazanavir, rilpivirine, dolutegravir, and a combination thereof. In a further embodiment, the one or more additional active agents are one or more immune system suppressing agents. In a further embodiment, the one or more additional active agents are selected from the group consisting of cyclosporine, tacrolimus, prednisolone, hydrocortisone, sirolimus, everolimus, azathioprine, mycophenolic acid, methotrexate, basiliximab, daclizumab, rituximab, anti-thymocyte globulin, anti-lymphocyte globulin, and a combination thereof.

Certain embodiments include methods for monitoring and/or predicting the treatment efficacy of the present treatment as described herein. Such methods include detecting the level of one or more biological molecules, such as for example, biomarkers, in a subject (or in a biological sample from the subject) treated for fibrosis or a fibrotic disease or condition, wherein an increase or decrease in the level of one or more biological molecules compared to a predetermined standard level indicates or is predictive of the treatment efficacy of the present treatment.

In one embodiment, the invention provides a method of treatment, comprising detecting the level of one or more biological molecules in the subject treated for fibrosis or the fibrotic disease or condition, and determining a treatment regimen based on an increase or decrease in the level of one or more biological molecules, wherein the biological molecule is selected from the group consisting of lipopolysaccharide (LPS), LPS-binding protein (LBP), 16S rDNA, sCD14, intestinal fatty acid binding protein (I-FABP), zonulin-1, Collagen 1a1 and 3a1, TGF-$\beta$, fibronectin-1, hs-CRP, IL-1$\beta$, IL-6, IL-33, fibrinogen, MCP-1, MIP-1$\alpha$ and -1$\beta$, RANTES, sCD163, TGF-$\beta$, TNF-a, a biomarker of hepatocyte apoptosis such as CK-18 (caspase-cleaved and total), or biomarkers of bacterial translocation such as LPS, LBP, sCD14, and I-FABP, or a combination thereof.

In one embodiment, the invention provides a method of treatment, comprising detecting the level of one or biological molecules in the subject treated for fibrosis or the fibrotic disease or condition, wherein an increase or decrease in the level of one or more biological molecules compared to a predetermined standard level is predictive of the treatment efficacy of fibrosis or the fibrotic disease or condition.

. In a further embodiment, the one or more biological molecules are measured in a biological sample from a subject treated for fibrosis or the fibrotic disease or condition. In yet a further embodiment, the biological sample is selected from blood, skin, hair follicles, saliva, oral mucous, vaginal mucous, sweat, tears, epithelial tissues, urine, semen, seminal fluid, seminal plasma, prostatic fluid, pre-ejaculatory fluid (Cowper's fluid), excreta, biopsy, ascites, cerebrospinal fluid, lymph, brain, and tissue extract sample or biopsy sample.

*Dosages and Administration:*

A dosage of a particular subject can be determined according to the subject's age, weight, general health conditions, sex, meal, administration time, administration route, excretion rate and the degree of particular disease conditions to be treated by taking into consideration of these and other factors.

The present invention provides a method of treatment, wherein the cenicriviroc or a salt or solvate thereof is formulated as an oral composition.

The present invention provides a method of treatment, wherein the cenicriviroc or a salt or solvate thereof is administered, for example, once per day or twice per day. The dosage form can be administered for a duration of time sufficient to treat the fibrotic disease or condition.

In the case of oral administration, a daily dosage is in a range of about 5 to 1000 mg, preferably about 10 to 600 mg, and more preferably about 10 to 300 mg, most preferably about 15 to 200 mg as the active ingredient (i.e. as the compound of the invention) per an adult of body weight of 50 kg, and the medicine may be administered, for example, once, or in 2 to 3 divided doses a day.

The cenicriviroc or a salt or solvate thereof may be formulated into any dosage form suitable for oral or injectable administration. When the compound is administered orally, it can be formulated into solid dosage forms for oral administration, for example, tablets, capsules, pills, granules, and so on. It also can be formulated into liquid dosage forms for oral administration, such as oral solutions, oral suspensions, syrups and the like. The term "tablets" as used herein, refers to those solid preparations which are prepared by homogeneously mixing and pressing the compounds and suitable auxiliary materials into circular or irregular troches, mainly in common tablets for oral administration, including also buccal tablets, sublingual tablets, buccal wafer, chewable tablets, dispersible tablets, soluble tablets, effervescent tablets, sustained-release tablets, controlled-release tablets, enteric-coated tablets and the like. The term "capsules" as used herein, refers to those solid preparations which are prepared by filling the compounds, or the compounds together with suitable auxiliary materials into hollow capsules or sealing into soft capsule materials. According to the solubility and release property, capsules can be divided into hard capsules (regular capsules), soft capsules (soft shell capsules), sustained-release capsules, controlled-release capsules, enteric-coated capsules and the like. The term "pills" as used herein, refers to spherical or near-spherical solid preparations which are prepared by mixing the compounds and suitable auxiliary materials via suitable methods, including dropping pills, dragee, pilule and the like. The term "granules" as used herein, refers to dry granular preparations which are prepared by mixing the compounds and suitable auxiliary materials and have a certain particle size. Granules can be divided into soluble granules (generally referred to as granules), suspension granules, effervescent granules, enteric-coated granules, sustained-release granules, controlled-release granules and the like. The term "oral solutions" as used herein, refers to a settled liquid preparation which is prepared by dissolving the compounds in suitable solvents for oral administration. The term "oral suspensions" as used herein, refers to suspensions for oral administration, which are prepared by dispersing the insoluble compounds in liquid vehicles,

also including dry suspension or concentrated suspension. The term "syrups" as used herein, refers to a concentrated sucrose aqueous solution containing the compounds. The injectable dosage form can be produced by the conventional methods in the art of formulations, and aqueous solvents or non-aqueous solvents may be selected. The most commonly used aqueous solvent is water for injection, as well as 0.9% sodium chloride solution or other suitable aqueous solutions. The commonly used non-aqueous solvent is vegetable oil, mainly soy bean oil for injection, and others aqueous solutions of alcohol, propylene glycol, polyethylene glycol, and *etc.*

In one embodiment, a pharmaceutical composition comprising cenicriviroc or a salt thereof and fumaric acid is provided. In certain embodiments, the cenicriviroc or salt thereof is cenicriviroc mesylate.

In further embodiments, the weight ratio of cenicriviroc or salt thereof to fumaric acid is from about 7:10 to about 10:7, such as from about 8:10 to about 10:8, from about 9:10 to about 10:9, or from about 95:100 to about 100:95. In other further embodiments, the fumaric acid is present in an amount of from about 15% to about 40%, such as from about 20% to about 30%, or about 25%, by weight of the composition. In other further embodiments, the cenicriviroc or salt thereof is present in an amount of from about 15% to about 40%, such as from about 20% to about 30%, or about 25%, by weight of the composition.

In other further embodiments, the composition of cenicriviroc or a salt thereof and fumaric acid further comprises one or more fillers. In more specific embodiments, the one or more fillers are selected from microcrystalline cellulose, calcium phosphate dibasic, cellulose, lactose, sucrose, mannitol, sorbitol, starch, and calcium carbonate. For example, in certain embodiments, the one or more fillers is microcrystalline cellulose. In particular embodiments, the weight ratio of the one or more fillers to the cenicriviroc or salt thereof is from about 25:10 to about 10:8, such as from about 20:10 to about 10:10, or about 15:10. In other particular embodiments, the one or more fillers are present in an amount of from about 25% to about 55%, such as from about 30% to about 50% or about 40%, by weight of the composition. In other further embodiments, the composition further comprises one or more disintegrants. In more specific embodiments, the one or more disintegrants are selected from cross-linked polyvinylpyrrolidone, cross-linked sodium carboxymethyl cellulose, and sodium starch glycolate. For example, in certain embodiments, the one or more disintegrants is cross-linked sodium carboxymethyl cellulose. In particular embodiments, the weight ratio of the one or more disintegrants to the cenicriviroc or salt thereof is from about 10:10 to about 30:100, such as about 25:100. In other particular embodiments, the one or more disintegrants are present in an amount of from about 2% to about 10%, such as from about 4% to about 8%, or about 6%, by weight of the composition. In other further embodiments, the composition further comprises one or more lubricants. In more specific embodiments, the one or more lubricants are selected from talc, silica, stearin, magnesium stearate, and stearic acid. For example, in certain embodiments, the one or more lubricants is magnesium stearate. In particular embodiments, the one or more lubricants are present in an amount of from about 0.25% to about 5%, such as from about 0.75% to about 3%, or about 1.25%, by weight of the composition.

In other further embodiments, the composition of cenicriviroc or a salt thereof and fumaric acid is substantially similar to that of Table 2. In other further embodiments, the composition of cenicriviroc or a salt thereof and fumaric acid is substantially similar to that of Tables 3 and 4. In other further embodiments, any of the compositions of cenicriviroc or a salt thereof and fumaric acid is produced by a process involving dry granulation. In other further embodiments, any of the compositions of cenicriviroc or a salt thereof and fumaric acid has a water content of no more than about 4% by weight, such as no more than 2% by weight, after six weeks exposure to about 40° C at about 75% relative humidity when packaged with desiccant. In other further embodiments, any of the above-mentioned compositions has a total impurity level of no more than about 2.5%, such as no more than 1.5%, after 12 weeks of exposure to 40° C at 75% relative humidity when packaged with desiccant. In other further embodiments, the cenicriviroc or salt thereof of any of the above-mentioned compositions has a mean absolute bioavailability after oral administration that is substantially similar to the bioavailability of the cenicriviroc or salt thereof in a solution after oral administration. In yet further embodiments, the cenicriviroc or salt thereof has an absolute bioavailability of about 10% to about 50%, such as about 27%, in beagle dogs.

In another embodiment, a pharmaceutical formulation is provided that comprises a composition of cenicriviroc or a salt thereof and fumaric acid. In further embodiments, the composition in the formulation can be in the form of a granulate. In other further embodiments, the composition in the formulation is disposed in a capsule shell. In other further embodiments, the composition of the formulation is disposed in a sachet. In other further embodiments, the composition of the formulation is a tablet or a component of a tablet. In still other further embodiments, the composition of the formulation is one or more layers of a multi-layered tablet. In other further embodiments, the formulation comprises one or more additional pharmaceutically inactive ingredients. In other further embodiments, the formulation is substantially similar to that of Table 9. In other further embodiments, a tablet having a composition substantially similar to of Table 9 is provided. In other further embodiments, any of the above embodiments are coated substrates. In another embodiment, methods for preparing any of the above-mentioned embodiments are provided. In further embodiments, the method comprises admixing cenicriviroc or a salt thereof and fumaric acid to form an admixture, and dry granulating the admixture. In other further embodiments, the method further comprises admixing one or more fillers with the cenicriviroc or salt thereof and fumaric acid to form the admixture. In other further embodiments, the method further comprises admixing

one or more disintegrants with the cenicriviroc or salt thereof and fumaric acid to form the admixture. In other further embodiments, the method further comprises admixing one or more lubricants with the cenicriviroc or salt thereof and fumaric acid to form the admixture. In other further embodiments, the method further comprises compressing the dry granulated admixture into a tablet. In other further embodiments, the method comprises filling a capsule with the dry granulated admixture.

Further, the compound of the invention can be included or used in combination with blood for transfusion or blood derivatives. In one embodiment, the compound of the invention can be included or used in combination with one or more agents that purge latent HIV reservoirs and added to blood for transfusion or blood derivatives. Usually, blood for transfusion or blood derivatives are produced by mixing blood obtained form plural persons and, in some cases, uninfected cells are contaminated with cells infected with HIV virus. In such a case, uninfected cells are likely to be infected with HIV virus. When the compound of the present invention is added to blood for transfusion or blood derivatives along with one or more agents that purge latent HIV reservoirs, infection and proliferation of the virus can be prevented or controlled. Especially, when blood derivatives are stored, infection and proliferation of the virus is effectively prevented or controlled by addition of the compound of the present invention. In addition, when blood for transfusion or blood derivatives contaminated with HIV virus are administered to a person, infection and proliferation of the virus in the person's body can be prevented by adding the compound of the invention to the blood or blood derivatives in combination with one or more agents that purge latent HIV reservoirs. For example, usually, for preventing HIV infectious disease upon using blood or blood derivatives by oral administration, a dosage is in a range of about 0.02 to 50 mg/kg, preferably about 0.05 to 30 mg/kg, and more preferably about 0.1 to 10 mg/kg as the CCR5/CCR2 antagonist per an adult of body weight of about 60 kg, and the medicine may be administered once or 2 to 3 doses a day. As a matter of course, although the dosage range can be controlled on the basis of unit dosages necessary for dividing the daily dosage, as described above, a dosage of a particular subject can be determined according to the subject's age, weight, general health conditions, sex, meal, administration time, administration route, excretion rate and the degree of particular disease conditions to be treated by taking into consideration of these and other factors. In this case, the administration route is also appropriately selected and, the medicine for preventing HIV infectious disease of the present invention may be added directly to blood for transfusion or blood derivatives before transfusion or using blood derivatives. In such a case, desirably, the medicine of the present invention is mixed with blood or blood derivatives immediately to 24 hours before, preferably immediately to 12 hours before, more preferably immediately to 6 hours before transfusion or using blood derivatives.

Aside from blood for transfusion or blood derivatives, when the compositions of the invention is administered together with the blood for transfusion or blood derivatives and/or other active agents, the medicine is administered preferably at the same time of, to 1 hour before transfusion or using the blood derivatives. More preferably, for example, the medicine is administered once to 3 times per day and the administration is continued 4 weeks.

*Combination Therapy:*

The compound of the invention may be used alone or in combination with one or more additional active agents. The one or more additional active agents may be any compound, molecule, or substance which can exert therapeutic effect to a subject in need thereof. The one or more additional active agents may be "co-administered", i.e, administered together in a coordinated fashion to a subject, either as separate pharmaceutical compositions or admixed in a single pharmaceutical composition. By "co-administered", the one or more additional active agents may also be administered simultaneously with the present compound, or be administered separately with the present compound, including at different times and with different frequencies. The one or more additional active agents may be administered by any known route, such as orally, intravenously, intramuscularly, nasally,subcutaneously, intra-vaginally, intra-rectally, and the like; and the therapeutic agent may also be administered by any conventional route. In many embodiments, at least one and optionally both of the one or more additional active agents may be administered orally.

These one or more additional active agents include, but are not limited to, one or more anti-fibrotic agents, antiretroviral agents, immune system suppressing agents, and CCR2 and/or CCR5 inhibitors or treatments. When two or more medicines are used in combination, dosage of each medicine is commonly identical to the dosage of the medicine when used independently, but when a medicine interferes with metabolism of other medicines, the dosage of each medicine is properly adjusted. Each medicine may be administered simultaneously or separately in a time interval for example of less than 12 hours, 24 hours, 36 hours. A dosage form as described herein, such as a capsule, can be administered at appropriate intervals. For example, once per day, twice per day, three times per day, and the like. In particular, the dosage form is administered for example, once or twice per day. Even more particularly, the dosage form is administered once per day. In one embodiment, the one or more antiretroviral agents include, but are not limited to, entry inhibitors, nucleoside reverse transcriptase inhibitors, nucleotide reverse transcriptase inhibitors, non-nucleoside reverse transcriptase inhibitors, protease inhibitors, integrase inhibitors, maturation inhibitors, and combinations thereof. In one embodiment, the one or more additional antiretroviral agents include, but are not limited to, lamivudine, efavirenz, raltegravir, vivecon, bevirimat, alpha interferon, zidovudine, abacavir, lopinavir, ritonavir, tenofovir, tenofovir disoproxil,

tenofovir prodrugs, emtricitabine, elvitegravir, cobicistat, darunavir, atazanavir, rilpivirine, dolutegravir, and a combination thereof.

In one embodiment, the one or more immune system suppressing agents include, but are not limited to, cyclosporine, tacrolimus, prednisolone, hydrocortisone, sirolimus, everolimus, azathioprine, mycophenolic acid, methotrexate, basiliximab, daclizumab, rituximab, anti-thymocyte globulin, anti-lymphocyte globulin, and a combination thereof

The following Examples further illustrate the present invention in detail but are not to be construed to limit the scope thereof.

## EXAMPLES

*Example 1 - Cenicriviroc mesylate compositions*

A series of cenicriviroc mesylate compositions that were identical except for the identity of the acid solubilizer were prepared by wet granulation in a Key 1L bowl granulator, followed by tray drying, sieving, mixing and compression into tablets on a Carver press. The composition of the formulations is shown in Table 2.

Table 2

| Components | Unit Formula (mg/unit) | | | |
|---|---|---|---|---|
| | **Ex. 1a Citric Acid** | **Ex. 1b Fumaric Acid** | **Ex. 1c Maleic Acid** | **Ex. 1d Sodium Bisulfate** |
| Cenicriviroc Mesylate | 28.45 | 28.45 | 28.45 | 28.45 |
| Mannitol | 7.88 | 7.88 | 7.88 | 7.88 |
| Hydroxypropyl Cellulose | 2.62 | 2.62 | 2.62 | 2.62 |
| Croscarmellose Sodium | 1.75 | 1.75 | 1.75 | 1.75 |
| Croscarmellose Sodium | 1.75 | 1.75 | 1.75 | 1.75 |
| Citric Acid | 43.75 | - | - | - |
| Fumaric Acid | - | 43.75 | - | - |
| Maleic Acid | - | - | 43.75 | - |
| Sodium Bisulfate | - | - | - | 43.75 |
| Silicon Dioxide | 0.43 | 0.43 | 0.43 | 0.43 |
| Magnesium Stearate | 0.88 | 0.88 | 0.88 | 0.88 |
| Total | 87.5 | 87.5 | 87.5 | 87.5 |

The tablets were administered to beagle dogs. An oral solution was also administered as a control. The absolute bioavailabilities of the formulations and of the oral solution were determined, and are shown in Figure 2. The result shows that the cenicriviroc mesylate with fumaric acid has a significantly higher bioavailability than any of the other solubilizers tested.

*Example 2: Cenicriviroc mesylate compositions*

Cenicriviroc mesylate, fumaric acid, microcrystalline cellulose, cross-linked sodium carboxymethyl cellulose, and magnesium stearate were admixed, dry granulated, milled, blended with extragranular microcrystalline cellulose, cross-linked sodium carboxymethyl cellulose, and magnesium stearate and compressed into tablets having a hardness greater than 10 kP and friability less than 0.8% w/w. The resulting tablets had the composition shown in Table 3.

Table 3.

| Components | Unit Formula (mg/unit) | | | | |
|---|---|---|---|---|---|
| | **Ex. 2a** | **Ex. 2b** | **Ex. 2c** | **Ex. 2d** | **Ex. 2e** |
| Cenicriviroc Mesylate | 170.69[a] | 170.69[a] | 170.69[a] | 170.69[a] | 170.69[a] |
| Fumaric Acid | 160.00 | 160.00 | 160.00[b] | 160.00 | 80.00 |
| Microcrystalline Cellulose | 252.68 | 272.18 | 272.18 | 272.18 | 66.35 |
| Crospovidone | - | - | - | 19.50 | - |
| Croscarmellose Sodium | 58.50 | 39.00 | 39.00 | 19.50 | 20.70 |
| Magnesium Stearate | 8.13 | 8.13 | 8.13 | 8.13 | 2.55 |
| Total | 650.0 | 650.0 | 650.0 | 650.0 | 340.0 |
| a. Equivalent to 150 mg cenicriviroc freebase.<br>b. Added in the extragranular portion of the powder blend. | | | | | |

By way of illustration, the concentration percentage and mass per tablet of the components in Example 2b (i.e., Ex. 2b)are given in Table 4.

Table 4

| Component | Concentration (% w/w) | Mass (mg) per tablet |
|---|---|---|
| Cenicriviroc mesylate | 26.26 | 170.69[a] |
| Fumaric acid | 24.62 | 160.00 |
| Microcrystalline cellulose | 41.87 | 272.18 |
| Cross-linked sodium carboxymethyl cellulose | 6.00 | 39.00 |
| Magnesium stearate | 1.25 | 8.13 |
| Total | 100.0 | 650.0 |
| [a] equivalent to 150 mg cenicriviroc free base | | |

Example 3: Cenicriviroc mesylate compositions

Cenicriviroc mesylate, microcrystalline cellulose, cross-linked sodium carboxymethyl cellulose, and magnesium stearate were admixed, dry granulated, dried, milled, blended with extragranular microcrystalline cellulose, cross-linked sodium carboxymethyl cellulose, fumaric acid, colloidal silicon dioxide, and magnesium stearate and compressed into tablets having a hardness greater than 10 kP and friability less than 0.8% w/w. The resulting tablets had the composition shown in Table 5.

Table 5

| Component | Concentration (% w/w) | Mass (mg) per tablet |
|---|---|---|
| Cenicriviroc mesylate | 26.26 | 28.45[a] |
| Fumaric acid | 24.62 | 26.67 |
| Microcrystalline cellulose | 41.87 | 45.36 |
| Cross-linked sodium carboxymethyl cellulose | 6.00 | 39.00 |
| Magnesium stearate | 1.25 | 1.35 |
| Total | 100.0 | 108.3 |
| [a] equivalent to 25 mg cenicriviroc free base | | |

Notably, the formulation of Table 5 has the same ratio of components as that of Table 3b, and differs only in the total amount of the components that are used for each tablet. Thus, Table 4 shows tablets with 150 mg cenicriviroc (based on free base), whereas Table CC-1 shows tablets with 25 mg cenicriviroc (based on free base) with the same ratio of components as the 150 mg tablets of Example 2b, shown in Table 4.

Example 4 - Reference

The citric acid based formulation of Table 6 was prepared as follows. Cenicriviroc, hydroxypropyl cellulose, mannitol, and cross-linked sodium carboxymethyl cellulose were admixed, wet granulated, dried, milled, and blended with microcrystalline cellulose, cross-linked sodium carboxymethyl cellulose, citric acid, colloidal silicon dioxide, talc, and magnesium stearate. The resulting blend was compressed into tablets having a hardness greater than 10 kP and friability less than 0.8% w/w. The tablets were coated with hydroxypropyl methylcellulose, polyethylene glycol 8000, titanium dioxide, and yellow iron oxide. The coated tablets thus produced were substantially identical to those disclosed in U.S. Patent Application Publication No. 2008/031942 (see, e.g., Table 3).

Table 6

| Component | mg/tablet | %w/w |
|---|---|---|
| Cenicriviroc mesylate | 28.91 | 4.68 |
| Mannitol | 341.09 | 56.85 |
| Microcrystalline cellulose | 80.00 | 12.94 |
| Colloidal silicon dioxide | 12.00 | 2.00 |
| Citric acid anhydrous | 75.00 | 12.14 |
| Hydroxypropyl cellulose | 12.00 | 1.94 |
| Cross-linked sodium carboxymethyl cellulose | 30.00 | 4.85 |
| Talc | 12.00 | 1.94 |
| Magnesium stearate | 9.00 | 1.46 |
| Hydroxypropyl methylcellulose | 11.71 | 1.89 |
| Polyethylene glycol 8000 | 2.69 | 0.44 |
| Titanium dioxide | 3.03 | 0.49 |
| Yellow iron oxide | 0.57 | 0.09 |

*Example 5- Reference*

Cenicriviroc and hypromellose acetate succinate were dissolved in methanol and spray dried into a fine powder containing 25% cenicriviroc by weight (based on the weight of cenicriviroc free base). The powder was admixed with colloidal silicon dioxide, microcrystalline cellulose, mannitol, sodium lauryl sulfate, cross-linked sodium carboxymethyl cellulose, and magnesium stearate. The admixture was compressed into tablets having a hardness greater than 10 kP and friability less than 0.8% w/w. The final composition of the tablets is shown in Table 7.

**Table 7**

| Component | Weight % | Mass (mg) |
|---|---|---|
| Cenicriviroc (as mesylate salt) | 8.33 | 50.00 |
| Hypromellose acetate succinate | 25.00 | 150.00 |
| Sodium lauryl sulfate | 2.00 | 12.00 |
| Cross-linked sodium carboxymethyl cellulose | 6.00 | 36.00 |
| Microcrystalline cellulose | 27.83 | 167.00 |
| Mannitol | 27.83 | 167.00 |

(continued)

| Component | Weight % | Mass (mg) |
|---|---|---|
| Colloidal silicon dioxide | 1.00 | 6.00 |
| Magnesium stearate | 2.00 | 12.00 |
| Total | 100.0 | 600.0 |

*Example 6: Bioavailibility of CVC formulation*

The absolute bioavailability of the tablets of Example 3 in beagle dogs was compared to that of the tablets of Examples 4 and 5, as well as to both an oral solution of cenicriviroc mesylate and a gelatin capsule containing cenicriviroc mesylate powder. The results are shown in Table 8.

Table 8

| Component | Absolute bioavailability(%) |
|---|---|
| Oral Solution | 25.8 |
| Powder in capsule | 6.4 |
| Example 3 | 26.6 |
| Example 4 | 21.1 |
| Example 5 | 12.4 |

This example demonstrates that the bioavailability of cenicriviroc in dry granulated tablets with fumaric acid (Ex. 3) is substantially similar to that of an oral solution, and is significantly higher than the bioavailability of cenicriviroc in wet granulated tablets with fumaric (Ex. 1b) or citric acid (Ex. 4), and over double that of cenicriviroc in tablets with amorphous cenicriviroc in a spray dried dispersion with HPMC-AS (Ex. 5). These results are surprising, because there was no reason to suspect that dry granulation of crystalline API provides a significant increase in bioavailability over wet granulation and amorphous spray dried dispersions. This is especially so because amorphous spray dried dispersions are frequently used to increase the bioavailability of poorly water soluble drugs. These results are also surprising because fumaric acid has a slower dissolution time than citric acid and was used at a lower mass ratio of acid relative to CVC API (3:1 for citric acid: API versus 1.06:1 fumaric acid : API). Hence it was therefore surprising that fumaric acid proved to be a more effective solubilizer than citric acid for CVC.

*Example 7: Accelerated stability of CVC formulation*

The accelerated stability of the tablets of Example 2b was compared to that of the tablets of Examples 1b, 4, and 5 via exposure to an environment of 75% relative humidity at 40° C. All tablets were packaged with a desiccant during the study. As shown in Figure 3, the tablets of Examples 2b are surprisingly much more stable than the other wet granulated tablets, and similarly stable as the spray dried dispersion tablets. This difference in stability between the tablets of Examples 2b and Example 4 is particularly surprising since the only significant difference between the two is the method of making the formulations (dry granulation vs. wet granulation). These results are also surprising, because it was not previously known that the method of granulation could have an effect on both cenicriviroc bioavailability and stability.

*Example 8: Stability of CVC formulation*

The stability of the tablets of Examples 2 and 3 was tested by exposing the tablets to an environment of 75% relative humidity at 40° C for six weeks. All tablets were packaged with a desiccant during the study. The results are shown in Table 9, which shows that the tablets are very stable under these conditions.

**Table 9**

| Time (Weeks) | Water content (%) | Strength (%) | Total Impurities (%) |
|---|---|---|---|
| 0 | 1.5 | 99.1 | 1.2 |

(continued)

| Time (Weeks) | Water content (%) | Strength (%) | Total Impurities (%) |
|---|---|---|---|
| 2 | 1.4 | 99.2 | 1.1 |
| 4 | 1.4 | 98.0 | 1.0 |
| 6 | 1.4 | 98.6 | 1.0 |

*Example 9: Stability of CVC formulations*

Dynamic vapor sorption isotherms at 25° C correlate to the stability of the tablets of Examples 3 and 4 with that of cenicriviroc mesylate. Sorption was performed from 0% relative humidity to 90% relative humidity at 5% intervals. At each interval, each sample was equilibrated for no less than 10 minutes and no longer than 30 minutes. Equilibration was stopped when the rate of mass increase was no more than 0.03% w/w per minute or after 30 minutes, whichever was shorter. The result, which appears in Figure 4, shows that tablets of Example 2b are significantly more stable than those of Example 4. This result is consistent with Example 3 being significantly less hygroscopic than Example 4. The increased hygroscopicity of Example 4, in comparison to Examples 2b, can be associated with a higher mobile water content which can in turn cause partial gelation and subsequent decreased stability of Example 4.

*Example 10: Bioavailability of CVC formulations*

The bioavailability of the tablets of Example 3 was compared to that of Example 5 and cenicriviroc mesylate powder in a gelatin capsule in different stomach states in beagle dogs. The bioavailability was tested under different pre-treatment states, each of which alters the gastric pH. Specifically, pentagastric pretreatment provides the lowest pH, no treatment provides an intermediate pH, and famotidine treatment provides the highest pH.

The result, which appears in Figure 5, shows that the tablets of Example 3 has a higher bioavailability under all conditions that were tested. The bioavailability of Example 3 varied less between pentagastrin treated and untreated dogs, whereas Example 4 showed a significant loss of bioavailability in fasted, non-treated dogs (intermediate gastric pH) compared to that in pentagastrin treated dogs (lowest gastric pH). Pretreatment with famotidine, an H2 receptor agonist that suppresses stomach acidity and raises gastric pH decreased bioavailability for all samples, however, the reduction for Example 3 was much less than that for Example 4.

These results demonstrate an additional unexpected benefit of dry granulated cenicriviroc compositions with fumaric acid. Specifically, the pharmacokinetics of such formulations do not vary as much as those of the spray dried dispersion (Example 4) when administered across a the full range of potential human gastric pH conditions. This result is unexpected and surprising, because the bioavailability of other weakly basic antiretroviral drugs, such as atazanavir, is greatly effected by the gastric pH. For such drugs, changes in gastric pH, which can be caused by a disease or medical condition, such as achlorohydric patients, or by coadministration of drugs such as antacids, proton pump inhibitors, or H2 receptor agonists, can lower the bioavailability to sub-therapeutic levels. These results showing that the dry granulated, fumaric acid based cenicriviroc mesylate formulation of Example 3 is less prone to bioavailability changes as the gastric pH changes shows that Example 3 is a more robust formulation that can be used in patients who have or are likely to have varying gastric pH levels.

*Examples 11a-11c: Preparation of Cenicriviroc mesylate and lamivudine formulations*

The formulations of cenicriviroc mesylate and lamivudine of Table 10 were prepared as follows. First, the intragranular components were admixed and dry granulated to form a composition as a dry granulated admixture. This dry granulated admixture was then further admixed with the extragranular components to form a mixture. The mixture was compressed into tablets. The absolute bioavailability of the cenicriviroc (CVC) and lamivudine (3TC) in beagle dogs in the 150 mg CVC strength tablets (Examples 11b and 11c) were measured. The results are shown in Figure 6.

**Table 10**

| | Example 12a | | Example 12b | | Example 12c | |
|---|---|---|---|---|---|---|
| | 25 mg cenicriviroc and 300 mg lamivudine | | 150 mg cenicriviroc and 300 mg lamivudine | | 150 mg cenicriviroc and 300 mg lamivudine | |
| | % w/w | mg/tablet | % w/w | mg/tablet | % w/w | mg/tablet |
| **Intragranular Components** | | | | | | |
| Cenicriviroc mesylate | 5.69 | 28.45 | 17.97 | 170.69 | **21.34** | **170.69** |
| Fumaric Acid | 5.33 | 26.67 | 16.84 | 160.00 | 20.00 | 160.00 |
| Microcrystalline cellulose | 5.82 | 29.11 | 18.39 | 19.50 | 2.64 | 21.10 |
| Cross-linked sodium carboxymethyl cellulose | 0.65 | 3.25 | 2.05 | 19.50 | 2.64 | 21.10 |
| Magnesium stearate | 0.16 | 0.81 | 0.51 | 4.88 | 0.53 | 4.20 |
| **Extragranular Components** | | | | | | |
| Lamivudine (3TC) | 60.00 | 300.00 | 31.58 | 600.00 | 37.50 | 300.00 |
| Microcrystalline cellulose | 16.34 | 81.71 | 6.39 | 60.75 | 3.78 | 30.21 |
| Cross-linked sodium carboxymethyl cellulose | 5.00 | 25.00 | 5.26 | 50.00 | 5.00 | 40.00 |
| Magnesium stearate | 1.00 | 5.00 | 1.00 | 9.50 | 1.00 | 8.00 |
| **Total per tablet** | 100.00 | 500.00 | 100.00 | 950.00 | 100.00 | 800.00 |

*Example 12: Anti-fibrotic And Anti-Inflammatory Activity Of The Dual CCR2 And CCR5 Antagonist Cenicriviroc In A Mouse Model Of NASH*

**Background:** Non-alcoholic steatohepatitis (NASH) is characterized by fat accumulation, chronic inflammation (including pro-inflammatory monocytes and macrophages) and when fibrosis is present, it can lead to cirrhosis or hepatocellular carcinoma. There are currently no approved therapies for NASH. Evidence suggests that C-C chemokine receptor (CCR) type 2 and its main ligand, monocyte chemotactic protein-1, contribute to pro-inflammatory monocyte recruitment in the liver. Cenicriviroc (CVC) is an oral, potent, dual CCR2/CCR5 antagonist that showed favorable safety and tolerability in a 48-week Phase 2b study in 143 HIV-1-infected adults (NCT01338883). CVC was evaluated in a mouse model of diet-induced NASH that leads to hepatocellular carcinoma; data from the first, fibrotic stage of the model are presented.

**Methods:** NASH was induced in male mice by a single injection of 200μg streptozotocin 2 days after birth (causing impaired glucose control), followed by a high fat diet from 4 weeks of age. From 6 to 9 weeks of age, 3 groups of animals (n=6/group) were administered CVC doses of 0 (vehicle), 20 (low dose) or 100 (high dose) mg/kg/day, via twice daily oral gavage. Animals were sacrificed at 9 weeks of age, and biochemical, gene expression, and histologic evaluations of the liver were conducted.

**Results:** CVC treatment had no effect on body or liver weight, whole blood glucose, or liver triglycerides. Mean (±SD) alanine aminotransferase levels were significantly decreased in both CVC treatment groups compared to control (58±12, 51±13 and 133±80 U/L for low dose, high dose and vehicle, respectively; p<0.05) and liver hydroxyproline tended to decrease in treated groups. By real-time RT-PCR, collagen type 1 mRNA in whole liver lysates decreased by 27-37% with CVC treatment. The percentage of fibrosis area (by Sirius red staining) was significantly decreased by CVC treatment relative to control (p<0.01): 0.66% ± 0.16, 0.64% ± 0.19 and 1.10% ± 0.31 for 20 mg/kg/day, 100 mg/kg/day and control, respectively, when perivascular space was included; 0.29% ± 0.14, 0.20% ± 0.06, and 0.61%± 0.23, respectively, when perivascular space was subtracted. Importantly, the histologic non-alcoholic fatty liver disease activity score (score is 0 for untreated mice in this model) was significantly decreased with CVC treatment (4.0±0.6, 3.7±0.8

and 5.3±0.5 for low dose, high dose and vehicle, respectively; $p<0.05$), primarily due to reduced inflammation and ballooning scores. As previously shown in humans, a CVC dose-related compensatory increase in plasma monocyte chemotactic protein-1 levels was observed in mice (1.1- and 1.5-fold increase for low and high dose, respectively), consistent with antagonism of CCR2.

**Conclusions:** These data suggest that CVC, an investigational agent currently in human trials for HIV-1, has anti-fibrotic and anti-inflammatory activity in a mouse model of NASH, warranting clinical investigation. These findings provide further evidence that disrupting the CCR2/monocyte chemotactic protein-1 axis may be a novel treatment approach for NASH.

*Example 13: Significant Anti-fibrotic Activity Of Cenicriviroc, A Dual CCR2/CCR5 Antagonist, In A Rat Model Of Thioacetamide-Induced Liver Fibrosis And Cirrhosis*

**Background:** C-C chemokine receptor (CCR) types 2 and 5 are expressed on pro-inflammatory monocytes and macrophages, Kupffer cells and hepatic stellate cells (HSCs), which contribute to inflammation and fibrogenesis in the liver. Cenicriviroc (CVC; novel, potent, oral, dual CCR2/CCR5 antagonist) had favorable safety/tolerability in a 48-week Phase 2b study in 143 HIV-1-infected adults (NCT01338883). This study evaluates the in vivo anti-fibrotic effect of CVC, and timing of treatment intervention relative to disease onset, in rats with emerging hepatic fibrosis due to thioacetamide (TAA)-induced injury.

**Methods:** Fibrosis was induced in male Sprague-Dawley rats by intraperitoneal administration of TAA 150 mg/kg 3 times/week for 8 weeks. Rats (n=4-8/group) received CVC 30 mg/kg/day (a), CVC 100 mg/kg/day (b) or vehicle control (c), concurrently with TAA for the first 8 weeks (Group 1; early intervention), during Weeks 4-8 (Group 2; emerging fibrosis) or during Weeks 8-12 following completion of TAA administration (Group 3; cirrhosis reversal). Biochemical, gene expression and histologic evaluations of the liver were conducted.

**Results:** When started concurrently with TAA (Group 1), CVC at 30 mg (Group 1a) and 100 mg (Group 1b) significantly reduced fibrosis (by 49% and 38%, respectively; $p<0.001$), as assessed by collagen morphometry. Protein levels for collagen type 1 were reduced by 30% and 12% for Groups 1a and 1b, respectively, while $\alpha$-SMA was reduced by 17% and 22%, respectively. When treatment started 4 weeks after TAA-induced injury (Group 2), a statistically significant anti-fibrotic effect was observed for CVC 30 mg (Group 2a, 36% reduction in collagen; $p<0.001$), but not for CVC 100 mg (Group 2b).. When treatment was started at Week 8 (cirrhosis present) and continued for 4 weeks (Group 3), there was no significant effect of CVC on fibrogenic gene expression or fibrosis.

**Conclusions:** CVC is a potent anti-fibrotic agent in non-cirrhotic hepatic fibrosis due to TAA. The drug was effective in early intervention (Group 1) and in emerging fibrosis (Group 2a), but not when cirrhosis was already established (Group 3).

*Example 14: Cenicriviroc Achieves High CCR5 Receptor Occupancy at Low Nanomolar Concentrations*

**Background:** Cenicriviroc (CVC) is a novel, once-daily, potent, CCR5 and CCR2 antagonist that has completed Phase 2b evaluation for the treatment of HIV-1 infection in treatment-naive adults (NCT01338883). The aims of this study were to evaluate in vitro receptor occupancy and biology after treatment with CVC, BMS-22 (TOCRIS, a CCR2 antagonist) and an approved CCR5 antagonist, Maraviroc (MVC).

**Methodology:** PBMCs from 5 HIV+ and 5 HIV- subjects were incubated with CVC, BMS-22 or MVC, followed by either no treatment or treatment with a RANTES (CCR5 ligand) or MCP-1 (CCR2 ligand). The capacity of each drug to inhibit CCR5 or CCR2 internalization was evaluated. Cell-surface expression of CCR5 and CCR2 was assessed by flow cytometry, and fluorescence values were converted into molecules of equivalent soluble fluorescence (MESF).

**Results:** Both CVC and MVC, in the absence of RANTES, increased cell-surface expression of CCR5. This effect was seen to a much greater degree in HIV-negative subjects (CD4+ and CD8+ T cells). CVC prevented RANTES-induced CCR5 internalization at lower effective concentrations than MVC. The effective concentration at which saturation of CCR5 was reached for CVC was 3.1 nM for CD4+ and 2.3 nM for CD8+ T cells (~91% and ~90% receptor occupancy, respectively). MVC reached saturation at 12.5 nM for both CD4+ and CD8+ T cells, representing ~86% and ~87% receptor occupancy, respectively. CVC and MVC achieved high but incomplete saturation of CCR5, an effect that may be amplified by the observation of increased CCR5 expression with both agents in the absence of RANTES. In the absence of MCP-1, CVC induced CCR2 internalization and decreased cell-surface expression on monocytes. BMS-22 slightly increased CCR2 cell-surface expression. CVC prevented MCP-1-induced CCR2 internalization at lower concentrations than BMS-22. Saturation of monocyte CCR2 was reached at 6 nM of CVC, representing ~98% CCR2 occupancy. To reach >80% receptor occupancy, an average of 18 nM of BMS-22 was required, compared to 1.8 nM of CVC.

**Conclusions:** CVC more readily prevented RANTES-induced CCR5 internalization (at lower concentration) than MVC in vitro, indicating CVC more be more effective at preventing cellular activation by RANTES than MVC in vivo.

Baseline CCR5 expression levels in treated subjects may be a determinant of CCR5 antagonist activity in vivo. CVC achieved ~98% receptor occupancy of CCR2 on monocytes at low nanomolar concentrations in vitro, and reduced CCR2 expression on monocytes in the absence of MCP-1. High saturation of CCR2 by CVC paired with reduced expression may explain the potent CCR2 blockade observed with CVC in the clinic. CVC has potent immunomodulatory activities in vitro, and may be an important combined immunotherapeutic and anti-retroviral in chronic HIV infection.

*Example 15: CVC Blocks HIV Entry But Does Not Lead To Redistribution Of HIV Into Extracellular Space Like MVC*

*Background:* In vivo, CVC has shown efficacy during monotherapy of treatmentexperienced individuals harbouring CCR5-tropic virus 7. In the phase IIb clinical study (652-2-202; NCT01338883), CVC demonstrated similar efficacy at 24 weeks (primary analysis) to the non-nucleoside reverse transcriptase inhibitor (NNRTI) efavirenz (EFV), and a superior toxicity profile than the non-nucleoside reverse transcriptase inhibitor (NNRTI) efavirenz (EFV),each when both were administered in combination with emtricitabine (FTC) and tenofovir (TDF), with favorable safety and tolerability. We hypothesized that the antiretroviral efficacy of CVC in Study 202 (Example 22) might have been underestimated as a result of the rebound phenomenon observed with MVC. Accordingly we conducted an ex vivo sub-analysis of Study 202 (Example 22) by measuring intracellular HIV DNA declines in stored PBMCs from 30 subjects who achieved virologic success at week 24 of the study. We also performed in vitro assays to determine and compare the extent of any cell-free virion redistribution that CVC or MVC might cause.

We now show that CVC does not trigger viral particle rebound. Indeed, comparable declines in intracellular DNA were seen in individuals treated with either CVC or EFV, suggesting that plasma viral load is an accurate measure of CVC treatment success. Structural modeling provides a potential explanation for differences between results obtained with MVC and CVC.

*Methods:* Cells. PM-1 cells that express CD4, CCR5, and CXCR4 were maintained in RPMI-1640 medium containing 10% fetal bovine serum (R10 medium) at 37C, 5% CO2. 293T cells used for transfection were maintained in DMEM at 10% FBS, L-glutamine, and antibiotics (D10 medium) at 37C, 5% CO2. Virus Stocks. HIV-1 BaL virus was produced by transfecting 293T cells with the plasmid pWT/BaL. Lipofectamine 2000 was used as a transfection agent. Culture supernatants were collected at 48 hrs post-transfection, filtered through a 0.45 μm pore filter, and treated with 50 units of benzonase per ml of virus stock for 20 minutes at 37C to remove contaminating plasmid DNA. Virus stocks were frozen at -80C to halt benzonase activity. Benzonase-treated virus stocks were propagated in cord blood mononuclear cells (CBMCs). CBMCs were stimulated for 72 h with phytohemagluttinin (PHA-M) in R10 medium prior to infection with HIV-1 BaL. The viral amplification culture was subsequently grown in R10 supplemented with interleukin 2 (IL-2) and incubated at 37C, 5% CO2.

*Infections:* We exposed PM-1 cells to HIV-1 BaL in the presence of inhibitory concentrations of CVC (20 nM) and MVC (50 nM). Both drugs were incubated with PM-1 cells for 1 hr at 37C prior to the addition of virus. 500 ng of p24 antigen of HIV-1 BaL were incubated per 5 X 105 cells in 1 ml of R10 media. Virus only controls, described as "no cell" in the text, were used to measure viral decay. Viral adsorption was measured in the no-drug controls, whereby 500 ng of p24 Ag of HIV-1 Bal were added per 5 X 105 PM-1 cells that were preincubated at 37C for 1 hr in the absence of drug treatment. Each drug treatment and control was performed in duplicate. Viral RNA was extracted from 140 μl of supernatant fluid using the QIAamp Viral RNA mini kit according to manufacturer's instructions. Samples were stored at - 80C until analysis. Supernatant viral loads were measured using quantitative real-time reverse transcription PCR (qRT-PCR) with the primers US1SSF (5'-AACTAGGGAACCCACTGCTTAA-3'), US1SSR (5'-TGAGGGATCTCTAGTTACCAGAGTCA-3') and US1SS probe (5'- (FAM) CCTCAATAAAGCTTGCCTTGAGTGCTTCAA) and the Invitrogen qRT-PCR Supermix Kit. Cycling parameters were: 50°C for 15 minutes, 95°C for 10 minutes, followed by 50 cycles of 95°C for 15 seconds, and 60°C for 1 minute. All values are the result of replicate testing over 2 independent experiments. RNA copy number was quantified by use of 10-fold serial dilutions of pBaL/wt to generate standard curves for each assay and calibrated against samples with known copy numbers from previous studies.

*Patient Samples:* Peripheral blood mononuclear cells (PBMC) samples were obtained from 30 patients (10, 13 and 7 on CVC 100 mg, CVC 200 mg and EFV, respectively) who achieved virologic success at week 24 in Study 202 a phase IIb clinical trial comparing the efficacy, safety, and tolerability of CVC (100 mg or 200 mg) or EFV in combination with emtricitabine/tenofovir disproxil fumarate (FTC/TDF) in HIV-1 infected, treatment-naive patients harboring CCR5-tropic virus. Samples at baseline and 24 weeks were taken from participants possessing baseline viral loads of <100,000 but >1,000 viral RNA copies/ml, with CD4 counts ≥ 200 cells/μl that were randomly assigned to receive either CVC or EFV.

Intracellular DNA qPCR. Total DNA was extracted, quantified, and stored at -80C. Intracellular strong-stop DNA levels were quantified with the US1SS primer/probe set described above. Intracellular full-length DNA levels were quantified using the US1FL primer/probe set (Forward: 5'-AACTAGGGAACCCACTGCTTAA; Reverse: 5'-CGAGTCCTGCGTCGAGAGA; Probe: 5'-[FAM] -CCTCAATAAAGCTTGCCTTGAGTGCTTCAA). Both DNA levels were multiplexed with a GAPDH primer/probe set (Forward: 5'-ACCGGGAAGGAAATGAATGG; Reverse: 5'-GCAGGAGCGCAGGGTTAGT; Probe: 5'-(VIC)-ACCGGCAGGCTTTCCTAACGGCT) to normalize DNA inputs and verify sample integrity.

*Statistical Analysis:* The Mann-Whitney test was used to analyze in vitro intracellular HIV DNA levels for all three treatment groups. All data were analyzed using Prism 5 software.

*Molecular Docking of Cenicriviroc in CCR5*

The crystal structure of the CCR5 chemokine receptor (Protein Data Bank identificationNo. [PDB ID] 4MBS) was obtained through the Research Collaboratory for Structural Bioinformatics (RCSB) Protein Data Bank and used as a docking target. The structure of the CCR5-receptor antagonist, cenicriviroc, (formerly TAK-652/TBR-652) was obtained from PubChem and used as a ligand. Minimization of ligand-docked structures was facilitated by the use of a UCSF Chimera, that prepared CCR5 and CVC as inputs for DOCK calculations, that predict the orientation of the ligand in the CCR5 seven-transmembrane (7TM) $\alpha$-helix receptor cavity. Docking calculations were performed and a maximum sized grid box was used to include all possible docking sites into CCR5. The binding site consists of all residues less than 15 Å from the 7TM cavity (around residues Glu283 and Tyr10). Docking results were processed to identify intermolecular interactions. The test nine poses were kept for further analysis. In order to validate the accuracy of the docking system, MVC was docked to CCR5 using the same method and its orientation with respect to the crystal structure was determined. The root mean square deviation (RMSD), calculated using PyMOL, between the observed crystal structure and the predicted conformation obtained from AutoDock Vina was 0.275 Å, indicating that the protocol was sound.

Results

First we quantified HIV intracellular DNA in order to validate measures of viral load that were obtained during the Study 202 clinical trial. *Ex vivo* analyses of full-length intracellular HIV DNA levels (indicative of early reverse transcription) in PBMCs isolated from participants in this clinical trial were similar across all groups (CVC 100 mg, CVC 200 mg, EFV 600 mg) at week 24 (Figure 7A). The mean fold-changes from baseline were 0.643 and 0.787 for the CVC groups 100 mg (n = 10) and CVC 200 mg (n = 11), respectively. The EFV 600 mg group (n = 7) had a mean fold change from baseline of 0.825 at 24 weeks. The differences were not statistically significant.

Next, strong-stop intracellular HIV DNA levels (indicative of late reverse transcription) were measured concomitantly with full-length levels at week 24 (Figure 7B). The mean fold-change from baseline was 0.49 for the CVC 100 mg group, 0.63 for the CVC 200 mg group, and 1.01 for the EFV 600 mg group. The means were not statistically significant.

*In vitro* experiments measuring extracellular viral levels following CVC and MVC exposure were also performed. Levels of virus in culture fluids were measured by qRT-PCR and P24 ELISA at 4 hrs following infection of entry-inhibitor exposed cells. After 4 hrs, culture fluids from the MVC-treated cells exhibited higher RNA levels compared to baseline (baseline: 1.19 X $10^{10}$ copies/ml, 4 hrs: 1.67 X $10^{10}$ copies/ml) (Figure 8A) than did CVC-treated cells. (baseline: 506 ng/ml, 4 hrs: 520 ng/ml) (Figure 8B). Viral RNA in culture fluids from CVC-treated cells did not change significantly after 4 hrs (baseline: 1.19 X $10^{10}$ copies/ml, 4 hrs: 1.26 X $10^{10}$ copies/ml) (Figure 8A). P24 levels declined from baseline after 4 hrs with CVC treatment (baseline: 506 ng/ml, 4 hrs: 192 ng/ml) (Figure 8B) the viral RNA declines for the no cell and no drug controls were similar after 4 hrs, 1.14 X $10^{10}$ copies/ml, and 1.1 X $10^{10}$ copies/ml respectively (Figure 8A). Following a baseline p24 level of 506 ng/ml, the p24 antigen level for the no cell control after 4 hrs was 138 ng/ml. The p24 no drug control level was 244 ng/ml (Figure 8B).

These differences in extracellular virus levels following CVC and MVC treatment prompted us to examine intracellular strong-stop HIV DNA levels in PM-1 cells exposed to either CVC or MVC for 1 hr before being infected with HIV-1 BaL. Total DNA was extracted from cell pellets after 4 hrs. Intracellular strong-stop HIV DNA levels of CVC or MVC-treated cells were compared to no drug controls (Figure 9). We observed a relative DNA level of 0.02 in MVC-treated cells compared to the no drug control whereas CVC-treated cells exhibited a relative intracellular DNA level of only 0.1. The difference between relative DNA levels of MVC and CVC-treated cells was significant.

A crystal structure exists of the CCR5 7TM complexed with MVC (PDB ID 4MBS) and this was used to generate a model of CCR5 with CVC docked into the binding pocket. We predicted docked poses that were also assessed by re-docking MRV into CCR5; the top poses with the most favorable energies had the proper orientation and overlap with the conformation in the crystal structure (RMSD < 0.3 Å). *In silico* CCR5 docking simulations indicated that CVC binds only at the hydrophobic pocket in the CCR5 structure, also known as the ligand-binding pocket (Figure 10). Only the top 9 poses were kept for further analysis. There are three different conformations that CVC exhibits post-docking into CCR5 and they are clustered into three sites (Figure 10A, B). The first site (site 1) spans deep into the hydrophobic pocket and fills a large volume (Figure 10A). The second site (site 2) is partially positioned in the middle of the pocket but also bulges outward from the CCR5 between TM1 and TM7 (Figure 10A). At the third site (site 3), few CVC poses are located near the entrance of the receptor cavity.

Site-directed mutagenesis of residues within the extracellular loops and transmembrane domain in CCR5 have identified key residues that are involved in gp120 binding; mutations at the different positions either abolished, compromised or affected gp120 binding to CCR5. The thirteen key residues that were identified to be important for gp120 binding

within CCR5 are Tyr37, Trp86, Trp94, Leu104, Tyr108, Phe109, Phe112, Thr177, Ile198, Trp248, Tyr251, Leu255 and Glu283. Figure 11 shows a molecular surface representation of CCRS with docked poses of CVC (left) and MVC (right) in the binding pocket. CVC and MVC have molecular surface areas of ~1285 and 1790 $Å^2$ (calculated using PyMOL), respectively. MVC occupies the middle of the binding pocket. All thirteen residues that were determined to be important for gp120 binding are within 4 Å from MVC, as measured by PyMOL (cut off distance used in this study for electrostatic and/or hydrophobic interactions). In contrast, the docked CVC poses occupy the same pocket but not at the center as seen for MVC (Figure 11). Rather, CVC shifts to one side of the pocket (Figure 12A/B) and a consensus of residues in CCR5 within 4 Å of CVC was determined. Even though CVC occupies a larger surface area than MVC, only seven of the thirteen residues that are important for gp120 binding are within 4 Å of CVC i.e. Tyr37, Trp86, Tyr108, Phe109, Ile198, Leu255 and Glu283. Overall, these simulations suggest that CVC occupies a region similar to MVC in the binding pocket of CCR5.

**Discussion:** In this study, we observe that CVC and MVC, both CCR5 antagonists preventing HIV entry, have a differential effect on extracellular virus levels.

In a phase IIb double-blind, double-dummy study comparing CVC with EFV, both with FTC/TDF in treatment-naive subjects, 76% of patients receiving CVC 100mg achieved virologic success (HIV RNA <50 copies/ml) at 24 weeks compared to 73% of patients receiving CVC 200mg and 71% of patients receiving EFV. We previously showed that MVC might artificially increase viral load, because cell-free virions can be repelled from the target cell following a failed attempt at entry in the presence of MVC. The current study was designed to address whether the same effect might occur for CVC and whether intracellular DNA measurements might be a more accurate representation of antiviral efficacy when comparing entry and reverse transcriptase inhibitors.

In fact, intracellular DNA levels across Study 202 treatment arms were similar at 24 weeks (Figure 7) in selected samples, reflecting the trend observed during the intent to treat (ITT) analysis. Full-length HIV-DNA levels were also similar for all groups at week 24, suggesting similar antiviral efficacy for both CVC and EFV. Differences in strong-stop HIV DNA levels were observed between the CVC and EFV groups, whereas both CVC groups exhibited steeper declines in viral load compared to EFV. As strong-stop HIV DNA levels are directly impacted by entry inhibitors, this result was expected. The similarities between EFV and CVC in terms of virologic success and intracellular HIV DNA levels suggest that the antiviral potency of this dual CCR5 and CCR2 inhibitor is not masked by viral load measurements.

We also asked whether CVC can result in virus repulsion as seen for MVC *in vitro*. Two separate measurements of virus quantitation, qRT-PCR and p24 ELISA, showed that MVC treatment maintained extracellular viral levels up to 4 hrs post-infection. In contrast, treatment with CVC resulted in a decline in viral levels decline at 4 hrs, comparable to that of the no drug or no cell controls (Figure 8). Despite an ostensibly similar antiviral mechanism, there appear to be differences between CVC and MVC in regard to interactions between cell-free virus and CCR5.

A further examination of intracellular strong-stop DNA *in vitro* showed that CVC caused a slight albeit significant increase in levels compared to MVC (Figure 9). This may be due to the differential effect of both inhibitors on CCR5, which, in turn, affects the rate of dissociation between virus and receptor. This raises the possibility that gp120 may associate more durably with CVC-bound CCR5 compared to MVC.

We also aimed to understand how CVC inhibits HIV entry into target cells by examining the binding site of CCR5. An engineered human CCR5 construct has been previously crystalized in complex with MVC at a resolution of 2.7 Å. Although this is not a full-length crystal structure of CCR5, it was utilized to better understand CVC interactions with CCR5 in *in silico* docking assays. All purported docking models for CVC imply a deep penetration of the drug into the 7TM cavity of CCR5, as is also seen for MVC. However, the CVC docked poses were not in close proximity to extracellular loop 2, ECL2 remained accessible post-docking. Other groups have reported that the CCR5 N-terminus and ECL2 domains both play a critical role in the interaction of HIV-1 with CCR5. In addition, the stem region of the V3 loop of gp120 is reported to bind to the CCR5 N-terminus while the V3 crown interacts with ECL2 and with residues inside the binding pocket. Based on our model, we can assume that CVC does not interfere directly with the gp120 V3 loop interaction with ECL2, since ECL2 appears to be exposed in the model.

It is conceivable that CVC can block CCR5 activation if CCR5 remains in an inactive state. Two residues, Tyr37 and Trp248, in the 7TM region have been shown to be important for CCR5 activation upon binding chemokine ligands, and this has also been shown to be important for MVC binding. Similar to MVC, different docked poses of CVC are buried in the hydrophobic binding site. Our model shows that access to Trp248 is blocked by CVC; Trp248 has been shown to be important for CCR5 activation, explaining the inactivation of the chemokine receptor. A second hypothesis is that the binding of MVC to CCR5, may cause CCR5 to undergo a global conformational change, that may be less altered in the presence of CVC.

Based on site-directed mutagenesis experiments by other groups and the tissue culture experiments and docking simulations presented in this study, we hypothesize that MVC occupies the middle of the hydrophobic pocket, potentially leading to an inaccessibility of some of residues in CCR5 that are important for gp120 binding. These residues may also be important for gp120 binding through direct electrostatic, or hydrophobic interactions and/or water-mediated hydrogen bonds. In contrast, CVC occupies the binding site, and it may be that gp120 can still access some of the

residues important for CCR5 binding even in the presence of docked CVC. This hypothesis is supported by site-directed mutagenesis studies that suggest that gp120 partly fills the receptor cavity while occupying the entirety of ECL2. However, the degree to which the V3 loop of gp120 penetrates the CCR5 7TM remains unknown. It has also been reported that dissociation rates of gp120 from CCR5 are accelerated in the presence of MVC, since the latter hinders the tight association between ECL2 and the V3 loop. Based on these studies, CVC may have a different effect on the ECL2/V3 interaction than does MVC. Dissociation and surface plasma resonance studies as well as crystallization of CCR5 in complex with CVC will provide valuable information on this topic.

Site-directed mutagenesis and biochemical studies are required to elucidate the residues that are important for CCR5 interaction with CVC. Determining the proximal location of the N terminus of CCR5 is also of interest.

In this study, we have demonstrated that, viral load quantification is an accurate measurement of the antiviral efficacy of CVC, and that inhibition of viral entry by CVC does not lead to the rebound of viral particles from the cell surface to the extracellular environment. Our *in silico* structure modeling provides a potential explanation for functional differences between CVC and MVC. Further studies are required to understand how CVC affects gp120 binding to CCR5.

*Example 16: Anti-fibrotic Activity of Dual CCR5/CCR2 Antagonist Cenicriviroc in a Mouse Model of Renal Fibrosis*

**Background:** Cenicriviroc (CVC) is a novel, oral, once-daily, dual CCR5/CCR2 antagonist that has completed Phase 2b HIV development (Study 202; NCT01338883). CVC has a favorable safety profile with 555 subjects having been treated with at least one dose, including 115 HIV-1-infected adults treated with CVC over a 48-week duration. Recently, CVC demonstrated significant anti-fibrotic activity in a mouse model of diet-induced, non-alcoholic steatohepatitis (NASH) and a rat model of thioacetamide-induced fibrosis. Here, we evaluated CVC in a well-established mouse model of renal fibrosis induced by unilateral ureter occlusion (UUO).

**Methodology:** Test animals were allocated to weight-matched treatment groups on the day prior to the surgical procedure (Day -1). Male CD-1 mice (N=51; age, 7-8 weeks) underwent either sham surgery or total ligation of the right ureter, i.e. UUO, via aseptic laparotomy (Figure 12). From Days 0 to 5: mice undergoing sham surgery received vehicle control (0.5% methylcellulose + 1% Tween-80) via twice-daily oral gavage; mice with permanent UUO received either vehicle control, CVC 7 mg/kg/day or CVC 20 mg/kg/day via twice-daily oral gavage. Another group received the anti-transforming growth factor TGF-β1 antibody, compound 1D11 (positive control) at 3 mg/kg/day from Days -1 to 4, injected intraperitoneally once daily, and vehicle control from Days 0 to 5. A CVC 100 mg/kg/day group (N=9) was initially included in the study but was terminated early due to moribundity (no analyses were conducted because no animal reached Day 5). CVC doses up to 2000 mg/kg/day were well tolerated in mouse toxicity studies that did not involve surgical procedures. On Day 5, animals were anaesthetised, blood and tissues were collected prior to sacrifice.

**Study endpoints:** Study endpoints included: a) body and kidney weights; b) fibrosis in obstructed kidney evaluated via histological quantitative image analysis of picrosirius red staining (ten images/depth/kidney obtained and assessed in a blinded fashion using light microscopy [at 200x] to enable sampling of 60-70% of the renal cortical area) and quantified by a composite Collagen Volume Fraction (CVF [% total area imaged]) score expressed as the average positive stain across three anatomically distinct (200-250 μM apart) tissue sections, or depths, from the obstructed kidney; c) hydroxyproline content of frozen renal cortical tissue biopsies as assessed by biochemical analyses; d) mRNA expression of profibrotic and inflammatory biomarkers (including MCP-1, Collagen 1a1, Collagen 3a1, TGF-β1, Fibronectin-1, α-smooth muscle actin (α-SMA) and connective tissue growth factor-1 (CTGF-1); assessed via Luminex® (Life Technologies™, Carlsbad, CA, USA) assay with relative expression normalised to HPRT (hypoxanthine phosphoribosyltransferase).

**Statistical analysis:** Data are expressed as mean ± standard error of mean (SEM). Statistical analyses were performed using GraphPad Prism® (GraphPad Software, Inc., San Diego, CA, USA). Treatment differences between sham-surgery+vehiclecontrol and UUO+vehicle-control groups, and between UUO+vehicle-control and UUO+compound-1D11 (positive control) groups, were analysed by unpaired t-Test. Treatment differences between UUO+vehicle-control and CVC-dose groups were analysed by one-way ANOVA (analysis of variance) with Dunnett's test (post-hoc).

**Methods:** CVC demonstrated significant antifibrotic effects, as defined by reductions in Collagen Volume Fraction or CVF (% area stained positively for collagen in histological obstructed-kidney sections), in a well-established mouse UUO model of renal fibrosis. Trends were observed for decreases in Collagen 1a1, Collagen 3a1, TGF-β1 and Fibronectin-1 mRNA expression in the obstructed kidney, but these did not achieve statistical significance. Taken together, CVC's mode of action, antifibrotic activity in animal models (kidney and liver), and extensive safety database support further evaluation in fibrotic diseases. A proof-of-concept study in non-HIV-infected patients with NASH and liver fibrosis is planned. Phase III trials in HIV-1-infected patients are also planned to evaluate a fixed-dose combination of CVC/lamivudine (3TC) as a novel 'backbone' versus tenofovir disoproxil fumarate/emtricitabine (TDF/FTC) when co-administered with guideline-preferred third agents.

**Results:** Body weight and obstructed kidney weight: CVC 7 mg/kg/day and compound 1D11 (positive control) had no effect on body weight, whereas CVC 20 mg/kg/day led to a modest, but significant, decrease (5%) in body weight,

relative to that of the UUO+vehicle-control group at Day 5 ($p<0.05$) (Figure 13; change in body weight shown in grams [g]). No significant treatment effects (CVC or compound 1D11 [positive control]) were observed on obstructed or contralateral kidney weight or kidney weight index versus the UUO+vehicle-control group (data not shown). Histology: The composite measure of CVF (% area averaged across three depths [±SEM]) was significantly higher in the UUO+vehicle-control group compared with that in the sham-surgery group (11.4±1.0-fold; $p<0.05$) (Figure 14). CVC 7 and 20 mg/kg/day and compound 1D11 (positive control) significantly attenuated UUO-induced increases in the composite measure of CVF (averaged across three depths [±SEM]) relative to that of the UUO+vehicle-control group (28.6±8.8%, 31.8±6.8% and 50.3±7.3% reduction, respectively; $p<0.05$).

**Hydroxyproline content:** Hydroxyproline content (% of protein) in obstructed kidneys from the UUO+vehicle-control group increased significantly relative to the sham-surgery group (0.72% vs 0.27%; $p<0.05$) (data not shown). Neither dose of CVC tested affected UUO-induced increases in obstructed kidney hydroxyproline content relative to the UUO+vehicle-control group; however, the compound 1D11 (positive control) group had significantly lower levels (0.55% vs 0.72%; $p<0.05$) (data not shown).

**Profibrotic and inflammatory biomarker mRNA expression:** For each of the biomarkers evaluated (MCP-1, Collagen 1a1, Collagen 3a1, TGF-β1, Fibronectin-1, α-SMA and CTGF-1), expression of mRNA in the UUO+vehicle-control group increased significantly compared with that in the shamsurgery group ($p<0.05$) (Figure 15). CVC 7 and 20 mg/kg/day attenuated UUO-induced increases in Collagen 1a1, Collagen 3a1, TGF-β1 and Fibronectin-1 mRNA expression. However, these reductions, compared with the UUO+vehicle-control group, did not reach statistical significance. Compound 1D11 (positive control) significantly reduced UUO-induced increases in mRNA expression of Collagen 1a1, Collagen 3a1, TGF-β1 and Fibronectin-1 relative to the UUO+vehicle-control group ($p<0.05$). CVC 7 and 20 mg/kg/day and compound 1D11 (positive control) did not have significant effects on UUO-induced increases in obstructed kidney cortical MCP-1, α-SMA and CTGF-1 mRNA expression, compared with the UUO+vehicle-control group (data not shown for α-SMA and CTGF-1 mRNA).

**Conclusions:** CVC demonstrated significant antifibrotic effects, as defined by reductions in Collagen Volume Fraction or CVF (% area stained positively for collagen in histological obstructed-kidney sections), in a well-established mouse UUO model of renal fibrosis. Trends were observed for decreases in Collagen 1a1, Collagen 3a1, TGF-β1 and Fibronectin-1 mRNA expression in the obstructed kidney, but these did not achieve statistical significance. Taken together, CVC's mode of action, antifibrotic activity in animal models (kidney and liver), and extensive safety database support further evaluation in fibrotic diseases. A proof-of-concept study in non-HIV-infected patients with NASH and liver fibrosis is planned. Phase III trials in HIV-1-infected patients are also planned to evaluate a fixed-dose combination of CVC/lamivudine (3TC) as a novel 'backbone' versus tenofovir disoproxil fumarate/emtricitabine (TDF/FTC) when co-administered with guideline-preferred third agents.

*Example 17: Improvements in APRI and FIB-4 fibrosis scores correlate with decreases in sCD14 in HIV-1 infected adults receiving cenicriviroc over 48 weeks*

**Background and aims:** Cenicriviroc (CVC), a novel, oral, once-daily CCR2/CCR5 antagonist, has demonstrated favorable safety and anti-HIV activity in clinical trials. CVC demonstrated antifibrotic activity in two animal models of liver disease. Post-hoc analyses were conducted on APRI and FIB-4 scores in Study 202 (NCT01338883).

**Methods:** 143 adults with CCR5 tropic HIV-1, BMI≤35kg/m2 and no apparent liver disease (ie, ALT/AST Grade≤2, total bilirubin≤ULN, no HBV, HCV, active or chronic liver disease, or cirrhosis) were randomized 4:1 to CVC or efavirenz (EFV). APRI and FIB-4 scores were calculated. Change in score category from baseline (BL) to Weeks 24 and 48 was assessed in patients with non-missing data. Correlations between changes from BL in APRI and FIB-4 scores, and MCP-1 (CCR2 ligand) and sCD14 (inflammatory biomarker) levels were evaluated.

**Results:** At BL, more patients on CVC than EFV had APRI≥0.5 and FIB-4≥1.45; proportion of CVC patients above these thresholds decreased at Weeks 24 and 48 (Table). Significant correlations were observed at Week 24 between changes in APRI score and MCP-1 levels ($p=0.014$), and between FIB-4 score and sCD14 levels ($p=0.011$), and at Week 48, between changes in APRI ($p=0.028$) and FIB-4 scores ($p=0.007$) and sCD14 levels. (Table 11).

Table 11

*[Table]*

| | Fibrosis index | | CVC | | | EFV | |
|---|---|---|---|---|---|---|---|
| | | **Baseline (n-113)** | **Week 24 (n-92)** | **Week 48 (n-80)** | **Baseline (n-28)** | **Week 24 (n-20)** | **Week 48 (n=17)** |
| **APRI category** | <0.5 | 84% | 93% | 91% | 96% | 100% | 100% |
| | 0.5-1.5 | 14% | 7% | 8% | 4% | - | - |
| | >1.5 | 2% | - | 1% | - | - | - |
| | *Decreased 1 category from baseline* | *N/A* | 14% | 10% | *N/A* | 5% | 6% |
| **FIB-4 category** | <1.45 | 82% | 93% | 94% | 100% | 100% | 94% |
| | 1.45-3.25 | 17% | 7% | 5% | - | - | 6% |
| | >3.25 | 1% | - | 1% | - | - | - |
| | *Decreased 1 category from beseline* | *N/A* | 13% | 14% | *N/A* | - | - |

**Conclusions:** In this population with no apparent liver disease, CVC treatment was associated with improvements in APRI and FIB-4 scores, and correlations were observed between changes in APRI and FIB-4 scores and sCD14 levels at Week 48. Proven CCR2/CCR5 antagonism, antifibrotic effects in animal models and extensive clinical safety data all support clinical studies of CVC in liver fibrosis.

*Example 18: In Vivo Efficacy Study of Cenicriviroc in STAM Model of Non-alcoholic Steatohepatitis*

This *in vivo* efficacy study was performed to examine the effects of Cenicriviroc in the STAM ™ mouse model of Non-alcoholic Steatohepatitis.

Materials and Methods

Experimental Design and Treatment

Study groups

Group 1-Vehicle: Eighteen NASH mice were orally administered vehicle at a volume of 10 mL/kg twice daily (9:00 and 19:00) from 6 weeks of age.

Group 2-Cenicriviroc 20 mg/kg (CVC-low): Eighteen NASH mice were orally administered vehicle supplemented with Cenicriviroc at a dose of 10 mg/kg twice daily (20 mg/kg/day) (9:00 and 19:00) from 6 weeks of age.

Group 3 - Cenicriviroc 100 mg/kg (CVC-high): Eighteen NASH mice were orally administered vehicle supplemented with Cenicriviroc at a dose of 50 mg/kg twice daily (100 mg/kg/day) (9:00 and 19:00) from 6 weeks of age.

Table 12 summarizes the treatment schedule:

Table 12

| Group | No. mice | Mice | Test substance | Dose (mg/kg) | Volume (mL/kg) | Regimen | Sacrifice (wks) |
|---|---|---|---|---|---|---|---|
| 1 | 18 | STAM | Vehicle | - | 10 | Oral, twice daily, 6-9 wks, 6 -18wks | 9 and 18 |
| 2 | 18 | STAM | CVC-low | 20 | 10 | Oral, twice daily,6-9 wks, 6 -18wks | 9 and 18 |

(continued)

| Group | No. mice | Mice | Test substance | Dose (mg/kg) | Volume (mL/kg) | Regimen | Sacrifice (wks) |
|---|---|---|---|---|---|---|---|
| 3 | 18 | STAM | CVC-high | 100 | 10 | Oral, twice daily,6-9 wks, 6 -18wks | 9 and 18 |

Results

Part 1: Study for Assessing the Anti-NASH/Fibrosis Effects of CVC

Body weight changes and general condition until Week 9 (Figure 16)

Body weight gradually increased during the treatment period. There were no significant differences in mean body weight between the Vehicle group and either the CVC-low or the CVC-high groups during the treatment period. None of the animals in the present study showed deterioration in general condition throughout the treatment period.

Body weight at the day of sacrifice at Week 9 (Figure 17A and Table 13)

There were no significant differences in mean body weight between the Vehicle group and either the CVC-low or the CVC-high groups (Vehicle: 18.9 ± 3.3 g, CVC-low: 19.5 ± 2.0 g, CVC-high: 18.7 ± 0.9 g).

Table 13: Body Weight and Liver Weight at Week 9

| Parameter (Mean ± SD) | Vehicle (n-6) | Cenicriviroc-low (n-6) | Cenicriviroc-high (n=6) |
|---|---|---|---|
| Body weight (g) | 18.9 ± 3.3 | 19.5 ± 2.0 | 18.7 ± 0.9 |
| Liver weight (mg) | 1270 ± 326 | 1334 ± 99 | 1307 ± 119 |
| Liver-to-body weight ratio (%) | 6.6 ± 0.8 | 6.9 ± 1.0 | 7.0 ± 0.8 |

Liver weight and liver-to-body weight ratio at week 9 (Figures 17 B & C and Table 13)

There were no significant differences in mean liver weight between the Vehicle group and either the CVC-low or the CVC-high groups (Vehicle: 1270 ± 326 mg, CVC-low: 1334 ± 99 mg, CVC-high: 1307 ± 119 mg).

There were no significant differences in mean liver-to-body weight ratio between the Vehicle group and either the CVC-low or the CVC-high groups (Vehicle: 6.6 ± 0.8%, CVC-low: 6.9 ±1.0%, CVC-high: 7.0 ± 0.8%).

Whole blood and biochemistry at week 9

Whole blood glucose data are shown in Figures 18A-D and Table 14.

There were no significant differences in blood glucose levels between the Vehicle group and either the CVC-low or the CVC-high groups (Vehicle: 590 ± 108 mg/dL, CVC-low: 585 ± 91 mg/dL, CVC-high: 585 ± 91 mg/dL). 4.4.2. Plasma ALT (Figure 18B, Table 14). The CVC-low and the CVC-high groups showed significant decreased in plasma ALT levels compared with Vehicle group (Vehicle: 133 ± 80 U/L, CVC-low: 58 ± 12 U/L, CVC-high: 52 ± 13 U/L).

Table 14: Blood and Liver Biochemistry at Week 9

| Parameter (Mean ± SD) | Vehicle (n=6) | Cenicriviroc-low (n=6) | Cenicriviroc-high (n=6) |
|---|---|---|---|
| Whole blood glucose (mg/dL) | 590 ± 108 | 585 ± 91 | 585 ± 91 |
| Plasma ALT (U/L) | 133 ± 80 | 58 ± 12 | 52 ± 13 |
| Plasma MCP-1 (pg/mL) | 60 ± 4 | 68 ± 16 | 91 ± 14 |
| Plasma MIP-1β (pg/mL) | 18 ± 5 | 18 ± 2 | 20 ± 4 |
| Liver triglyceride (mg/g liver) | 40.8 ± 20.4 | 48.5 ± 16.1 | 51.7 ± 14.1 |
| Liver hydroxyproline (μg/mg total protein) | 0.75 ± 0.18 | 0.63 ± 0.05 | 0.62 ± 0.09 |

Plasma MCP-1 data are shown in Figure 18C and Table 14. The CVC-high group showed a significant increase in plasma MCP-1 levels compared with the Vehicle group. There were no significant differences in plasma MCP-1 levels between the Vehicle group and the CVC-low group (Vehicle: 60 ± 4 pg/mL, CVC-low: 68 ± 16 pg/mL, CVC-high: 91 ±

14 pg/mL).

Plasma MIP-1β data are shown in Figure 18D, Table 14. There were no significant differences in plasma MIP-1β levels between the Vehicle group and either the CVC-low or the CVC-high groups (Vehicle: 18 ± 5 pg/mL, CVC-low: 18 ± 2 pg/mL, CVC-high: 20 ± 4 pg/mL). Liver Biochemistry at Week 9

Liver triglyceride content data are shown in Figure 18D and Table 14. There were no significant differences in liver triglyceride content between the Vehicle group and either the CVC-low or the CVC-high groups (Vehicle: 40.8 ± 20.4 mg/g liver, CVC-low: 48.5 ± 16.1 mg/g liver, CVC-high: 51.7 ± 14.1 mg/g liver).

Liver hydroxyproline content data are shown in Figure 18E and Table 14. The liver hydroxyproline content tended to decease in the CVC-low and the CVC-high groups compared with the Vehicle group (Vehicle: 0.75 ± 0.18 μg/mg, CVC-low: 0.63 ± 0.05 μg/mg, CVC-high: 0.62 ± 0.09 μg/mg).

Histological Analyses at Week 9

HE staining and NAFLD Activity score data are shown in Figures 19 and 20, and Table 15. Liver sections from the Vehicle group exhibited severe micro- and macrovesicular fat deposition, hepatocellular ballooning and inflammatory cell infiltration. The CVC-low and the CVC-high groups showed moderate improvements in inflammatory cell infiltration and hepatocellular ballooning, with a significant reduction in NAS compared with the Vehicle group (Vehicle: 5.3 ± 0.5, CVC-low: 4.0 ± 0.6, CVC-high: 3.7 ± 0.8). Representative photomicrographs of the HE-stained sections are shown in Figure 19.

Table 15: NAFLD Activity Score at Week 9

| | | Score | | | | | | | | | | | |
|---|---|---|---|---|---|---|---|---|---|---|---|---|---|
| | | Steatosis | | | | Lobular inflammation | | | | Hepatocyte ballooning | | | |
| Group | n | 0 | 1 | 2 | 3 | 0 | 1 | 2 | 3 | 0 | 1 | 2 | NAS (Mean±SD) |
| Vehicle | 6 | - | 4 | 2 | - | - | - | 6 | - | - | - | 6 | 5.3 ± 0.5 |
| Cenicriviroc-low | 6 | - | 6 | - | - | - | 3 | 3 | - | - | 3 | 3 | 4.0 ± 0.6 |
| Cenicriviroc-high | 6 | 1 | 5 | - | - | - | 3 | 3 | - | 1 | 2 | 3 | 3.7 ± 0.8 |

**Definition of NAS Components**

| **Item** | **Score** | **Extent** |
|---|---|---|
| **Steatosis** | 0 | <5% |
| | 1 | 5-33% |
| | 2 | >33-66% |
| | 3 | >66% |
| **Hepatocyte ballooning** | 0 | None |
| | 1 | Few balloon cells |
| | 2 | Many cells/prominent ballooning |
| **Lobular inflammation** | 0 | No foci |
| | 1 | <2 foc/200x |
| | 2 | 2-4 foci/200x |
| | 3 | >4 foci/200x |

Sirius red staining data are shown in Figures 21, 22, 23 and Table 16. Liver sections from the Vehicle group showed collagen deposition in the pericentral region of the liver lobule. Compared with the Vehicle group, collagen deposition in the pericentral region was markedly reduced in the CVC-low and the CVC-high groups. The fibrosis area (Sirius red-positive area) significantly decreased in the CVC-low and the CVC-high groups compared with the Vehicle group (Vehicle: 1.10 ± 0.31%, CVC-low: 0.66 ± 0.16%, CVC-high: 0.64 ± 0.19%). The modified fibrosis areas were also significantly reduced in the CVC-low and the CVC-high groups compared with the Vehicle group (Vehicle: 0.61 ± 0.23%, CVC-low: 0.29 ± 0.14%, CVC-high: 0.20 ±0.06%).

Table 16: Histological Analyses at Week 9

| Parameter (Mean ± SD) | Vehicle (n=6) | Cenicriviroc-low (n=6) | Cenicriviroc-high (n=6) |
|---|---|---|---|
| Sirius red-positive area (%) | 1.10 ± 0.31 | 0.66 ± 0.16 | 0.64 ± 0.19 |

(continued)

| Parameter (Mean ± SD) | Vehicle (n=6) | Cenicriviroc-low (n=6) | Cenicriviroc-high (n=6) |
|---|---|---|---|
| Modified Sirius red-positive area | 0.61 ± 0.23 | 0.29 ± 0.14 | 0.20 ± 0.06 |
| F4/80-positive area (%) | 4.99 ± 1.10 | 4.77 ± 1.02 | 4.96 ± 0.60 |
| F4/80 and CD206-positive cells (%) | 34.3 ± 4.2 | 34.7 ± 6.3 | 33.1 ± 3.0 |
| F4/80 and CD16/32-positive cells (%) | 33.5 ± 3.7 | 38.7 ± 7.6 | 41.5 ± 8.2 |
| M1/M2 ratio (%) | 99.6 ± 20.2 | 112.3 ± 17.0 | 125.1 ± 219 |
| Oil red-positive area (%) | 9.66 ± 5.02 | 6.51 ± 3.88 | 7.23 ± 3.59 |
| TUNEL-positive cells (%) | 36.0 ± 3.7 | 43.3 ± 2.9 | 39.0 ± 5.3 |

| Cenicriviroc-high | | | | | | | |
|---|---|---|---|---|---|---|---|
| Mouse ID | Photo No. | Total area (pix) | Total positive area (pix) | Positive perivascular area (pix) | Modified positive area (pix) | Modified positive area (%) | Modified positive area (%) |
| 301 | 1 | 1284424 | 9749 | 6409 | 3340 | 0.26 | 0.18 |
| | 2 | 1291238 | 3234 | 2491 | 743 | 0.06 | |
| | 3 | 1289200 | 4737 | 3491 | 1246 | 0.10 | |
| | 4 | 1252731 | 17225 | 12045 | 5180 | 0.41 | |
| | 5 | 1277575 | 6253 | 5119 | 1134 | 0.09 | |
| 302 | 1 | 1217885 | 16038 | 13242 | 2796 | 0.23 | 0.20 |
| | 2 | 1248706 | 7010 | 4876 | 2134 | 0.17 | |
| | 3 | 1253036 | 14194 | 10634 | 3560 | 0.28 | |
| | 4 | 1301898 | 4914 | 2070 | 2844 | 0.22 | |
| | 5 | 1268269 | 7439 | 6404 | 1035 | 0.08 | |
| 303 | 1 | 1285828 | 4306 | 3322 | 984 | 0.08 | 0.12 |
| | 2 | 1297994 | 2159 | 1550 | 609 | 0.05 | |
| | 3 | 1279156 | 3201 | 2025 | 1176 | 0.09 | |
| | 4 | 1285026 | 12648 | 8537 | 4111 | 0.32 | |
| | 5 | 1285009 | 4011 | 3119 | 892 | 0.07 | |
| 304 | 1 | 1294810 | 3685 | 1677 | 2008 | 0.16 | 0.26 |
| | 2 | 1274697 | 2221 | 1222 | 999 | 0.08 | |
| | 3 | 1286001 | 11356 | 8814 | 2542 | 0.20 | |
| | 4 | 1236232 | 10705 | 8252 | 2453 | 0.20 | |
| | 5 | 1217017 | 18761 | 10537 | 8224 | 0.68 | |
| 305 | 1 | 1287425 | 5774 | 2832 | 2942 | 0.23 | 0.17 |
| | 2 | 1278985 | 2638 | 1733 | 905 | 0.07 | |
| | 3 | 1272127 | 7654 | 4214 | 3440 | 0.27 | |
| | 4 | 1289371 | 5726 | 3563 | 2163 | 0.17 | |
| | 5 | 1200639 | 3654 | 2171 | 1483 | 0.12 | |

(continued)

| Cenicriviroc-high | | | | | | | |
|---|---|---|---|---|---|---|---|
| Mouse ID | Photo No. | Total area (pix) | Total positive area (pix) | Positive perivascular area (pix) | Modified positive area (pix) | Modified positive area (%) | Modified positive area (%) |
| 306 | 1 | 1236260 | 6253 | 2852 | 3401 | 0.28 | 0.27 |
| | 2 | 1270484 | 12655 | 11196 | 1459 | 0.11 | |
| | 3 | 1144610 | 20504 | 12793 | 7711 | 0.67 | |
| | 4 | 1292425 | 7266 | 4401 | 2865 | 0.22 | |
| | 5 | 1295488 | 1921 | 976 | 945 | 0.07 | |

Representative photomicrographs of Sirius red-stained sections of livers are shown in Figure 21.

F4/80 immunohistochemistry data are shown Figures 22 and 23, and Table 16. F4/80 immunostaining of liver sections form the Vehicle group demonstrated accumulation of F4/80+ cells in the liver lobule. There were no significant differences in the number and size of F4/80+ cells between the Vehicle group and either the CVC-low or the CVC-high groups, as well as in the percentage of inflammation area (F4/80-positive area) (Vehicle: 4.99 ± 1.10%, CVC-low: 4.77 ± 1.02%, CVC-high: 4.96 ± 0.60%).

Representative photomicrographs of the F4/80-immunostained sections are shown in Figure 22.

F4/80+CD206+ and F4/80+CD16/32+ immunohistochemistry data are shown in Figures 24, 25, 26, 27, 28, and Table 16). There were no significant differences in the percentages of F4/80+CD206+ cells in macrophages between the Vehicle group and either the CVC-low or the CVC-high groups (Vehicle: 34.3 ± 4.2%, CVC-low: 34.7 ± 6.3%, CVC-high: 33.1 ± 3.0%). There was no significant difference in the percentages of F4/80+CD16/32+ cells in macrophages between the Vehicle group and the CVC-low group. The percentages of F4/80+CD16/32+ cells tended to increase in the CVC-high group compared with the Vehicle (Vehicle: 33.5 ± 3.7%, CVC-low: 38.7 ± 7.6%, CVC-high: 41.5 ± 8.2%). There was no significant difference in the M1/M2 ratio between the Vehicle group and the CVC-low group. In the CVC-high group, the M1/M2 ratio tended to increase compared with the Vehicle (Vehicle: 99.6 ± 20.2%, CVC-low: 112.3 ± 17.0%, CVC-high: 125.1 ± 21.9%).

Representative photomicrographs of the F4/80 and CD206, F4/80 and CD16/32 double-immunostained sections are shown in Figures 24 and 26.

Oil red staining data are shown in Figures 29, 30, and Table 16. There were no significant differences in the fat deposition between the Vehicle group and either the CVC-low or the CVC-high groups, as well as in the percentage of fat deposition area (oil-positive area) (Vehicle: 9.66 ± 5.02%, CVC-low: 6.51 ± 3.88%, CVC-high: 7.23 ± 3.59%).

Representative photomicrographs of the oil red-stained sections are shown in Figure 29.

TUNEL staining data are shown in Figures 31, 32 and Table 16. The percentages of TUNEL-positive cells significantly increased in the CVC-low group compared with the Vehicle group. There was no significant difference in percentages of TUNEL-positive cells between the Vehicle group and the CVC-high group (Vehicle: 36.0 ± 3.7%, CVC-low: 43.3 ± 2.9%, CVC-high: 39.0 ± 5.3%).

Representative photomicrographs of TUNEL-positive cells in livers are shown in Figure 31.

Gene Expression Analysis at Week 9 data are shown in Figure 33 and Tables 17-18.

Table 17: Gene Expression Analysis at Week 9

| Parameter (Mean ± SD) | Vehicle (n=6) | Cenicriviroc-low (n-6) | Cenicriviroc-high (n=6) |
|---|---|---|---|
| TNF-α | 1.00 ± 0.24 | 1.16 ± 0.39 | 1.09 ± 0.23 |
| MCP-1 | 1.00 ± 0.31 | 1.05 ± 0.50 | 1.00 ± 0.53 |
| Collagen Type 1 | 1.00 ± 0.42 | 0.63 ± 0.10 | 0.73 ± 0.04 |
| TIMP-1 | 1.00 ± 0.46 | 0.75 ± 0.32 | 0.80 ± 0.20 |

Table 18: P values at Week 9

| P values (Student's t-test, one-tailed) | | | Body weight | Liver weight | Liver-to-body weight ratio |
|---|---|---|---|---|---|
| Vehicle | v.s. | Cenicriviroc-low | 0.3517 | 0.3265 | 0.2732 |
| | v.s. | Cenicriviroc-high | 0.4487 | 0.3993 | 0.1929 |

| P values (Student's t-test, one-tailed) | | | Whole blood glucose | Plasma ALT | Plasma MCP-1 | Plasma MIP-1β | Liver triglyceride | Liver hydroxyproline |
|---|---|---|---|---|---|---|---|---|
| Vehicle | v.s. | Cenicriviroc-low | 0.4629 | 0.0239 | 0.1329 | 0.3861 | 0.2421 | 0.0794 |
| | v.s. | Cenicriviroc-high | 0.4651 | 0.0177 | 0.0003 | 0.1587 | 0.1545 | 0.0661 |

| P values (Student's t-test, one-tailed) | | | TNF-α | MCP-1 | Collagen type 1 | TIMP-1 |
|---|---|---|---|---|---|---|
| Vehicle | v.s. | Cenicriviroc-low | 0.2054 | 0.4149 | 0.0312 | 0.1473 |
| | v.s. | Cenicriviroc-high | 0.2611 | 0.4982 | 0.0738 | 0.173 |

| P values (Student's t-test, one-tailed) | | | NAFLD Activity score | Sirius red-positive area | Modified Sirius red-positive area | F4/80 positive area | F4/80 and CD206 positive cells | F4/80 and CD 16/32 positive cells | M1/M2 ratio | Oil red-positive area | TUNEL-positive cells |
|---|---|---|---|---|---|---|---|---|---|---|---|
| Vehicle | v.s. | Cenicriviroc-low | 0.0013 | 0.0058 | 0.0067 | 0.3633 | 0.4525 | 0.0818 | 0.1333 | 0.1261 | 0.0017 |
| | v.s. | Cenicriviroc-high | 0.0009 | 0.0054 | 0.0008 | 0.481 | 0.292 | 0.0273 | 0.0311 | 0.1791 | 0.1416 |

TNFα

There were no significant differences in TNFα mRNA expression levels between the Vehicle group and either the CVC-low or the CVC-high groups (Vehicle: 1.00 ± 0.24, CVC-low: 1.16 ± 0.39, CVC-high: 1.09 ± 0.23).

MCP-1

There were no significant differences in MCP-1 mRNA between the Vehicle group and either the CVC-low or the CVC-high groups (Vehicle: 1.00 ± 0.31, CVC-low: 1.05 ± 0.50, CVC-high: 1.00 ± 0.53).

Collagen Type 1

Collagen Type 1 mRNA expression levels were significantly down-regulated in the CVC-low group compared with the Vehicle group. Collagen Type 1 mRNA expression levels tended to be down-regulated in the CVC-high group compared with the Vehicle group. (Vehicle: 1.00 ± 0.42, CVC-low: 0.63 ± 0.10, CVC-high: 0.73 ± 0.04).

TIMP-1

There were no significant differences in TIMP-1 mRNA expression levels between the Vehicle group and either the CVC-low and the CVC-high groups (Vehicle: 1.00 ± 0.46, CVC-low: 0.75 ± 0.32, CVC-high: 0.80 ± 0.20).

Part 2: study for assessing the anti-HCC effects of CVC

Body weight changes until week 18 (Figure 35)

Body weight gradually increased during the treatment period. There were no significant differences in mean body weight between the Vehicle group and either the CVC-low or the CVC-high groups during the treatment period.

Survival analysis data are shown in Figure 36. Four out of twelve mice died at day 59 (ID112), day 75 (ID113, 115) and day 84 (ID116) in the Vehicle group (The first day of administration was designed as day 0). Six out of twelve

mice died at day 62 (ID209), day 64 (ID217), day 75 (ID212), day 76 (ID213), day 84 (ID215) and day 86 (ID208) in the CVC-low group. Five out of twelve mice died at day 62 (ID317), day 65 (ID312), day 70 (ID316), day 78 (ID314) and day 85 (ID309) in the CVC-high group. There were no abnormal necropsy findings in the dead animals except for the typical hepatic lesions of NASH. There were no significant differences in survival rate between the Vehicle group and either the CVC-low or the CVC-high groups. By consigner instruction, the rest of the animals were sacrificed earlier than scheduled at 18 weeks of age (scheduled sacrificed at 20 weeks of age).

Body Weight at the Day of Sacrifice at Week 18 data are shown in Figure 37A and Table 19. The body weight tended to decrease in the CVC-high group compared with the Vehicle group. There was no significant difference in mean body weight between the Vehicle group and the CVC-low group (Vehicle: 23.0 ± 2.3 g, CVC-low: 22.9 ± 3.5 g, CVC-high: 20.8 ± 2.7 g).

Table 19: Body Weight and Liver Weight at Week 18

| Parameter (Mean ± SD) | Vehicle (n=8) | Cenicriviroc-low (n-6) | Cenicriviroc-high (n=7) |
|---|---|---|---|
| Body weight (g) | 23.0 ± 2.3 | 22.9 ± 3.5 | 20.8 ± 2.7 |
| Liver weight (mg) | 1782 ± 558 | 1837 ± 410 | 1817 ± 446 |
| Liver-to-body weight ratio (%) | 7.7 ± 2.2 | 8.3 ± 2.8 | 8.8 ± 2.3 |

Liver Weight and Liver-to-Body Weight Ratio at Week 18 data are shown in Figures 37B & C and Table 19. There were no significant differences in mean liver weight between the Vehicle group and either the CVC-low or the CVC-high groups (Vehicle: 1782 ± 558 mg, CVC-low: 1837 ± 410 mg, CVC-high: 1817 ± 446 mg). There were no significant differences in mean liver-to-body weight ratio between the Vehicle group and either the CVC-low or the CVC-high groups (Vehicle: 7.7 ± 2.2%, CVC-low: 8.3 ± 2.8%, CVC-high: 8.8 ± 2.3%).

Macroscopic Analyses of Liver at Week 18

Macroscopic appearance of livers is shown in Figures 38A-C.

Number of visible tumor nodules formed on liver surface are shown in Figure 39 and Table 20. There were no significant differences in the number of hepatic tumor nodules per individual mouse between the Vehicle group and either the CVC-low or the CVC-high groups (Vehicle: 2.4 ± 4.1, CVC-low: 1.5 ± 1.9, CVC-high: 3.6 ± 2.5).

Table 20: Macroscopic Analyses of Liver at Week 18

| Parameter (Mean ± SD) | Vehicle (n=8) | Cenicriviroc-low (n=6) | Cenicriviroc-high (n=7) |
|---|---|---|---|
| Number of visible tumor nodules | 2.4 ± 4.1 | 1.5 ± 1.9 | 3.6 ± 2.5 |
| Maximum diameter of visible tumor nodules (mm) | 4.0 ± 4.7 | 4.8 ± 5.4 | 5.3 ± 5.1 |

Maximum diameters of visible tumor nodules formed on liver surface are shown in Figure 40 and Table 20. There were no significant differences in maximum diameter of tumor between the Vehicle group and either the CVC-low or the CVC-high groups (Vehicle: 4.0 ± 4.7 mm, CVC-low: 4.8 ± 5.4 mm, CVC-high: 5.3 ± 5.1 mm).

Histological Analyses at Week 18

HE staining data are shown in Figure 41. HE staining revealed infiltration of inflammatory cells, macro- and microvesicular fat deposition, hepatocellular ballooning, altered foci and nodular lesions in the Vehicle group. Six out of eight mice in the Vehicle group exhibited HCC lesions. HCC lesions were detected in five out of six mice in the CVC-low group and six out of seven mice in the CVC-high group. No obvious differences were found between the Vehicle group and either the CVC-low or the CVC-high groups.

Representative photomicrographs of the HE-stained sections are shown in Figure 41.

GS immunohistochemistry data are shown in Figure 42. GS-positive nodules in the sections were detected in six out of eight mice in the Vehicle group, five out of six mice in the CVC-low group and seven out of seven mice in the CVC-high group, respectively.

Representative photomicrographs of the GS-stained sections are shown in Figure 42.

CD31 immunohistochemistry data are shown in Figures 43 and 44 and Table 21. The CD31-positive area

tended to decrease in the CVC-low group compared with the Vehicle group. The CD31-positive area tended to increase in the CVC-high group compared with the Vehicle group (Vehicle: 2.71 ± 1.36%, CVC-low: 1.47 ± 1.10%, CVC-high: 3.68 ± 1.37%).

Representative photomicrographs of the CD31-stained sections are shown in Figure 43.

Table 21: Histological Analyses at Week 18

| Parameter (Mean ± SD) | Vehicle (n=8) | Cenicriviroc-low (n=6) | Cenicriviroc-high (n=7) |
|---|---|---|---|
| CD31-positive area (%) | 2.71 ± 1.36 | 1.47 ± 1.10 | 3.68 ± 1.37 |

Table 22: P Values at Week 18

| P value (Student's t-test, one-tailed) | Body Weight | Liver Weight | Liver-to-body weight ratio | The number of visible tumor nodules | Maximum diameter of visible tumor nodules | CD31-positive area |
|---|---|---|---|---|---|---|
| Vehicle vs Cenicriviroc-low | 0.4758 | 0.4215 | 0.341 | 0.3191 | 0.3812 | 0.0456 |
| Vehicle vs Cenicriviroc-high | 0.0574 | 0.4476 | 0.184 | 0.2578 | 0.3096 | 0.0972 |
| | | | | | | |
| P values (Logrank-test) | Survival Curve | | | | | |
| Vehicle vs Cenicriviroc-low | 0.7513 | | | | | |
| Vehicle vs Cenicriviroc-high | 0.5701 | | | | | |

*SUMMARY AND DISCUSSION*

In the analyses at week 9, treatment with low and high dose of CVC significantly reduced fibrosis area in a dose dependent manner, demonstrating anti-fibrotic effect of CVC in the present study. Treatment with low and high dose of CVC also reduced the mRNA expression levels of Collagen Type 1 and liver hydroxyproline content, supporting its anti-fibrotic property. CVC treatment groups significantly decreased plasma ALT levels and NAS compared with the Vehicle group in a dose dependent manner. The improvement in NAS was attributable to the reduction in lobular inflammation and hepatocyte ballooning. Since hepatocyte ballooning is derived from oxidative stress-induced hepatocellular damage and is associated with disease progression of NASH [26; 27], it is strongly suggested that CVC improved NASH pathology by inhibiting hepatocyte damage and ballooning. Together, CVC have potential anti-NASH and hepatoprotective effects in this study.

As shown in humans, plasma MCP-1 levels increased by the treatment with CVC in the present study, indicating dose-dependent antagonism of CCR2 by CVC, but plasma MIP-1β levels did not show any significant changes by the treatment. To investigate the mechanism of action of CVC, we evaluated the effect of CVC on population of the macrophages. Preliminary results demonstrated that CVC showed the tendency of high M1/M2 ratio compared with Vehicle group, suggesting that CVC might inhibit the fibrogenesis by regulating the balance of macrophage subpopulation in the inflamed liver. This will be further investigated in the future.

In the analyses at week 18, the effect on NASH-derived HCC was not observed in the CVC treatment groups. In conclusion, CVC showed anti-NASH, hepatoprotective and anti-fibrotic effects in the present study.

*Example 19: Receptor-Binding Properties of CVC and Metabolites*

CVC has the unique property in vitro of being a CCR2 antagonist with 50% inhibitory concentrations (IC50) of 5.9 nmol/L. CVC dose-dependently inhibited the binding of RANTES, MIP-1α, and MIP-1β to CCR5-expressing Chinese hamster ovary (CHO) cells with an IC50 of 3.1, 2.3, and 2.3 nmol/L, respectively. CVC achieved ≥ 90% receptor occupancy for CCR5 at concentrations of 3.1 nM for CD4+ and 2.3 nM for CD8+ T-cells ex vivo in humans [4]. CVC inhibited the

binding of MCP-1 to CCR2b with an IC50 of 5.9 nmol/L. CVC achieved ~98% receptor occupancy for CCR2 on monocytes at 6 nM ex vivo in humans and reduced CCR2 expression on monocytes in the absence of MCP-1. CVC only weakly inhibited ligand binding to CCR3 and CCR4. CVC did not inhibit ligand binding to CCR1 or CCR7. CVC blocked RANTES-induced Ca2+ mobilization.

Two metabolites of CVC (M-I and M-II) were detected in animal studies (see Example 20); M-II was a major metabolite in monkeys and dogs, M-I was a minor metabolite in all species. M-I inhibited the binding of RANTES to CCR5-expressing cells with an IC50 of 6.5 nmol/L, which is approximately 2-fold the IC50 of CVC. M-II had no effect on binding of RANTES.

*Example 20: Identification of Metabolites*

After single-dose, oral administration of [14C]-CVC at 3 mg/kg to fed animals, unchanged CVC was the major component detected in the plasma of rats and dogs, the AUC0-24 ratio of CVC to total 14C being 58.9% and 47.4%, respectively [44]. In monkeys, this ratio was only 12.9%, whereas a relatively large amount of metabolite M-II was detected, the AUC0-24 ratio of M-II to total 14C being 34.3%. Especially in dogs and monkeys, the amounts of M-II were significantly greater after oral administration than after IV administration. These results suggest that CVC can be metabolized to M-II before reaching the systemic circulation. Minor metabolites, including M-I, T-1184803, and T-1169518, were also detected in the plasma of rats, dogs, and monkeys. It is postulated that the metabolite M-I is formed by oxidation of the sulfinyl moiety of CVC and that M-II is formed by the subsequent reduction of the sulfinyl moiety with cleavage of the C-S bond of the [(1-propyl-1*H*-imidazol-5-yl)methyl]sulfinyl group, followed by S-methylation.

*CLINICAL TRIALS*

*Example 21: Short-Term Efficacy Data in HIV-1 Infected Adult Subjects*

*Methods*

A Phase 2a double-blind, randomized, placebo-controlled, dose-escalating study evaluating the antiviral activity, PK, safety, and tolerability of monotherapy of CVC for 10 days in subjects with CCR5-tropic HIV-1 infection. Participants were required to be antiretroviral treatment-experienced, CCR5 antagonist-naive, with HIV-1 RNA levels of at least 5000 copies/mL and CD4+ cell counts of at least 250 cells/$mm^3$ was performed. Groups of 10 subjects were sequentially enrolled in a ratio of 4:1 subjects per cohort to receive CVC (25, 50, 75, 100, or 150 mg) or matching placebo. All subjects received once-daily doses of CVC or placebo for 10 days and were followed to Day 40.

Demographics and Other Baseline Characteristics

A total of 54 subjects were enrolled into this study. Demographics were generally similar across the dose groups. A majority of the subjects in each dose group were male (66.7% to 100%), and median age ranged from 33.5 years (placebo group) to 45.0 years (150-mg group). Most subjects were Caucasian or African American. Median BMI ranged from 22.9 kg/m2 (100-mg group) to 27.4 kg/m2 (25-mg group). Median HIV-1 RNA values ranged from 4.00 log10 copies/mL (150-mg group) to 4.60 log10 copies/mL (75-mg group). Median CD4+ cell count was highest in the 150-mg group (508.0 cells/$mm^3$) and ranged from 402.0 to 460.0 cells/$mm^3$ across the remaining groups.

Efficacy and Safety Results

CVC showed a potent effect on HIV-1 RNA levels that persisted after completion of treatment. The median nadir changes from baseline for the 25-, 50-, 75-, and 150-mg doses were -0.7, -1.6, -1.8, and -1.7 $log^{10}$ copies/mL, respectively, in CCR5-antagonist naive, treatment-experienced HIV-1 infected subjects. These results demonstrate the potent antagonistic CCR5 activity of CVC. The mean changes in HIV-1 RNA levels are shown in Figure 45.

Exploratory assessment of changes in MCP-1 (a ligand of CCR2, which is a chemokine co-receptor expressed on pro-inflammatory monocytes, also known as CCL2), hs-CRP, and IL-6 were performed and significant dose-dependent increases in MCP-1 were observed (Table 23).

On Day 10, least square mean MCP-1 levels were 56.3, 94.2, 34.4, and 334.3 pg/mL higher than at Baseline in the 25-, 50-, 75-, and 150-mg dose groups, respectively, compared to a slight decline in the placebo group. At the 50- and 150-mg doses, these results were statistically significant (p=0.024 and p<0.001, respectively). These results demonstrate the potent antagonistic CCR2 activity of CVC. CVC had no effect on hs-CRP or IL-6 levels overall in this 10-day study.

Table 23

| Parameter | Placebo | CVC 25mg | CVC 50 mg | CVC 75 mg | CVC 150 mg |
|---|---|---|---|---|---|
| Baseline, pg/mL | n-10 | n-9 | n=7 | n-7 | n=8 |
| Mean | 22.4 | 20.0 | 12.6 | 26.6 | 31.6 |
| Median | 18.5 | 16.0 | 6.0 | 8.0 | 19.5 |
| Range | 6-50 | 7-44 | 5-37 | 5-92 | 8-82 |
| Day 10, pg/mL | n=10 | n-9 | n-7 | n=7 | n=8 |
| Mean | 21.0 | 75.3 | 101.3 | 59.1 | 372.0 |
| Median | 12.5 | 39.0 | 65.0 | 43.5 | 368.0 |
| Range | 5-52 | 10-287 | 21-266 | 20-128 | 79-605 |
| Change from Baseline to Day 10 | n=10 | n=9 | n=7 | n=7 | n=8 |
| LS mean | -1.9 | +56.3 | +94.2 | +34.4 | +333.4 |
| P-valuea | -- | 0.095 | 0.024 | 0.222 | <0.001 |
| Median | 0.0 | +25.0 | +56.0 | +36.0 | +322.0 |

Abbreviation: LS, least squares
a P-values were one-sided and based on comparison of each dose of CVC with placebo without multiple comparisons adjustment.

Adverse Events

Cenicriviroc was generally well tolerated at the doses studied and no safety concerns were identified. There were no deaths, SAEs, or other significant AEs, and there were no discontinuations because of an AE. Most treatment-emergent AEs were mild or moderate in severity. Subjects who received 150 mg of CVC (ie, the highest dose studied) had more Aes compared to subjects in the other dose groups, although the severity of AEs was comparable across all dose groups. The most common (≥ 10%) treatment-emergent AEs in this study were nausea (18.5%), diarrhea (16.7%), headache (14.8%), and fatigue (11.0%).

Laboratory Safety

There were 6 subjects with ALT and/or AST elevations in the 25 mg (2 subjects), 50 mg (2 subjects), 100 mg (1 subject), and 150 mg (1 subject) dose groups, and 1 subject with an AST elevation in the placebo group during the observation period. All elevations were Grade 1, were isolated except in 2 subjects (both in the 50-mg dose group) who had more than a single elevation, and resolved without sequelae. The 2 subjects who had more than a single elevation were in the 50 mg dose group, and one of these subjects had a Grade 1 elevated AST at baseline. The AST elevations observed in subjects in the 100 mg and 150 mg dose groups during treatment (observed in 1 subject in each dose group), returned to normal values during continuation of treatment. No Grade 2-4 elevations in ALT or AST occurred during the study.

The only Grade 3 or higher laboratory abnormalities were a Grade 3 hypophosphatemia in the 25 mg dose group that was present before dosing, a Grade 4 elevated triglyceride in the 50 mg dose group in a subject who had a Grade 3 triglyceride at baseline, and Grade 3 and 4 amylase and lipase, respectively, in a subject with a prior history of pancreatitis.

Cardiovascular Safety and Physical Examinations

A Grade 3 systolic hypertension was observed in a subject in the 150-mg dose group who had a Grade 2 elevation in systolic blood pressure at baseline. There were no clinically relevant physical examination or ECG findings.

As previously described, CVC has a dual activity as a CCR5 and CCR2 antagonist. Exploratory assessment of changes in MCP-1 (the ligand of CCR2, also known as CCL2), hs-CRP, and IL-6 were performed and significant dose-dependent increases in MCP-1 were observed (see Table 24). On Day 10, least square mean MCP-1 levels were 56.3, 94.2, 34.4, and 334.3 pg/mL higher than at Baseline in the 25, 50, 75, and 150 mg dose groups, respectively, compared to a slight decline in the placebo group. At the 50 and 150 mg doses, these results were statistically significant (p=0.024 and p<0.001, respectively). These results demonstrate the potent antagonistic CCR2 activity of CVC. CVC had no effect on hs-CRP or IL-6 levels overall in this 10-day study.

Table 24: Summary of MCP-1 Levels by Cohort - Study 201

| Parameter | Placebo | CVC 25 mg | CVC 50 mg | CVC 75 mg | CVC 150 mg |
|---|---|---|---|---|---|
| Baseline | n=10 | n=9 | n=7 | n=7 | n=8 |
| Mean | 22.4 | 20.0 | 12.6 | 26.6 | 31.6 |
| Median | 18.5 | 16.0 | 6.0 | 8.0 | 19.5 |
| Range | 6-50 | 7-44 | 5-37 | 5-92 | 8-82 |
| Day 10, pg/mL | n=10 | n=9 | n=7 | n=8 | n=8 |
| Mean | 21.0 | 75.3 | 101.3 | 59.1 | 372.0 |
| Median | 12.5 | 39.0 | 65.0 | 43.5 | 368.0 |
| Range | 5-52 | 10-287 | 21-266 | 20-128 | 79-605 |
| Change from Baseline to Day 10 | n=10 | n=9 | n=7 | n=7 | n=8 |
| LS mean | -1.9 | +56.3 | +94.2 | +34.4 | +334.3 |
| P value[a] | - | 0.095 | 0.024 | 0.222 | <0.001 |
| Median | 0.0 | +25.0 | +56.0 | +36.0 | +322.0 |
| Abbreviation: LS, least squares<br>[a] P-values were one-sided and based on comparison of each dose of CVC with placebo without multiple comparisons adjustment | | | | | |

*Resistance Data*

In Study 201, drug resistance testing was performed at Baseline, Day 7, and Day 40 (or at the "Early Termination" visit, if applicable). All subjects with evaluable samples remained fully susceptible to CVC.

*Viral Tropism*

All subjects in Study 201 were tested for viral tropism to exclude that their virus was CXCR4 tropic or dual/mixed. All subjects had CCR5-tropic virus at screening (based on the enhanced sensitivity profile assay). A total of 39 subjects on CVC had evaluable samples following treatment, and one of these subjects (in the CVC 150 mg dose group) was found to have dual/mixed-tropic virus on Day 10. Further testing (at another laboratory using a different assay) revealed that this subject had mainly CXCR4-tropic virus at Baseline, therefore, this subject should not have been enrolled in the study according to the inclusion criteria. This subject did not respond to CVC treatment; the largest decrease in HIV-1 RNA of this subject was 0.13 $\log_{10}$ copies/mL below the baseline value.

*Pharmacokinetic/Pharmacodynamic Relationships*

For all doses tested in Study 201, a more than dose proportional increase in exposure was observed for "Formulation F1", which was used for all but the 100 mg dose cohort.

Drug response was characterized using the following maximum effect ($E_{max}$) model:

$$E = E_0 + \frac{(I_{max} - E_0) \cdot C^{\gamma}}{IC_{50}{}^{\gamma} + C^{\gamma}}$$

where E is effect, E0 is the baseline effect (fixed to 0), $I_{max}$ is the maximum inhibition, C denotes the PK variable ($AUC_{0-24}$, $C_{max}$, or steady-state concentration [$C_{ss}$]), $IC_{50}$ is the value of the PK variable which corresponds to 50% of the maximum inhibition and $\gamma$ is the shape parameter which describes the degree of sigmoidicity.

The Emax of CVC in the PK/PD model was -1.43 $\log_{10}$ copies/mL. Based on the Emax model, average $C_{ss}$ of CVC for the 25, 50, 75, and 150 mg doses were expected to result in 54.9%, 79.8%, 85.9%, and 95.9% of the maximum inhibitory effect of the drug. Thus, dose levels of 75 and 150 mg QD displayed potent antiviral activity, with PD effects

greater than 80% of the $E_{max}$ of CVC in HIV-1-infected subjects.

*Example 22: Long-Term Efficacy Data in HIV-1 Infected Adult Subjects*

*Efficacy Results of Study 202*

*Study Design and Objectives*

This was a randomized, double-blind, double-dummy, 48-week comparative study evaluating efficacy and safety of CVC 100 mg and CVC 200 mg compared to approved antiretroviral agent efavirenz (EFV, Sustiva®), all administered in combination with approved antiretroviral agents emtricitabine/tenofovir disoproxil fumarate (FTC/TDF), in HIV-1 infected, antiretroviral treatment-naive adult subjects with only CCR5-tropic virus. Subjects with a history of HIV-2, hepatitis B and/or C, cirrhosis of the liver or any known active or chronic active liver disease were excluded from the study.

Approximately 150 subjects were planned to be randomized (143 subjects were actuall randomized) in a 2:2:1 ratio to CVC 100 mg + placebo, CVC 200 mg + placebo or the approved antiviral agent efavirenz (EFV) + placebo, all in combination with approved antiviral agents emtricitabine/tenofovir disoproxil fumarate (FTC/TDF) provided as open label study drug in a fixed dose combination formulation (TRUVADA®). A pharmacokinetic assessment was conductedin the first 25 study subjects to confirm that adequate CVC plasma exposures were achieved at the selected doses of CVC 100 mg and CVC 200 mg prior to enrolling the remainder of the study population.

*Demographic and Baseline Characteristics*

Most subjects were male (94%) and white (62%), with a mean age of 35 years and a mean body mass index of 26.2 kg/$m^2$. In total, 32% of subjects were Black/African American. In addition, 24% of the randomized subjects were of Hispanic ethnicity.

At Baseline, the median duration of HIV-1 infection (ie, time [months] since first positive HIV-1 test to informed consent date) was 8 months, the mean HIV-1 RNA was 4.50 $log^{10}$ copies/mL. (80% of subjects had viral load < 100,000 copies/mL), and the mean CD4+ cell count was 402 cells/$mm^3$ (58% of subjects had CD4+ cell counts ≥ 350 cells/mm3).

Primary Efficacy Results

The primary efficacy endpoint was virologic response at Week 24, defined as HIV-1 RNA < 50 copies/mL using the FDA Snapshot Algorithm. The percentage of subjects with virologic success (response) was comparable among the 3 treatment arms (76% with CVC 100 mg, 73% with CVC 200 mg, and 71% with EFV). More subjects in the EFV arm prematurely discontinued the study (11 out of 28 subjects, 39%) than in the CVC 100 mg arm (17 out of 59 subjects, 29%) and the CVC 200 mg arm (15 out of 56 subjects, 27%).

The Week 48 data were consistent with the data observed at Week 24. The percentage of subjects with virologic success over time was generally comparable among the 3 treatment arms, although higher in the CVC arms compared to the EFV arm at Week 48 (68% with CVC 100 mg, 64% with CVC 200 mg, and 50% with EFV).

*Secondary and Exploratory Analyses*

*Biomarkers of Inflammation*

As an exploratory analysis, levels of inflammation biomarkers MCP-1, sCD14, high sensitivity C-reactive protein [hs-CRP], interleukin-6 [IL-6], D-dimer, and fibrinogen) were measured. Baseline values and changes from baseline at Week 24 and Week 48 of MCP-1, sCD14, hs-CRP, IL-6, D-dimer, and fibrinogen are summarized in Table 25.

Table 25

| | CVC 100 mg | | CVC 200 mg | | EFV 600 mg | |
|---|---|---|---|---|---|---|
| **Parameter** | **N** | **Mean (SE) Median (min); max)** | **N** | **Mean (SE) Median (min: max)** | **N** | **Mean (SE) Median (min; max)** |
| **MCP-1 (pg/mL)** | | | | | | |
| **Baseline value** | 55 | 128 (8.3) 110 (57; 337) | 54 | 153 (8.4) 137 (68; 393) | 28 | 139 (19.2) 122 (57; 608) |

(continued)

| Parameter | CVC 100 mg | | CVC 200 mg | | EFV 600 mg | |
|---|---|---|---|---|---|---|
| | N | Mean (SE) Median (min); max) | N | Mean (SE) Median (min: max) | N | Mean (SE) Median (min; max) |
| **MCP-1 (pg/mL)** | | | | | | |
| **Changes from baseline at Week 24** | 48 | 493 (46.2)* 429 (184; 2352) | 44 | 753 (502)* 695 (48; 1557) | 21 | -44 (24.1) -17 (-471; 77) |
| **Changes from baseline at Week 48** | 41 | 636 (63.8)* 523 (220; 2616) | 39 | 900 (90.9)* 756 (121; 3259) | 18 | 4.2(29.48) 33.6 (-437; 175) |
| **sCD14 (× $10^6$ pg/mL) (original values)** | | | | | | |
| **Baseline value** | 55 | 1.80 (0.062) 1.73 (1.07; 3.77) | 54 | 1.88 (0.069) 1.86 (1.05; 3.76) | 28 | 2.00 (0.105) 2.02 (0.93; 3.95) |
| **Changes from baseline at Week 24** | 48 | -0.19 (0.064)* -0.18 (-1.33; 0.95) | 44 | -0.23 (0.066)* -0.19 (-1.78; 0.80) | 21 | 0.23 (0.143) 0.13 (-1.60; 1.33) |
| **Changes from baseline at Week 48** | 41 | 0.10 (0.070)* 0.10 (-0.63; 1.96) | 39 | -0.04 (0.081)* -0.04 (-1.24; 1.15) | 18 | 0.64 (0.178) 0.46 (-0.50; 2.51) |
| **hs-CRP (mg/dL)** | | | | | | |
| **Baseline value** | 57 | 0.39 (0.128) 0.15 (0.01; 6.48) | 54 | 0.46 (0.149) 0.15 (0.02; 6.81) | 28 | 0.81 (0.374) 0.14 (0.02; 9.81) |
| **Changes from baseline at Week 24** | 52 | -0.16 (0.121) -0.03 (-6.07; 0.86) | 49 | -0.04 (0.138) -0.04 (-4.03; 4.72) | 21 | -0.46 (0.529) -0.01 (-9.26; 4.12) |
| **Changes from baseline at Week 48** | 44 | -0.08 (0.161) -0.01 (-6.22; 2.72) | 40 | -0.18 (0.114) -0.04 (-4.13; 0.67) | 20 | -0.71 (0.484) -0.03 (-8.93; 0.17) |
| **IL-6 (pg/mL)** | | | | | | |
| **Baseline value** | 57 | 2.51 (0.306) 1.90 (1.90; 18.00) | 52 | 3.34 (0.561) 1.90 (1.90; 21.50) | 28 | 13.81 (9.418) 1.90 (190; 264.00) |
| **Changes from baseline at Week 24** | 52 | 0.42 (0.375) 0.00 (-4.30; 12.80) | 47 | 0.81 (0.877) 0.00 (-12.10; 33.80) | 21 | -8.72 (7.518) 0.00 (-149.00; 29.70) |
| **Changes from baseline at Week 48** | 44 | 0.29 (0.362) 0.00 (-5.20; 10.90) | 38 | -0.04 (0.471) 0.00 (-12.10; 7.70) | 20 | -13.11 (10.320) 0.00 (-204.10; 5.00) |
| **D-dimer (ng/mL)** | | | | | | |
| **Baseline value** | 56 | 187 (21.1) 150 (49; 800) | 54 | 184 (19.0) 125 (49; 750) | 27 | 163 (19.0) 150 (49; 450) |
| **Changes from baseline at Week 24** | 51 | -32 (25.4) -1.0 (-550; 801) | 49 | -64 (16.2) -50 (-500; 100) | 20 | -53 (24.7) -26 (-350; 150) |
| **Changes from baseline at Week 48** | 42 | -41 (23.1) -1.0 (-650; 250) | 40 | -70 (21.3) -50 (-701; 100) | 19 | -34 (25.7) 0.0 (-300; 150) |

(continued)

| | | | | | | |
|---|---|---|---|---|---|---|
| **Fibrinogen (mg/dL)** | | | | | | |
| **Baseline value** | 55 | 236 (6.7) 229 (134; 409) | 54 | 248 (8.6) 260 (86; 429) | 28 | 258 (16.9) 245 (139; 510) |
| **Changes from baseline at Week 24** | 50 | -3 (8.0) -14 (-121; 198) | 49 | -7 (11.7) -8 (-187; 231) | 21 | -28 (19.0) -31 (-227; 174) |
| **Changes from baseline at Week 48** | **41** | 11 (10.2)# 15 (-127; 186) | 40 | -10 (8.8)# -13 (-103; 140) | 20 | -30 (15.9) -22 (-164; 109) |

N = number of subjects.
*Note:* Baseline was defined as the last non-missing assessment prior to initiation of study treatment
* Pairwise comparisons with the EFV mn, using LSMeans based on an ANCOVA model with factors for treatment, baseline, and HIV-1 RNA at Baseline, showed p-values $<0.001$.
# Differences between treatment arms, as assessed with a van Elteren test controlling for baseline HIV-1 RNA, is statistically significant (p-value: 0.048).

A dose-response was observed with CVC in increases over time of MCP-1, a ligand of CCR2, while MCP-1 remained at baseline values in the EFV arm (see Figure 46). The differences in changes from baseline of plasma MCP-1 between the EFV and CVC 100 mg and CVC 200 mg treatment arms were statistically significant ($p<0.001$) at Week 24 and Week 48 (see Table 25).

In addition, a decrease over 48 weeks of treatment was observed for sCD14 (linear mixed-model analysis of repeat sCD14 analysis, see below) in both CVC treatment arms, while an increase was observed for sCD14 in the EFV arm during the same observation period (see Figure 47). Soluble CD14 is a biomarker of monocyte activation and has been independently associated with morbidity and mortality in large, long-term cohort studies in HIV-infected patients and with worse clinical outcomes in patients with chronic viral hepatitis and patients with severe hepatic fibrosis.

The sCD14 samples were originally analyzed in 2 separate batches: Batch 1 included samples leading up to the Week 24 primary analysis and Batch 2 included Week 32 and Week 48 (end of study) samples. Results for changes in sCD14 from baseline from the 2-batch analysis are presented in Table 25. A repeat analysis of archived samples all analyzed in one batch was conducted for consistency in analysis across time points. To control for the effects of covariates, a linear mixed-model repeated-measures analysis was conducted on the changes from baseline in sCD14 (analysis dated September 2013). With the exception of changes from baseline to Week 32 in the CVC 200 mg arm, reductions in sCD14 levels observed with CVC at both doses (100 and 200 mg) over 48 weeks of treatment (LS means) were statistically significant compared to increases observed with EFV ($p<0.05$) (see Table 26 and Figure 47).

Table 26

| | **CVC 100 mg** | | **CVC 200 mg** | | **EFV 600 mg** | |
|---|---|---|---|---|---|---|
| **Parameter** | **N** | **Mean (SE) Median (min; max)** | **N** | **Mean (SE) Median (min; max)** | **N** | **Mean (SE) Median (min; max)** |
| **Original values: sCD14 (x $10^6$ pg/mL) Week 48 Final Analysis (June 2013)** | | | | | | |
| **Baseline value** | 55 | 1.80 (0.062) 1.73 (1.07; 3.77) | 54 | 1.88 (0.069) 1.86 (1.05; 3.76) | 28 | 2.00 (0.105) 2.02 (0.93; 3.95) |
| **Changes from baseline at Week 12** | 51 | -0.14 (0.054)* -0.16 (-1.14; 0.95) | 50 | -0.23 (0.070)* -0.21 (-2.39; 0.83) | 22 | 0.09 (0.160) 0.18 (-1.45; 1.61) |
| **Changes from baseline at Week 24** | 48 | -0.19 (0.064)* -0.18 (-1.33; 0.95) | 44 | -0.23 (0.066)* -0.19 (-1.78; 0.80) | 21 | 0.23 (0.143) 0.13 (-1.60; 1.33) |
| **Changes from baseline at Week 32** | 44 | 0.11 (0.072)# 0.12 (-0.68; 1.39) | 43 | -0.02 (0.084)* -0.02 (-1.53; 1.00) | 19 | 0.48 (0.186) 0.17 (-0.97; 2.18) |
| **Changes from baseline at Week 48** | 41 | 0.10 (0.070)* 0.10 (-0.63; 1.96) | 39 | -0.04 (0.081)* -0.04 (-1.24; 1.15) | 18 | 0.64 (0.178) 0.46 (-0.50; 2.51) |

Changes in other biomarkers of inflammation (hs-CRP, IL-6, D-dimer) were similar in the CVC and EFV treatment groups.

APRI and FIB-4 Scores

Furthermore, in post-hoc analyses of data from this study that enrolled subjects with no apparent liver disease according to stringent eligibility criteria (HIV-1 infection and without ALT/AST Grade ≥ 2, total bilirubin > ULN, HBV and/or HCV, active or chronic liver disease, cirrhosis or BMI > 35 kg/m2), improvements in AST-to-platelet ratio index (APRI) and noninvasive hepatic fibrosis index score combining standard biochemical values, platelets, ALT, AST, and age (FIB-4) scores were observed over time in ≥ 10% of all CVC-treated subjects (pooled data for CVC 100 mg and 200 mg) (Figure 48). In the EFV arm, 5% of subjects at Week 24 and 6% of subjects at Week 48 had a decrease in APRI score by one category from baseline; no subject treated with EFV decreased in FIB-4 score by one category where all subjects had scores < 1.45 at baseline.

As mentioned above, in this study, CVC also had a significant effect on sCD14, an important marker of monocyte activation. In the same post-hoc analyses described above, statistically significant correlations were observed between changes in FIB-4 score and sCD14 levels in CVC-treated subjects at Week 24, and between changes in APRI and FIB-4 scores and sCD14 levels at Week 48. The Week 48 results are shown in Figure 49 and Figure 50.

Safety Results

Extent of Exposure

The mean duration of intake of study medication (CVC or EFV) was longer in the CVC arms than in the EFV treatment arm (41.2 and 40.9 weeks with CVC 100 mg and 200 mg, respectively, versus 36.2 weeks with EFV), which was driven by the higher discontinuation rate in the EFV arm.

Summary of All Adverse Events

In total, 51 subjects (88%), 48 subjects (84%), and 27 subjects (96%) had at least 1 AE in, respectively, the CVC 100 mg, CVC 200 mg, and the EFV arm. The most frequently reported AEs (preferred terms in ≥ 10% of subjects in any of the 3 treatment arms) were nausea, upper respiratory tract infection, diarrhea, headache, rash events, fatigue, dizziness, nasopharyngitis, abnormal dreams, insomnia, lymphadenopathy, depression, and syphilis (Table 27). From these most frequently reported AEs, headache, fatigue, and upper respiratory tract infection were reported more frequently in the CVC arms than in the EFV arm; and dizziness, abnormal dreams, insomnia, lymphadenopathy, depression, and syphilis were reported more frequently in the EFV arm than in the CVC arms.

Table 27

| Preferred Term, n (%) | CVC 100 mg (N = 58) | CVC 200 mg (N = 57) | All CVC (N = 115) | EFV (N = 28) |
|---|---|---|---|---|
| ***Mean (SE) duration of Intake study medication (weeks)[a]*** | ***41.2 (1.89)*** | ***40.9 (1.88)*** | ***41.1 (1.33)*** | ***36.2 (3.64)*** |
| ***Any AE*** | ***51 (88%)*** | ***49 (84%)*** | ***99 (86%)*** | ***17 (96%)*** |
| Nausea | 10 (17%) | 8 (14%) | 18 (16%) | 6 (21%) |
| Upper respiratory tract infection | 9 (16%) | 9 (16%) | 18 (16%) | 2 (7%) |
| Diarrhoea | 7 (12%) | 10 (18%) | 17 (15%) | 3 (11%) |
| Headache | 9 (16%) | 7 (12%) | 16 (14%) | 0 |
| Rash[b] | 7 (12%) | 7 (12%) | 14 (12%) | 5 (18%) |
| Fatigue | 6 (10%) | 8 (14%) | 14 (12%) | 1 (4%) |
| Dizziness | 5 (9%) | 6 (11%) | 11 (10%) | 8 (29%) |

(continued)

| Preferred Term, n (%) | CVC 100 mg (N = 58) | CVC 200 mg (N = 57) | All CVC (N = 115) | EFV (N = 28) |
|---|---|---|---|---|
| Nasopharyngitis | 2 (3%) | 8 (14%) | 10 (9%) | 1 (4%) |
| Abnormal dreams | 6 (10%) | 3 (5%) | 9 (8%) | 6 (21%) |
| Insomnia | 0 | 7 (12%) | 7 (6%) | 4 (14%) |
| Lymphadenopathy | 3 (5%) | 4 (7%) | 7 (6%) | 4 (14%) |
| Depression | 2 (3%) | 1 (2%) | 3 (3%) | 3 (11%) |
| Syphilis | 1 (2%) | 0 | 1 (1%) | 3 (11%) |

N = number of subjects; n = number of observations.
*Note-*. Adverse events were coded using MedDRA version 13.1. Only adverse events with an onset date from the date of the first dose of study drag to within 30 days of discontinuing study drag are reported. For subjects who experienced the same coded event more than once, only the event with the highest severity is presented.
[a] Note that exposure is based on ITT population.
[b] Included rash, rash maculopapular, rash pruritic, rash generalized, and rash papular.

Most AEs were mild or moderate (Grade 1 or Grade 2). Grade 3 or 4 AEs are summarized in Table 29. The percentage of subjects who experienced a Grade ≥ 3 AE was lower in the CVC arms (total of 4%) than in the EFV arm (15%). One subject (Subject 06007) in the EFV arm had a Grade 4 AE of suicidal ideation, which was considered serious. No Grade 4 AEs were reported in CVC-treated subjects. None of the Grade ≥ 3 AEs (preferred terms) were reported in more than 1 subject. Table 28 provides an overview of deaths, SAEs, AEs, AEs by severity, AEs related to study medication, and AE leading to discontinuation.

Table 28

| Number of Subjects with AE, n (%) | CVC 100 mg (N = 58) | CVC 200 mg (N = 57) | All CVC (N = 115) | EFV (N = 28) |
|---|---|---|---|---|
| ***Mean (SE) duration of intake study medication (weeks)***[a] | ***41.2 (1.89)*** | ***40.9 (1.88)*** | ***41.1 (1.33)*** | ***36.2 (3.64)*** |
| Subjects with ≥ 1 AE | 51 (88%) | 48 (84%) | 99 (86%) | 27 (96%) |
| Subjects with AEs, by worst grade severity: | | | | |
| - Grade 1 | 31 (53%) | 19 (33%) | 50 (43%) | 10 (36%) |
| - Grade 2 | 18 (31%) | 26 (46%) | 44 (38%) | 13 (46%) |
| - Grade 3 | 2 (3%) | 3 (5%) | 5 (4%) | 3 (11%) |
| - Grade 4 | 0 | 0 | 0 | 1 (4%) |
| Subjects with AEs related to study medication[b] | 29 (50%) | 25 (44%) | 54 (47%) | 20 (71%) |
| Subjects with AEs leading to discontinuation of study medication | 0 | 1 (2%) | 1 (1%) | 6 (21%) |
| Subjects with serious AEs | 1 (2%) | 1 (2%) | 2 (2%) | 1 (4%) |
| Deaths | 0 | 0 | 0 | 0 |

N = number of subjects; n = number of observations.
*Note*: Adverse events were coded using MedDRA version 13.1. Only adverse events with an onset date from the date of the first dose of study drug to within 30 days of discontinuing study drug are reported. For subjects who experienced the same coded event more than once, only the event with the highest severity is presented.
a Note that exposure is based on ITT population.
b AEs considered to be at least possibly related to study medication (ie. CVC, EFV, or FTC/TDF) according to the investigator.

Table 29

| System Organ Classification Preferred Term, n (%) | CVC 100 mg (N = 58) | CVC 200 mg (N = 57) | AH CVC (N = 115) | EFV (N = 28) |
|---|---|---|---|---|
| ***Mean (SE) duration** of **intake study medication (weeks)**[a]* | ***41.2 (1.89)*** | ***40.9 (1.88)*** | ***41.1 (1.33)*** | ***36.2 (3.64)*** |
| ***Any Grade 3 AE*** | ***2** (3%)[b]* | ***3 (5%)**[c]* | ***5(4%)*** | ***3 (11%)**[d]* |
| ***Any Grade 4 AE*** | *0* | ***0*** | ***0*** | ***1 (4%)*** |
| **Investigations** | 0 | **1 (2%)** | **1 (1%)** | **1 (4%)** |
| Blood creatine phosphokinase increased | 0 | 1 (2%) | 1 (1%) | 0 |
| Weight decreased | 0 | 0 | 0 | 1 (4%) |
| **Psychiatric disorders** | **1 (2%)** | 0 | **1 (1%)** | **1 (4%)** |
| Depression | 0 | 0 | 0 | 1 (4%) |
| Stress | 1 (2%) | 0 | 1(1%) | 0 |
| Suicidal ideation | 0 | 0 | 0 | 1 (4%)[e] |
| **Cardiac disorders** | **1 (2%)** | **0** | **1 (1%)** | **0** |
| **Palpitations** | 1 (2%) | 0 | 1 (1%) | 0 |
| **Ear and labyrinth disorders** | **0** | **0** | **0** | **1 (4%)** |
| Tinnitus | 0 | 0 | 0 | 1 (4%) |
| **Eye disorders** | **0** | **1 (2%)** | **1 (1%)** | **0** |
| Blindness unilateral | 0 | 1 (2%) | 1 (1%) | 0 |
| **Gastrointestinal disorders** | **1 (2%)** | **0** | **1 (1%)** | **0** |
| Abdominal pain | 1 (2%) | 0 | 1 (1%) | 0 |
| **General disorders and administration site conditions** | **0** | **1 (2%)** | **1 (1%)** | **0** |
| Pyrexia | 0 | 1 (2%) | 1 (1%) | 0 |
| **Infections and infestations** | **0** | **1** (2%) | **1 (1%)** | **0** |
| Corneal infection | 0 | 1 (2%) | 1 (1%) | 0 |
| **Skin and subcutaneous tissue disorders** | **0** | **0** | **0** | **1 (4%)** |
| Dermatitis allergic | 0 | 0 | 0 | 1 (4%) |

N = number of subjects: n = number of observations.

*Note*: Adverse events were coded using MedDRA version 13.1. Only adverse events with an onset date from the date of the first dose of study drug to within 30 days of discontinuing study drug are reported. For subjects who experienced the same coded event more than once, only the event with the highest severity is presented.

a Note that exposure is based on ITT population.

b Subjects 10004 and 54001 in CVC 100 mg arm

c Subjects 06009, 42001 and 45005 in CVC 200 mg arm

d Subjects 06005, 06007, 46003 and 48001 in EFV arm

e Note: This (suicidal ideation in EFV arm) was a Grade 4 event; all other events were Grade 3.

Serious adverse events are summarized in Table 30.

Table 30 - Number of Subjects (%) With Serious Adverse Events through Week 48 - Safety Population

| System Organ Classification Preferred Term, n (%) | CVC 100 mg (N = 58) | CVC 200 mg (N = 57) | All CVC (N = 115) | EFV (N = 28) |
|---|---|---|---|---|
| ***Mean (SE) duration of intake study medication (weeks)**[a]* | ***41.2 (1.89)*** | ***40.9 (1.88)*** | ***41.1 (1.33)*** | ***36.2 (3.64)*** |
| ***Any SAE*** | **1 (2%)** | **1 (2%)** | ***2 (2%)*** | ***1 (4%)*** |

(continued)

| System Organ Classification Preferred Term, n (%) | CVC 100 mg (N = 58) | CVC 200 mg (N = 57) | All CVC (N = 115) | EFV (N = 28) |
|---|---|---|---|---|
| **Infections and infestations** | **1 (2%)** | **1 (2%)** | **2 (2%)** | **0** |
| Corneal infection | 0 | 1 (2%) | 1 (1%) | 0 |
| Gastroenteritis | **1 (2%)** | 0 | **1 (1%)** | 0 |
| **Eye disorders** | 0 | **1 (2%)** | **1 (1%)** | **0** |
| Blindness unilateral | 0 | 1 (2%) | 1 (1%) | 0 |
| **Psychiatric disorders** | 0 | 0 | 0 | **1 (4%)** |
| Depression | 0 | 0 | 0 | 1 (4%) |
| Suicidal ideation | 0 | 0 | 0 | 1 (4%) |

N = number of subjects; n = number of observations.
*Note*: Adverse events were coded using MedDRA version 13.1. Only adverse events with an onset date from the date of the first dose of study drug to within 30 days of discontinuing study drug are reported. For subjects who experienced the same coded event more than once, only the event with the highest severity is presented.
a Note that exposure is based on ITT population.

Adverse Events Leading to Discontinuation

AEs leading to discontinuation of study medication are summarized in Table 31. In total, Aes leading to discontinuation of study medication occurred in 1 subject (2%) in the CVC 200 mg arm and in 6 subjects (21%) in the EFV arm. AEs (preferred terms) leading to discontinuation of study medication that were reported in more than 1 subject were insomnia and dizziness, reported in 3 and 2 subjects, respectively, in the EFV arm, and depression, that was reported in 1 subject in the CVC 200 mg arm and in 1 subject in the EFV arm (insomnia, dizziness, and depression are all common AEs for EFV).

Table 31 - Number of Subjects (%) With Adverse Events Leading to Discontinuation of Study Medication through Week 48 - Safety Population

| System Organ Classification Preferred Term, n (%) | CVC 100 mg (N = 58) | CVC 200 mg (N = 57) | All CVC (N = 115 | EFV (N = 28) |
|---|---|---|---|---|
| ***Mean (SE) duration of intake study medication (weeks)[a]*** | ***41.2 (1.89)*** | ***40.9 (1.88)*** | ***41.1 (1.33)*** | ***36.2 (3.64)*** |
| ***Any AE tending to discontinuation of study drug*** | ***0*** | ***1 (2%)[b]*** | ***1 (1%)*** | ***6 (21%)[c]*** |
| **Nervous system disorders** | **0** | **0** | **0** | **4 (14%)** |
| Dizziness | 0 | 0 | 0 | 2 (7%) |
| Disturbance in attention | 0 | 0 | 0 | 1 (4%) |
| Hypoaesthesia | 0 | 0 | 0 | 1 (4%) |
| **Psychiatric disorders** | 0 | **1(2%)** | **1 (1%)** | **3 (11%)** |
| Insomnia | 0 | 0 | 0 | 3 (11%) |
| Depression | 0 | 1 (2%) | 1 (1%) | 1 (4%) |
| Abnormal dreams | 0 | 0 | 0 | 1 (4%) |
| Aggression | 0 | 1 (2%) | 1 (1%) | 0 |
| Anxiety | 0 | 0 | 0 | 1 (4%) |
| Tachyphrenia | 0 | 0 | 0 | 1 (4%) |
| Thinking abnormal | 0 | 1 (2%) | 1 (1%) | 0 |
| **Skin and subcutaneous tissue disorders** | **0** | **0** | **0** | 2 (7%) |
| Dermatitis allergic | 0 | 0 | 0 | 1 (4%) |
| Rash | 0 | 0 | 0 | 1 (4%) |
| **Ear and labyrinth disorders** | **0** | **0** | **0** | **1 (4%)** |

(continued)

| System Organ Classification Preferred Term, n (%) | CVC 100 mg (N = 58) | CVC 200 mg (N = 57) | All CVC (N = 115 | EFV (N = 28) |
|---|---|---|---|---|
| Tinnitus | 0 | 0 | 0 | 1 (4%) |
| **Eye disorders** | **0** | **0** | **0** | **1 (4%)** |
| Photophobia | 0 | 0 | o | 1 (4%) |
| **Gastrointestinal disorders** | 0 | 0 | 0 | **1 (4%)** |
| Nausea | 0 | 0 | 0 | 1 (4%) |
| **General disorders and administration site conditions** | **0** | **1 (2%)** | **1 (1%)** | **0** |
| Malaise | 0 | 1 (2%) | 1 (1%) | 0 |
| **Musculoskeletal and connective tissue disorders** | **0** | **0** | **0** | **1 (4%)** |
| Musculoskeletal disconfort | 0 | 0 | 0 | 1 ( 4%) |

N = number of subjects; n = number of observations.
*Note:* Adverse events were coded using MedDRA version 13.1. Only adverse events with an onset date from the date of the first dose of study drug to within 30 days of discontinuing study drug are reported. For subjects who experienced the same coded event more than once, only the event with the highest severity is presented.
a Note that exposure is based on ITT population.
b Subject 06001 in CVC 200 mg arm.
c Subject 02016. 16031. 20004. 26001. 46003 and 48001 in EFV arm.

An overview of the number of subjects with graded treatment-emergent laboratory abnormalities is given in Table 32.

Table 32

| Laboratory Parameter Worst Grade, n (%)[a] | CVC 100 mg (N = 58) | CVC 200 mg (N = 57) | All CVC (N = 115) | EFV (N = 28) |
|---|---|---|---|---|
| **Any graded (Grade 1-4) abnormality** | **51 (88%)** | **55 (96%)** | **106 (92%)** | **25 (89%)** |
| Grade 1 | 21 (36%) | 16 (28%) | 37 (32%) | 13 (46%) |
| Grade 2 | 23 (40%) | 27 (47%) | 50 (43%) | 8 (29%) |
| Grade 3 | 4 (7%) | 9 (16%) | 13 (11%) | 3 (11%) |
| Grade 4 | 3 (5%) | 3 (5%) | 6 (5%) | 1 (4%) |

N = number of subjects: n = number of observations.
a Percentages are based on the number of subjects with a given laboratory assessment.

Grade 3 or 4 (worst toxicity grades) treatment-emergent laboratory abnormalities are summarized in Table 33. Except for abnormalities in CPK that were observed more frequently in the CVC 200 mg arm, there were no differences in percentages of subjects with Grade 3 or Grade 4 laboratory abnormalities between the treatment arms.

Table 33 - Treatment-Emergent Grade 3 or Grade 4 (Worst Grade; DAIDS) Laboratory Parameters through Week 48 - Safety Population

| Laboratory Parameter Worst Grade, n (%)[a] | CVC 100 mg (N = 58) | CVC 200 mg (N = 57) | All CVC (N = 115) | EFV (N = 28) |
|---|---|---|---|---|
| *Any Grade 3 or Grade 4 abnormality* | *7 (12%)* | *12 (21%)* | *19 (17%)* | *4 (14%)* |
| *Any Grade 3 abnormality* | *4 (7%)* | *9 (16%)* | *13 (11%)* | *3 (11%)* |
| *Any Grade 4 abnormality* | *3 (5%)* | *3 (5%)* | *6 (5%)* | *1 (4%)* |
| CHEMISTRY | | | | |

(continued)

| Laboratory Parameter Worst Grade, n (%)[a] | CVC 100 mg (N = 58) | CVC 200 mg (N = 57) | All CVC (N = 115) | EFV (N = 28) |
|---|---|---|---|---|
| **Aspartate aminotransferase (AST) increased (Grade 3 or 4)** | **1 (2%)** | **0** | **1 (<1%)** | 0 |
| Grade 3 | **1 (2%)** | 0 | 1 (<1%) | 0 |
| Grade 4 | 0 | 0 | 0 | 0 |
| **Creatine pbosphokinase (CPK) increased (Grade 3 or 4)** | **3 (5%)** | **9 (16%)** | **12 (10%)** | **2 (7%)** |
| Grade 3 | 2 (3%) | 6 (11%) | 8 (7%) | 2 (7%) |
| Grade 4 | 1 (2%) | 3 (5%) | 4 (3%) | 0 |
| **Phosphate decreased (Grade 3 or 4)** | **2 (3%)** | **2 (4%)** | **4 (3%)** | **1 (4%)** |
| Grade 3 | 2 (3%) | 2 (4%) | 4 (3%) | 1 (4%) |
| Grade 4 | 0 | 0 | 0 | 0 |
| **COAGULATION** | | | | |
| **Prothrombin time / international normalized ratio increased (Grade 3 or 4)** | **1 (2%)** | 0 | **1 (< 1%)** | 0 |
| Grade 3 | 0 | 0 | 0 | 0 |
| Grade 4 | 1 (2%) | 0 | 1 (< 1%) | 0 |
| **HEMATOLOGY** | | | | |
| **Fibrinogen decreased (Grade 3 or 4)** | 0 | **2 (4%)** | **2 (2%)** | 0 |
| Grade 3 | 0 | 2 (4%) | 2 (2%) | 0 |
| Grade 4 | 0 | 0 | 0 | 0 |
| **Hemoglobin decreased (Grade 3 or 4)** | **1 (2%)** | 0 | **1 (< 1%)** | 0 |
| Grade 3 | 0 | 0 | 0 | 0 |
| Grade 4 | 1 (2%) | 0 | 1 (< 1%) | 0 |
| **Neutrophils decreased (Grade 3 or 4)** | **2 (3%)** | 0 | **2 (2%)** | **1 (4%)** |
| Grade 3 | 2 (3%) | 0 | 2 (2%) | 0 |
| Grade 4 | 0 | 0 | 0 | (4%) |

N = number of subjects; n = number of observations.
a Percentages are based on the number of subjects with a given laboratory assessment.

Grade 3 or 4 increases in creatine phosphokinase (CPK) were observed more frequently in the CVC 200 mg arm than in the other two treatment arms. From the 12 subjects with Grade 3 or 4 increases in CPK in the CVC arms (3 subjects with CVC 100 mg and 9 subjects with CVC 200 mg), 11 subjects had CPK elevations (8 subjects had Grade 3 and 3 subject had Grade 4 elevations) that were observed at one single time point (note: 1 of these 11 subjects [Subject 48015] had isolated Grade 3 CPK elevations at Week 8 and Week 36). The 12th subject (Subject 42001) had 2 consecutive CPK elevations (Grade 3 followed by Grade 4) that returned to normal values while continuing treatment at a subsequent visit. None of the CPK elevations were associated with clinical symptoms; no subjects discontinued due to CPK elevations and there were no differences in AEs related to musculoskeletal disorders between the CVC and EFV arms.

Changes from baseline in CPK are shown in Figure 51. No obvious trend was observed for CPK in the actual values over time or in the changes from baseline in any of the treatment arms.

The number of subjects with graded treatment-emergent laboratory abnormalities in selected liver parameters of interest is shown in Table 34. No Grade 4 ALT or AST elevations were observed. Except for one Grade 3 AST elevation, all ALT and AST elevations were Grade 1 or Grade 2. The Grade 3 AST elevation in 1 subject (48015 in the CVC 100-mg arm) was observed at one single time point and was asymptomatic; the subject did not discontinue study medication due to the Grade 3 AST elevation and did not report an AE related to the AST elevation. In addition, this subject with a Grade 3 AST elevation did not have any graded bilirubin elevations, but had one single Grade 3 CPK increase at the same study visit as the Grade 3 AST elevation. All abnormalities in bilirubin were Grade 1 or Grade 2. The majority of ALT, AST, and bilirubin elevations were transient, returned to baseline values at subsequent visits upon continued

treatment, were not associated with any clinical symptoms, and did not result in discontinuation

Table 34 -Treatment-Emergent Worst Grade (DAIDS) Laboratory Abnormalities in Selected Liver Parameters through Week 48 - Safety Population

| **Laboratory Parameter Worst Grade, n (%)[a]** | **CVC 100 mg (N = 58)** | **CVC 200 mg (N = 57)** | **All CVC (N = 115)** | **EFV (N = 28)** |
|---|---|---|---|---|
| **Alanine aminotransferase (ALT)** | **7 (12%)** | **8 (14%)** | **15 (13%)** | **2 (7%)** |
| Grade 1 | 4 (7%) | 6 (11%) | 10 (9%) | **2 (7%)** |
| Grade 2 | **3 (5%)** | **2 (4%)** | **5 (4%)** | 0 |
| Grade 3 | 0 | 0 | 0 | 0 |
| Grade 4 | 0 | 0 | 0 | 0 |
| **Aspartate aminotransferase (AST)** | **11 (19%)** | **10 (18%)** | **21 (18%)** | **3 (11%)** |
| Grade 1 | 8 (14%) | 6 (11%) | 14 (12%) | 3 (11%) |
| Grade 2 | 2 (3%) | 4 (7%) | 6 (5%) | 0 |
| Grade 3 | 1 (2%) | 0 | 1 (< 1%) | 0 |
| Grade 4 | 0 | 0 | 0 | 0 |
| **Bilirubin** | **4 (7%)** | **3 (5%)** | **7 (6%)** | **1 (4%)** |
| Grade 1 | 1 (2%) | 2 (4%) | 3 (3%) | 1 (4%) |
| Grade 2 | 3 (5%) | 1 (2%) | 4 (3%) | 0 |
| Grade 3 | 0 | 0 | 0 | 0 |
| Grade 4 | 0 | 0 | 0 | 0 |

N = number of subjects; n = number of observations.
**a** Percentages are based an the number of subjects with a given laboratory assessment.

Exploratory analyses were performed at Weeks 24 and 48 to evaluate CVC exposures in subjects with treatment-emergent laboratory adverse events. Of specific interest were CPK elevations, given the increased incidence of CPK abnormalities in the CVC 200-mg arm, and liver parameters of interest (AST, ALT, and bilirubin). Both exposure parameters (Cavg and Cmin) were considered reasonable to explore possible relationships with laboratory abnormalities; however $C_{avg}$ was considered most relevant given that it is reflective of overall CVC exposure.

Despite the possible signal for a dose-response relationship for CPK elevations by virtue of the differences among the study treatment arms, none of these extensive exploratory analyses were able to uncover any exposure-response relationship. Logistic regression analysis outputs evaluating Ln exposures versus probability of CPK severity Grade > 2 did not identify an association between CVC exposure and CPK elevation. There are no trends in either increasing frequency or severity of CPK elevation versus CVC exposure.

Similar analyses were conducted for ALT, AST, and bilirubin elevations, and also did not reveal any apparent relationship between CVC exposure and liver-related laboratory abnormalities (Figures 52 - Figure 55).

Metabolic Parameters

The number of subjects with graded treatment-emergent fasting laboratory abnormalities at fasting visits is shown in Table 35. All abnormalities in total cholesterol, LDL cholesterol, triglycerides, or glucose were Grade 1 or Grade 2. The percentage of subjects with abnormalities in total cholesterol and LDL cholesterol was lower in the CVC arms than in the EFV arm, which is in line with the decreases over time in cholesterol during CVC treatment (Figure 56).

Table 35- Treatment-Emergent Worst Grade (DAIDS) Fasting Laboratory Abnormalities at Fasting Visits through Week 48

| **Laboratory Parameter Worst Grade, n (%)[a]** | **CVC 100 mg (N = 58)** | **CVC 200 mg (N = 57)** | **All CVC (N = 115)** | **EFV (N = 28)** |
|---|---|---|---|---|
| ***Any graded (Grade 1-4) fasting laboratory abnormality*** | ***4 (7%)*** | ***12 (21%)*** | ***16 (14%)*** | ***9 (32%)*** |
| ***Grade 1*** | ***3 (5%)*** | ***6 (11%)*** | ***9 (9%)*** | ***6 (21%)*** |

(continued)

| Laboratory Parameter Worst Grade, n (%)[a] | CVC 100 mg (N = 58) | CVC 200 mg (N = 57) | All CVC (N = 115) | EFV (N = 28) |
|---|---|---|---|---|
| ***Grade 2*** | ***1 (2%)*** | ***6 (11%)*** | ***7 (6%)*** | ***3 (11%)*** |
| ***Grade 3*** | ***0*** | ***0*** | ***0*** | ***0*** |
| ***Grade 4*** | ***0*** | ***0*** | ***0*** | ***0*** |
| **Total cholesterol** | **3 (5%)** | **5 (9%)** | **8 (7%)** | **9 (32%)** |
| Grade 1 | 3 (5%) | 2 (4%) | 5 (4%) | 6 (21%) |
| Grade 2 | 0 | 3 (5%) | 3 (3%) | 3 (11%) |
| Grade 3 | 0 | 0 | 0 | 0 |
| Grade 4 | 0 | 0 | 0 | 0 |
| **Glucose (serum, high)** | **0** | **5 (9%)** | **5 (4%)** | **2 (7%)** |
| Grade 1 | 0 | 3 (5%) | 3 (3%) | 2 (7%) |
| Grade 2 | 0 | 2 (4%) | 2 (2%) | 0 |
| Grade 3 | 0 | 0 | 0 | 0 |
| Grade 4 | 0 | 0 | 0 | 0 |
| **LDL cholesterol** | **2 (3%)** | **4 (7%)** | **6 (5%)** | **6 (21%)** |
| Grade 1 | 1 (2%) | 2 (4%) | 3 (3%) | 3 (11%) |
| Grade 2 | 1 (2%) | 2 (4%) | 3 (3%) | 3 (11%) |
| Grade 3 | 0 | 0 | 0 | 0 |
| Grade 4 | 0 | 0 | 0 | 0 |
| **Triglycerides** | **0** | **1 (2%)** | **1 (< 1%)** | **0** |
| Grade 1 | 0 | 0 | 0 | 0 |
| Grade 2 | 0 | 1 (2%) | 1 (< 1%) | 0 |
| Grade 3 | 0 | 0 | 0 | 0 |
| Grade 4 | 0 | 0 | 0 | 0 |

N = number of subjects; n = number of observations.
Note: Grade 4 abnormalities in (LDL) cholesterol and grade 1 abnormalities in triglycerides are not available with the DAIDS grading scale.
a Percentage are based on the number of subjects with a given laboratory assessment.

Mean baseline values and changes from baseline in HbA1c, HOMA-IR, fasting LDL, fasting HDL, fasting total cholesterol, fasting total cholesterol/HDL ratio, and fasting triglycerides are shown in Table 36. Mean change from baseline in metabolic parameters are shown in Figure 56. A decrease was observed during CVC treatment (both CVC 100 mg and 200 mg) in total cholesterol, mainly due to decreases in LDL cholesterol (see Table 36). In contrast, increases were observed during EFV treatment in LDL cholesterol as well as HDL cholesterol. Small and comparable decreases in fasting total cholesterol/HDL ratio were observed in all treatment arms. No notable changes over time were observed in glucose, insulin, HOMA-IR, HbA1c, and triglycerides (see Table 36).

Table 36 - Mean Changes From Baseline in Fasting Metabolic Laboratory Parameters through Week 48 - Safety Population

| Laboratory Parameter | N | CVC 100 mg | N | CVC 200 mg | N | All CVC | N | EFV |
|---|---|---|---|---|---|---|---|---|
| **HbA1c, %Hb** | | | | | | | | |
| Baseline, mean (SE) | 54 | 5.41 (0.074) | 55 | 5.39 (0.049) | 109 | 5.40 (0.044) | 28 | 5.43 (0.080) |
| Mean change (SE) from baseline at: | | | | | | | | |
| - Week 4 | 51 | 0.01 (0.050) | 50 | -0.08 (0.038) | 101 | -0.03 (0.031) | 24 | -0.01 (0.067) |

(continued)

| Laboratory Parameter | N | CVC 100 mg | N | CVC 200 mg | N | All CVC | N | EFV |
|---|---|---|---|---|---|---|---|---|
| **HbA1c, %Hb** | | | | | | | | |
| - Week 12 | 51 | -0.04 (0.048) | 47 | -0.08 (0.043) | 98 | -0.06 (0.032) | 23 | -0.07 (0.065) |
| - Week 24 | 48 | 0.06 (0.053) | 48 | 0.06 (0.046) | 96 | 0.06 (0.035) | 21 | -0.01 (0.093) |
| - Week 48 | 40 | 0.09 (0.065) | 40 | 0.10 (0.055) | 80 | 0.10 (0.042) | 19 | -0.08 (0.108) |
| **HOMA-IR** | | | | | | | | |
| Baseline. mean (SE) | 52 | 5.08 (1.154) | 50 | 4.25 (0.698) | 102 | 4.67 (0.678) | 28 | 4.45 (0.830) |
| Mean change (SE) from baseline at: | | | | | | | | |
| - Week 4 | 46 | 0.11 (1.678) | 45 | -0.71 (0.792) | 91 | -0.30 (0.930) | 22 | 0.30 (0.738) |
| - Week 12 | 48 | -0.59 (1.113) | 44 | -0.53 (0.842) | 92 | -0.56 (0.703) | 21 | 0.06 (1.296) |
| - Week 24 | 44 | -1.42 (1.355) | 39 | 0.15 (0.458) | 83 | -0.68 (0.751) | 21 | -1.27 (0.851) |
| - Week 48 | 40 | -156 (1.411) | 34 | 0.17 (0.771) | 74 | -0.76 (0.842) | 17 | -012 (1.313) |
| **Fasting LDL, mg/dL** | | | | | | | | |
| Baseline, mean (SE) | 58 | 94.72 (3.344) | 54 | 98.30 (3.964) | 112 | 96.45 (2.573) | 28 | 91.00 (4.976) |
| Mean change (SE) from baseline at: | | | | | | | | |
| - Week 4 | 51 | -10.90 (2.721) | 48 | -8.46 (2.533) | 99 | -9.72 (1.858) | 21 | 8.62 (4.018) |
| - Week 12 | 51 | -11.20 (2.894) | 49 | -11.69 (2.685) | 100 | -11.44 (1.967) | 22 | 7.59 (5.120) |
| - Week 24 | 47 | -10.21 (3.111) | 43 | -6.93 (3.464) | 90 | -8.64 (2.313) | 20 | 13.40 (6.210) |
| - Week 48 | 43 | -11.16 (3.340) | 35 | -5.20 (3.442) | 78 | -8.49 (2.412) | 16 | 11.19 (8.464) |
| **Fasting HDL, mg/dL** | | | | | | | | |
| Baseline, mean (SE) | 58 | 48.21 (1.901) | 56 | 43.75 (1.602) | 114 | 46.02 (1.259) | 28 | 42.00 (1.909) |
| Mean change (SE) from baseline at: | | | | | | | | |
| - Week 4 | 51 | -3.98 (1.065) | 50 | -1.84 (0.966) | 101 | -2.92 (0.724) | 21 | 5.90 (1.790) |
| - Week 12 | 51 | -2.96 (1.663) | 51 | -1.22 (0.989) | 102 | -2.09 (0.966) | 22 | 9.45 (1.965) |
| - Week 24 | 48 | -2.15 (1.539) | 45 | -0.71 (1.269) | 93 | -1.45 (1.001) | 20 | 12.75 (2.100) |
| - Week 48 | 43 | -1.63 (1.908) | 38 | -0.21 (1.391) | 81 | -0.96 (1.200) | 16 | 11.94 (2.128) |
| **Fasting total cholesterol, mg/dL** | | | | | | | | |
| Baseline, mean (SE) | 58 | 166 (4.6) | 56 | 168 (4.2) | 114 | 167 (3.1) | 28 | 155 (5.2) |

(continued)

| | | | | | | | | |
|---|---|---|---|---|---|---|---|---|
| **Fasting total cholesterol, mg/dL** | | | | | | | | |
| Mean change (SE) from baseline at: | | | | | | | | |
| - Week 4 | 51 | -16 (3.6) | 50 | -12 (2.9) | 101 | -14 (2.3) | 21 | 19 (4.2) |
| - Week 12 | 51 | -17 (3.8) | 51 | -16 (3.1) | 102 | -17 (2.5) | 22 | 18 (5.5) |
| - Week 24 | 48 | -14 (3.9) | 45 | -12 (4.0) | 93 | -13 (2.8) | 20 | 24 (6.2) |
| - Week 48 | 43 | -14 (3.9) | 38 | -9 (3.9) | 81 | -12 (2.8) | 16 | 26 (9.4) |
| **Fasting total cholesterol/HDL ratio** | | | | | | | | |
| Baseline, mean (SE) | 58 | 3.70 (0.175) | 56 | 4.13 (0.196) | 114 | 3.91 (0.132) | 28 | 3.92 (0.233) |
| Mean change (SE) from baseline at: | | | | | | | | |
| - Week 4 | 51 | -0.11 (0.146) | 50 | -0.22 (0.100) | 101 | -0.17 (0.089) | 21 | -0.07 (0.141) |
| - Week 12 | 51 | -0.06 (0.264) | 51 | -0.36 (0.105) | 102 | -0.21 (0.142) | 22 | -0.41 (0.166) |
| - Week 24 | 48 | -0.19 (0.165) | 45 | -0.41 (0.128) | 93 | -0.30 (0.105) | 20 | -0.47 (0.154) |
| - Week 48 | 43 | 0.02 (0.290) | 38 | -0.31 (0.118) | 81 | -0.14 (0.164) | 16 | -0.35 (0.221) |
| **Fasting triglycerides, mg/dL** | | | | | | | | |
| Baseline, mean (SE) | 58 | 118 (10.8) | 56 | 133 (11.9) | 114 | 125 (8.0) | 28 | 111 (12.7) |
| Mean change (SE) from baseline at: | | | | | | | | |
| - Week 4 | 51 | -8 (8.3) | 50 | -2 (7.0) | 101 | -5 (5.4) | 21 | 23 (14.4) |
| - Week 12 | 51 | -16 (9.0) | 51 | -13 (7.2) | 102 | -15 (5.9) | 22 | 3 (14.4) |
| - Week 24 | 48 | -8 (10.0) | 45 | -23 (9.4) | 93 | -15 (6.9) | 20 | -10 (12.9) |
| - Week 48 | 43 | -9 (8.2) | 38 | -16 (11.7) | 81 | -12 (7.0) | 16 | 14 (19.4) |

HbA1c = hemoglobin type $A_{tc}$; HDL = high-density lipoprotein: HOMA-IR = Homeostasis Model of Assessment - Insulin Resistance: LDL = low-density lipoprotein; N = number of subjects.
Note: Baseline was defined as the last non-missing assessment prior to initiation of study treatment.

No notable changes from baseline were observed in any of the treatment arms in waist-to-hip ratio at Week 24 and Week 48.

Cardiovascular Safety

Worst treatment-emergent ECG abnormalities during the treatment period are summarized in Table 37. The proportion of subjects with QTc increase of > 30-60 msec was lower for the CVC arms compared to the EFV arm. Only 1 subject had QTc increase of > 60 msec in the CVC 100 mg arm. No subjects had prolonged or pathologically prolonged QTc.

No clinically relevant changes in ECG parameters were observed during the treatment period in any of the treatment arms.

Table 37- Worst Treatment-Emergent ECG Abnormalities During the Treatment Period through Week 48

| Parameter n (%) | CVC 100 mg (N = 58) | CVC 200 mg (N = 57) | ALL CVC (N = 115) | EFV (N = 28) |
|---|---|---|---|---|
| **QTcF interval[a]** | | | | |
| Borderline | 1 (2%) | 1 (2%) | 2 (2%) | 0 |

(continued)

| Parameter n (%) | CVC 100 mg (N = 58) | CVC 200 mg (N = 57) | ALL CVC (N = 115) | EFV (N = 28) |
|---|---|---|---|---|
| **QTcF interval[a]** | | | | |
| Prolonged | 0 | 0 | 0 | 0 |
| Pathologically prolonged | 0 | 0 | 0 | 0 |
| Increase by >30-60 ms | 4 (8%) | 3 (6%) | 7 (7%) | 4 (14%) |
| Increase by >60 ms | 1 (2%) | 0 | 1 (1%) | 0 |
| **QTcB interval[b]** | | | | |
| Borderline | 4 (8%) | 2 (4%) | 6 (6%) | 3 (11%) |
| Prolonged | 0 | 0 | 0 | 0 |
| Pathologically prolonged | 0 | 0 | 0 | 0 |
| Increase by >30-60 ms | 6 (12%) | 3 (6%) | 9 (9%) | 4 (14%) |
| Increase by >60 ms | 1 (2%) | 0 | 1 (1%) | 0 |
| **QRS[c]** | | | | |
| Abnormally low | 0 | 0 | 0 | 0 |
| Abnormally high | 1 (2%)[d] | 0 | 1 (1%)[d] | 0 |
| **PR[e]** | | | | |
| Abnormally high | 2 (3%) | 1 (2%) | 3 (3%) | 1 (4%) |
| **HR[f]** | | | | |
| Abnormally low | 0 | 0 | 0 | 0 |
| Abnormally high | 0 | 0 | 0 | 0 |

N = number of subjects; n = number of observations.
Note: Percentages are based on the number of subjects with a given ECG parameter.
a QTcF: normal < 450 ms ≤ borderline ≤ 480 ms < prolonged ≤ 500 ms < pathological.
b QTcB: normal < 450 ms ≤ borderline ≤ 480 ms < prolonged ≤ 500 ms < pathological.
c Abnormal QRS: abnormally low ≤ 50 ms < normal < 120 ms ≤ abnormally high.
d This subject (Subject 06004) had a QRS value of 120 ms at Week 24. and had a screening value of 125 ms and **a** baseline value < 120 ms (ie. 111 ms; see Listing 16.2.8.7).
e Abnormal PR. normal < 210 ms ≤ abnormally high
f Abnormal HR: abnormally low ≤ 50 bpm < normal < 120 bpm ≤ abnormally high.

Vital Signs

No clinically relevant mean changes were observed for any of the vital signs parameters (systolic and diastolic blood pressure, heart rate) in any of the treatment arms. Data Observations Regarding MCP-1 from the Phase 2 Trials MCP-1 protein and gene expression were shown to be up-regulated in hepatic tissue of patients with chronic liver disease with different degrees of liver damage and fibrosis. As previously shown, compensatory increases in plasma MCP-1 levels were observed following CVC treatment in nonclinical and clinical studies, suggesting potent CCR2 blockade. Although the impact of prolonged compensatory increases in MCP-1 levels secondary to CCR2 antagonism by CVC in man is currently unknown, available data do not suggest an increased risk of hepatobiliary disorders or abnormalities in liver parameters based on 48 weeks of safety data.

No indication of inflammation was seen in clinical pathology parameters or in any tissue, including the liver, by microscopic evaluation at the high dose of 1000 mg/kg/day where plasma MCP-1 levels in the chronic (3- and 9-month) monkey toxicity studies were ~ 5-fold over controls.

In fact, anti-fibrotic effects of CVC at the 100 mg/kg/day dose observed in the mouse model of NASH were seen in conjunction with significantly increased plasma MCP-1 levels. In addition, improvements in APRI and FIB-4 fibrosis index scores observed in CVC-treated subjects over 48 weeks occurred despite significant and sustained MCP-1 elevations. Also in this study, CVC was generally well tolerated in 115 subjects treated with CVC 100 mg and 200 mg for up to 48 weeks.

Changes in NAS and in hepatic fibrosis stage (NASH CRN system and Ishak) at Year 1 and 2 will be assessed

by histology. Changes in morphometric quantitative assessment of collagen on liver biopsy will also be assessed. Correlations between efficacy endpoints and MCP-1 plasma levels will be evaluated to determine whether or not prolonged MCP-1 increases observed with CVC treatment pose a potential risk in subjects with liver fibrosis due to NASH.

*Example 23: Biomarkers of Inflammation and Immune Function*

A dose-response was observed with CVC in increases over time of MCP-1, the ligand of CCR2, which is a chemokine receptor found on monocytes, while MCP-1 remained at baseline values in the EFV arm. The differences in changes from baseline of plasma MCP-1 between the EFV and CVC 100 mg and CVC 200 mg treatment arms were statistically significant ($p<0.001$) at Week 24 and Week 48, suggesting potent and dose-dependent CCR2 blockade by CVC. Furthermore, a decrease over the first 24 weeks was observed for sCD14, a biomarker of monocyte activation and an independent predictor of mortality in HIV infection, in both CVC treatment arms, while an increase was observed for sCD14 in the EFV arm during the same observation period. Between Weeks 24 and 48, sCD14 levels returned to baseline values in CVC-treated subjects whereas they continued to rise in EFV-treated subjects. The differences in changes from baseline between the CVC arms and the EFV arm were statistically significant ($p<0.001$) at Week 24 and Week 48 and also at Week 48 in a repeat analysis. These results indicate a potential effect of CVC on decreasing monocyte activation.

No meaningful differences between the treatment arms were observed in changes from Baseline in other inflammation biomarkers (hs-CRP, fibrinogen, IL-6, and D-dimer) and biomarkers of immune function (total CD38+ expression and total HLA DR+ expression on CD4+ T cells or on CD8+ T cells).

*Example 24: Measurement of biomarkers associated with bacterial translocation*

Decreases in sCD14 levels in CVC-treated subjects could also equate to decreases in bacterial translocation, a phenomenon commonly observed in patients with HIV infection [15] as well those with NASH [16-18], alcoholic liver disease [17,19], HIV/HCV co-infection [20] and cirrhosis [21]. Bacterial translocation comes as result of breakdown of enterocyte tight junctions (TJs), which compromises intestinal mucosal barrier, a phenomenon commonly described as the leaky gut. Decrease in gut integrity has been associated with immune deficiency and/or significant changes in gut microbiota, also referred to as dysbiosis and bacterial overgrowth. Subsequent translocation of microbial products, such as lipopolysaccharide (LPS) and 16S ribosomal DNA (16S rDNA), contributes to immune activation. LPS, a component of the cell wall of gram-negative bacteria, binds membrane or soluble CD14 (sCD14; produced upon LPS activation of monocytes) and the myeloid differentiation-2 (MD-2)-TLR4 complex [14].

Lipopolysaccharide is the most potent inducer of inflammatory cytokines, particularly TNF-$\alpha$, in monocytes and macrophages. High plasma sCD14 levels predicted disease progression in HBV and HCV infection independent of other markers of hepatic inflammation, fibrosis, and disease progression [20]. Exposure to bacterial products of intestinal origin, most notably endotoxin, including LPS, leads to liver inflammation, hepatocyte injury and hepatic fibrosis [22]. Activation of Kupffer cells via TLR4-dependent mechanism and subsequent activation hepatic stellate cells are both potent drivers of fibrogenesis [19].

This hypothesis will be evaluated by testing biomarkers of bacterial translocation in archived samples from Study 652-2-202, upcoming hepatic impairment Study 652-1-121 and liver fibrosis PoC Study 652-2-203. These biomarkers will include LPS, LPS-binding protein (LBP), sCD14, intestinal fatty acid binding protein (I-FABP).

*Example 25 - Conclusions Based on CVC Clinical Phase 1 Data and Phase 2*

Data in HIV-infected Subjects CVC has been evaluated in 14 single-dose and multiple-dose bioavailability studies and DDI studies in healthy volunteer subjects (n=390), as well as two Phase 2 studies in HIV-infected subjects (n=159), including 115 subjects treated with CVC for up to 48 weeks.

The most frequent adverse events observed in the Phase 1 studies in which CVC alone was given were consistent with conditions commonly reported in Phase 1 study units. Overall, the pattern of adverse events suggests that CVC was generally well tolerated in these Phase 1 studies evaluating single doses of CVC up to 800 mg and at multiple daily doses of up to 200 mg for 10 days. The frequency and magnitude of transaminase elevations observed across these studies was consistent with the pattern described for Phase 1 studies in scientific literature. CVC has been evaluated in a Phase 2a 10-day CVC monotherapy study at 25- to 150-mg doses (n=44) and in a Phase 2b 48-week efficacy and safety study at doses of CVC 100 mg and CVC 200 mg (n=115). In both studies and at all doses CVC presented a favorable adverse event profile. Based on 48-week data from the Phase 2b study, CVC was not associated with an increased risk of hepatobiliary disorders or transaminase elevations. Decreases in total and LDL cholesterol were observed in CVC-treated subjects in this study. No clinically relevant changes in ECG parameters or changes for any vital sign parameters were observed during the 48-week treatment period. No apparent dose or exposure relationship

for adverse events, laboratory abnormalities (including CPK, ALT, AST and bilirubin elevations) or dose-limiting toxicities were observed.

Based on data from the Phase 1 program and Phase 2 data from studies of HIV-infected subjects, we paln to evaluate CVC 150 mg taken once daily in the treatment of subjects with hepatic fibrosis due to NASH over a period of 2 years in Study 652-2-203 (with the primary study endpoint at Year 1). The study's crossover design will evaluate the safety and efficacy of 2 continuous years of CVC treatment as well as 1 year of placebo treatment followed by 1 year of CVC treatment. Standard assessments of the impact of CVC treatment on hepatic fibrosis due to NASH will be conducted based on histological data from liver biopsies and other measures of histologic improvement. Safety and tolerability will be assessed, and careful monitoring for signs of hepatic or other organ toxicities will be conducted, including periodic data review by an independent data monitoring committee. The study is expected to elucidate the anti-inflammatory and anti-fibrotic activity of CVC and its impact on hepatic fibrosis due to NASH, and to provide additional data for the assessment of the safety and tolerability of CVC 150 mg.

*Example 26 - Study of CVC to Evaluate Hepatic Histological Improvement in NASH*

Based on the nonclinical and clinical data indicating that CVC has anti-inflammatory and anti-fibrotic activity and is generally well tolerated, Tobira plans to investigate CVC in a Phase 2 study in subjects with hepatic fibrosis due to NASH. This Phase 2 study will evaluate the efficacy of CVC for the treatment of NASH in adult subjects with liver fibrosis who are at risk of disease progression due to the presence of at least one contributing factor, including type 2 diabetes mellitus (T2DM), high body mass index (BMI) (> 25 kg/m2) with at least 1 criterion of the metabolic syndrome (MS) as defined by the National Cholesterol Education Program (NCEP), bridging fibrosis, and/or definite NASH (NAS ≥ 5).

The Phase 2 study is designed to evaluate the potential of CVC to treat this serious condition and to address the significant unmet medical need of patients with hepatic fibrosis due to NASH. This study is a randomized, double-blind, placebo-controlled study designed to evaluate the efficacy and safety of CVC 150 mg when compared to placebo in subjects with hepatic fibrosis due to NASH. The study population consists of subjects with liver fibrosis (NASH Clinical Research Network [CRN] Stage 1-3) due to NASH (NAS ≥ 4) at risk of disease progression.

A dose of CVC 150 mg (DP7 formulation) will be evaluated for the treatment of NASH in subjects with liver fibrosis in Study 652-2-203 based on the following considerations:

CVC is expected to provide both anti-inflammatory and anti-fibrotic activity, primarily due to its antagonism of CCR2 and CCR5 co-receptors and the resulting effects on recruitment, migration and infiltration of pro-inflammatory monocytes to the site of liver injury. Therefore, a primary consideration for selecting a dose for use in this study is to ensure that CVC plasma exposures are sufficient to provide near maximal antagonism of CCR2 and CCR5.

CCR2 and CCR5 antagonism by CVC have been evaluated in in vitro and ex vivo studies and in 2 clinical studies of CVC in the treatment of HIV-1 infection (Phase 2a Study 652-2-201 and Phase 2b Study 652-2-202). In each case, potent and concentration-dependent antagonism of CCR2 and CCR5 was observed. Clinical evidence of CCR2 and CCR5 antagonism was established by measuring changes from baseline in plasma MCP-1 (a ligand of CCR2) concentrations and changes in plasma HIV-RNA (CCR5 co-receptor required for HIV entry), respectively, in these 2 Phase 2 Studies.

In Study 652-2-202, doses of CVC 100 mg and CVC 200 mg (DP6 formulation) were evaluated in 115 HIV-1 infected subjects for up to 48 weeks (mean [SE] duration of CVC intake: 41.1 [1.33] weeks) and were found to be effective and well tolerated in the treatment of HIV infection. Based on exposure-response analyses, which showed that increasing CVC plasma concentrations correlated with an improved virologic outcome, CVC 200 mg was considered an appropriate dose for further evaluation of CVC as an antiviral agent for the treatment of HIV infection in Phase 3 studies.

CVC plasma exposures, however, appear to be higher in non-HIV infected healthy volunteer subjects as compared to HIV-infected subjects when CVC is administered under the same dosing conditions (Studies 652-1-111, 652-1-110, 652-2-202). A dose of CVC 150 mg will be evaluated for the treatment of NASH in subjects with liver fibrosis in Study 652 2 203. Based on the referenced available data, this dose is considered to be in a therapeutically relevant range and is expected to provide exposures in subjects with NASH and liver fibrosis that are comparable to those of CVC 200 mg, which was evaluated in Study 652-2-202 and found to result in potent CCR2 and CCR5 antagonism.

A total of 250 subjects (125 subjects per treatment arm) are planned, and total study treatment duration will be 2 years. The study population will include subjects with NASH (NAS ≥ 4) and liver fibrosis (Stages 1 to 3 [NASH CRN system]) who are at increased risk of disease progression due to the presence of ≥ 1 contributing factor(s):

Documented evidence of type 2 diabetes mellitus
High BMI (> 25 kg/m2) with at least 1 of the following criteria of the metabolic syndrome, as defined by the NCEP:
Central obesity: waist circumference ≥ 102 cm or 40 inches (male), ≥ 88 cm or 35 inches (female)
Dyslipidemia: TG ≥ 1.7 mmol/L (150 mg/dL)
Dyslipidemia: HDL-cholesterol < 40 mg/dL (male), < 50 mg/dL (female)

Blood pressure ≥ 130/85 mmHg (or treated for hypertension)
Fasting plasma glucose ≥ 6.1 mmol/L (110 mg/dL); or
Bridging fibrosis (NASH CRN Stage 3) and/or definite NASH (NAS ≥ 5).

There will be 2 treatment periods. Treatment Period 1 will consist of double-blind randomized treatment (CVC 150 mg or matching placebo) for 1 year. Subjects and investigators will remain blinded to treatment assignment during Period 1. During Treatment Period 2, subjects originally randomized to CVC 150 mg will continue to receive that treatment for an additional year, and subjects originally randomized to placebo will cross over from placebo to CVC 150 mg.

Subjects will receive study drug, once daily (QD), for 2 years. The study will comprise 2 treatment periods: Treatment Period 1 (first year) and Treatment Period 2 (second year). Eligible subjects will be assigned to receive CVC (n=126) or matching placebo (n=126) during the first year of treatment (Treatment Period 1). For Treatment Period 2, half of the placebo-treated subjects (randomized at Baseline) will cross-over to CVC and the other half will remain on placebo for the second year of treatment. At Baseline (Day 1), following Screening evaluations, eligible subjects will be assigned to the treatment arms using permuted block randomization stratified by NAS at Screening (4 or ≥ 5) and fibrosis stage (≤ 2 or > 2). Eligible subjects will be randomized in a 2:1:1 ratio to one of the following 3 treatment arms:

Table 38

| Arm | N | Treatment Period 1 | Treatment Period 2 |
|---|---|---|---|
| A | 126 | CVC 150 mg, QD | CVC 150 mg, QD |
| B | 63 | Matching placebo, QD | CVC 150 mg, QD |
| C | 63 | Matching placebo, QD | Matching placebo, QD |

CVC and matching placebo will be administered as double-blinded study drug. Study drug (CVC/matching placebo) should be taken every morning with food.

The primary endpoint (Year 1) biopsy must be performed within 1 month prior to the end of Treatment Period 1 before starting Treatment Period 2. The final (Year 2) biopsy must be performed within 1 month prior to end of treatment with study drug.

Enrollment will be initiated at a limited number of sites until up to 20 subjects have been randomized and treated and safety data have been reviewed by the Data Monitoring Committee (DMC). The first DMC review will occur within 3 months of the first subject enrolled or, when up to 20 subjects have been randomized and at least 10 subjects have been treated for 1 month, whichever comes first. Subsequent enrollment of the remainder of study subjects will occur once the DMC has evaluated the safety data for these first 10-20 subjects and has determined that the study may continue.

During Treatment Period 1, all subjects will undergo safety assessments at Weeks 2 and 4 of Month 1. In addition, the first 20 subjects will undergo safety assessments at Weeks 1 and 3 of Month 1. All subjects will undergo study visit assessments every 2 weeks during Month 2, monthly visits during Months 3 to 6, and at Months 8, 10, and 12. During Treatment Period 2, subjects will undergo monthly visits during Months 13 to 15, and at Months 18, 21 and 24.

Key Assessments

During the study:

Liver biopsies will be taken at Screening, at the primary endpoint (Year 1: within 1 month prior to end of Treatment Period 1 and before starting Treatment Period 2), and at Year 2 (within 1 month prior to end of treatment)

Pro-inflammatory cytokines, biomarkers of inflammation, biomarkers of hepatocyte apoptosis, biomarkers of bacterial translocation, fasting metabolic parameters, renal parameters, and eGFR will be measured at Baseline and Months 3, 6, 12, 15, 18, and 24.

At sites where available, assessment of non invasive liver imaging (e.g., ultrasound transient elastography [TE], two-dimensional magnetic resonance elastography [MRE], acoustic radiation force impulse [ARFI]) will be performed at Baseline and at Months 6, 12, 18, and 24.

Pharmacokinetic samples for CVC will be collected at Baseline (pre-dose sample just before starting treatment), at Months 0.5, 3 and 15 (pre-dose and at least 1 hour post-dose), and at Months 6, 12, 18 and 24 (pre-dose).

Weight, waist circumference, hip circumference, arm circumference, and tricep skinfold will be performed at Baseline and at Months 3, 6, 12, 15, 18, and 24. Height will be performed at Screening and Month 12.

Physical examinations and laboratory analyses will be performed at each visit. ECGs will be performed at Baseline and at Months 3, 6, 12, 15, 18, and 24.

Adverse events and concomitant medications will be assessed at each visit.

The informed consent and patient education materials about NASH, liver fibrosis, and liver biopsy procedures will be reviewed at the screening visit.

Study drug diaries will be provided to each subject at the same time that study drug is dispensed. The diary will be reviewed at all On-treatment Visits and the Early Discontinuation Visit.

Subjects will return to the clinic 1 month after receiving their last treatment for an end of study follow-up evaluation.

The primary efficacy objective of the study will be to evaluate hepatic histological improvement in nonalcoholic fatty liver disease (NAFLD) activity score (NAS) at Year 1 relative to screening biopsy, defined by a minimum 2-point improvement in NAS with at least a 1-point improvement in both the lobular inflammation and ballooning categories and no concurrent worsening of fibrosis stage (with worsening defined as progression to bridging fibrosis or cirrhosis).

Secondary efficacy objectives include evaluation of the resolution of NASH with no concurrent worsening of fibrosis stage (worsening defined as progression to bridging fibrosis or cirrhosis) at Year 2; the resolution of NASH with no concurrent worsening of fibrosis stage (worsening defined as progression to bridging fibrosis or cirrhosis) at Year 1; the safety and tolerability of CVC over 1 and 2 years of treatment of NASH in adult subjects with liver fibrosis; characterization of the plasma PK of CVC in a population PK analysis; evaluation of the hepatic histological improvement in NAS at Year 2, defined by a minimum 2-point improvement in NAS with at least a 1-point improvement in more than 1 category and with no concurrent worsening of fibrosis stage (worsening defined as progression to bridging fibrosis or cirrhosis); evaluation of the efficacy of CVC versus placebo in adult subjects with liver fibrosis as determined by change in morphometric quantitative collagen on liver biopsy at Years 1 and 2; evaluation of the change in histologic fibrosis stage (nonalcoholic steatohepatitis clinical research network [NASH CRN] system and Ishak) at Years 1 and 2; evaluation of the change from in hepatic tissue fibrogenic protein (alpha-smooth muscle actin [a-SMA]) at Years 1 and 2; evaluation of the change from Baseline in noninvasive hepatic fibrosis markers (APRI, FIB-4, hyaluronic acid, FibroTest (FibroSure), NAFLD fibrosis score [NFS] and enhanced liver fibrosis test [ELF]) at Months 3, 6, 12, 15, 18, and 24; evaluation of the change from Baseline in biomarkers of hepatocyte apoptosis at Years 1 and 2; evaluation of the change from Baseline in liver parameters and fasting metabolic parameters at Months 3, 6, 12, 15, 18, and 24; evaluation of the change from Baseline in weight, BMI, waist circumference, waist-hip ratio, arm circumference, and tricep skinfold at Months 3, 6, 12, 15, 18, and 24.

Tertiary Objectives include evaluation of the change from Baseline in non-invasive liver imaging method (e.g., ultrasound transient elastography [TE], 2-dimensional magnetic resonance elastography [MRE], acoustic radiation force impulse [ARFI]) at Months 6, 12, 18, and 24 (at sites where available); the change from Baseline in pro-inflammatory cytokines and biomarkers of inflammation at Months 3, 6, 12, 15, 18, and 24; the change from Baseline in estimated glomerular filtration rate (eGFR) and in renal parameters at Months 3, 6, 12, 15, 18, and 24; and the change from Baseline in biomarkers associated with bacterial translocation at Months 3, 6, 12, 15, 18, and 24.

The detailed description herein describes various aspects and embodiments of the invention, however, unless otherwise specified, none of those are intended to be limiting. Indeed, a person of skill in the art, having read this disclosure, will envision variations, alterations, and adjustments that can be made without departing from the scope and spirit of the invention, all of which should be considered to be part of the invention unless otherwise specified. Applicants thus envision that the invention described herein will be limited only by the appended claims.

**References:**

1. Saiman Y, Friedman SL. The role of chemokines in acute liver injury. 2012;3:213.
2. Zimmermann HW, Tacke F. Modification of chemokine pathways and immune cell infiltration as a novel therapeutic approach in liver inflammation and fibrosis. Inflamm Allergy Drug Targets. 2011;10:509-536.
3. Seki E, De Minicis S, Gwak GY, Kluwe J, Inokuchi S, Bursill CA, Llovet JM, Brenner DA, Schwabe RF. CCR1 and CCR5 promote hepatic fibrosis in mice. J Clin Invest. 2009;119:1858-1870.
4. Seki E, de Minicis S, Inokuchi S, Taura K, Miyai K, van Rooijen N, Schwabe RF, Brenner DA. CCR2 promotes hepatic fibrosis in mice. Hepatology. 2009;50:185-197.
5. Miura K, Yang L, van Rooijen N, Ohnishi H, Seki E. Hepatic recruitment of macrophages promotes nonalcoholic steatohepatitis through CCR2. Am J Physiol Gastrointest Liver Physiol. 2012;302:G1310-1331.
6. Mitchell C, Couton D, Couty JP, Anson M, Crain AM, Bizet V, Rénia L, Pol S, Mallet V, Gilgenkrantz H. Dual role of CCR2 in the constitution and the resolution of liver fibrosis in mice. Am J Pathol. 2009;174:1766-1775.
7. Xia Y, Entman ML, Wang Y. CCR2 regulates the uptake of bone marrow-derived fibroblasts in renal fibrosis. PLoS ONE 2013; 8(10): e77493. doi:10.1371/journal.pone.0077493
8. Karlmark KR, Wasmuth HE, Trautwein C, Tacke F. Chemokine-directed immune cell infiltration in acute and chronic liver disease. Expert Review Gastroenterology Hepatology. 2008; 2: 233-242

9. Vielhauer V, Anders H-J, Mack M, Cihak J, Strutz F, Stangassinger M, Luckow B, Gröne H-J, Schlöndorff D. Obstructive nephropathy in the mouse: Progressive fibrosis correlates with tubulointerstitial chemokine expression and accumulation of CC chemokine receptor 2- and 5-positive leukocytes. J Am Soc Nephrol 2001;12:1173-1187.
10. Segerer S, Mack M, Regele H, Kerjaschki D, Schlöndorff D. Expression of the C-C chemokine receptor 5 in human kidney disease. Kidney Int 1999; 56:52-64.
11. Wai C-T, Greenson J, Fontana R, Kalbfleisch J, Marrero J, Conjeevaram H, Lok A. A simple noninvasive index can predict both significant fibrosis and cirrhosis in patients with chronic hepatitis C. Hepatology 2003;38:518-526.
12. Vallet-Pichard A, Mallet V, Nalpas B, Verkarre V, Nalpas A, Dhalluin-Venier V, Fontaine H, Pol S. FIB-4: an inexpensive and accurate marker of fibrosis in HCV infection. Comparison with liver biopsy and FibroTest. Hepatology 2007;46:32-36.
13. Sandler NG, Wand H, Roque A, et al. Plasma levels of soluble CD14 independently predict mortality in HIV infection. JID 2011;203:780-790.
14. Brenchley J, Price DA, Schacker TW, Asher TE, Silvestri G, Rao S, Kazzaz Z, Bomstein E, Lambotte O, Altmann D, Blazar BR, Rodriguez B, Teixeira-Johnson L, Landay A, Martin JN, Hecht FM, Picker LJ, Lederman MM, Deeks SG, Douek DC. Microbial translocation is a cause of systemic immune activation in chronic HIV infection.Nature Medicine 2006;12:1365-1371.
15. Vajro P, Paolella G, Fasano A. Microbiota and Gut-Liver Axis: Their Influences on Obesity and Obesity-Related Liver Disease JPGN 2013;56: 461-468.
16. Roh YS, Seki E. Toll-like receptors in alcoholic liver disease, non-alcoholic steatohepatitis and carcinogenesis Journal of Gastroenterology and Hepatology 2013; 28: 38-42.
17. Ilan Y. Leaky gut and the liver: A role for bacterial translocation in nonalcoholic steatohepatitis. World J Gastroenterol 2012 June 7; 18: 2609-2618.
18. Petrasek J, Mandrekar P, Szabo G. Toll-Like Receptors in the Pathogenesis of Alcoholic Liver Disease. Gastroenterology Research and Practice 2010, Article ID 710381, 12 pages. doi:10.1155/2010/710381.
19. Sandler N, Koh C, Roque A, Eccleston J, Siegel R, Demino M, Kleiner D, Deeks S, Liang T-J, Heller T, Douek D. Host Response to Translocated Microbial Products Predicts Outcomes of Patients With HBV or HCV Infection. Gastroenterology 2011;141:1220-1230. doi:10.1053/j.gastro.2011.06.063.
20. Wiest R, Lawson M, Geuking M. Pathological bacterial translocation in liver cirrhosis. J Hepatology 2014; 60(1):197-209.
21. Shanab AA, Scully P, Crosbie O, Buckley M, O'Mahony L, Shanahan F, Gazareen S, Murphy E, Quigley EM. Small intestinal bacterial overgrowth in nonalcoholic steatohepatitis: association with toll-like receptor 4 expression and plasma levels of interleukin 8. Dig Dis Sci 2011; 56: 1524-1534
22. Henao-Mejia J, Elinav E, Jin C, Hao L, Mehal WZ, Strowig T, Thaiss CA, Kau AL, Eisenbarth SC, Jurczak MJ, Camporez J-P, Shulman GI, Gordon JI, Hoffman HM; Flavell RA. Inflammasome-mediated dysbiosis regulates progression of NAFLD and obesity. Nature 2012; 492: 179-185. doi:10.1038/naturel0809.
23. Klibanov, Olga M.; Williams, Shannon H.; Iler, Cameron A (2010). "Cenicriviroc, an orally active CCR5 antagonist for the potential treatment of HIV infection". Current Opinion in Investigational Drugs 11 (8): 940-950.
**24.** Kleiner DE. et al., Hepatology, Design and validation of a histological scoring system for nonalcoholic fatty liver disease; 2005;41:1313-1321.
25. Fujii H et al. J. Atheroscler. Thromb. 2009;16:893.
26. Rangwala F et al. J. Pathol. 2011; 224:401.
27. Seki et al.CCR1 and CCR5 promote hepatic fibrosis in mice. J. Clin. Invest. 2009; 119(7):1858-1870.
28. Xu et al. Liver fibrosis: mechanisms of immune-mediated liver injury. Cellular & Mol. Immunol.; 2012; 9:296-301.
29. Karlmark et al, Expert Rev. Gastroenterol. Hepatol. 2(2), 233-242 (2008).
30. Mitchell C, et al. Dual role of CCR2 in the constitution and the resolution of liver fibrosis in mice. Am J Pathol. 2009;174:1766-1775.
31. Berres M-L, et al. Antagonism of the chemokine Ccl5 ameliorates experimental liver fibrosis in mice. Journal of Clin Invest. November 2010; 120(11):4129-4140
32. Benyon, RC and MJ Arthur, Extracellular matrix degradation and the role of hepatic stellate cells.Semin Liver Dis. 2001 Aug;21(3):373-84.
33. Sheth SG, Chopra S. Natural history and management of nonalcoholic fatty liver disease in adults, UpToDate; 2014
34. Chalasani N, Younossi Z, Lavine JE, Diehl AM, Brunt EM, Cusi K, et al. The diagnosis and management of non-alcoholic fatty liver disease: Practice Guideline by the American Association for the Study of Liver Diseases, American College of Gastroenterology, and the American Gastroenterological Association. Hepatology 2012;55:2005-23.
35. McCullough AJ. The clinical features, diagnosis and natural history of nonalcoholic fatty liver disease. Clin Liver Dis 2004;8(3):521-33.
36. Loomba R, Sirlin CB, Schwimmer JB, Lavine JE. Advances in pediatric nonalcoholic fatty liver disease. Hepatology 2009;50(4):1282-93.
37. Torres DM, Williams CD, Harrison SA. Features, diagnosis, and treatment of nonalcoholic fatty liver disease. Clin Gastroenterol Hepatol 2012;10(8):837-58.
38. Schwenger KJ, Allard JP. Clinical approaches to non-alcoholic fatty liver disease. World J Gastroenterol 2014;20(7):1712-23.
39. Koo SH. Nonalcoholic fatty liver disease: molecular mechanisms for the hepatic steatosis. Clin Mol Hepatol 2013;19(3):210-5.
40. Attar BM, Van Thiel DH. Current concepts and management approaches in nonalcoholic fatty liver disease. ScientificWorld Journal 2013;2013:481893
41. Basaranoglu M, Basaranoglu G, Sentürk H. From fatty liver to fibrosis: a tale of "second hit". World J Gastroenterol 2013;19(8):1158-65.
42. Cohen JC, Horton JD, Hobbs HH. Human fatty liver disease: old questions and new insights. Science 2011;332(6037):1519-23.
43. Matteoni CA, Younossi ZA, Gramlich T, et al. Nonalcoholic fatty liver disease: A spectrum of clinical and pathological severity. Gastroenterology 1999;116(6):1413-1419
44. World Gastroenterology Organisation Global Guidelines. Nonalcoholic Fatty liver Disease and Nonalcoholic Steatohepatitis. June 2012.
45. Ahmed MH, Abu EO, Byrne CD. Non-Alcoholic Fatty Liver Disease (NAFLD): new challenge for general practitioners and important burden for health authorities? Prim Care Diabetes. 2010;4:129-37.
46. Vernon G, Baranova A, Younossi ZM. Systematic review: the epidemiology and natural history of non-alcoholic fatty liver disease and nonalcoholic steatohepatitis in adults. Aliment Pharmacol Ther 2011;34:274-85.
47. The Gastroenterological Society of Australia/Australian Liver Association January 2013. http://static.squarespace.com/static/50ff0804e4b007d5a9abe0a5/t/53321aaee4b09f967eb0c7e5/1395792558684/gesa2013_revised%5B1%5D.pdf
48. Dixon JB, Bhathal PS, O'Brien PE. Nonalcoholic fatty liver disease: predictors of nonalcoholic steatohepatitis and liver fibrosis in the severely obese. Gastroenterol. 2001;121:91-100.
49. Williams CD, Stenger J, Asike MI, Torres DM, Shaw J, Contreras M, et al. Prevalence of nonalcoholic fatty liver disease and nonalcoholic steatohepatitis among a largely middle-aged population utilizing ultrasound and liver biopsy: a prospective study. Gastroenterology 2011;140:124-131.
50. Schattenberg JM, Schuppan D. Nonalcoholic steatohepatitis: the therapeutic challenge of a global epidemic. Curr Opin Lipidol 2011;22:479-88.
51. Bahrami H. Nonalcoholic fatty liver disease in developing countries. World J Gastroenterol 2005;11:3808-3809.
52. Tiniakos DG, Vos MB, Brunt EM. Nonalcoholic fatty liver disease: pathology and pathogenesis. Annu Rev Pathol 2010;5:145-71.
53. Vajro P, Paolella G, Fasano A. Microbiota and gut-liver axis: their influences on obesity and obesity-related liver disease. J Pediatr Gastroenterol Nutr 2013;56:461-8.
54. Roh YS, Seki E. Toll-like receptors in alcoholic liver disease, non-alcoholic steatohepatitis and carcinogenesis. J Gastroenterol Hepatol 2013;28 Suppl 1:38-42.
55. Ilan Y. Leaky gut and the liver: a role for bacterial translocation in nonalcoholic steatohepatitis. World J Gastroenterol 2012;18:2609-18.
56. Moschen AR, Kaser S, Tilg H. Non-alcoholic steatohepatitis: a microbiota-driven disease. Trends Endocrinol Metab 2013;24:537-45.
57. Sanyal AJ, Campbell-Sargent C, Mirshahi F, Rizzo WB, Contos MJ, Sterling RK, et al. Nonalcoholic steatohepatitis: association of insulin resistance and mitochondrial abnormalities. Gastroenterology 2001;120:1183-92.
58. McClain CJ, Mokshagundam SP, Barve SS, Song Z, Hill DB, Chen T, et al. Mechanisms of non-alcoholic steatohepatitis. Alcohol 2004;34:67-79.
59. Day CP, Saksena S. Non-alcoholic steatohepatitis: definitions and pathogenesis. J Gastroenterol Hepatol. 2002;17 Suppl 3:S377-S84.
60. Marra F, Gastaldelli A, Svegliati Baroni G, Tell G, Tiribelli C. Molecular basis and mechanisms of progression of non-alcoholic steatohepatitis. Trends Mol Med. 2008;14:72-81
61. Charlton MR, Burns JM, Pederson RA, Watt KD, Heimbach JK, Dierkhising RA. Frequency and outcomes of liver transplantation for nonalcoholic steatohepatitis in the United States. Gastroenterol. 2011;141:1249-53.
62. Vatche G. Agopian et al. Liver Transplantation for Nonalcoholic Steatohepatitis. Annals of Surgery 2012; 256(4); 624-633.
63. McCullough AJ. Epidemiology of the metabolic syndrome in the USA. J Dig Dis. 2011;12:333-40.

**Embodiments of the invention**

1. A method of treating fibrosis or a fibrotic disease or condition or condition in a subject in need thereof comprising co-administering to the subject a therapeutically effective amount of cenicriviroc or a salt or solvate thereof; and an additional active agent.

2. The method of 1, wherein the fibrosis or fibrotic disease or condition is liver fibrosis or renal fibrosis.

3. The method of 1, wherein the cenicriviroc or a salt or solvate thereof is formulated as a pharmaceutical composition comprising cenicriviroc or a salt or solvate thereof and fumaric acid.

4. The method of 2, wherein the liver fibrosis is associated with non-alcoholic steatohepatitis (NASH).

5. The method of 2, wherein the liver fibrosis is associated with non-alcoholic fatty liver disease (NAFLD).

6. The method of 2, wherein the liver fibrosis is associated with emerging cirrhosis.

7. The method of 2, wherein the liver fibrosis comprises non-cirrhotic hepatic fibrosis.

8. The method of 2, wherein the subject is infected by human immunodeficiency virus (HIV).

9. The method of any one of 1 to 8, wherein the subject has a disease or condition selected from the group consisting of alcoholic liver disease, HIV and HCV co-infection, viral hepatitis (such as HBV or HCV infection), type 2 diabetes mellitus (T2DM), metabolic syndrome (MS), and a combination thereof.

10. A method of treating NASH in a subject in need thereof comprising administering to the subject a therapeutically effective amount of cenicriviroc, or a salt or solvate thereof; wherein the NASH is associated with type 2 diabetes mellitus (T2DM).

11. A method of treating NASH in a subject in need thereof comprising administering to the subject a therapeutically effective amount of cenicriviroc, or a salt or solvate thereof; wherein the NASH is associated with metabolic syndrome (MS).

12. A method of treating NASH in a subject in need thereof comprising administering to the subject a therapeutically effective amount of cenicriviroc, or a salt or solvate thereof; wherein the NASH is associated with HIV and HCV co-infection.

13. The method of any of the preceding , wherein the cenicriviroc or salt or solvate thereof is formulated as an oral composition.

14. The method of any of the preceding, wherein the cenicriviroc or salt or solvate thereof is administered once per day or twice per day.

15. The method of any of the preceding , wherein the cenicriviroc or salt or solvate thereof is coadministered with one or more additional active agents.

16. The method of 15, wherein the one or more additional active agents are one or more antiretroviral agents selected from the group consisting of entry inhibitors, nucleoside reverse transcriptase inhibitors, nucleotide reverse transcriptase inhibitors, non-nucleoside reverse transcriptase inhibitors, protease inhibitors, integrase inhibitors, maturation inhibitors, and combinations thereof.

17. The method of 16, wherein the one or more additional antiretroviral agents are selected from the group consisting of lamivudine, efavirenz, raltegravir, vivecon, bevirimat, alpha interferon, zidovudine, abacavir, lopinavir, ritonavir, tenofovir, tenofovir disoproxil, tenofovir prodrugs, emtricitabine, elvitegravir, cobicistat, darunavir, atazanavir, rilpivirine, dolutegravir, and a combination thereof.

18. The method of 15, wherein the one or more additional active agents are one or more immune system suppressing agents.

19. The method of 18, wherein the one or more additional active agents are selected from the group consisting of cyclosporine, tacrolimus, prednisolone, hydrocortisone, sirolimus, everolimus, azathioprine, mycophenolic acid, methotrexate, basiliximab, daclizumab, rituximab, anti-thymocyte globulin, anti-lymphocyte globulin, and a combination thereof.

20. The method of any of the preceding , comprising detecting a level of one or more biological molecules in the subject treated for fibrosis or the fibrotic disease or condition or condition, and determining a treatment regimen based on an increase or decrease in the level of one or more biological molecules, wherein the biological molecule is selected from the group consisting of lipopolysaccharide (LPS), LPs-binding protein (LBP), 16S rDNA, sCD14, intestinal fatty acid binding protein (I-FABP), zonulin-1, Collagen lal and 3a1, TGF-β, fibronectin-1, hs-CRP, IL-1β, IL-6, IL-33, fibrinogen, MCP-1, MIP-1α and -1β, RANTES, sCD163, TGF-β, TNF-α, a biomarker of hepatocyte apoptosis such as CK-18 (caspase-cleaved and total), and a combination thereof.

21. The method of any of the preceding , comprising detecting a level of one or biological molecules in the subject treated for fibrosis or the fibrotic disease or condition or condition, wherein an increase or decrease in the level of one or more biological molecules compared to a predetermined standard level is predictive of the treatment efficacy of fibrosis or the fibrotic disease or condition, wherein the biological molecule is selected from the group consisting of lipopolysaccharide (LPS), LPs-binding protein (LBP), 16S rDNA, sCD14, intestinal fatty acid binding protein (I-FABP), zonulin-1, Collagen lal and 3a1, TGF-β, fibronectin-1, hs-CRP, IL-1β, IL-6, IL-33, fibrinogen, MCP-1, MIP-1α and -1β, RANTES, sCD163, TGF-β, TNF-α, a biomarker of hepatocyte apoptosis such as CK-18 (caspase-cleaved and total), and a combination thereof.

22. The method of 20 or 21, where the one or more biological molecules are measured in a biological sample from a subject treated for fibrosis or the fibrotic disease or condition.

23. The method of 22, where the biological sample is selected from blood, skin, hair follicles, saliva, oral mucous, vaginal mucous, sweat, tears, epithelial tissues, urine, semen, seminal fluid, seminal plasma, prostatic fluid, pre-ejaculatory fluid (Cowper's fluid), excreta, biopsy, ascites, cerebrospinal fluid, lymph, brain, and tissue extract sample or biopsy sample.

**Claims**

**1.** A pharmaceutical composition comprising cenicriviroc, or a salt or solvate thereof, fumaric acid, microcrystalline

cellulose, croscarmellose sodium, and magnesium stearate and one or more additional active agents for use in preventing or treating fibrosis or a fibrotic disease or condition in a subject in need thereof,
wherein the one or more additional active agent is an antiretroviral agent,
wherein the pharmaceutical composition is for administration to the subject in a therapeutically effective amount and wherein the pharmaceutical composition comprises a weight ratio of cenicriviroc or a salt or solvate thereof to fumaric acid from (a) 7:10 to 10:7 or (b) 8:10 to 10:8.

**2.** The pharmaceutical composition for use according to claim 1, wherein the fibrosis or fibrotic disease or condition is liver or renal fibrosis.

**3.** The pharmaceutical composition for use according to claim 2, wherein the liver fibrosis is associated with non-alcoholic steatohepatitis (NASH) or non-alcoholic fatty liver disease (NAFLD).

**4.** The pharmaceutical composition for use according to claim 3, wherein the NASH is associated with type 2 diabetes mellitus (T2DM), metabolic syndrome (MS), or HIV and HCV co-infection.

**5.** The pharmaceutical composition for use according to claim 1, wherein the pharmaceutical composition is formulated as an oral composition.

**6.** The pharmaceutical composition for use according to claim 1 or claim 5, wherein the pharmaceutical composition is administered once per day or twice per day.

**7.** The pharmaceutical composition for use according to claim 1, wherein the antiretroviral agent is selected from the group consisting of entry inhibitors, nucleoside reverse transcriptase inhibitors, nucleotide reverse transcriptase inhibitors, non-nucleoside reverse transcriptase inhibitors, protease inhibitors, integrase inhibitors, maturation inhibitors, and combinations thereof.

**8.** The pharmaceutical composition for use according to claim 7, wherein the one or more additional antiretroviral agents are selected from the group consisting of lamivudine, efavirenz, raltegravir, vivecon, bevirimat, alpha interferon, zidovudine, abacavir, lopinavir, ritonavir, tenofovir, tenofovir disoproxil, tenofovir prodrugs, emtricitabine, elvitegravir, cobicistat, darunavir, atazanavir, rilpivirine, dolutegravir, and a combination thereof.

**9.** The pharmaceutical composition for use according to any of the preceding claims wherein the level of one or more biological molecules in a biological sample from a subject treated for fibrosis or the fibrotic disease or condition is measured, and a treatment regimen is determined based on an increase or decrease in the level of one or more biological molecules, wherein the biological molecule is selected from the group consisting of lipopolysaccharide (LPS), LPs-binding protein (LBP), 16S rDNA, sCD14, intestinal fatty acid binding protein (I-FABP), zonulin-1, Collagen lal and 3a1, TGF-13, fibronectin-1, hs-CRP, IL-10, IL-6, IL-33, fibrinogen, MCP-1, MIP-la and -10, RANTES, sCD163, TGF-13, TNF-a, a biomarker of hepatocyte apoptosis such as CK-18 (caspase-cleaved and total), and a combination thereof.

**10.** The pharmaceutical composition for use according to any of the preceding claims wherein the level of one or more biological molecules in a biological sample from a subject treated for fibrosis or the fibrotic disease or condition or condition is measured, wherein an increase or decrease in the level of one or more biological molecules compared to a measured standard level is predictive of the treatment efficacy of fibrosis or the fibrotic disease or condition, wherein the biological molecule is selected from the group consisting of lipopolysaccharide (LPS), LPs-binding protein (LBP), 16S rDNA, sCD14, intestinal fatty acid binding protein (I-FABP), zonulin-1, Collagen lal and 3a1, TGF-13, fibronectin-1, hs-CRP, IL-l(3, IL-6, IL-33, fibrinogen, MCP-1, MIP-la and -10, RANTES, sCD163, TGF-13, TNF-a, a biomarker of hepatocyte apoptosis such as CK-18 (caspase-cleaved and total), and a combination thereof.

**11.** The pharmaceutical composition for use according to claim 9 or 10, where the biological sample is selected from blood, skin, hair follicles, saliva, oral mucous, vaginal mucous, sweat, tears, epithelial tissues, urine, semen, seminal fluid, seminal plasma, prostatic fluid, pre-ejaculatory fluid (Cowper's fluid), excreta, biopsy, ascites, cerebrospinal fluid, lymph, brain, and tissue extract sample or biopsy sample.

**12.** The pharmaceutical composition for use according to any of the preceding claims, wherein the prevention or treatment of fibrosis or a fibrotic disease or condition is measured by changes from baseline in AST-to-platelet ratio (APRI) and FIB-4 scores.

**13.** The pharmaceutical composition for use according to claim 1, wherein the pharmaceutical composition comprises:

a) from 15% to 40% cenicriviroc or a salt or solvate thereof;
b) from 25% to 55% microcrystalline cellulose;
c) from 15% to 40% fumaric acid;
d) from 2% to 10% croscarmellose sodium; and
e) from 0.25% to 5% magnesium stearate.

Figure 1

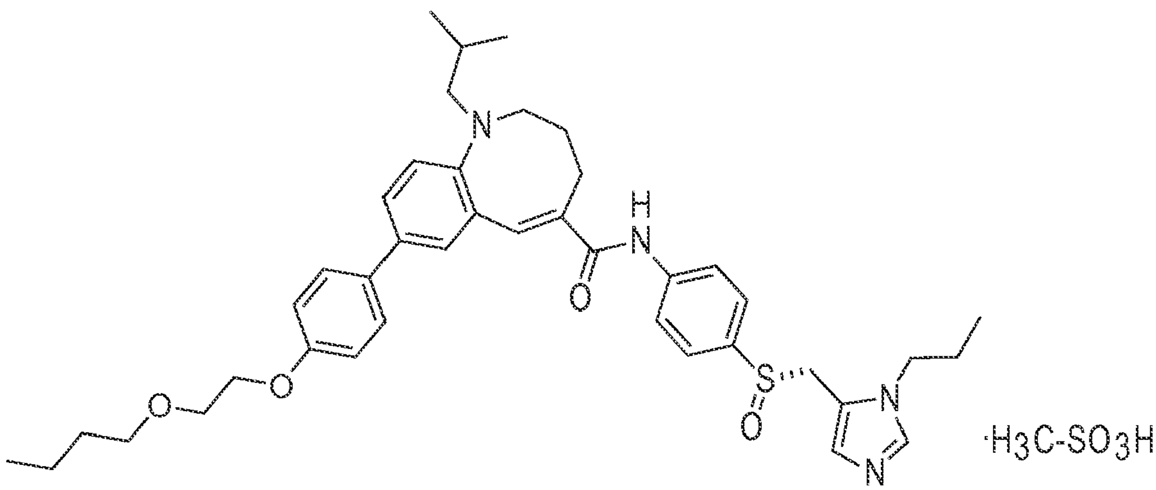

Figure 2

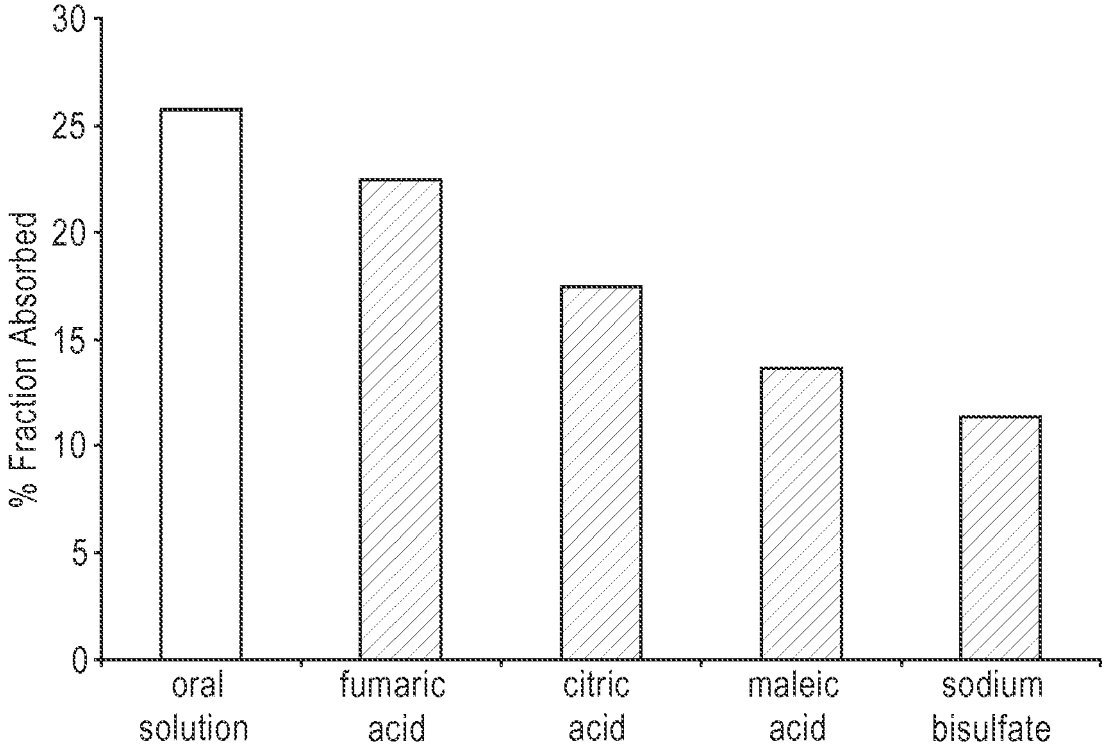
30
25
20
15
10
5
0
% Fraction Absorbed
oral solution
fumaric acid
citric acid
maleic acid
sodium bisulfate

Figure 3

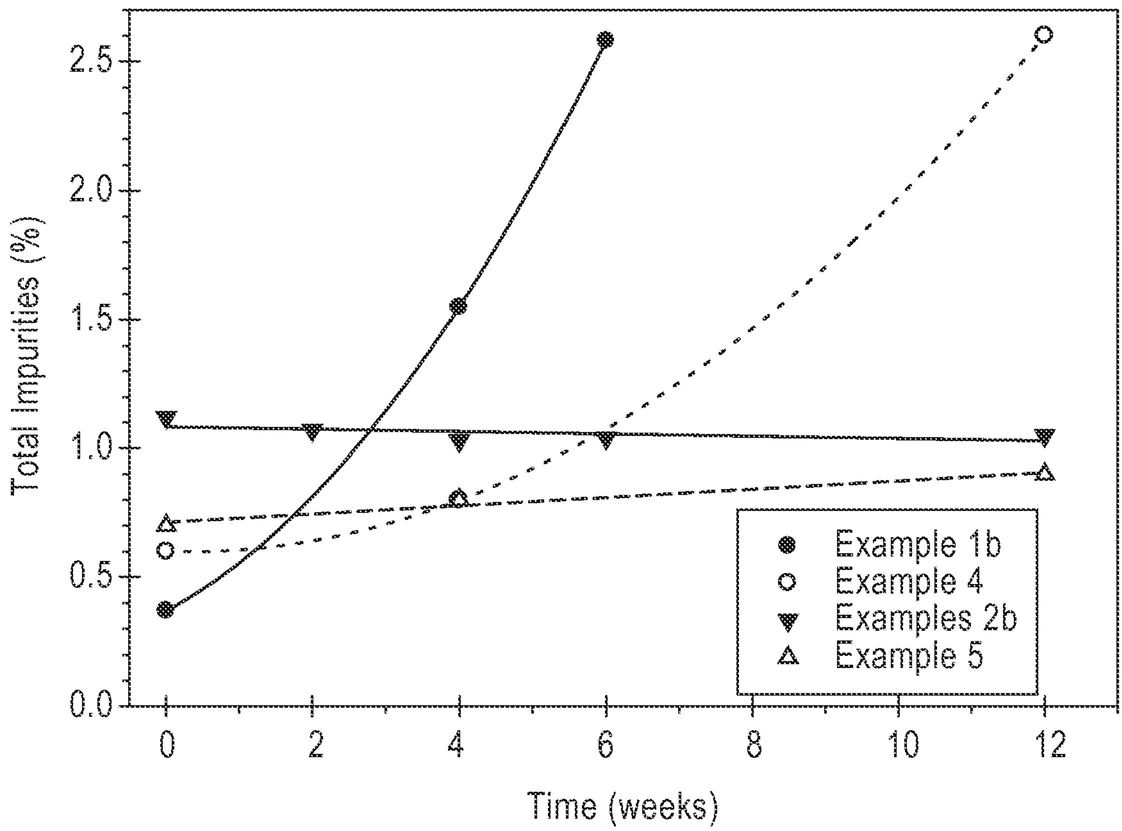
2.5
2.0
1.5
1.0
0.5
0.0
Total Impurities (%)
0
2
4
6
8
10
12
Time (weeks)
Example 1b
Example 4
Examples 2b
Example 5

Figure 4

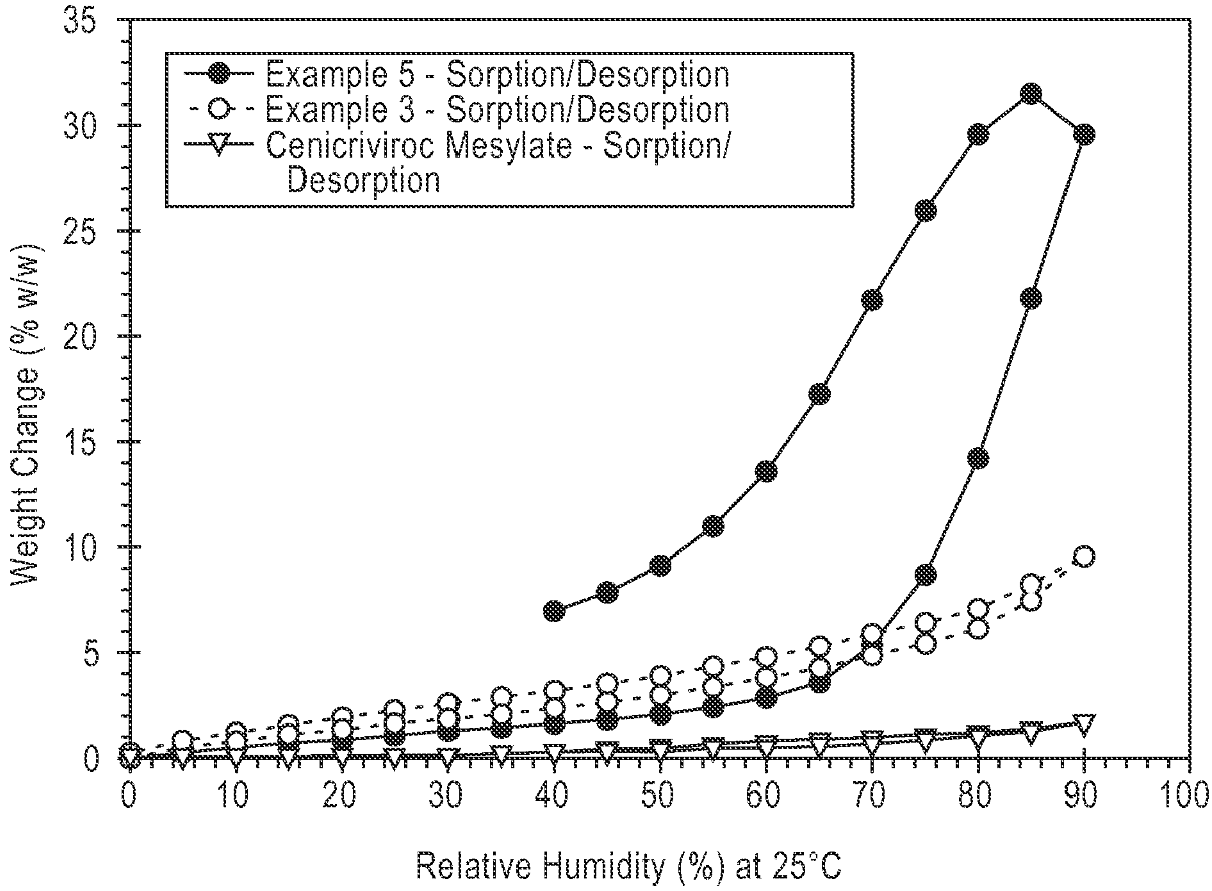
Example 5 - Sorption/Desorption
Example 3 - Sorption/Desorption
Cenicriviroc Mesylate - Sorption/ Desorption
Weight Change (% w/w)
Relative Humidity (%) at 25°C
0
5
10
15
20
25
30
35
0
10
20
30
40
50
60
70
80
90
100

Figure 5

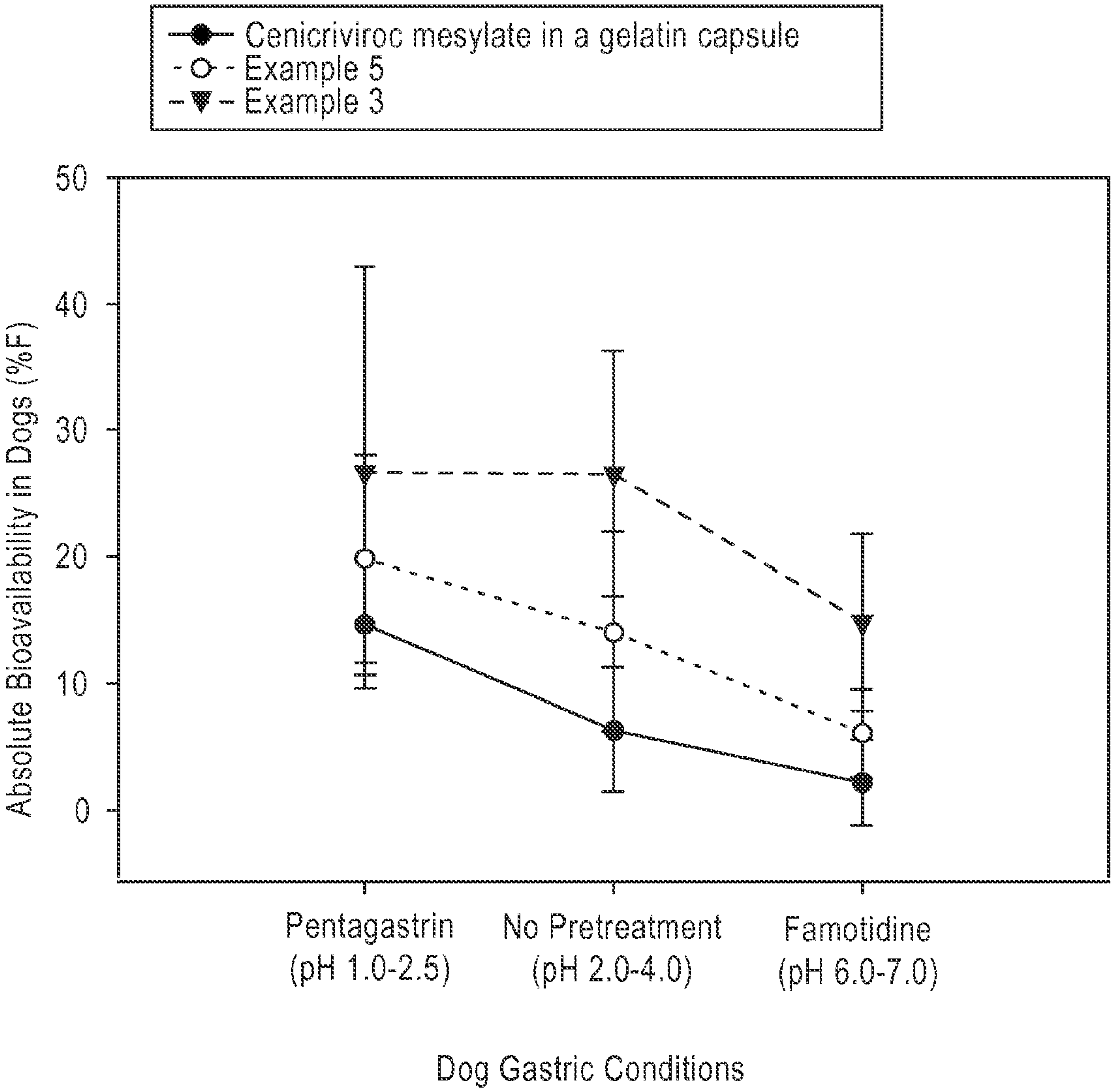
Cenicriviroc mesylate in a gelatin capsule
Example 5
Example 3
Absolute Bioavailability in Dogs (%F)
50
40
30
20
10
0
Pentagastrin
(pH 1.0-2.5)
No Pretreatment
(pH 2.0-4.0)
Famotidine
(pH 6.0-7.0)
Dog Gastric Conditions

Figure 6

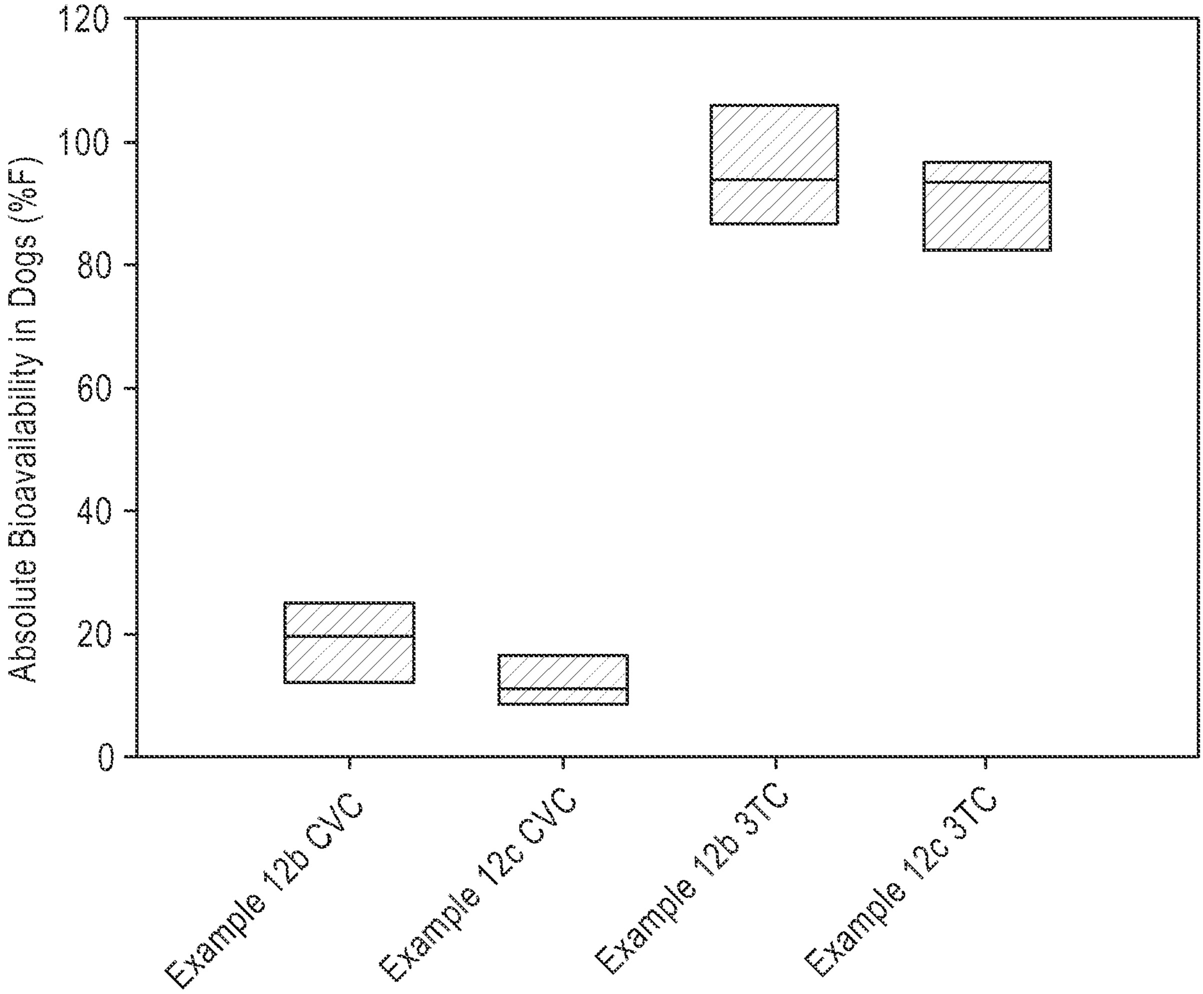
120
100
80
60
40
20
0
Absolute Bioavailability in Dogs (%F)
Example 12b CVC
Example 12c CVC
Example 12b 3TC
Example 12c 3TC

Figure 7

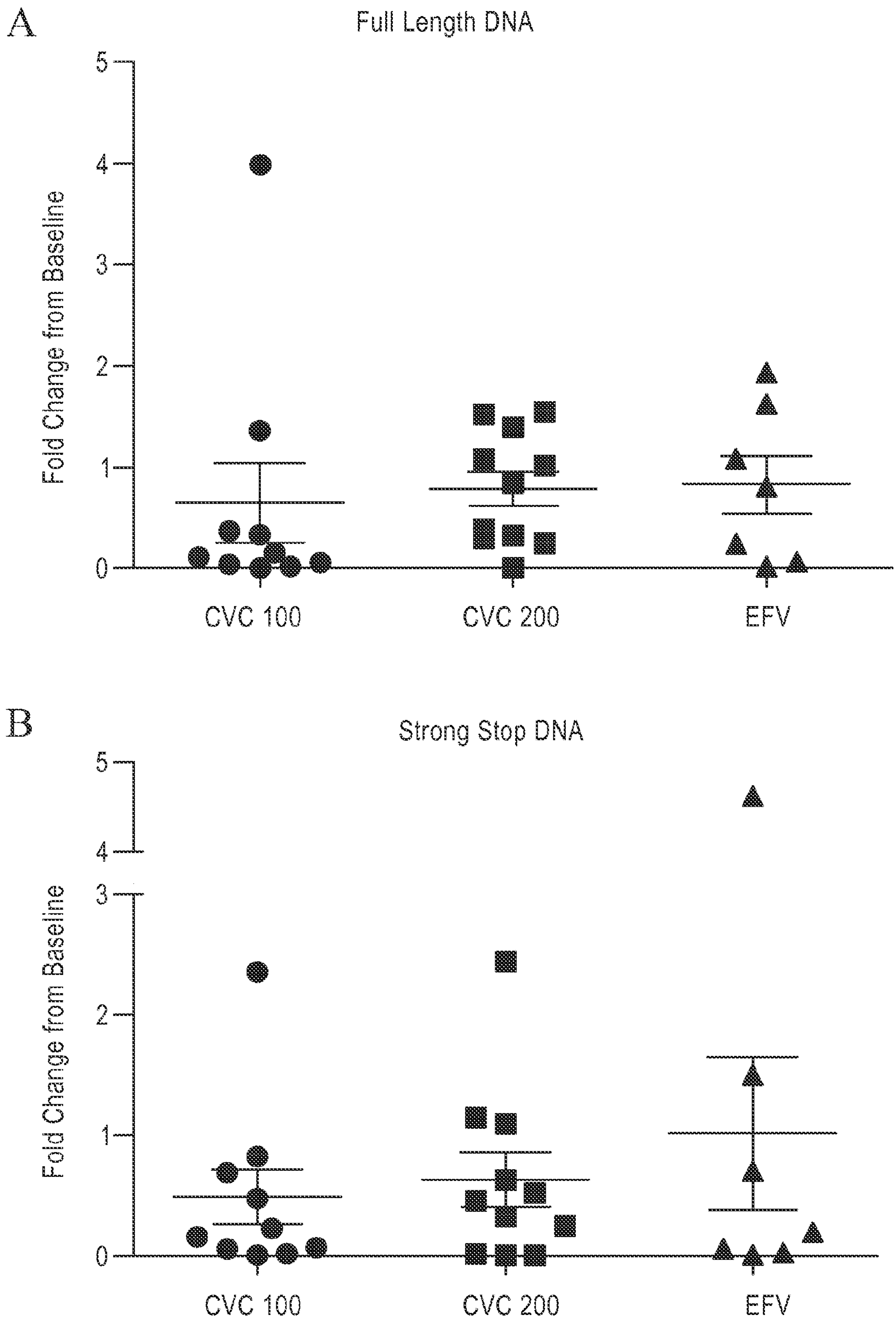
A
Full Length DNA
Fold Change from Baseline
5
4
3
2
1
0
CVC 100
CVC 200
EFV
B
Strong Stop DNA
Fold Change from Baseline
5
4
3
2
1
0
CVC 100
CVC 200
EFV

Figure 8

A

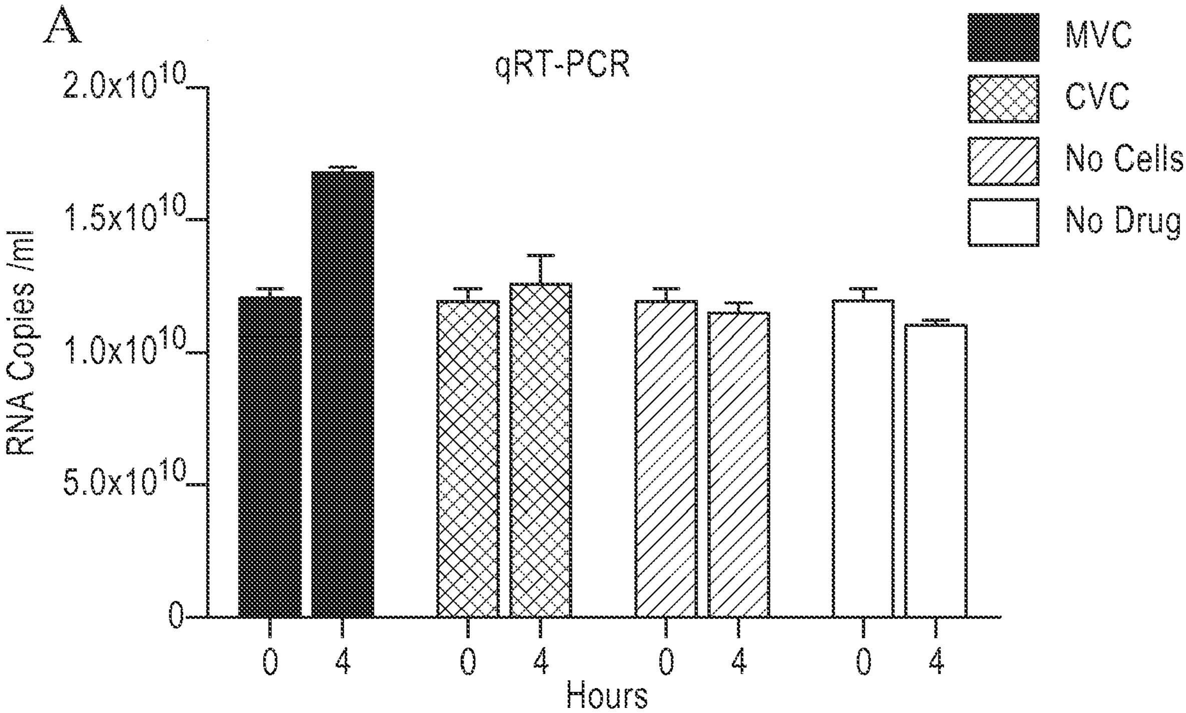
qRT-PCR
MVC
CVC
No Cells
No Drug
2.0x10^10
1.5x10^10
1.0x10^10
5.0x10^10
0
RNA Copies /ml
0
4
0
4
0
4
0
4
Hours

B

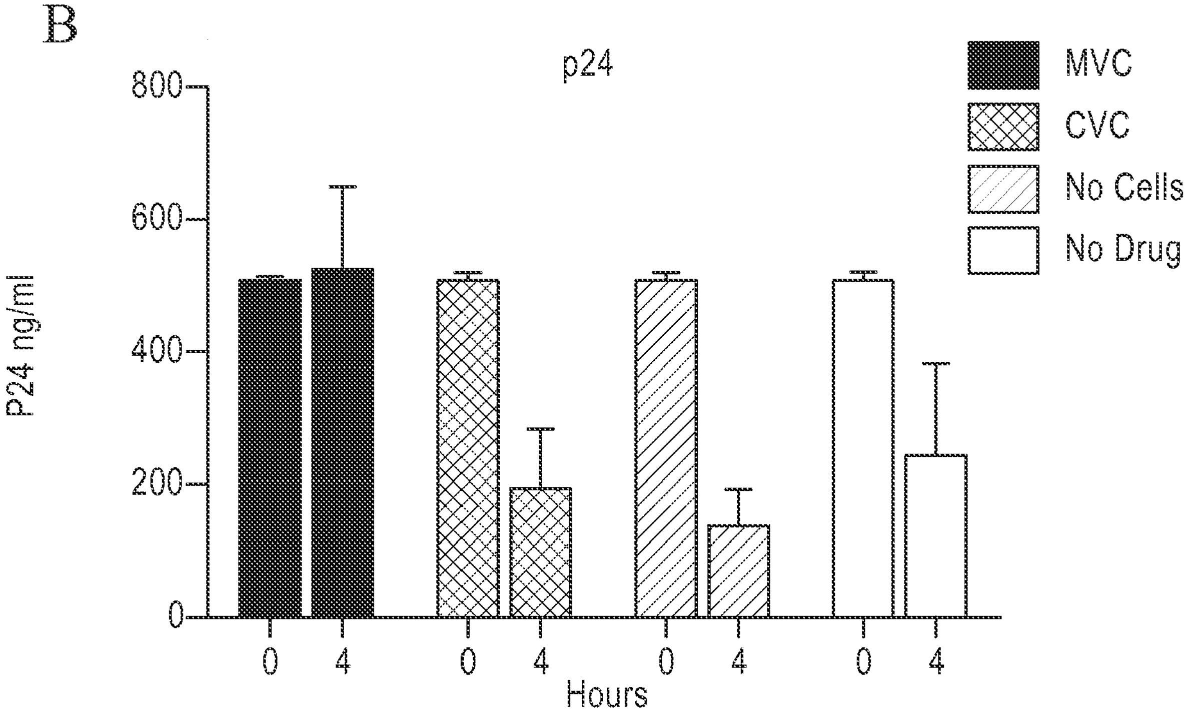
p24
MVC
CVC
No Cells
No Drug
800
600
400
200
0
P24 ng/ml
0
4
0
4
0
4
0
4
Hours

Figure 9

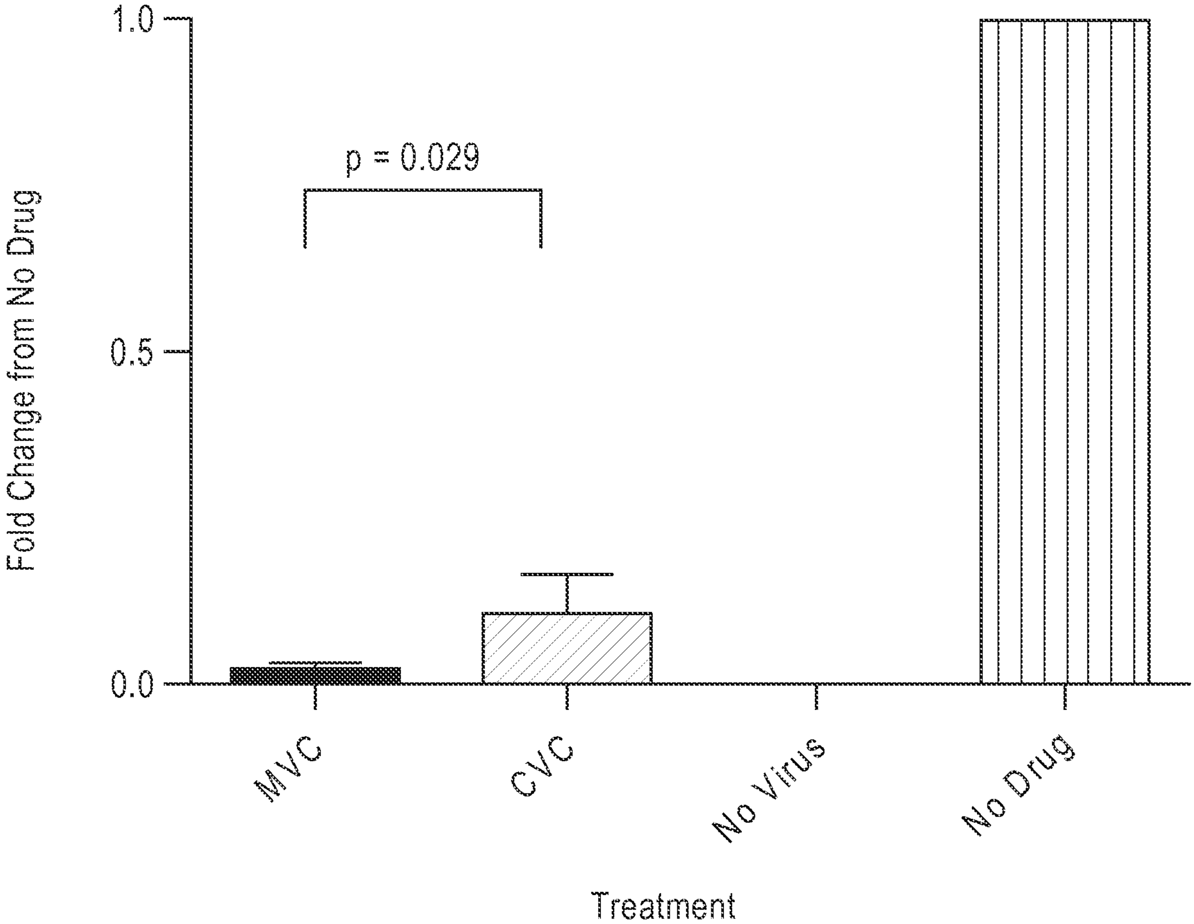
1.0
0.5
0.0
Fold Change from No Drug
p = 0.029
MVC
CVC
No Virus
No Drug
Treatment

Figure 10A

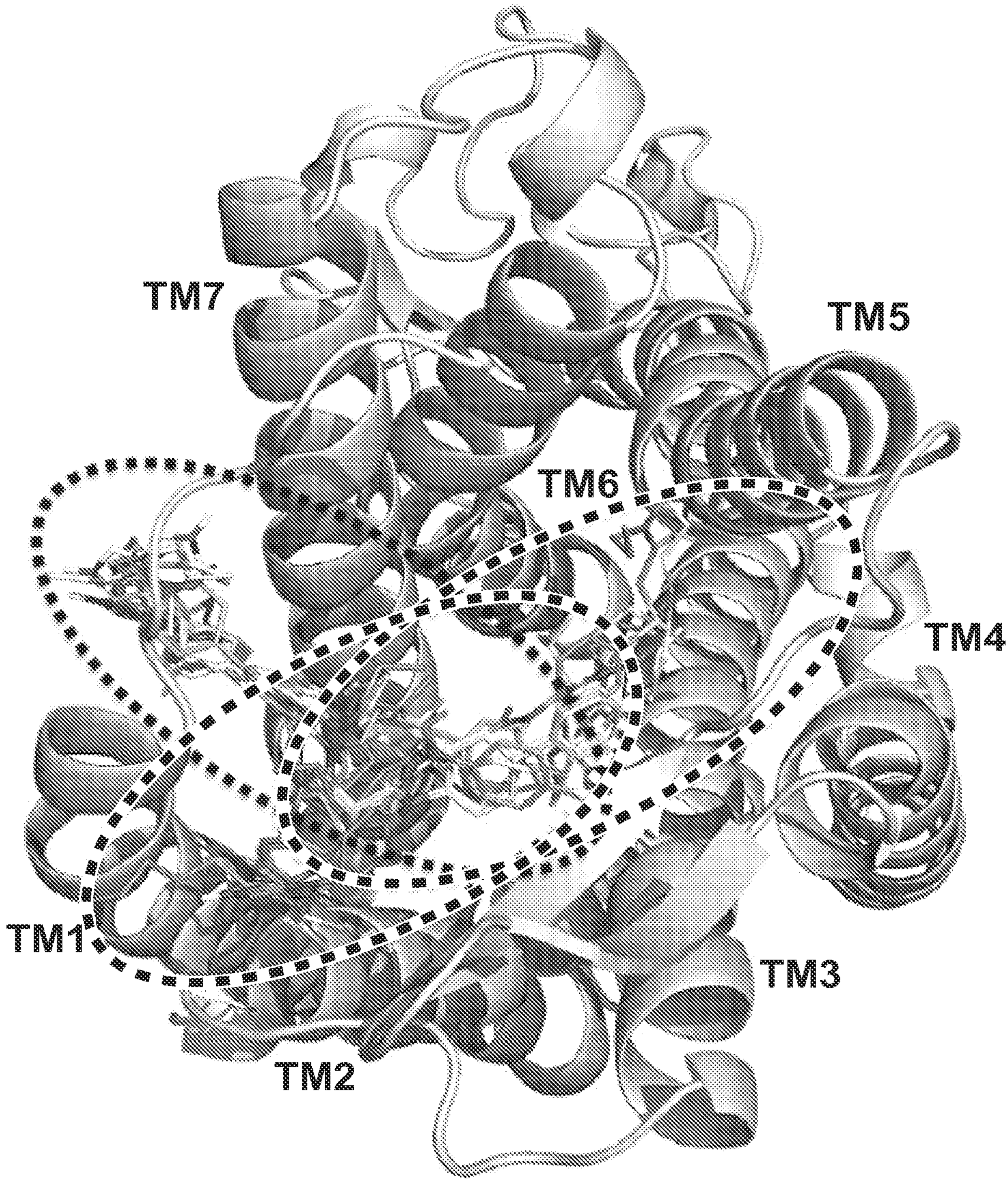

Figure 10B

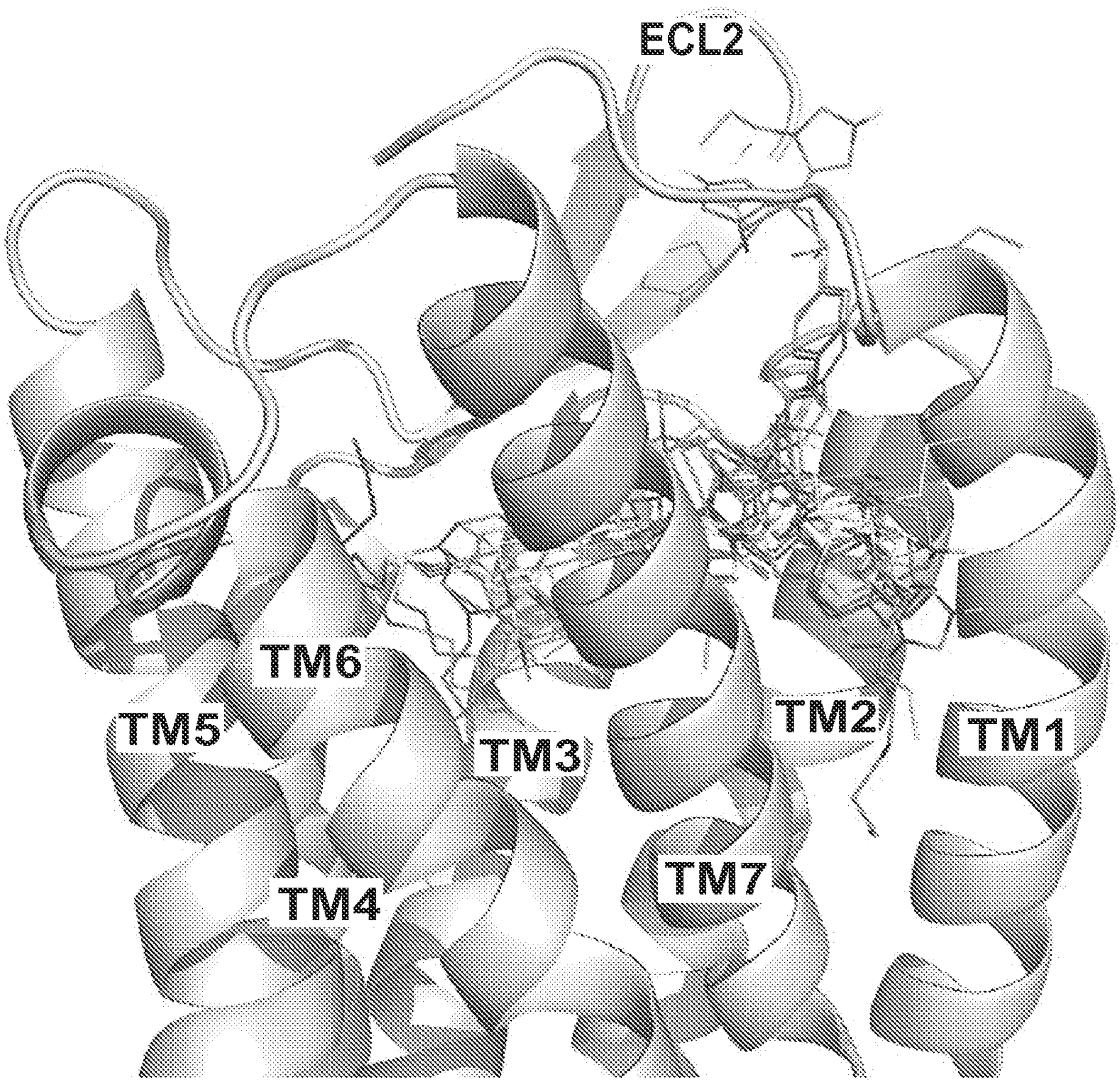

# Figure 11

**CVC**

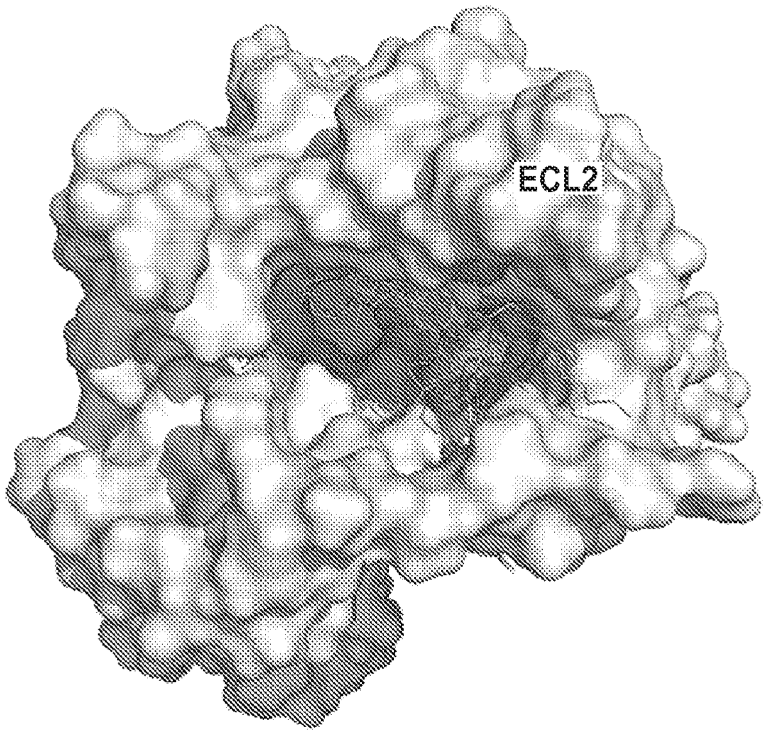


**MVC**

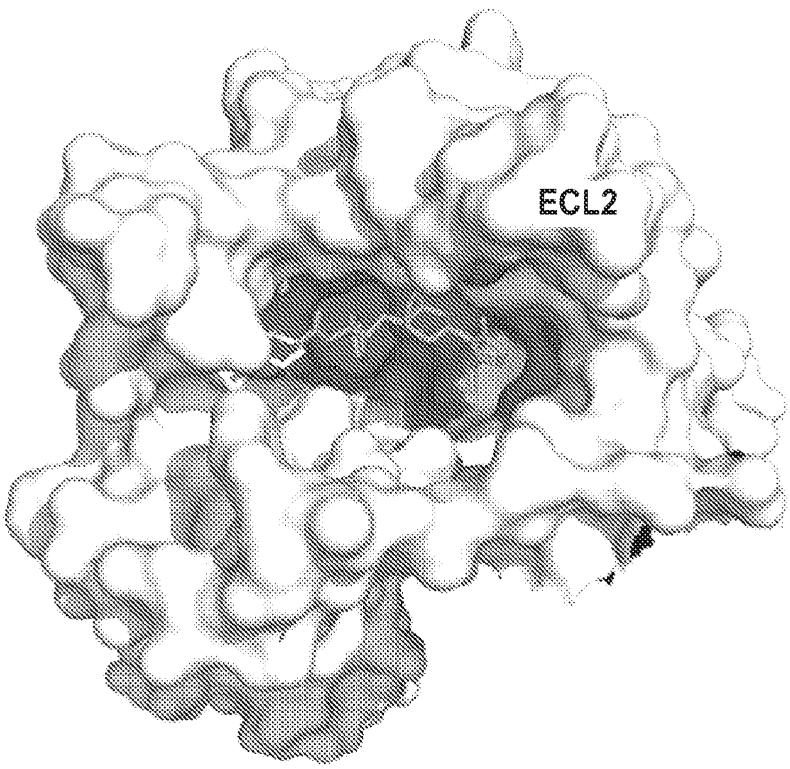

Figure 12

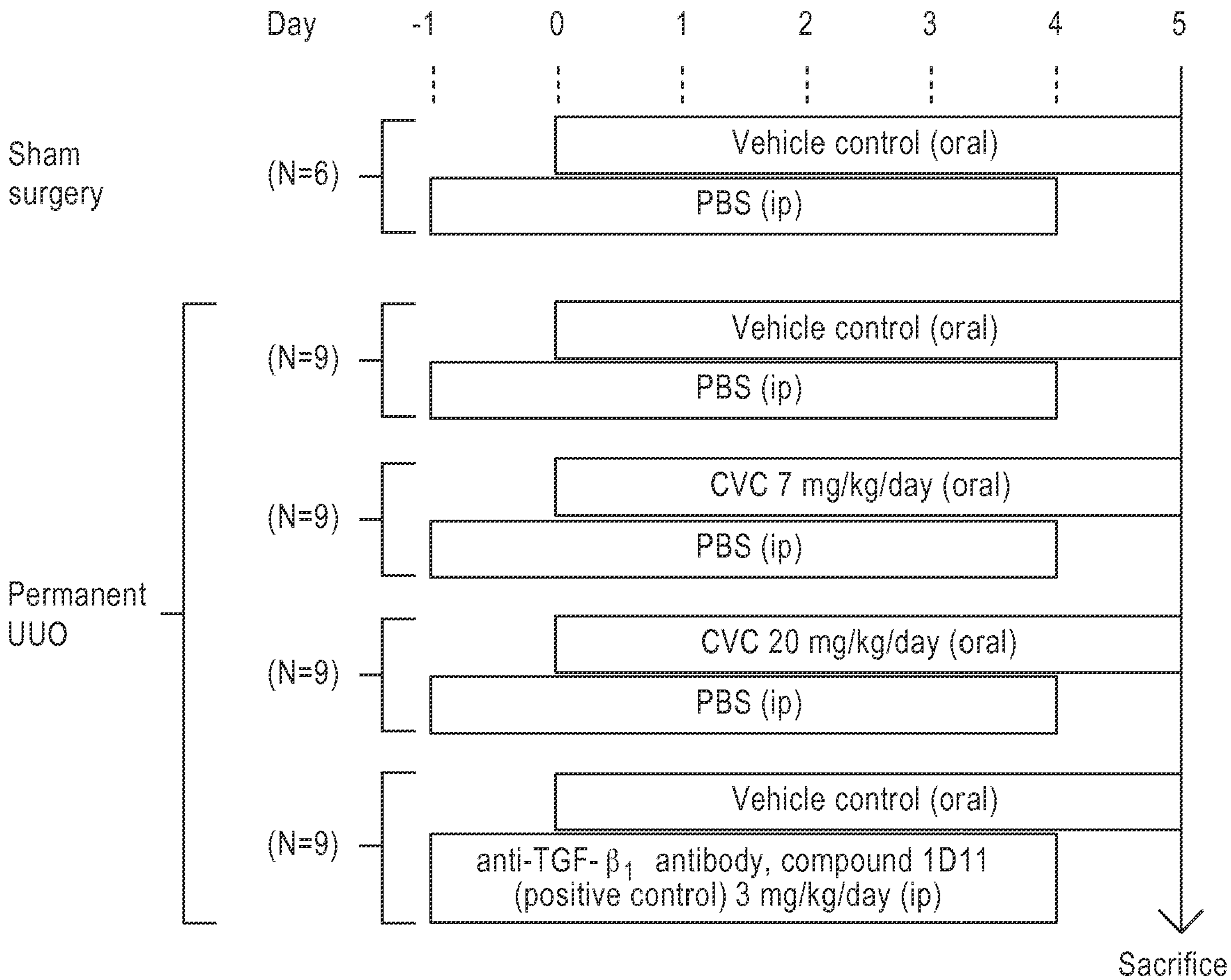
Day
-1
0
1
2
3
4
5
Sham
surgery
(N=6)
Vehicle control (oral)
PBS (ip)
Permanent
UUO
(N=9)
Vehicle control (oral)
PBS (ip)
(N=9)
CVC 7 mg/kg/day (oral)
PBS (ip)
(N=9)
CVC 20 mg/kg/day (oral)
PBS (ip)
(N=9)
Vehicle control (oral)
anti-TGF-β1 antibody, compound 1D11
(positive control) 3 mg/kg/day (ip)
Sacrifice

Figure 13

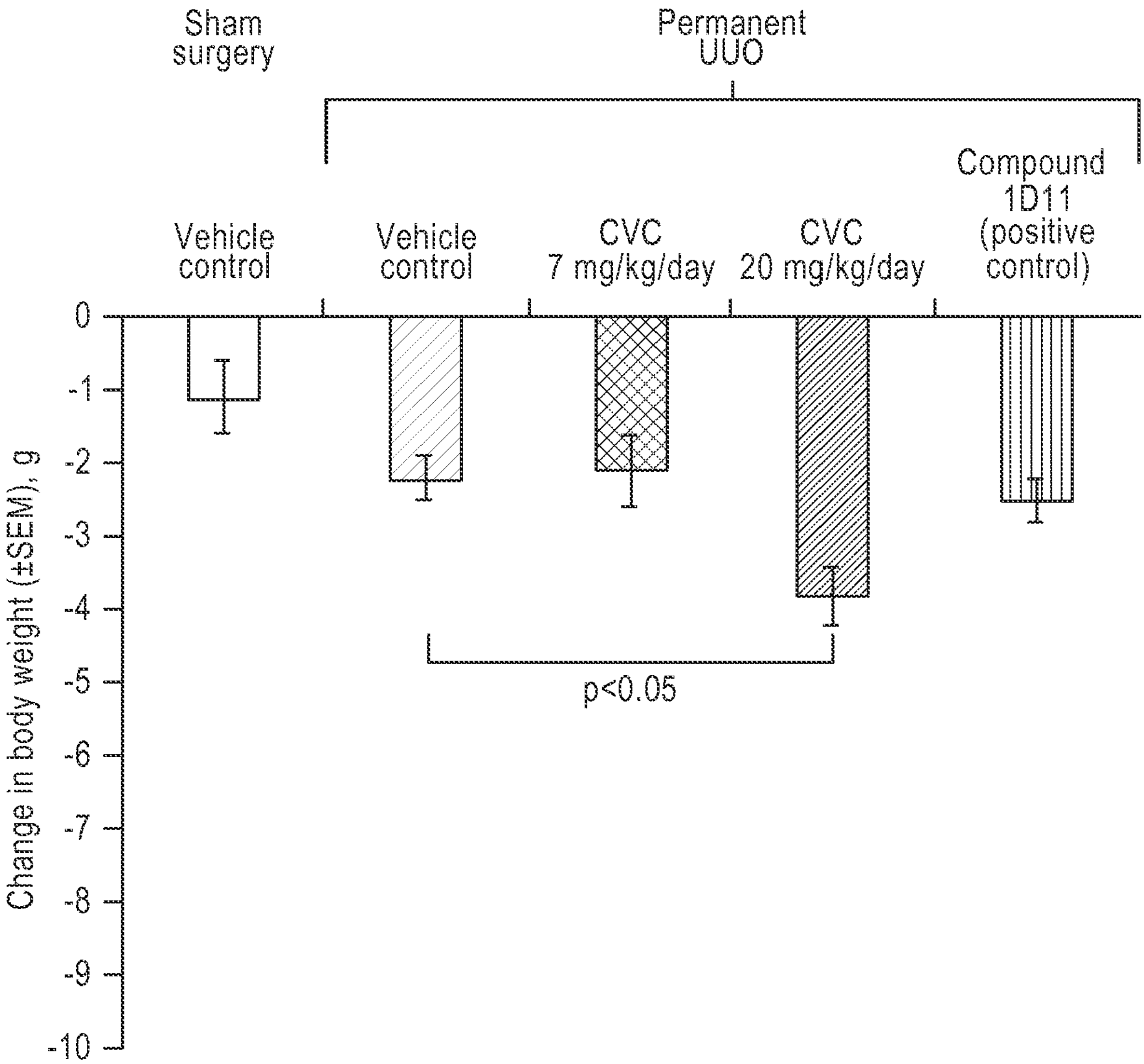
Sham surgery
Permanent UUO
Vehicle control
Vehicle control
CVC 7 mg/kg/day
CVC 20 mg/kg/day
Compound 1D11 (positive control)
0
-1
-2
-3
-4
-5
-6
-7
-8
-9
-10
Change in body weight (±SEM), g
p<0.05

Figure 14

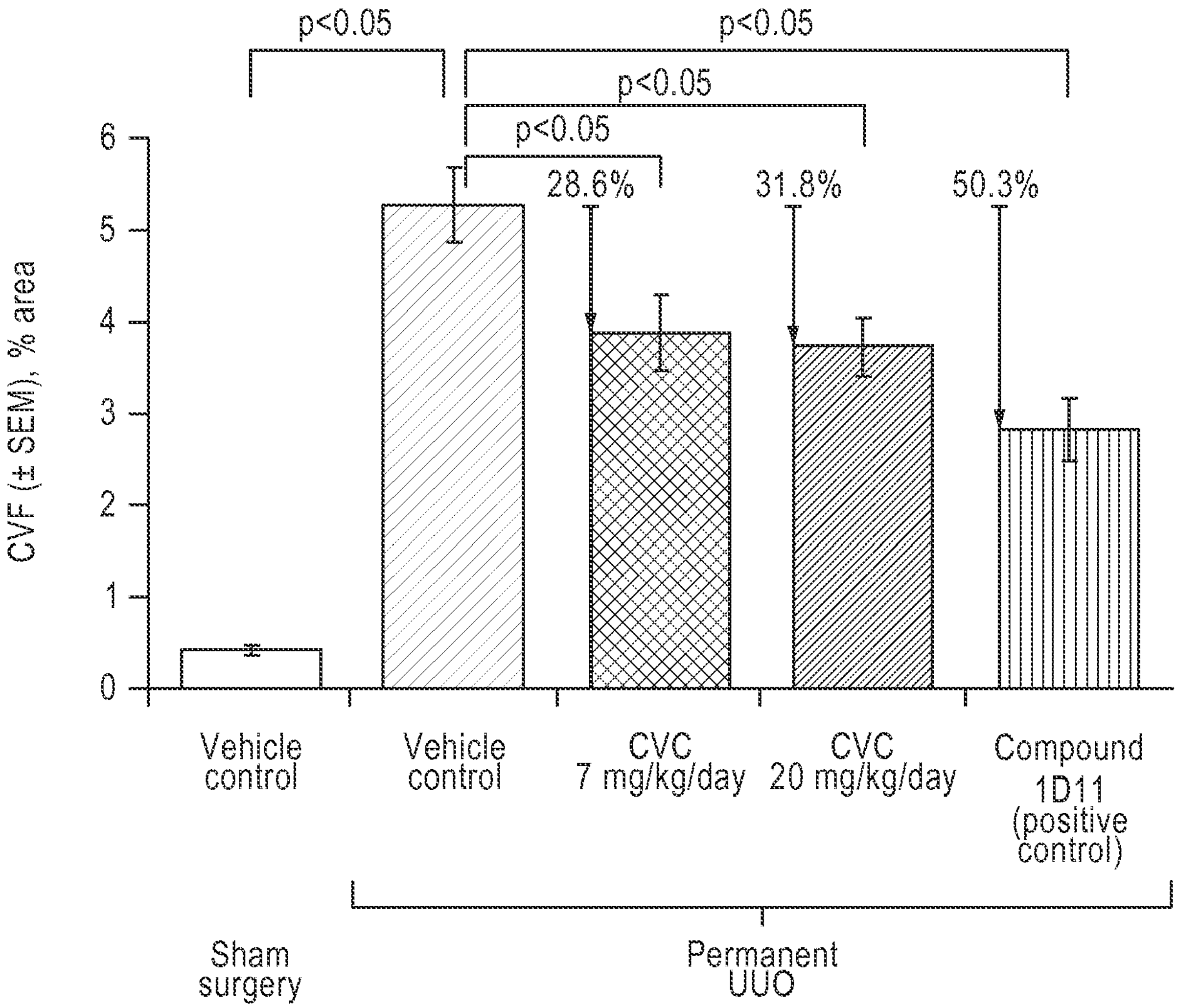
p<0.05
p<0.05
p<0.05
p<0.05
28.6%
31.8%
50.3%
CVF (± SEM), % area
0
1
2
3
4
5
6
Vehicle control
Vehicle control
CVC 7 mg/kg/day
CVC 20 mg/kg/day
Compound 1D11 (positive control)
Sham surgery
Permanent UUO

Figure 15

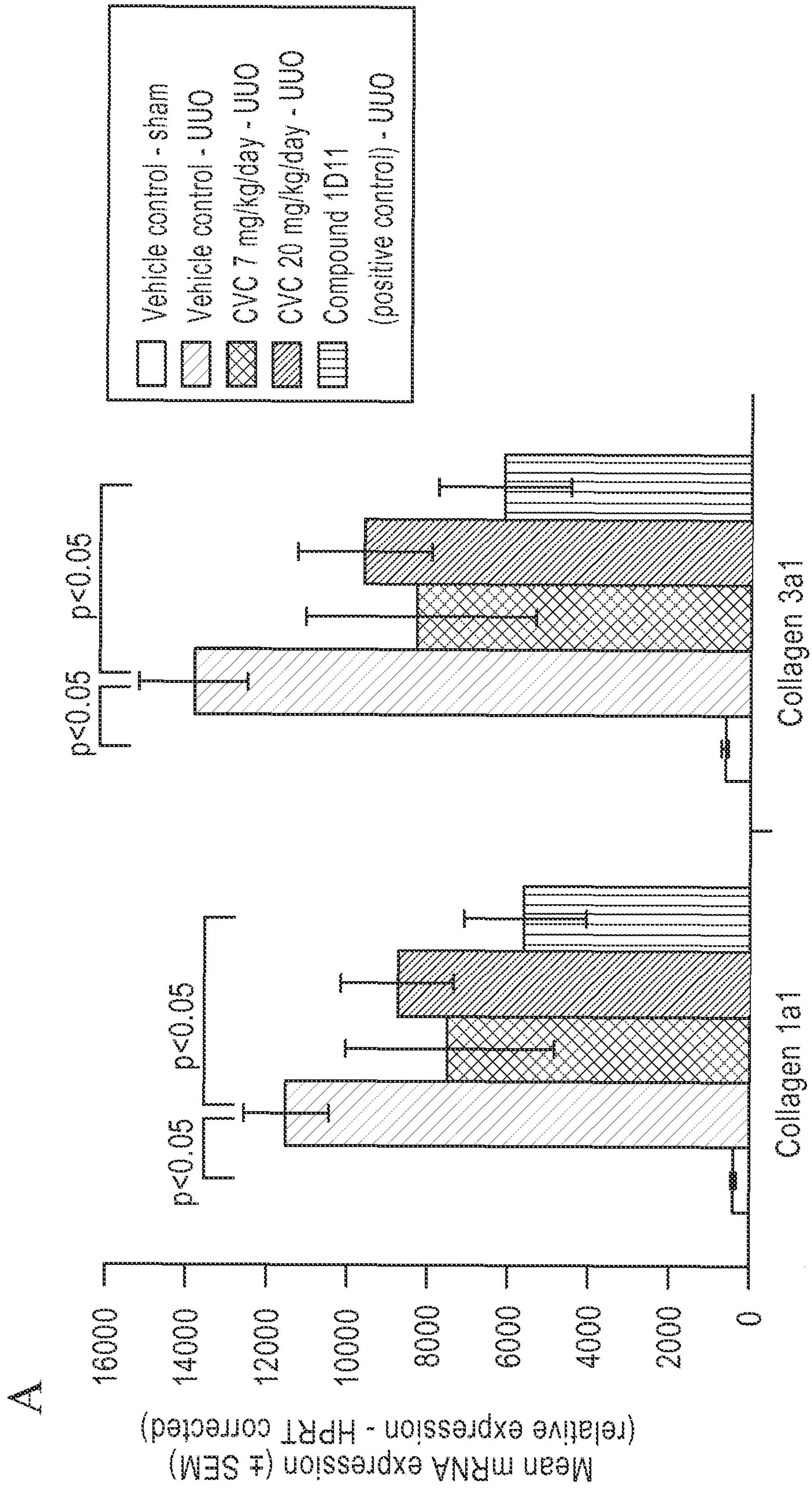
A
Mean mRNA expression (± SEM)
(relative expression - HPRT corrected)
16000
14000
12000
10000
8000
6000
4000
2000
0
p<0.05
p<0.05
p<0.05
p<0.05
Collagen 1a1
Collagen 3a1
Vehicle control - sham
Vehicle control - UUO
CVC 7 mg/kg/day - UUO
CVC 20 mg/kg/day - UUO
Compound 1D11
(positive control) - UUO

Figure 15

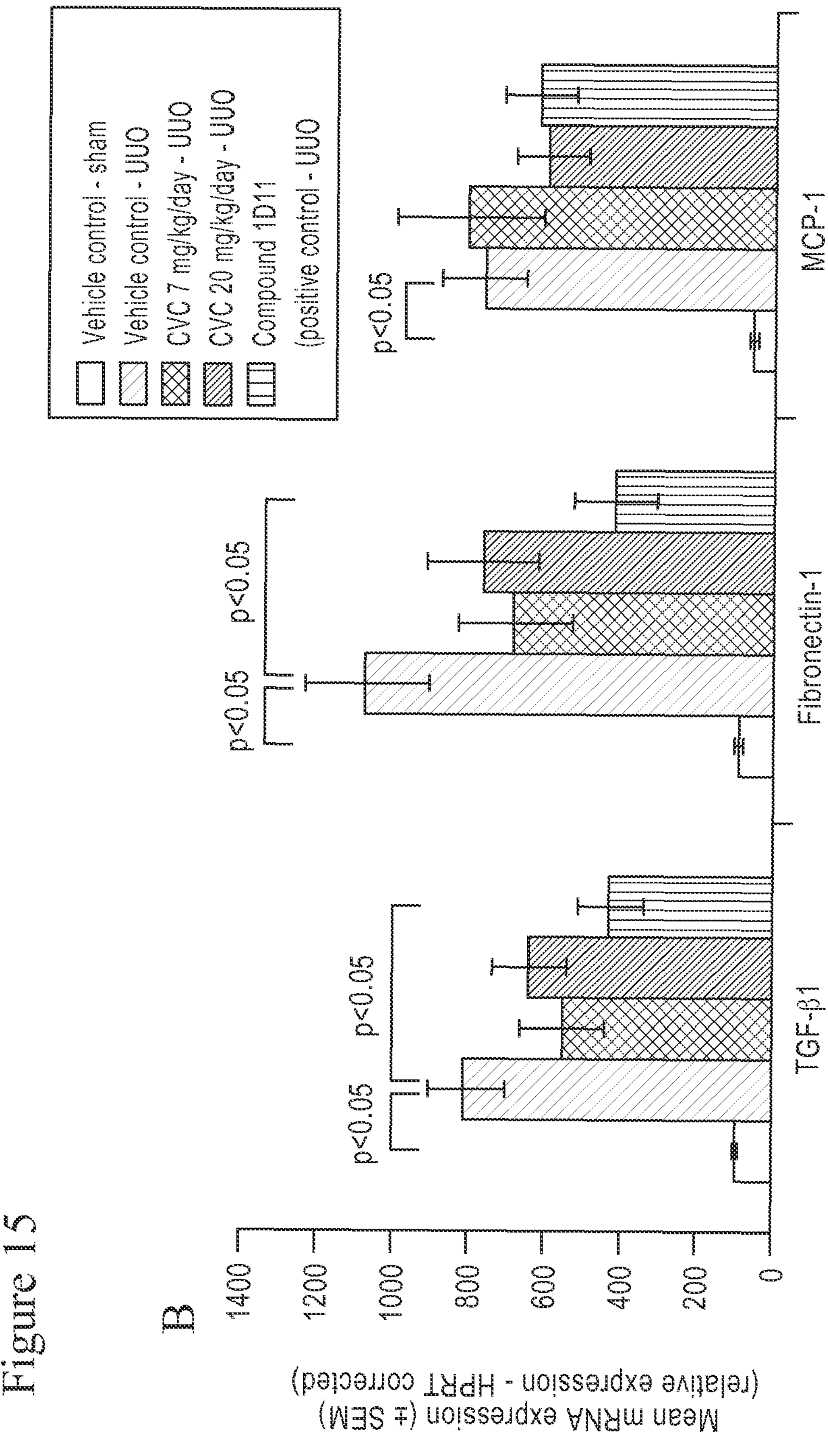
B
Mean mRNA expression (± SEM)
(relative expression - HPRT corrected)
0
200
400
600
800
1000
1200
1400
TGF-β1
Fibronectin-1
MCP-1
p<0.05
p<0.05
p<0.05
p<0.05
p<0.05
Vehicle control - sham
Vehicle control - UUO
CVC 7 mg/kg/day - UUO
CVC 20 mg/kg/day - UUO
Compound 1D11
(positive control - UUO)

Figure 16

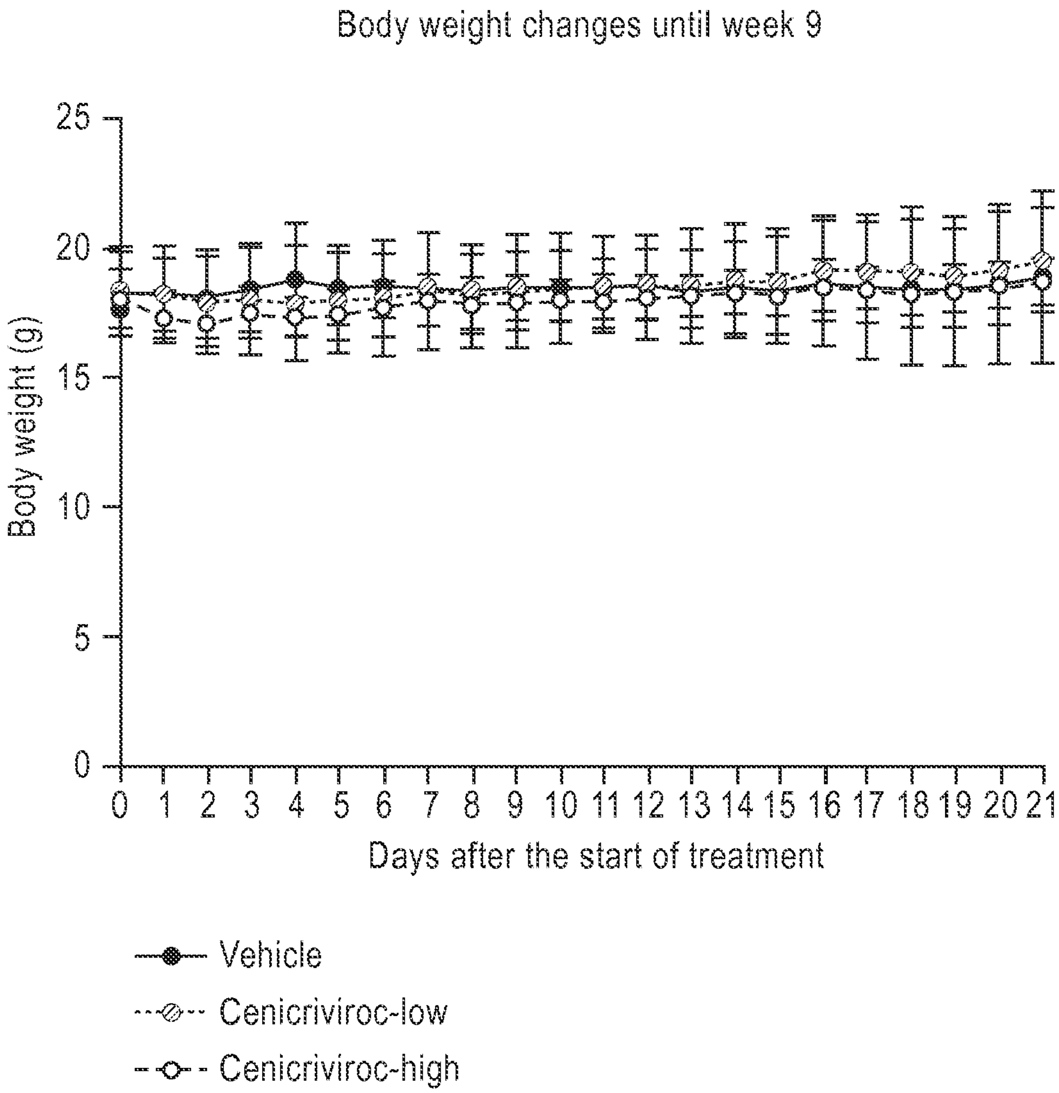
Body weight changes until week 9
Body weight (g)
25
20
15
10
5
0
0 1 2 3 4 5 6 7 8 9 10 11 12 13 14 15 16 17 18 19 20 21
Days after the start of treatment
Vehicle
Cenicriviroc-low
Cenicriviroc-high

Figure 17A

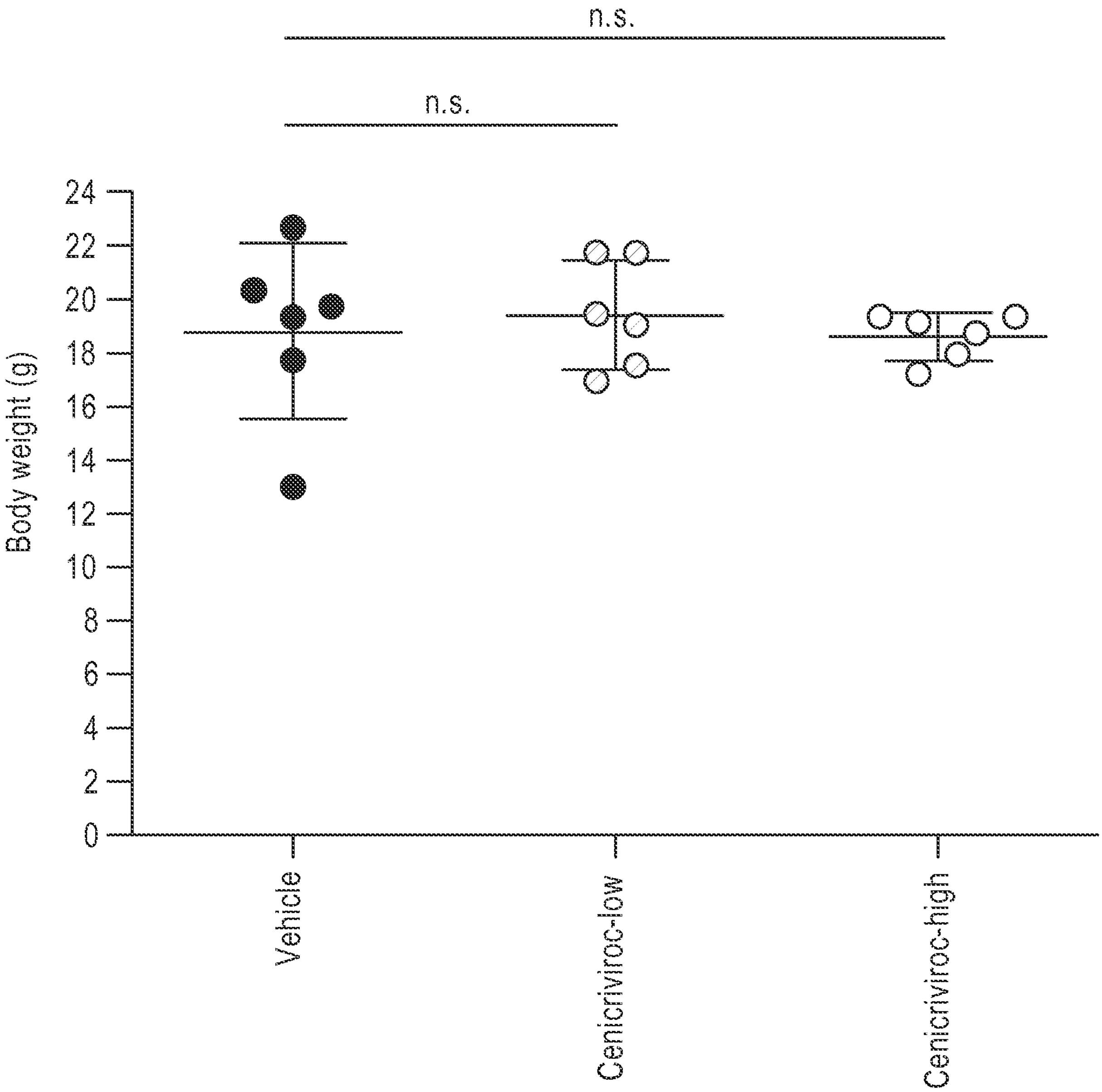
n.s.
n.s.
Body weight (g)
0
2
4
6
8
10
12
14
16
18
20
22
24
Vehicle
Cenicriviroc-low
Cenicriviroc-high

Figure 17B

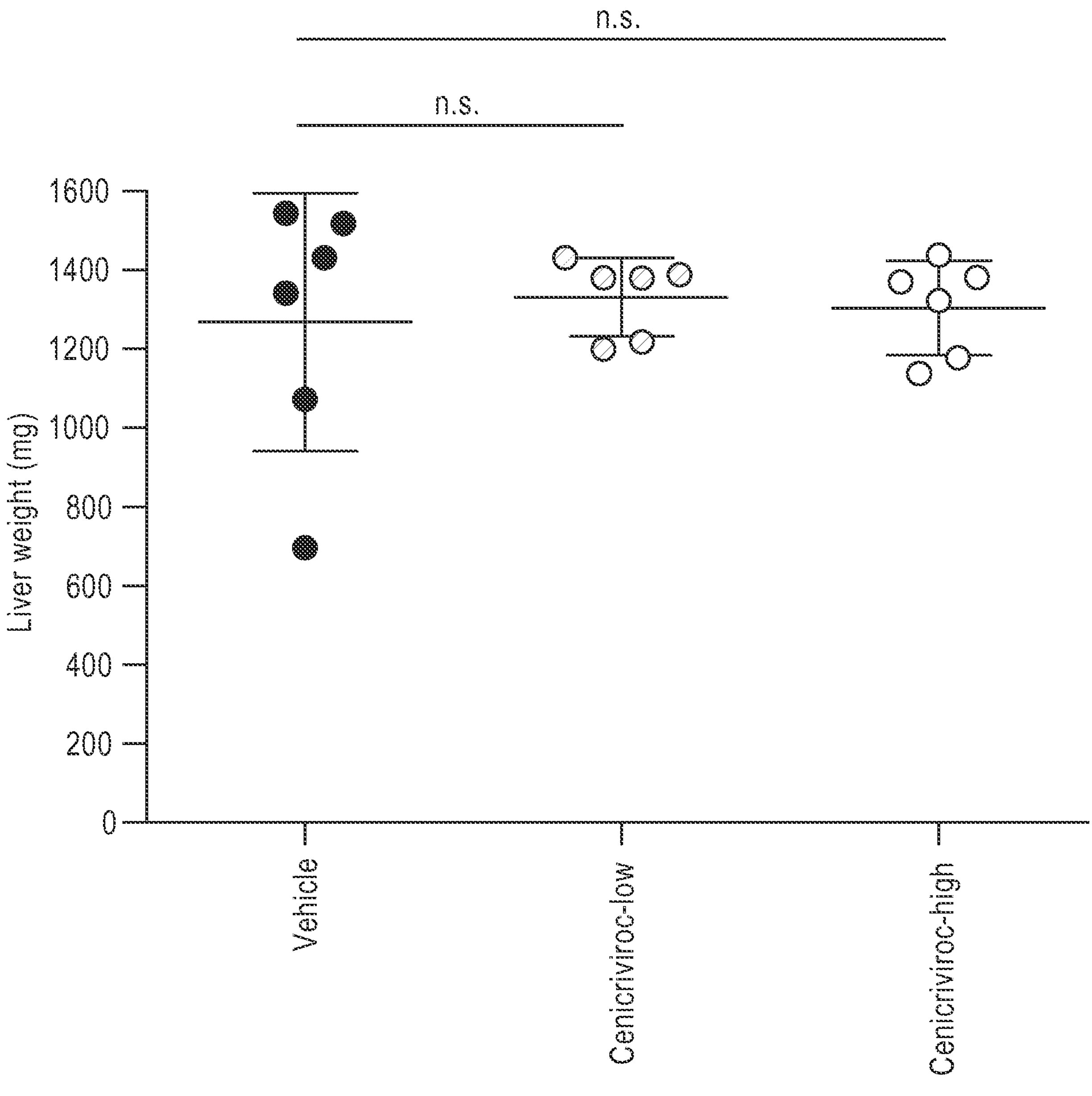
n.s.
n.s.
1600
1400
1200
1000
800
600
400
200
0
Liver weight (mg)
Vehicle
Cenicriviroc-low
Cenicriviroc-high

Figure 17C

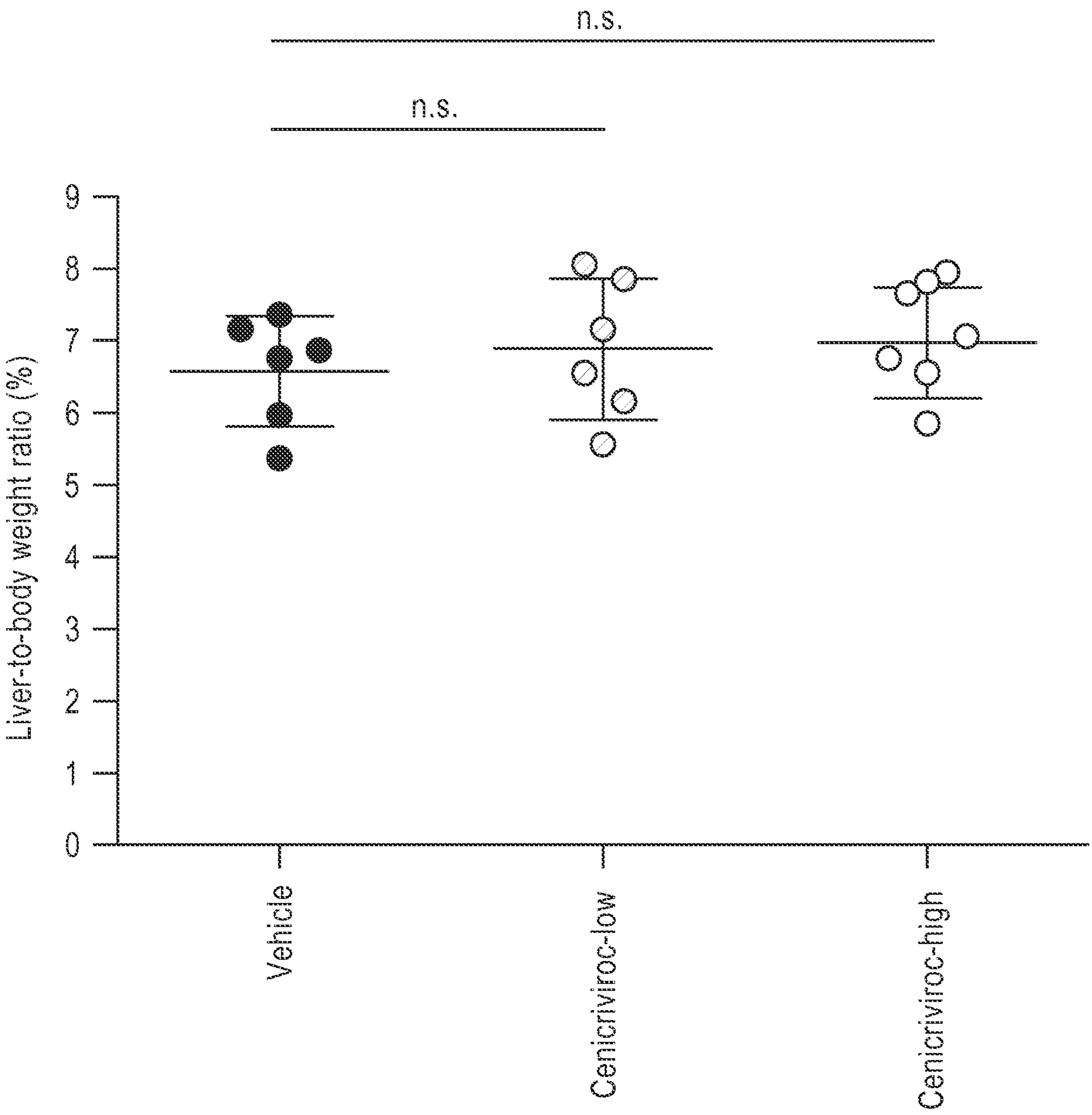
n.s.
n.s.
Liver-to-body weight ratio (%)
9
8
7
6
5
4
3
2
1
0
Vehicle
Cenicriviroc-low
Cenicriviroc-high

Figure 18

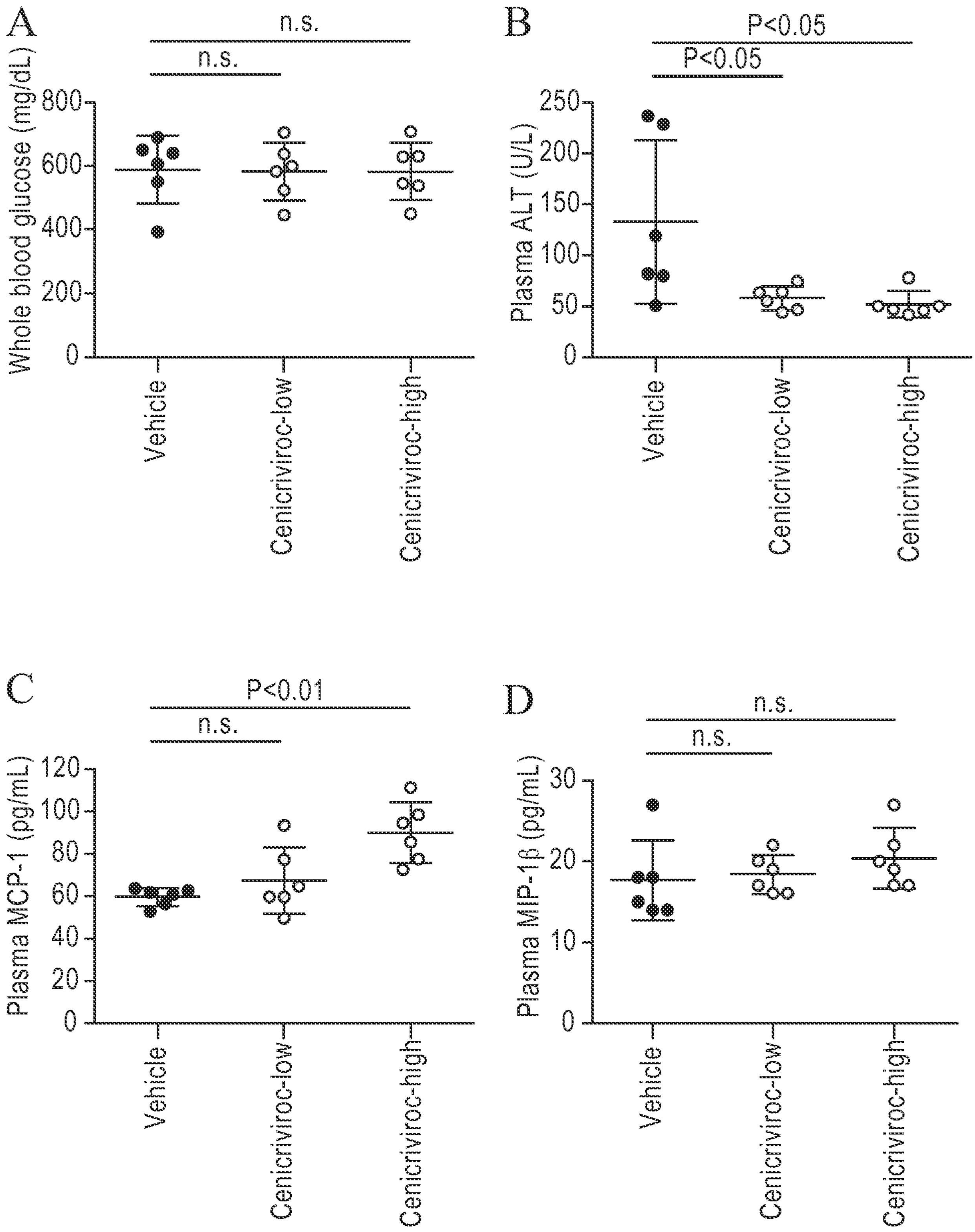
A
n.s.
n.s.
Whole blood glucose (mg/dL)
800
600
400
200
0
Vehicle
Cenicriviroc-low
Cenicriviroc-high
B
P<0.05
P<0.05
Plasma ALT (U/L)
250
200
150
100
50
0
Vehicle
Cenicriviroc-low
Cenicriviroc-high
C
P<0.01
n.s.
Plasma MCP-1 (pg/mL)
120
100
80
60
40
20
0
Vehicle
Cenicriviroc-low
Cenicriviroc-high
D
n.s.
n.s.
Plasma MIP-1β (pg/mL)
30
20
10
0
Vehicle
Cenicriviroc-low
Cenicriviroc-high

Figure 18

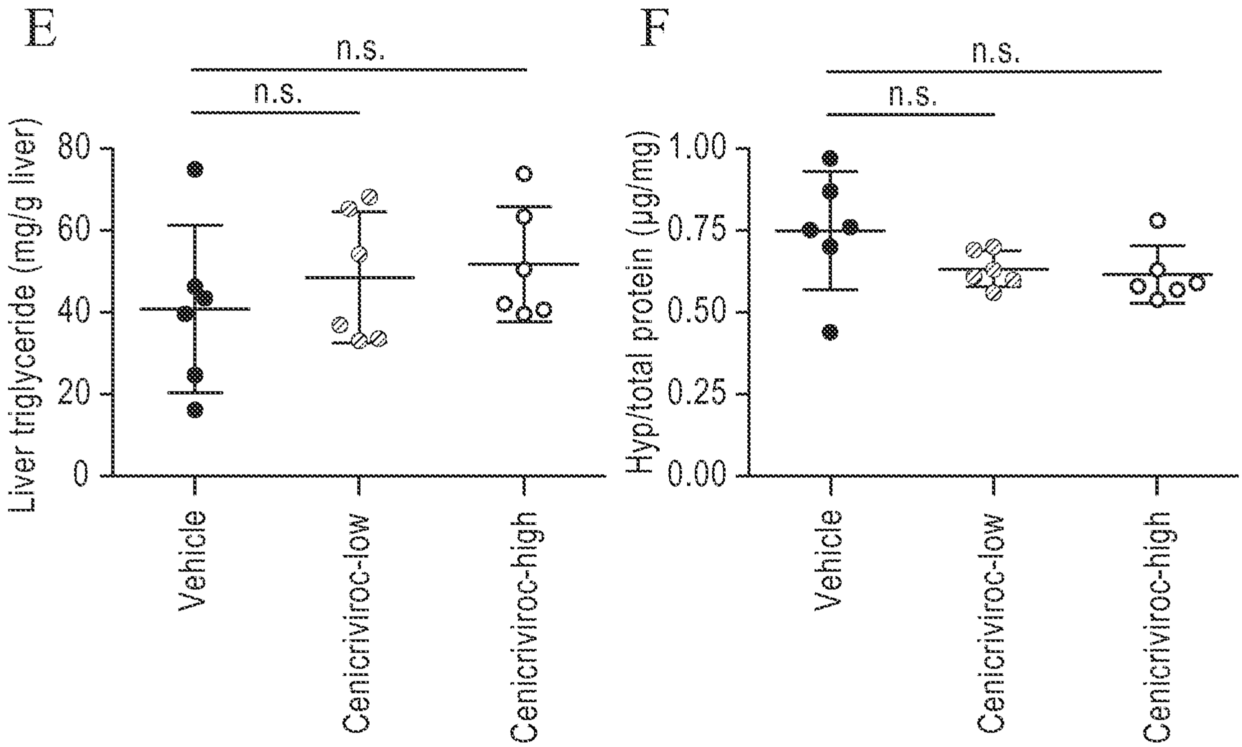
E
n.s.
n.s.
Liver triglyceride (mg/g liver)
80
60
40
20
0
Vehicle
Cenicriviroc-low
Cenicriviroc-high
F
n.s.
n.s.
Hyp/total protein (µg/mg)
1.00
0.75
0.50
0.25
0.00
Vehicle
Cenicriviroc-low
Cenicriviroc-high

Figure 19

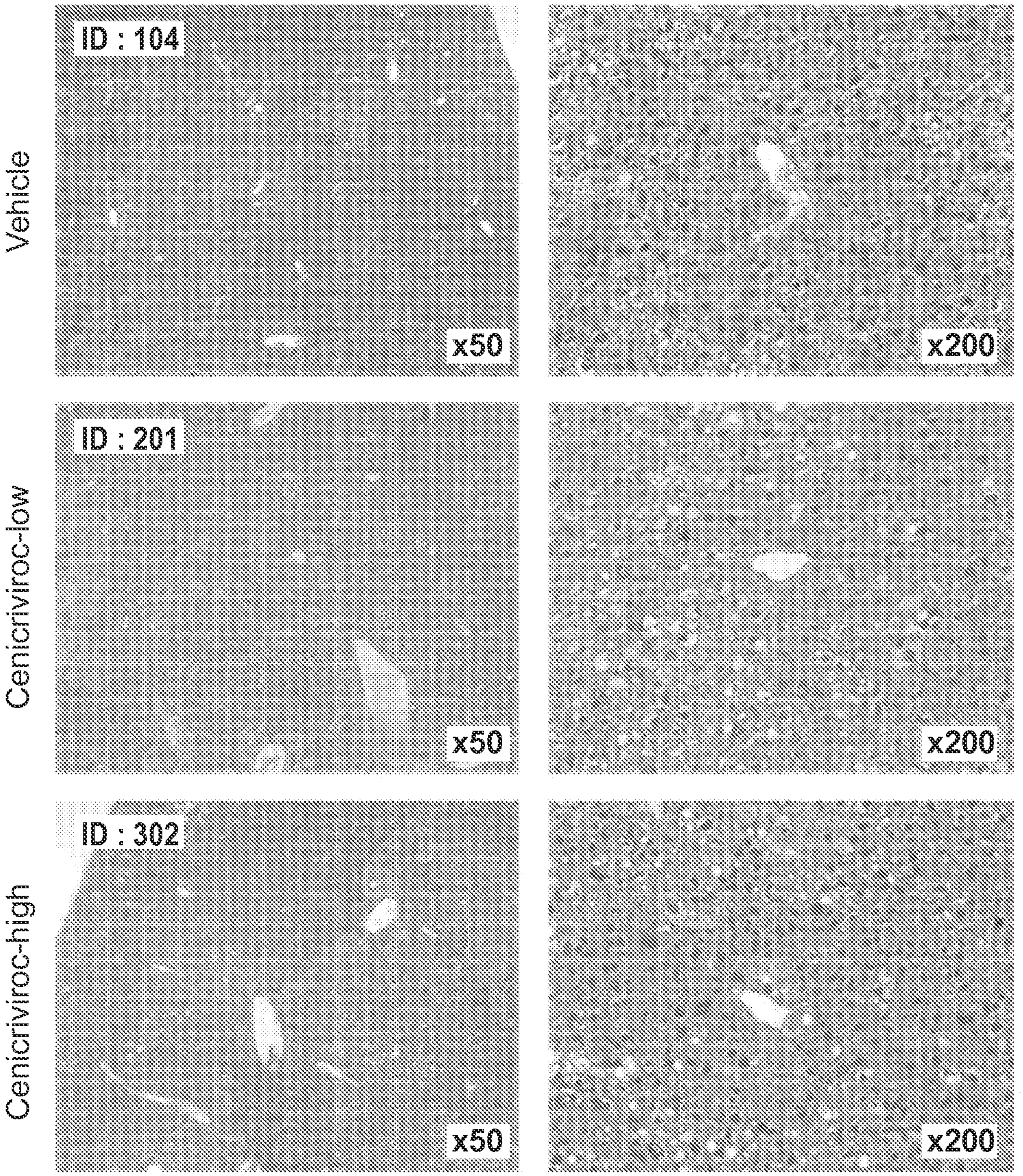
Vehicle
ID : 104
x50
x200
Cenicriviroc-low
ID : 201
x50
x200
Cenicriviroc-high
ID : 302
x50
x200

Figure 20

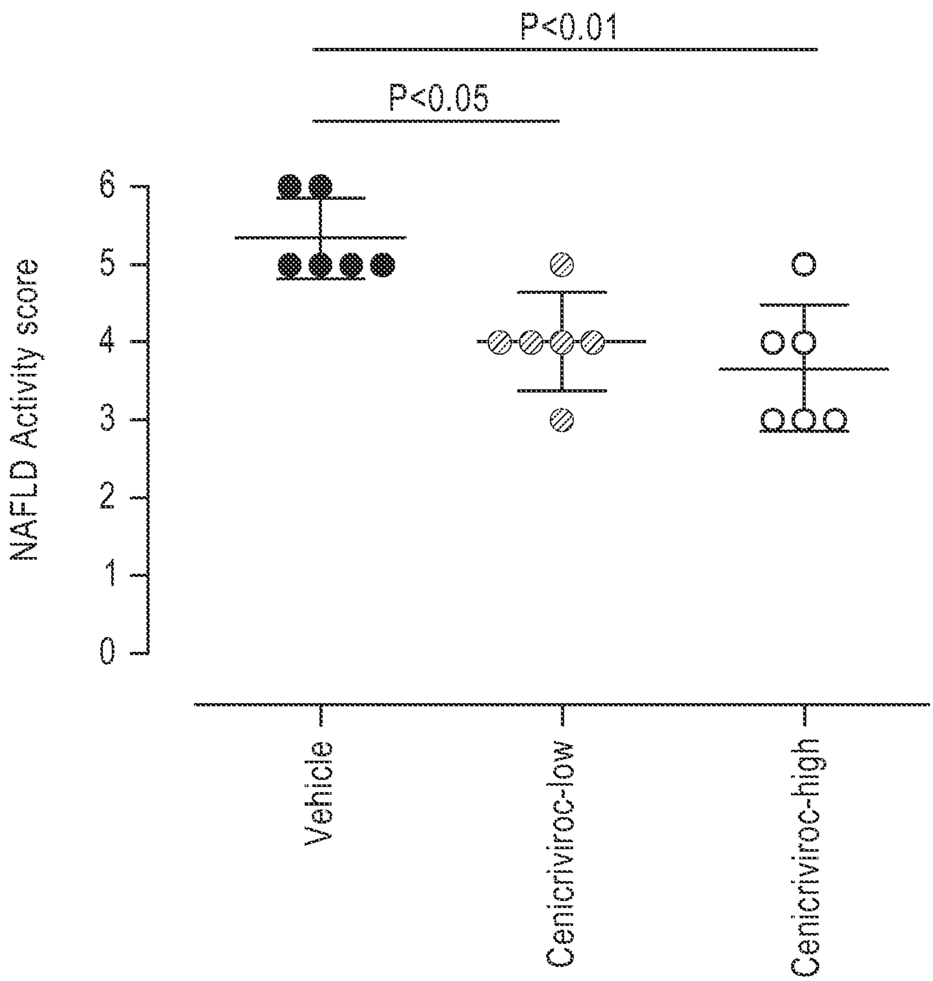
P<0.01
P<0.05
NAFLD Activity score
0
1
2
3
4
5
6
Vehicle
Cenicriviroc-low
Cenicriviroc-high

Figure 21

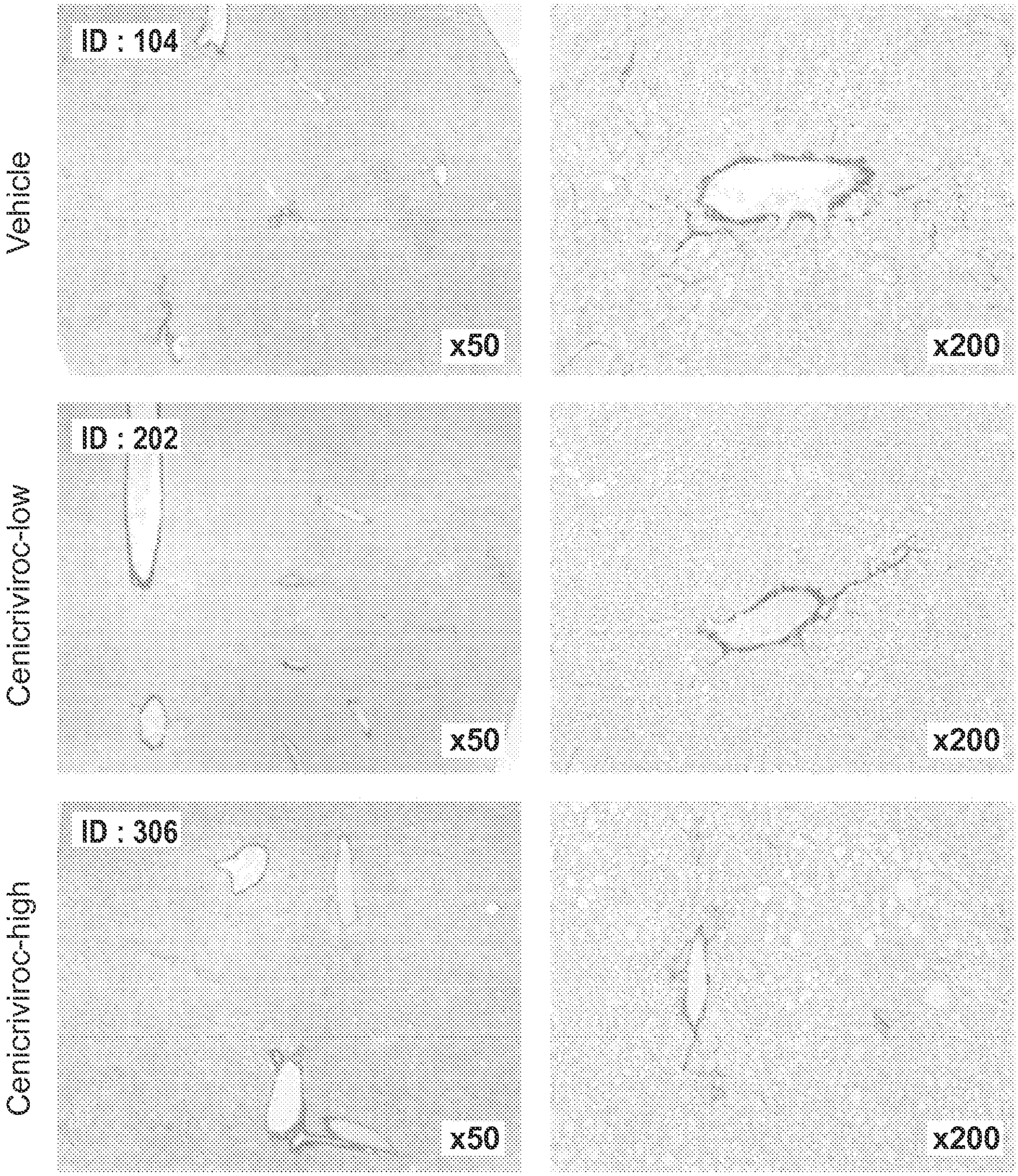
Vehicle
Cenicriviroc-low
Cenicriviroc-high
ID : 104
ID : 202
ID : 306
x50
x200
x50
x200
x50
x200

Figure 22

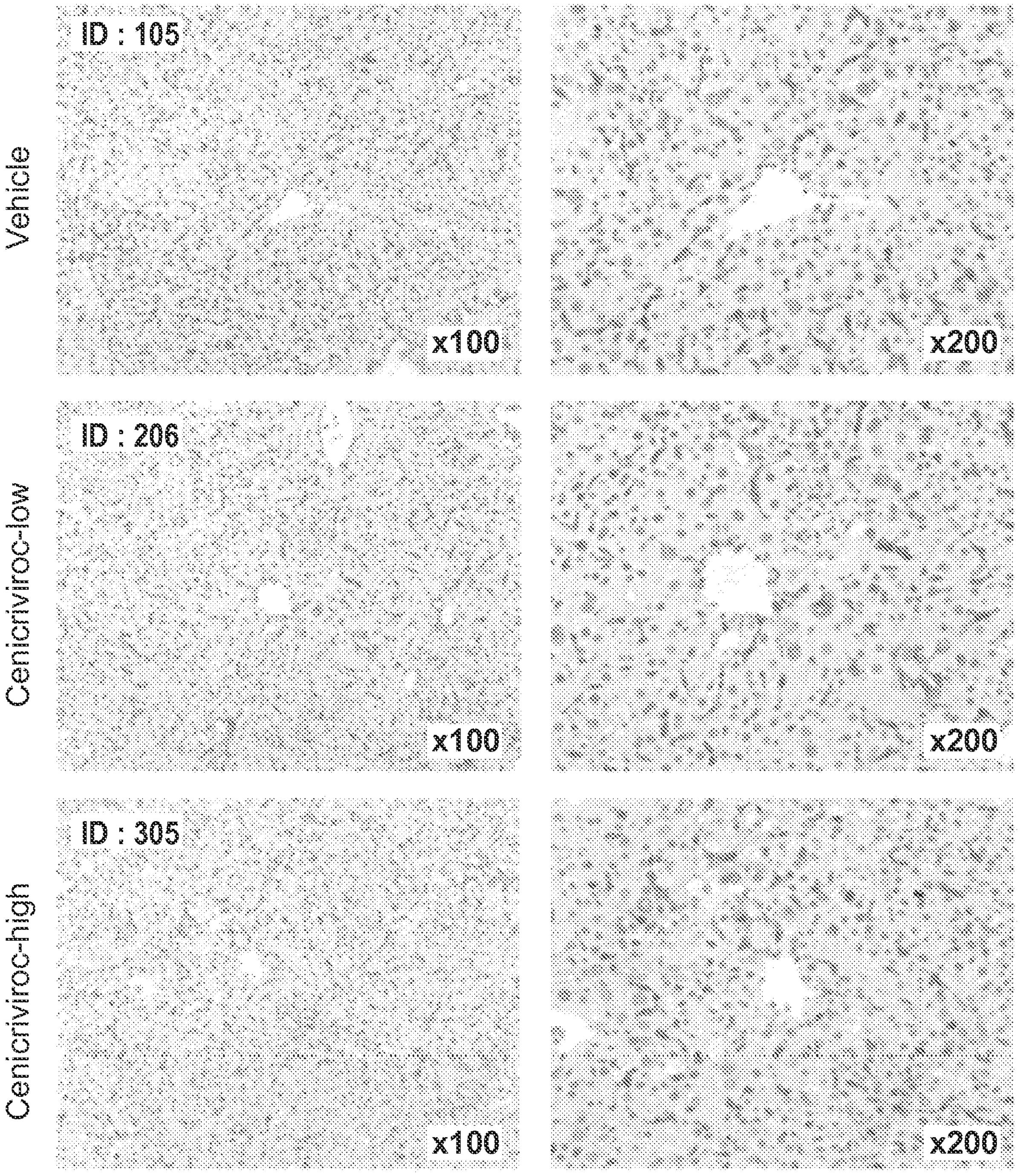
Vehicle
ID : 105
x100
x200
Cenicriviroc-low
ID : 206
x100
x200
Cenicriviroc-high
ID : 305
x100
x200

Figure 23

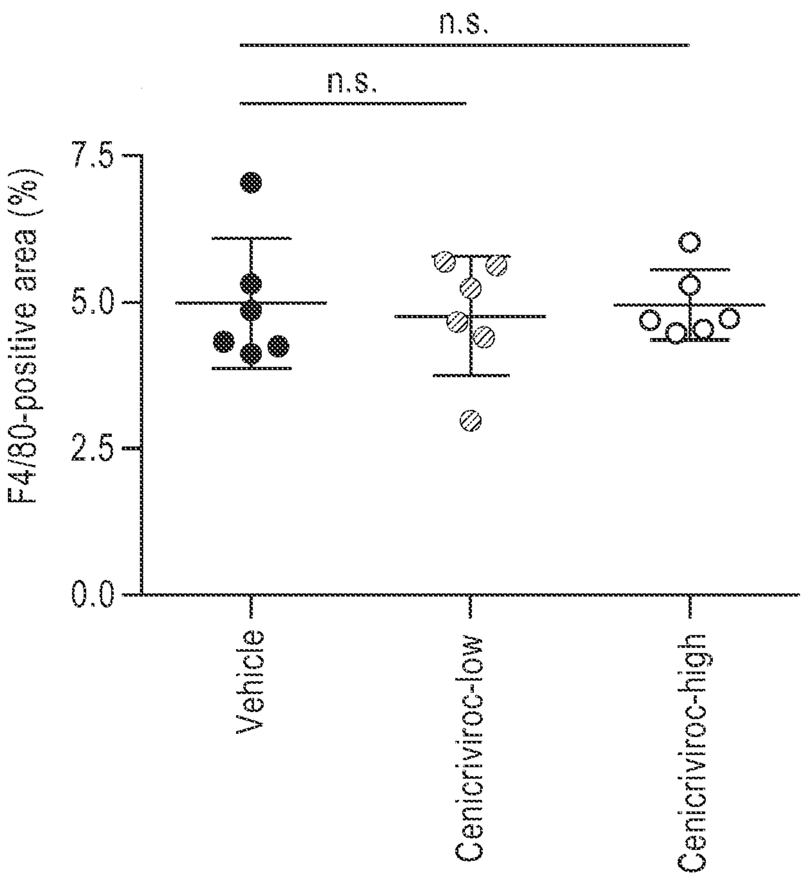
n.s.
n.s.
F4/80-positive area (%)
7.5
5.0
2.5
0.0
Vehicle
Cenicriviroc-low
Cenicriviroc-high

# Figure 24

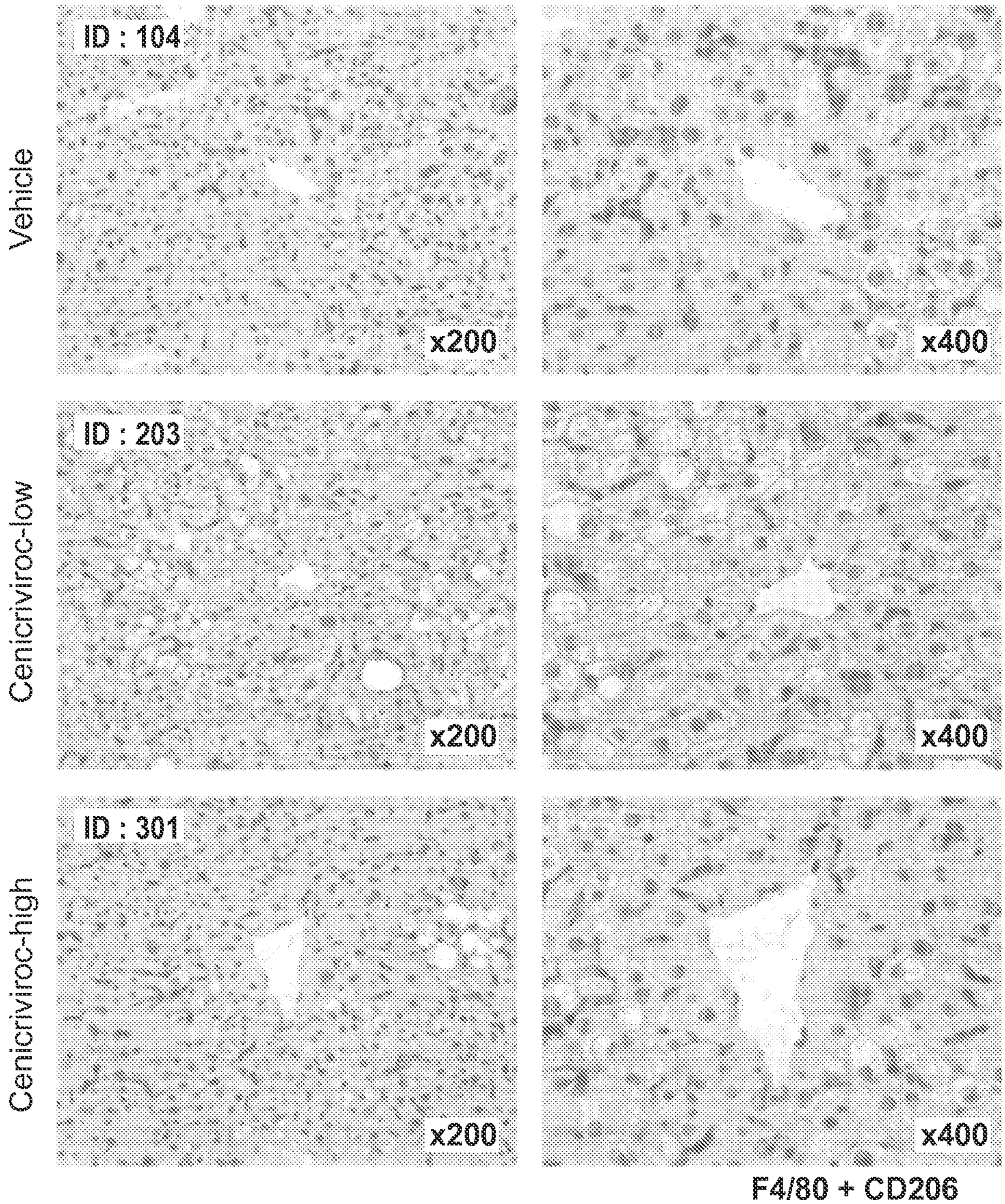
Vehicle
ID : 104
x200
x400
Cenicriviroc-low
ID : 203
x200
x400
Cenicriviroc-high
ID : 301
x200
x400
F4/80 + CD206

Figure 25

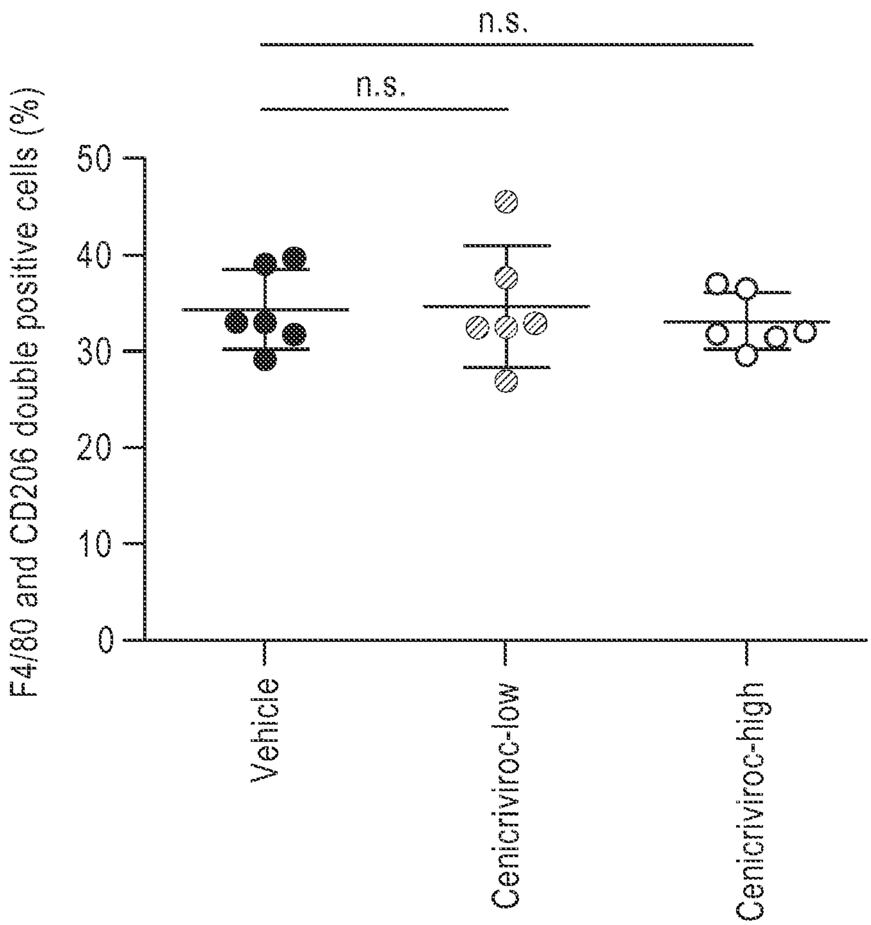
n.s.
n.s.
F4/80 and CD206 double positive cells (%)
50
40
30
20
10
0
Vehicle
Cenicriviroc-low
Cenicriviroc-high

Figure 26

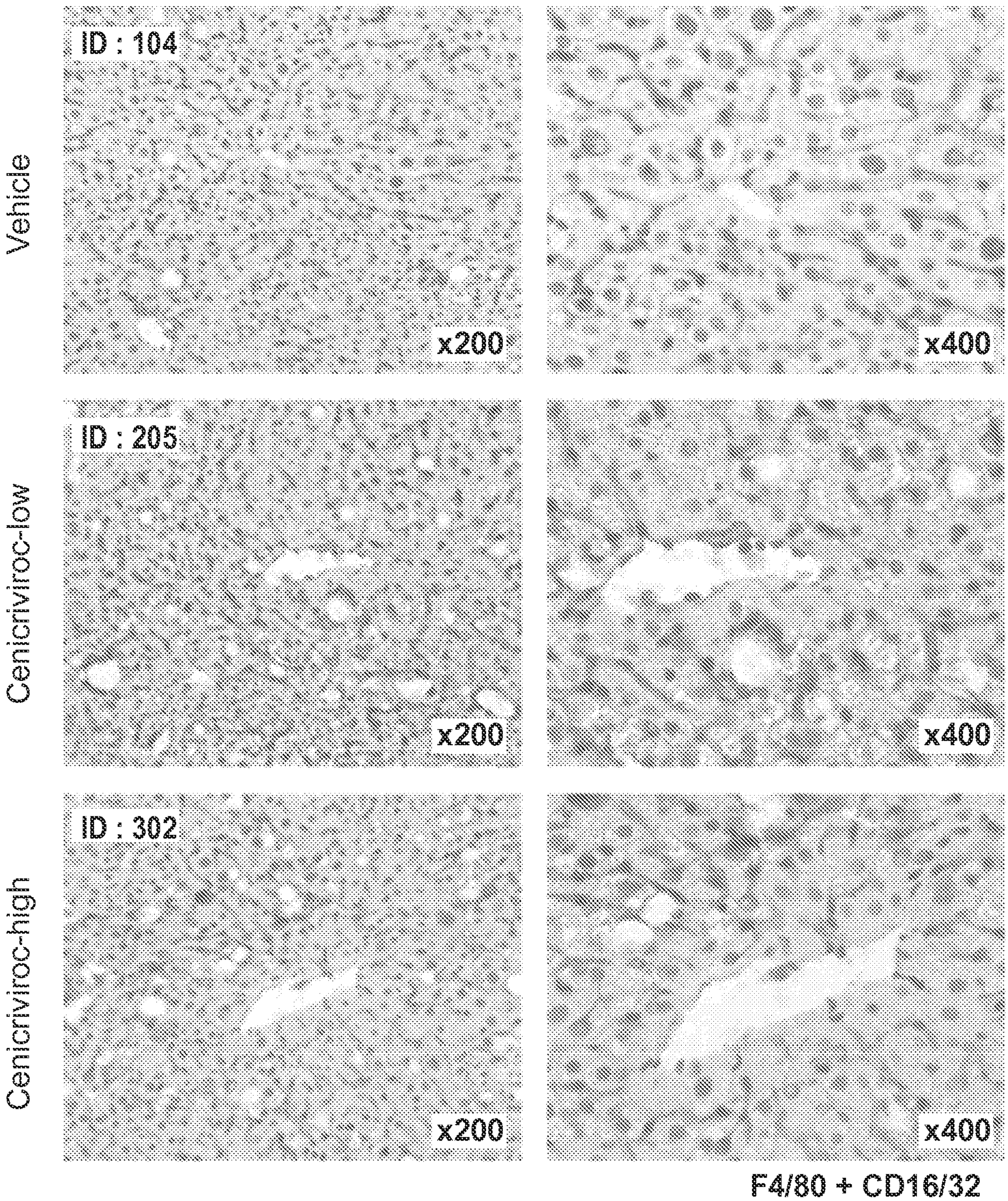
Vehicle
Cenicriviroc-low
Cenicriviroc-high
ID : 104
ID : 205
ID : 302
x200
x400
x200
x400
x200
x400
F4/80 + CD16/32

Figure 27

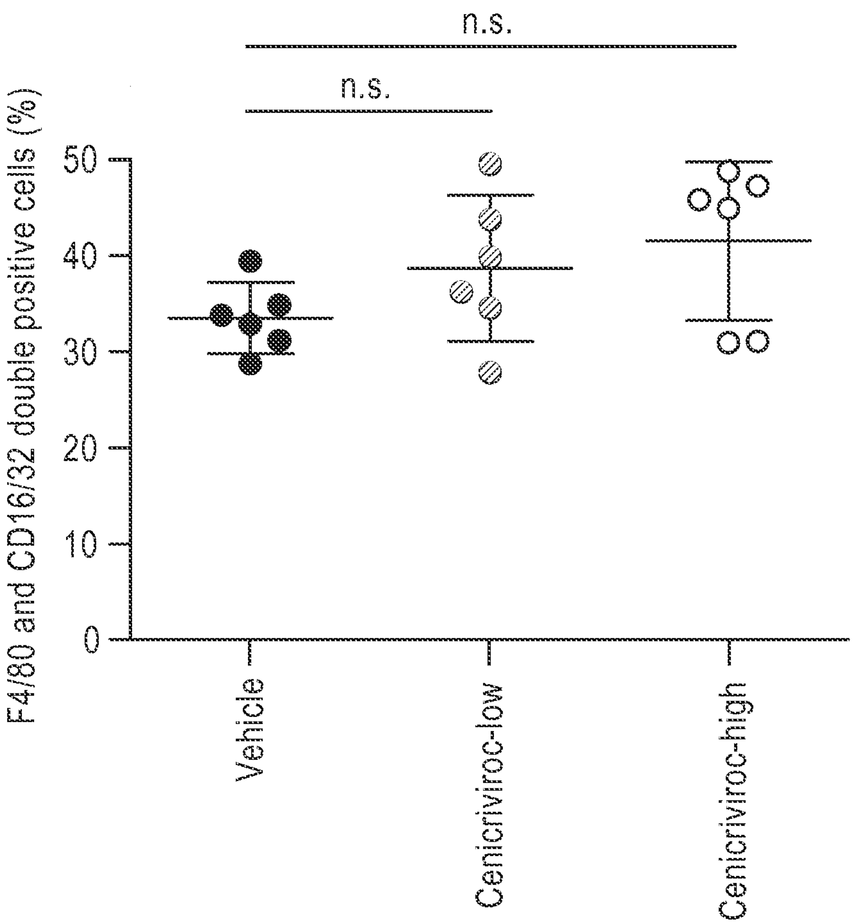
n.s.
n.s.
F4/80 and CD16/32 double positive cells (%)
50
40
30
20
10
0
Vehicle
Cenicriviroc-low
Cenicriviroc-high

Figure 28

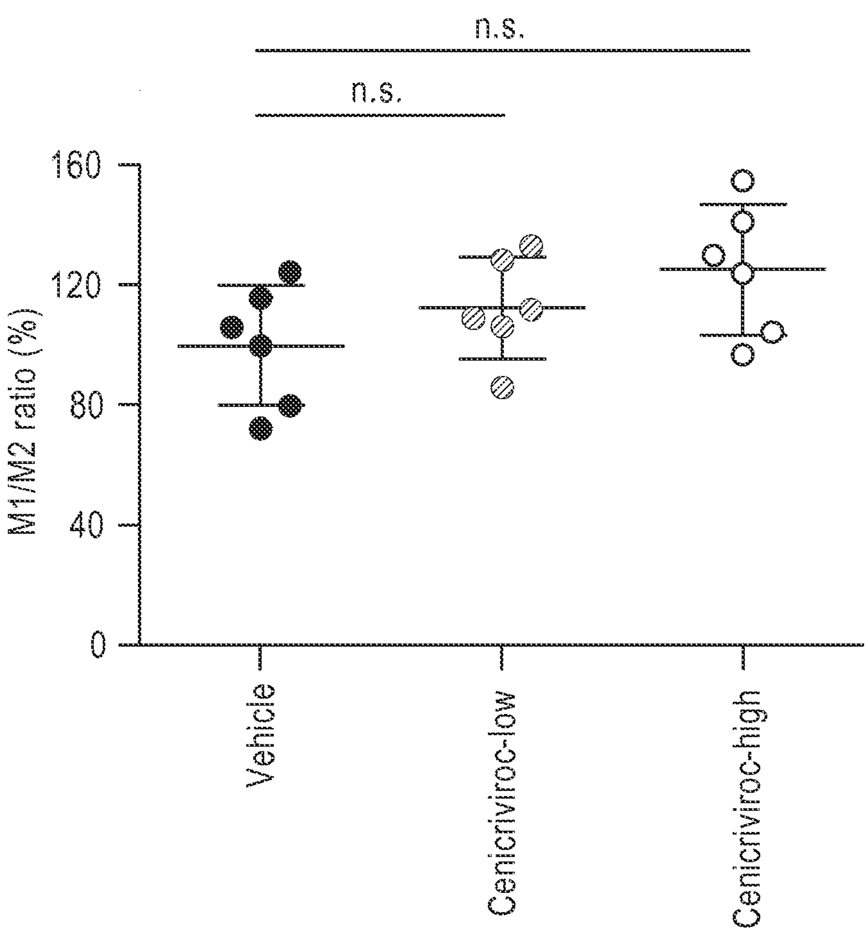
n.s.
n.s.
160
120
80
40
0
M1/M2 ratio (%)
Vehicle
Cenicriviroc-low
Cenicriviroc-high

Figure 29

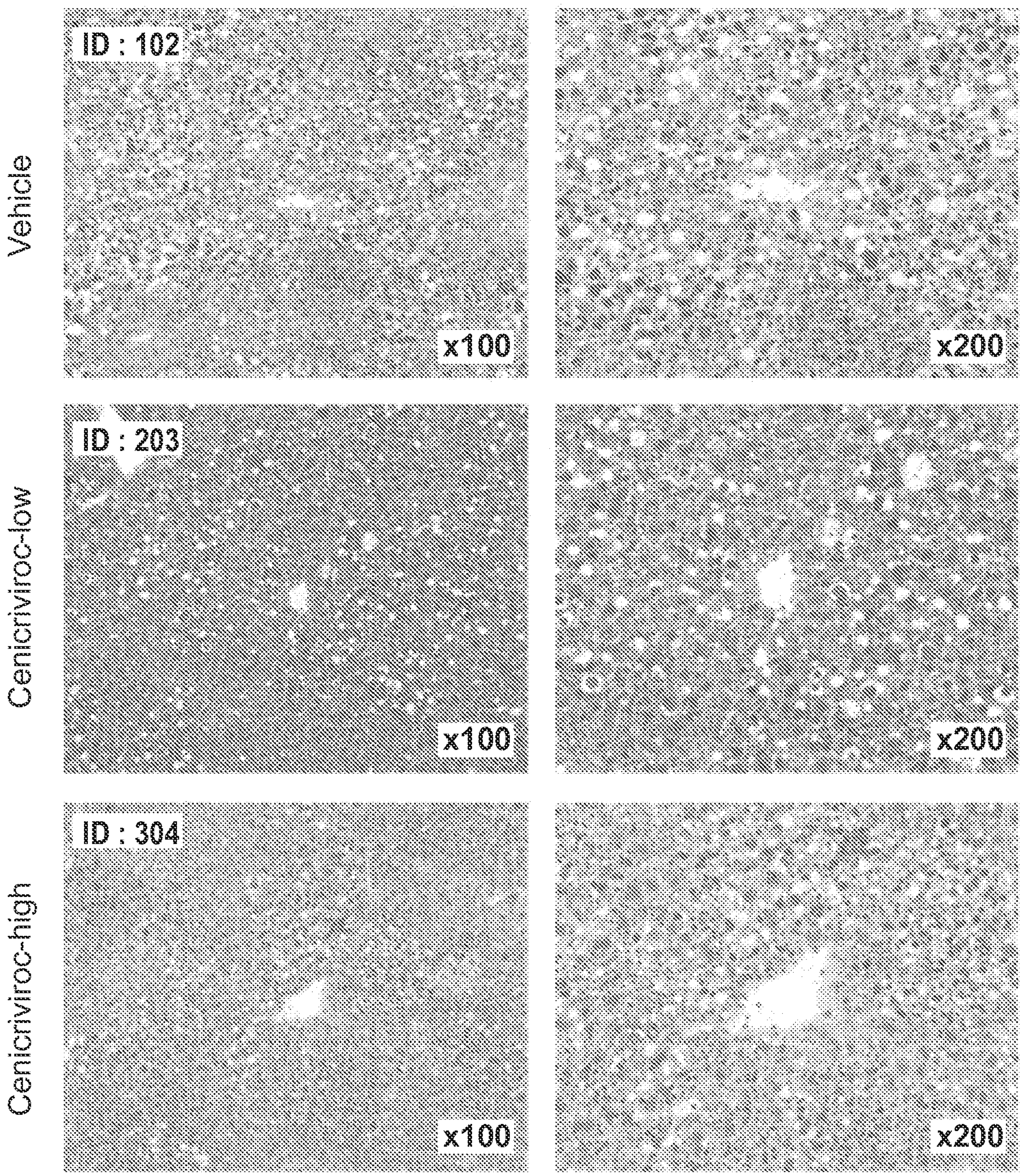
Vehicle
ID : 102
x100
x200
Cenicriviroc-low
ID : 203
x100
x200
Cenicriviroc-high
ID : 304
x100
x200

Figure 30

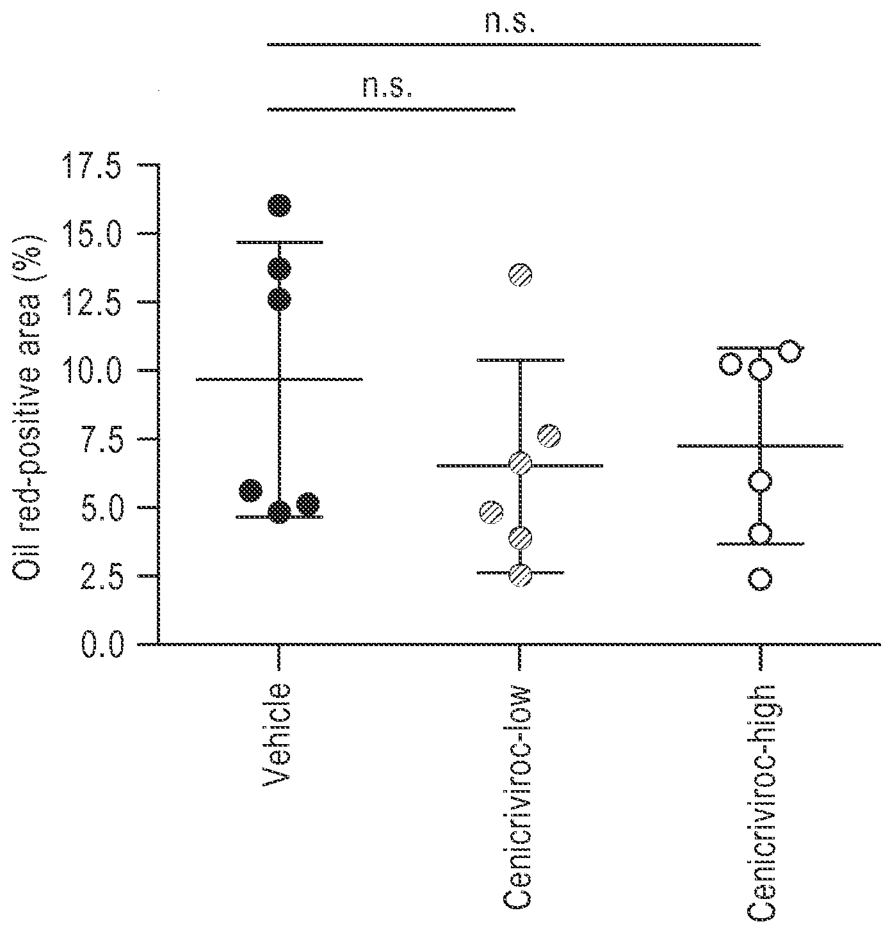
n.s.
n.s.
17.5
15.0
12.5
10.0
7.5
5.0
2.5
0.0
Oil red-positive area (%)
Vehicle
Cenicriviroc-low
Cenicriviroc-high

Figure 31

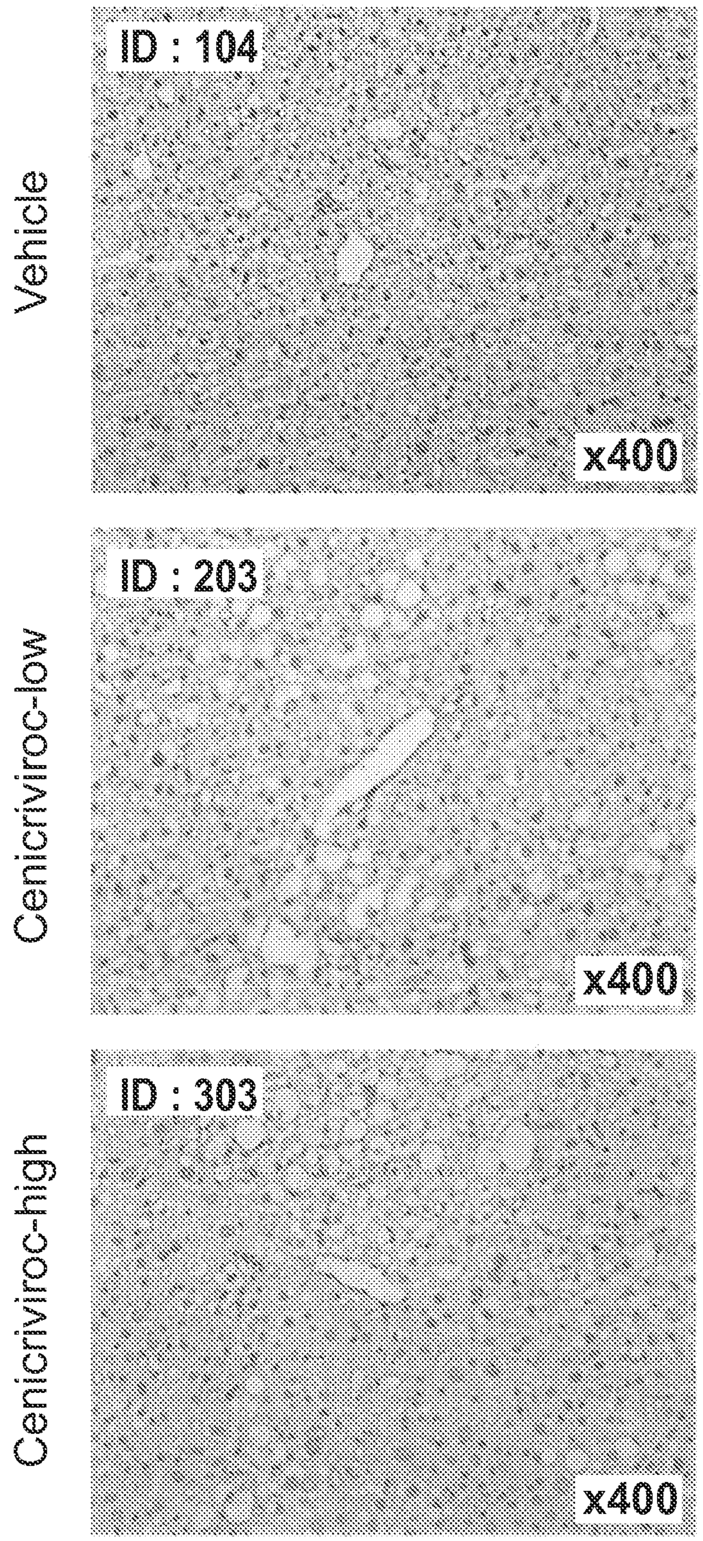
Vehicle
ID : 104
x400
Cenicriviroc-low
ID : 203
x400
Cenicriviroc-high
ID : 303
x400

Figure 32

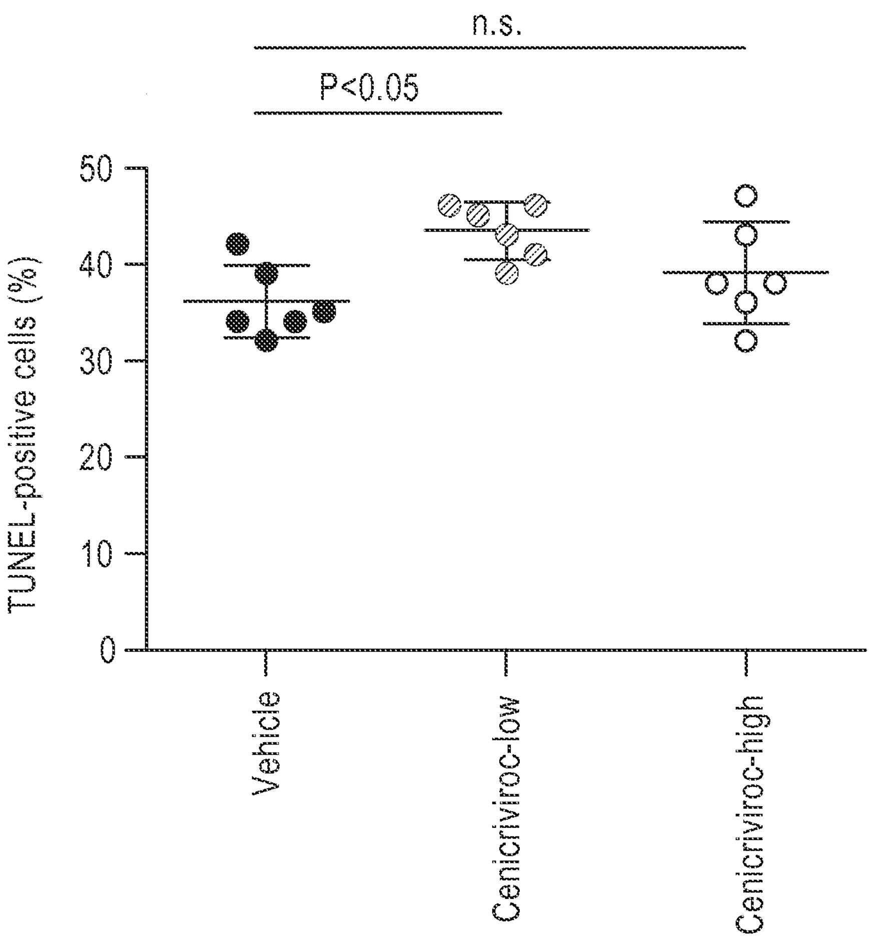
n.s.
P<0.05
TUNEL-positive cells (%)
50
40
30
20
10
0
Vehicle
Cenicriviroc-low
Cenicriviroc-high

Figure 33

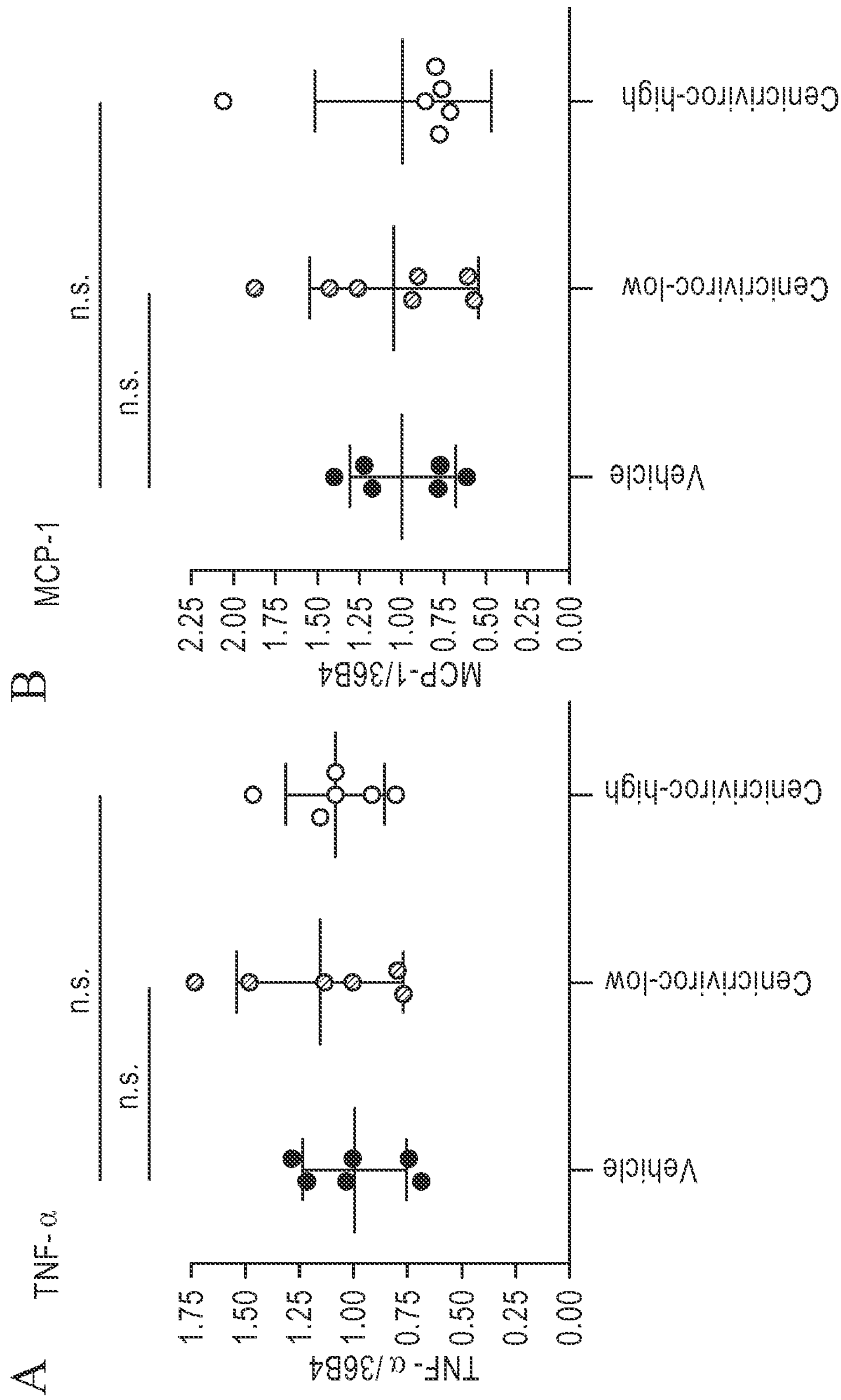
A
TNF-α
TNF-α/36B4
1.75
1.50
1.25
1.00
0.75
0.50
0.25
0.00
n.s.
n.s.
Vehicle
Cenicriviroc-low
Cenicriviroc-high
B
MCP-1
MCP-1/36B4
2.25
2.00
1.75
1.50
1.25
1.00
0.75
0.50
0.25
0.00
n.s.
n.s.
Vehicle
Cenicriviroc-low
Cenicriviroc-high

Figure 33

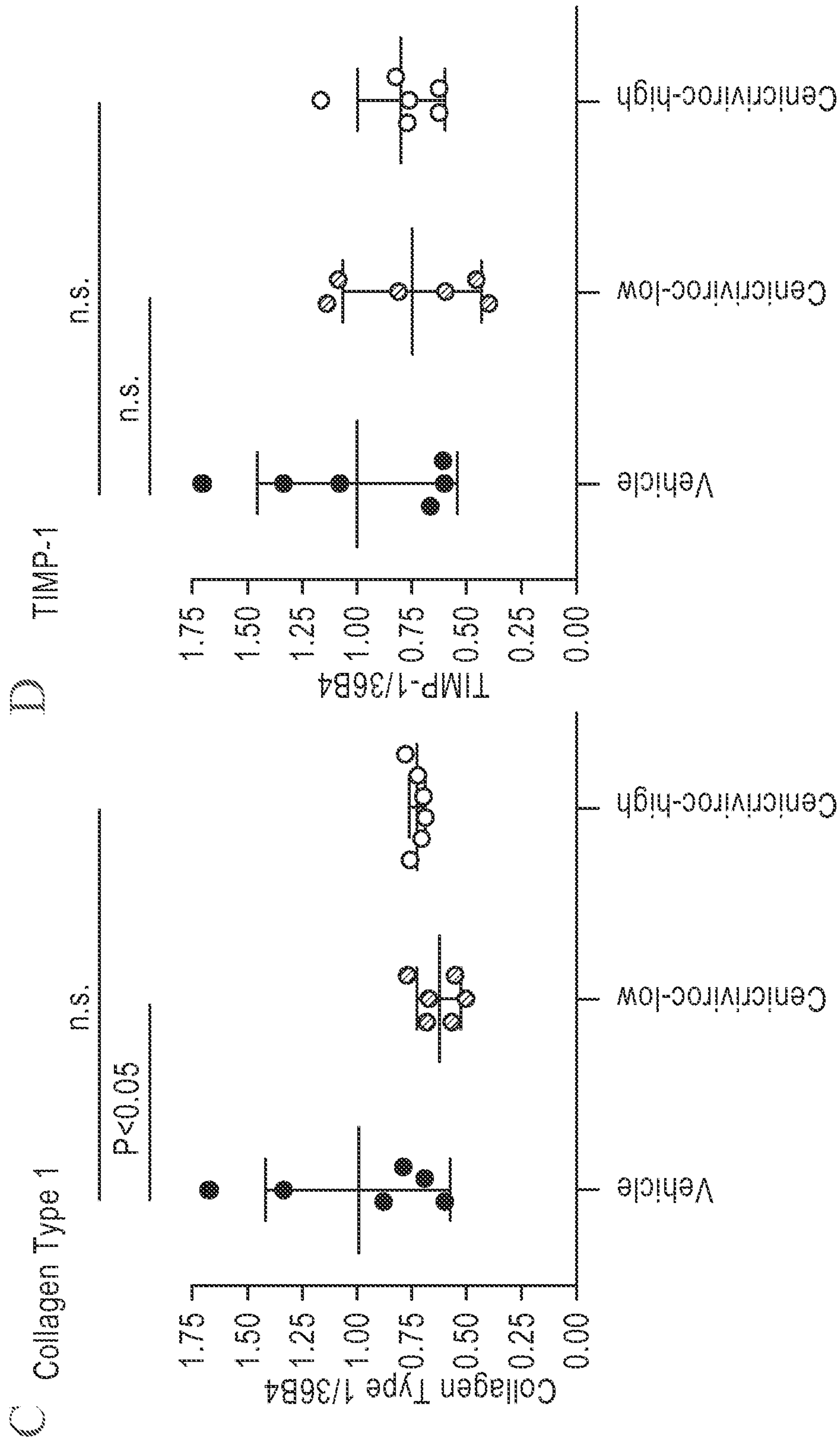
C
Collagen Type 1
Collagen Type 1/36B4
1.75
1.50
1.25
1.00
0.75
0.50
0.25
0.00
P<0.05
n.s.
Vehicle
Cenicriviroc-low
Cenicriviroc-high
D
TIMP-1
TIMP-1/36B4
1.75
1.50
1.25
1.00
0.75
0.50
0.25
0.00
n.s.
n.s.
Vehicle
Cenicriviroc-low
Cenicriviroc-high

Figure 34A Plate 1-1 : 36B4

| Well | Sample Type | Target Mark | Association Mark | Ct(SDM) | Init Qty | Qty(SDM) | Rel.Qty(SDM) |
|---|---|---|---|---|---|---|---|
| A2 | UNKN | A | 101 | 21.3 | - | 4.00E+00 | 3.67E+00 |
| A3 | UNKN | A | 101 | 21.49 | - | 3.54E+00 | 3.67E+00 |
| A4 | UNKN | A | 101 | 21.52 | - | 3.47E+00 | 3.67E+00 |
| B2 | UNKN | A | 102 | 21.37 | - | 3.82E+00 | 4.09E+00 |
| B3 | UNKN | A | 102 | 21.28 | - | 4.05E+00 | 4.09E+00 |
| B4 | UNKN | A | 102 | 21.15 | - | 4.40E+00 | 4.09E+00 |
| C2 | UNKN | A | 103 | 21.1 | - | 4.54E+00 | 4.21E+00 |
| C3 | UNKN | A | 103 | 21.31 | - | 3.97E+00 | 4.21E+00 |
| C4 | UNKN | A | 103 | 21.26 | - | 4.10E+00 | 4.21E+00 |
| D2 | UNKN | A | 104 | 21.06 | - | 4.66E+00 | 4.28E+00 |
| D3 | UNKN | A | 104 | 21.32 | - | 3.95E+00 | 4.28E+00 |
| D4 | UNKN | A | 104 | 21.21 | - | 4.23E+00 | 4.28E+00 |
| E2 | UNKN | A | 105 | 20.97 | - | 4.94E+00 | 4.86E+00 |
| E3 | UNKN | A | 105 | 21.1 | - | 4.54E+00 | 4.86E+00 |
| E4 | UNKN | A | 105 | 20.92 | - | 5.10E+00 | 4.86E+00 |
| F2 | UNKN | A | 106 | 20.9 | - | 5.16E+00 | 5.16E+00 |
| F3 | UNKN | A | 106 | 20.91 | - | 5.13E+00 | 5.16E+00 |
| F4 | UNKN | A | 106 | 20.89 | - | 5.19E+00 | 5.16E+00 |
| A5 | UNKN | A | 201 | 21.26 | - | 4.10E+00 | 4.30E+00 |
| A6 | UNKN | A | 201 | 21.11 | - | 4.51E+00 | 4.30E+00 |
| A7 | UNKN | A | 201 | 21.19 | - | 4.29E+00 | 4.30E+00 |
| B5 | UNKN | A | 202 | 20.96 | - | 4.97E+00 | 5.03E+00 |
| B6 | UNKN | A | 202 | 20.86 | - | 5.29E+00 | 5.03E+00 |
| B7 | UNKN | A | 202 | 21 | - | 4.84E+00 | 5.03E+00 |
| C5 | UNKN | A | 203 | 21.31 | - | 3.97E+00 | 4.20E+00 |
| C6 | UNKN | A | 203 | 21.02 | - | 4.78E+00 | 4.20E+00 |
| C7 | UNKN | A | 203 | 21.36 | - | 3.85E+00 | 4.20E+00 |
| D5 | UNKN | A | 204 | 21.17 | - | 4.34E+00 | 4.45E+00 |
| D6 | UNKN | A | 204 | 21.12 | - | 4.48E+00 | 4.45E+00 |
| D7 | UNKN | A | 204 | 21.11 | - | 4.51E+00 | 4.45E+00 |
| E5 | UNKN | A | 205 | 20.98 | - | 4.90E+00 | 4.77E+00 |
| E6 | UNKN | A | 205 | 21.1 | - | 4.54E+00 | 4.77E+00 |
| E7 | UNKN | A | 205 | 20.99 | - | 4.87E+00 | 4.77E+00 |
| F5 | UNKN | A | 206 | 21.11 | - | 4.51E+00 | 4.53E+00 |
| F6 | UNKN | A | 206 | 21.01 | - | 4.81E+00 | 4.53E+00 |
| F7 | UNKN | A | 206 | 21.2 | - | 4.26E+00 | 4.53E+00 |
| A8 | UNKN | A | 301 | 21.1 | - | 4.54E+00 | 4.49E+00 |
| A9 | UNKN | A | 301 | 21.1 | - | 4.54E+00 | 4.49E+00 |
| A10 | UNKN | A | 301 | 21.16 | - | 4.37E+00 | 4.49E+00 |
| B8 | UNKN | A | 302 | 21.34 | - | 3.90E+00 | 3.97E+00 |
| B9 | UNKN | A | 302 | 21.25 | - | 4.13E+00 | 3.97E+00 |
| B10 | UNKN | A | 302 | 21.34 | - | 3.90E+00 | 3.97E+00 |
| C8 | UNKN | A | 303 | 21.03 | - | 4.75E+00 | 4.79E+00 |
| C9 | UNKN | A | 303 | 20.97 | - | 4.94E+00 | 4.79E+00 |
| C10 | UNKN | A | 303 | 21.05 | - | 4.69E+00 | 4.79E+00 |
| D8 | UNKN | A | 304 | 20.87 | - | 5.26E+00 | 5.10E+00 |
| D9 | UNKN | A | 304 | 20.94 | - | 5.03E+00 | 5.10E+00 |
| D10 | UNKN | A | 304 | 20.95 | - | 5.00E+00 | 5.10E+00 |
| E8 | UNKN | A | 305 | 21.02 | - | 4.78E+00 | 4.82E+00 |
| E9 | UNKN | A | 305 | 20.98 | - | 4.90E+00 | 4.82E+00 |
| E10 | UNKN | A | 305 | 21.02 | - | 4.78E+00 | 4.82E+00 |
| F8 | UNKN | A | 306 | 21.17 | - | 4.34E+00 | 4.51E+00 |
| F9 | UNKN | A | 306 | 21.04 | - | 4.72E+00 | 4.51E+00 |
| F10 | UNKN | A | 306 | 21.13 | - | 4.46E+00 | 4.51E+00 |
| B11 | STD | A | | 19.8 | 1.00E+01 | 1.04E+01 | - |
| C11 | STD | A | | 19.92 | 1.00E+01 | 9.65E+00 | - |
| D11 | STD | A | | 19.8 | 1.00E+01 | 1.04E+01 | - |
| E11 | STD | A | | 22.06 | 2.50E+00 | 2.46E+00 | - |
| F11 | STD | A | | 22.1 | 2.50E+00 | 2.40E+00 | - |
| G11 | STD | A | | 22.02 | 2.50E+00 | 2.52E+00 | - |
| B12 | STD | A | | 24.11 | 6.25E-01 | 6.65E-01 | - |
| C12 | STD | A | | 24.29 | 6.25E-01 | 5.92E-01 | - |
| D12 | STD | A | | 24.33 | 6.25E-01 | 5.77E-01 | - |
| E12 | STD | A | | 26.3 | 1.56E-01 | 1.64E-01 | - |
| F12 | STD | A | | 26.11 | 1.56E-01 | 1.85E-01 | - |
| G12 | STD | A | | 26.63 | 1.56E-01 | 1.33E-01 | - |

# Figure 34B Plate 1-2 : TNF-α

| Well | Sample Type | Target Mark | Association Mark | Ct(SDM) | Init Qty | Qty(SDM) | Rel.Qty(SDM) |
|---|---|---|---|---|---|---|---|
| A2 | UNKN | A | 101 | 30.72 | - | 4.40E+00 | 4.47E+00 |
| A3 | UNKN | A | 101 | 30.65 | - | 4.59E+00 | 4.47E+00 |
| A4 | UNKN | A | 101 | 30.71 | - | 4.42E+00 | 4.47E+00 |
| B2 | UNKN | A | 102 | 30.44 | - | 5.21E+00 | 5.26E+00 |
| B3 | UNKN | A | 102 | 30.44 | - | 5.21E+00 | 5.26E+00 |
| B4 | UNKN | A | 102 | 30.39 | - | 5.37E+00 | 5.26E+00 |
| C2 | UNKN | A | 103 | 31.19 | - | 3.31E+00 | 2.88E+00 |
| C3 | UNKN | A | 103 | 31.53 | - | 2.69E+00 | 2.88E+00 |
| C4 | UNKN | A | 103 | 31.56 | - | 2.65E+00 | 2.88E+00 |
| D2 | UNKN | A | 104 | 30.9 | - | 3.94E+00 | 4.40E+00 |
| D3 | UNKN | A | 104 | 30.54 | - | 4.90E+00 | 4.40E+00 |
| D4 | UNKN | A | 104 | 30.73 | - | 4.37E+00 | 4.40E+00 |
| E2 | UNKN | A | 105 | 30.91 | - | 3.92E+00 | 3.62E+00 |
| E3 | UNKN | A | 105 | 31.05 | - | 3.60E+00 | 3.62E+00 |
| E4 | UNKN | A | 105 | 31.18 | - | 3.33E+00 | 3.62E+00 |
| F2 | UNKN | A | 106 | 30.49 | - | 5.05E+00 | 5.16E+00 |
| F3 | UNKN | A | 106 | 30.59 | - | 4.76E+00 | 5.16E+00 |
| F4 | UNKN | A | 106 | 30.3 | - | 5.67E+00 | 5.16E+00 |
| A5 | UNKN | A | 201 | 30.51 | - | 4.99E+00 | 4.86E+00 |
| A6 | UNKN | A | 201 | 30.69 | - | 4.48E+00 | 4.86E+00 |
| A7 | UNKN | A | 201 | 30.47 | - | 5.11E+00 | 4.86E+00 |
| B5 | UNKN | A | 202 | 30.31 | - | 5.63E+00 | 5.06E+00 |
| B6 | UNKN | A | 202 | 30.62 | - | 4.67E+00 | 5.06E+00 |
| B7 | UNKN | A | 202 | 30.55 | - | 4.87E+00 | 5.06E+00 |
| C5 | UNKN | A | 203 | 31.05 | - | 3.60E+00 | 3.23E+00 |
| C6 | UNKN | A | 203 | 31.18 | - | 3.33E+00 | 3.23E+00 |
| C7 | UNKN | A | 203 | 31.5 | - | 2.74E+00 | 3.23E+00 |
| D5 | UNKN | A | 204 | 31.17 | - | 3.35E+00 | 3.55E+00 |
| D6 | UNKN | A | 204 | 31.01 | - | 3.69E+00 | 3.55E+00 |
| D7 | UNKN | A | 204 | 31.04 | - | 3.62E+00 | 3.55E+00 |
| E5 | UNKN | A | 205 | 30.15 | - | 6.20E+00 | 7.06E+00 |
| E6 | UNKN | A | 205 | 29.78 | - | 7.76E+00 | 7.06E+00 |
| E7 | UNKN | A | 205 | 29.9 | - | 7.22E+00 | 7.06E+00 |
| F5 | UNKN | A | 206 | 29.9 | - | 7.22E+00 | 7.82E+00 |
| F6 | UNKN | A | 206 | 29.79 | - | 7.71E+00 | 7.82E+00 |
| F7 | UNKN | A | 206 | 29.62 | - | 8.55E+00 | 7.82E+00 |
| A8 | UNKN | A | 301 | 30.59 | - | 4.76E+00 | 5.19E+00 |
| A9 | UNKN | A | 301 | 30.47 | - | 5.11E+00 | 5.19E+00 |
| A10 | UNKN | A | 301 | 30.29 | - | 5.70E+00 | 5.19E+00 |
| B8 | UNKN | A | 302 | 30.72 | - | 4.40E+00 | 4.30E+00 |
| B9 | UNKN | A | 302 | 30.6 | - | 4.73E+00 | 4.30E+00 |
| B10 | UNKN | A | 302 | 30.97 | - | 3.78E+00 | 4.30E+00 |
| C8 | UNKN | A | 303 | 29.8 | - | 7.67E+00 | 7.02E+00 |
| C9 | UNKN | A | 303 | 30.04 | - | 6.63E+00 | 7.02E+00 |
| C10 | UNKN | A | 303 | 30.01 | - | 6.75E+00 | 7.02E+00 |
| D8 | UNKN | A | 304 | 30.93 | - | 3.87E+00 | 4.10E+00 |
| D9 | UNKN | A | 304 | 30.86 | - | 4.04E+00 | 4.10E+00 |
| D10 | UNKN | A | 304 | 30.72 | - | 4.40E+00 | 4.10E+00 |
| E8 | UNKN | A | 305 | 30.58 | - | 4.78E+00 | 4.40E+00 |
| E9 | UNKN | A | 305 | 30.75 | - | 4.32E+00 | 4.40E+00 |
| E10 | UNKN | A | 305 | 30.84 | - | 4.09E+00 | 4.40E+00 |
| F8 | UNKN | A | 306 | 30.59 | - | 4.76E+00 | 4.89E+00 |
| F9 | UNKN | A | 306 | 30.46 | - | 5.14E+00 | 4.89E+00 |
| F10 | UNKN | A | 306 | 30.59 | - | 4.76E+00 | 4.89E+00 |
| B11 | STD | A | | 29.33 | 1.00E+01 | 1.02E+01 | - |
| C11 | STD | A | | 29.36 | 1.00E+01 | 1.00E+01 | - |
| D11 | STD | A | | 29.44 | 1.00E+01 | 9.53E+00 | - |
| E11 | STD | A | | 31.59 | 2.50E+00 | 2.60E+00 | - |
| F11 | STD | A | | 31.8 | 2.50E+00 | 2.29E+00 | - |
| G11 | STD | A | | 31.75 | 2.50E+00 | 2.36E+00 | - |
| B12 | STD | A | | 33.59 | 6.25E-01 | 7.76E-01 | - |
| C12 | STD | A | | 34.07 | 6.25E-01 | 5.81E-01 | - |
| D12 | STD | A | | 33.71 | 6.25E-01 | 7.22E-01 | - |
| F12 | STD | A | | 36.38 | 1.56E-01 | 1.44E-01 | - |
| H11 | STD | A | | 36.37 | 1.56E-01 | 1.45E-01 | - |

Figure 34C Plate 1-3 : TIMP-1

| Well | Sample Type | Target Mark | Association Mark | Ct(SDM) | Init Qty | Qty(SDM) | Rel.Qty(SDM) |
|---|---|---|---|---|---|---|---|
| A2 | UNKN | A | 101 | 29.74 | - | 5.21E+00 | 5.14E+00 |
| A3 | UNKN | A | 101 | 29.77 | - | 5.12E+00 | 5.14E+00 |
| A4 | UNKN | A | 101 | 29.78 | - | 5.09E+00 | 5.14E+00 |
| B2 | UNKN | A | 102 | 29.14 | - | 7.52E+00 | 7.34E+00 |
| B3 | UNKN | A | 102 | 29.25 | - | 7.03E+00 | 7.34E+00 |
| B4 | UNKN | A | 102 | 29.15 | - | 7.48E+00 | 7.34E+00 |
| C2 | UNKN | A | 103 | 30.88 | - | 2.60E+00 | 2.65E+00 |
| C3 | UNKN | A | 103 | 30.78 | - | 2.76E+00 | 2.65E+00 |
| C4 | UNKN | A | 103 | 30.89 | - | 2.58E+00 | 2.65E+00 |
| D2 | UNKN | A | 104 | 30.58 | - | 3.12E+00 | 2.73E+00 |
| D3 | UNKN | A | 104 | 30.8 | - | 2.73E+00 | 2.73E+00 |
| D4 | UNKN | A | 104 | 31.05 | - | 2.34E+00 | 2.73E+00 |
| E2 | UNKN | A | 105 | 30.56 | - | 3.16E+00 | 3.38E+00 |
| E3 | UNKN | A | 105 | 30.52 | - | 3.24E+00 | 3.38E+00 |
| E4 | UNKN | A | 105 | 30.28 | - | 3.75E+00 | 3.38E+00 |
| F2 | UNKN | A | 106 | 29.36 | - | 6.57E+00 | 5.82E+00 |
| F3 | UNKN | A | 106 | 29.7 | - | 5.34E+00 | 5.82E+00 |
| F4 | UNKN | A | 106 | 29.64 | - | 5.54E+00 | 5.82E+00 |
| A5 | UNKN | A | 201 | 30.43 | - | 3.42E+00 | 3.66E+00 |
| A6 | UNKN | A | 201 | 30.17 | - | 4.01E+00 | 3.66E+00 |
| A7 | UNKN | A | 201 | 30.36 | - | 3.57E+00 | 3.66E+00 |
| B5 | UNKN | A | 202 | 30.38 | - | 3.52E+00 | 3.14E+00 |
| B6 | UNKN | A | 202 | 30.88 | - | 2.60E+00 | 3.14E+00 |
| B7 | UNKN | A | 202 | 30.49 | - | 3.30E+00 | 3.14E+00 |
| C5 | UNKN | A | 203 | 31.4 | - | 1.89E+00 | 1.75E+00 |
| C6 | UNKN | A | 203 | 31.63 | - | 1.64E+00 | 1.75E+00 |
| C7 | UNKN | A | 203 | 31.54 | - | 1.73E+00 | 1.75E+00 |
| D5 | UNKN | A | 204 | 31.22 | - | 2.11E+00 | 2.12E+00 |
| D6 | UNKN | A | 204 | 31.35 | - | 1.95E+00 | 2.12E+00 |
| D7 | UNKN | A | 204 | 31.08 | - | 2.30E+00 | 2.12E+00 |
| E5 | UNKN | A | 205 | 29.75 | - | 5.15E+00 | 5.44E+00 |
| E6 | UNKN | A | 205 | 29.66 | - | 5.47E+00 | 5.44E+00 |
| E7 | UNKN | A | 205 | 29.59 | - | 5.71E+00 | 5.44E+00 |
| F5 | UNKN | A | 206 | 29.65 | - | 5.51E+00 | 5.40E+00 |
| F6 | UNKN | A | 206 | 29.68 | - | 5.41E+00 | 5.40E+00 |
| F7 | UNKN | A | 206 | 29.72 | - | 5.28E+00 | 5.40E+00 |
| A8 | UNKN | A | 301 | 30.34 | - | 3.61E+00 | 3.89E+00 |
| A9 | UNKN | A | 301 | 30.05 | - | 4.31E+00 | 3.89E+00 |
| A10 | UNKN | A | 301 | 30.28 | - | 3.75E+00 | 3.89E+00 |
| B8 | UNKN | A | 302 | 30.81 | - | 2.71E+00 | 2.62E+00 |
| B9 | UNKN | A | 302 | 30.85 | - | 2.64E+00 | 2.62E+00 |
| B10 | UNKN | A | 302 | 30.94 | - | 2.50E+00 | 2.62E+00 |
| C8 | UNKN | A | 303 | 29.47 | - | 6.15E+00 | 5.89E+00 |
| C9 | UNKN | A | 303 | 29.6 | - | 5.68E+00 | 5.89E+00 |
| C10 | UNKN | A | 303 | 29.55 | - | 5.85E+00 | 5.89E+00 |
| D8 | UNKN | A | 304 | 30.05 | - | 4.31E+00 | 4.14E+00 |
| D9 | UNKN | A | 304 | 30.32 | - | 3.66E+00 | 4.14E+00 |
| D10 | UNKN | A | 304 | 30 | - | 4.45E+00 | 4.14E+00 |
| E8 | UNKN | A | 305 | 30.86 | - | 2.63E+00 | 3.18E+00 |
| E9 | UNKN | A | 305 | 30.47 | - | 3.34E+00 | 3.18E+00 |
| E10 | UNKN | A | 305 | 30.36 | - | 3.57E+00 | 3.18E+00 |
| F8 | UNKN | A | 306 | 30.31 | - | 3.68E+00 | 3.60E+00 |
| F9 | UNKN | A | 306 | 30.51 | - | 3.25E+00 | 3.60E+00 |
| F10 | UNKN | A | 306 | 30.23 | - | 3.86E+00 | 3.60E+00 |
| B11 | STD | A | | 28.72 | 1.00E+01 | 9.72E+00 | - |
| C11 | STD | A | | 28.81 | 1.00E+01 | 9.20E+00 | - |
| D11 | STD | A | | 28.8 | 1.00E+01 | 9.26E+00 | - |
| E11 | STD | A | | 31.05 | 2.50E+00 | 2.34E+00 | - |
| F11 | STD | A | | 30.82 | 2.50E+00 | 2.69E+00 | - |
| G11 | STD | A | | 30.75 | 2.50E+00 | 2.81E+00 | - |
| B12 | STD | A | | 33.04 | 6.25E-01 | 6.93E-01 | - |
| C12 | STD | A | | 32.89 | 6.25E-01 | 7.60E-01 | - |
| F12 | STD | A | | 36.03 | 1.56E-01 | 1.11E-01 | - |
| H11 | STD | A | | 35.34 | 1.56E-01 | 1.70E-01 | - |

Figure 34D Plate 1-4 : Collagen Type 1

| Well | Sample Type | Target Mark | Association Mark | Ct(SDM) | Init Qty | Qty(SDM) | Rel.Qty(SDM) |
|---|---|---|---|---|---|---|---|
| A2 | UNKN | A | 101 | 27.62 | - | 5.75E+00 | 5.44E+00 |
| A3 | UNKN | A | 101 | 27.83 | - | 5.07E+00 | 5.44E+00 |
| A4 | UNKN | A | 101 | 27.7 | - | 5.48E+00 | 5.44E+00 |
| B2 | UNKN | A | 102 | 27.15 | - | 7.64E+00 | 7.59E+00 |
| B3 | UNKN | A | 102 | 27.25 | - | 7.19E+00 | 7.59E+00 |
| B4 | UNKN | A | 102 | 27.09 | - | 7.92E+00 | 7.59E+00 |
| C2 | UNKN | A | 103 | 28.18 | - | 4.10E+00 | 4.08E+00 |
| C3 | UNKN | A | 103 | 28.24 | - | 3.96E+00 | 4.08E+00 |
| C4 | UNKN | A | 103 | 28.15 | - | 4.18E+00 | 4.08E+00 |
| D2 | UNKN | A | 104 | 28.48 | - | 3.42E+00 | 2.84E+00 |
| D3 | UNKN | A | 104 | 29 | - | 2.50E+00 | 2.84E+00 |
| D4 | UNKN | A | 104 | 28.94 | - | 2.59E+00 | 2.84E+00 |
| E2 | UNKN | A | 105 | 28 | - | 4.58E+00 | 4.24E+00 |
| E3 | UNKN | A | 105 | 28.34 | - | 3.73E+00 | 4.24E+00 |
| E4 | UNKN | A | 105 | 28.06 | - | 4.41E+00 | 4.24E+00 |
| F2 | UNKN | A | 106 | 28.28 | - | 3.86E+00 | 3.95E+00 |
| F3 | UNKN | A | 106 | 28.24 | - | 3.96E+00 | 3.95E+00 |
| F4 | UNKN | A | 106 | 28.21 | - | 4.03E+00 | 3.95E+00 |
| A5 | UNKN | A | 201 | 28.54 | - | 3.30E+00 | 3.23E+00 |
| A6 | UNKN | A | 201 | 28.45 | - | 3.49E+00 | 3.23E+00 |
| A7 | UNKN | A | 201 | 28.75 | - | 2.91E+00 | 3.23E+00 |
| B5 | UNKN | A | 202 | 28.59 | - | 3.21E+00 | 3.10E+00 |
| B6 | UNKN | A | 202 | 28.51 | - | 3.36E+00 | 3.10E+00 |
| B7 | UNKN | A | 202 | 28.85 | - | 2.74E+00 | 3.10E+00 |
| C5 | UNKN | A | 203 | 28.87 | - | 2.71E+00 | 2.65E+00 |
| C6 | UNKN | A | 203 | 28.78 | - | 2.86E+00 | 2.65E+00 |
| C7 | UNKN | A | 203 | 29.07 | - | 2.40E+00 | 2.65E+00 |
| D5 | UNKN | A | 204 | 28.99 | - | 2.52E+00 | 2.46E+00 |
| D6 | UNKN | A | 204 | 28.9 | - | 2.66E+00 | 2.46E+00 |
| D7 | UNKN | A | 204 | 29.21 | - | 2.20E+00 | 2.46E+00 |
| E5 | UNKN | A | 205 | 28.29 | - | 3.84E+00 | 3.55E+00 |
| E6 | UNKN | A | 205 | 28.42 | - | 3.55E+00 | 3.55E+00 |
| E7 | UNKN | A | 205 | 28.56 | - | 3.26E+00 | 3.55E+00 |
| F5 | UNKN | A | 206 | 28.37 | - | 3.66E+00 | 3.86E+00 |
| F6 | UNKN | A | 206 | 28.06 | - | 4.41E+00 | 3.86E+00 |
| F7 | UNKN | A | 206 | 28.44 | - | 3.51E+00 | 3.86E+00 |
| A8 | UNKN | A | 301 | 28.41 | - | 3.57E+00 | 3.41E+00 |
| A9 | UNKN | A | 301 | 28.64 | - | 3.11E+00 | 3.41E+00 |
| A10 | UNKN | A | 301 | 28.42 | - | 3.55E+00 | 3.41E+00 |
| B8 | UNKN | A | 302 | 28.41 | - | 3.57E+00 | 3.43E+00 |
| B9 | UNKN | A | 302 | 28.46 | - | 3.47E+00 | 3.43E+00 |
| B10 | UNKN | A | 302 | 28.57 | - | 3.24E+00 | 3.43E+00 |
| C8 | UNKN | A | 303 | 28.33 | - | 3.75E+00 | 3.83E+00 |
| C9 | UNKN | A | 303 | 28.25 | - | 3.93E+00 | 3.83E+00 |
| C10 | UNKN | A | 303 | 28.31 | - | 3.79E+00 | 3.83E+00 |
| D8 | UNKN | A | 304 | 28.13 | - | 4.23E+00 | 3.92E+00 |
| D9 | UNKN | A | 304 | 28.54 | - | 3.30E+00 | 3.92E+00 |
| D10 | UNKN | A | 304 | 28.13 | - | 4.23E+00 | 3.92E+00 |
| E8 | UNKN | A | 305 | 28.4 | - | 3.59E+00 | 4.04E+00 |
| E9 | UNKN | A | 305 | 28.17 | - | 4.13E+00 | 4.04E+00 |
| E10 | UNKN | A | 305 | 28.07 | - | 4.39E+00 | 4.04E+00 |
| F8 | UNKN | A | 306 | 28.47 | - | 3.45E+00 | 3.52E+00 |
| F9 | UNKN | A | 306 | 28.44 | - | 3.51E+00 | 3.52E+00 |
| F10 | UNKN | A | 306 | 28.4 | - | 3.59E+00 | 3.52E+00 |
| B11 | STD | A | | 26.55 | 1.00E+01 | 1.10E+01 | - |
| C11 | STD | A | | 26.75 | 1.00E+01 | 9.73E+00 | - |
| D11 | STD | A | | 26.87 | 1.00E+01 | 9.05E+01 | - |
| E11 | STD | A | | 29.08 | 2.50E+00 | 2.38E+00 | - |
| F11 | STD | A | | 29.14 | 2.50E+00 | 2.30E+00 | - |
| G11 | STD | A | | 28.88 | 2.50E+00 | 2.69E+00 | - |
| B12 | STD | A | | 31.01 | 6.25E-01 | 7.44E-01 | - |
| C12 | STD | A | | 31.26 | 6.25E-01 | 6.40E-01 | - |
| D12 | STD | A | | 31.31 | 6.25E-01 | 6.21E-01 | - |
| E12 | STD | A | | 33.4 | 1.56E-01 | 1.76E-01 | - |
| F12 | STD | A | | 33.54 | 1.56E-01 | 1.62E-01 | - |
| G12 | STD | A | | 34.05 | 1.56E-01 | 1.19E-01 | - |

Figure 34E Plate 2-1 : 36B4

| Well | Sample Type | Target Mark | Association Mark | Ct(SDM) | Init Qty | Qty(SDM) | Rel.Qty(SDM) |
|---|---|---|---|---|---|---|---|
| A2 | UNKN | A | 101 | 19.42 | - | 2.53E+01 | 2.53E+01 |
| A3 | UNKN | A | 101 | 19.47 | - | 2.54E+01 | 2.53E+01 |
| A4 | UNKN | A | 101 | 19.54 | - | 2.41E+01 | 2.53E+01 |
| B2 | UNKN | A | 102 | 19.3 | - | 2.86E+01 | 2.79E+01 |
| B3 | UNKN | A | 102 | 19.32 | - | 2.82E+01 | 2.79E+01 |
| B4 | UNKN | A | 102 | 19.38 | - | 2.70E+01 | 2.79E+01 |
| C2 | UNKN | A | 103 | 19.33 | - | 2.80E+01 | 2.83E+01 |
| C3 | UNKN | A | 103 | 19.37 | - | 2.72E+01 | 2.83E+01 |
| C4 | UNKN | A | 103 | 19.24 | - | 2.98E+01 | 2.83E+01 |
| D2 | UNKN | A | 104 | 19.26 | - | 2.94E+01 | 2.70E+01 |
| D3 | UNKN | A | 104 | 19.49 | - | 2.50E+01 | 2.70E+01 |
| D4 | UNKN | A | 104 | 19.41 | - | 2.65E+01 | 2.70E+01 |
| E2 | UNKN | A | 105 | 19.17 | - | 3.13E+01 | 2.94E+01 |
| E3 | UNKN | A | 105 | 19.3 | - | 2.86E+01 | 2.94E+01 |
| E4 | UNKN | A | 105 | 19.32 | - | 2.82E+01 | 2.94E+01 |
| F2 | UNKN | A | 106 | 19.03 | - | 3.46E+01 | 3.32E+01 |
| F3 | UNKN | A | 106 | 19.14 | - | 3.20E+01 | 3.32E+01 |
| F4 | UNKN | A | 106 | 19.1 | - | 3.29E+01 | 3.32E+01 |
| A5 | UNKN | A | 201 | 19.21 | - | 3.05E+01 | 3.20E+01 |
| A6 | UNKN | A | 201 | 19.03 | - | 3.46E+01 | 3.20E+01 |
| A7 | UNKN | A | 201 | 19.19 | - | 3.09E+01 | 3.20E+01 |
| B5 | UNKN | A | 202 | 19.11 | - | 3.27E+01 | 3.08E+01 |
| B6 | UNKN | A | 202 | 19.14 | - | 3.20E+01 | 3.08E+01 |
| B7 | UNKN | A | 202 | 19.35 | - | 2.76E+01 | 3.08E+01 |
| C5 | UNKN | A | 203 | 19.55 | - | 2.40E+01 | 2.26E+01 |
| C6 | UNKN | A | 203 | 19.53 | - | 2.43E+01 | 2.26E+01 |
| C7 | UNKN | A | 203 | 19.84 | - | 1.95E+01 | 2.26E+01 |
| D5 | UNKN | A | 204 | 19.45 | - | 2.57E+01 | 2.52E+01 |
| D6 | UNKN | A | 204 | 19.38 | - | 2.70E+01 | 2.52E+01 |
| D7 | UNKN | A | 204 | 19.62 | - | 2.28E+01 | 2.52E+01 |
| E5 | UNKN | A | 205 | 19.23 | - | 3.00E+01 | 3.03E+01 |
| E6 | UNKN | A | 205 | 19.15 | - | 3.15E+01 | 3.03E+01 |
| E7 | UNKN | A | 205 | 19.28 | - | 2.90E+01 | 3.03E+01 |
| F5 | UNKN | A | 206 | 19.37 | - | 2.72E+01 | 2.75E+01 |
| F6 | UNKN | A | 206 | 19.36 | - | 2.74E+01 | 2.75E+01 |
| F7 | UNKN | A | 206 | 19.34 | - | 2.78E+01 | 2.75E+01 |
| A8 | UNKN | A | 301 | 19.14 | - | 3.20E+01 | 3.00E+01 |
| A9 | UNKN | A | 301 | 19.23 | - | 3.00E+01 | 3.00E+01 |
| A10 | UNKN | A | 301 | 19.33 | - | 2.80E+01 | 3.00E+01 |
| B8 | UNKN | A | 302 | 19.46 | - | 2.55E+01 | 2.65E+01 |
| B9 | UNKN | A | 302 | 19.36 | - | 2.74E+01 | 2.65E+01 |
| B10 | UNKN | A | 302 | 19.41 | - | 2.65E+01 | 2.65E+01 |
| C8 | UNKN | A | 303 | 19.19 | - | 3.09E+01 | 3.18E+01 |
| C9 | UNKN | A | 303 | 19.1 | - | 3.29E+01 | 3.18E+01 |
| C10 | UNKN | A | 303 | 19.16 | - | 3.16E+01 | 3.18E+01 |
| D8 | UNKN | A | 304 | 19.27 | - | 2.92E+01 | 3.16E+01 |
| D9 | UNKN | A | 304 | 19.07 | - | 3.36E+01 | 3.16E+01 |
| D10 | UNKN | A | 304 | 19.15 | - | 3.18E+01 | 3.16E+01 |
| E8 | UNKN | A | 305 | 19.37 | - | 2.72E+01 | 2.84E+01 |
| E9 | UNKN | A | 305 | 19.22 | - | 3.03E+01 | 2.84E+01 |
| E10 | UNKN | A | 305 | 19.35 | - | 2.76E+01 | 2.84E+01 |
| F8 | UNKN | A | 306 | 19.46 | - | 2.55E+01 | 2.80E+01 |
| F9 | UNKN | A | 306 | 19.33 | - | 2.80E+01 | 2.80E+01 |
| F10 | UNKN | A | 306 | 19.21 | - | 3.05E+01 | 2.80E+01 |
| B11 | STD | A | | 18.7 | 4.00E+01 | 4.37E+01 | - |
| C11 | STD | A | | 18.83 | 4.00E+01 | 3.99E+01 | - |
| D11 | STD | A | | 18.86 | 4.00E+01 | 3.90E+01 | - |
| E11 | STD | A | | 20.9 | 1.00E+01 | 9.22E+00 | - |
| F11 | STD | A | | 20.81 | 1.00E+01 | 9.83E+00 | - |
| G11 | STD | A | | 20.81 | 1.00E+01 | 9.83E+00 | - |
| B12 | STD | A | | 22.69 | 2.50E+00 | 2.60E+00 | - |
| C12 | STD | A | | 22.71 | 2.50E+00 | 2.56E+00 | - |
| D12 | STD | A | | 22.8 | 2.50E+00 | 2.40E+00 | - |
| E12 | STD | A | | 24.77 | 6.25E-01 | 5.97E-01 | - |
| F12 | STD | A | | 24.68 | 6.25E-01 | 6.36E-01 | - |
| G12 | STD | A | | 24.63 | 6.25E-01 | 6.59E-01 | - |

Figure 34F Plate 2-2 : MCP-1

| Well | Sample Type | Target Mark | Association Mark | Ct(SDM) | Init Qty | Qty(SDM) | Rel.Qty(SDM) |
|---|---|---|---|---|---|---|---|
| A2 | UNKN | A | 101 | 28.15 | - | 4.51E+01 | 4.43E+01 |
| A3 | UNKN | A | 101 | 29.21 | - | 4.31E+01 | 4.43E+01 |
| A4 | UNKN | A | 101 | 28.16 | - | 4.48E+01 | 4.43E+01 |
| B2 | UNKN | A | 102 | 28.26 | - | 4.16E+01 | 4.12E+01 |
| B3 | UNKN | A | 102 | 28.18 | - | 4.41E+01 | 4.12E+01 |
| B4 | UNKN | A | 102 | 28.38 | - | 3.80E+01 | 4.12E+01 |
| C2 | UNKN | A | 103 | 29.26 | - | 1.98E+01 | 2.19E+01 |
| C3 | UNKN | A | 103 | 28.94 | - | 2.51E+01 | 2.19E+01 |
| C4 | UNKN | A | 103 | 29.19 | - | 2.08E+01 | 2.19E+01 |
| D2 | UNKN | A | 104 | 28.19 | - | 4.38E+01 | 4.12E+01 |
| D3 | UNKN | A | 104 | 28.44 | - | 3.64E+01 | 4.12E+01 |
| D4 | UNKN | A | 104 | 28.2 | - | 4.35E+01 | 4.12E+01 |
| E2 | UNKN | A | 105 | 28.71 | - | 2.97E+01 | 2.84E+01 |
| E3 | UNKN | A | 105 | 28.74 | - | 2.91E+01 | 2.84E+01 |
| E4 | UNKN | A | 105 | 28.87 | - | 2.64E+01 | 2.84E+01 |
| F2 | UNKN | A | 106 | 28.57 | - | 3.30E+01 | 3.25E+01 |
| F3 | UNKN | A | 106 | 28.65 | - | 3.11E+01 | 3.25E+01 |
| F4 | UNKN | A | 106 | 28.55 | - | 3.35E+01 | 3.25E+01 |
| A5 | UNKN | A | 201 | 28.34 | - | 3.92E+01 | 3.62E+01 |
| A6 | UNKN | A | 201 | 28.45 | - | 3.61E+01 | 3.62E+01 |
| A7 | UNKN | A | 201 | 28.56 | - | 3.33E+01 | 3.62E+01 |
| B5 | UNKN | A | 202 | 28.42 | - | 3.69E+01 | 3.60E+01 |
| B6 | UNKN | A | 202 | 28.5 | - | 3.48E+01 | 3.60E+01 |
| B7 | UNKN | A | 202 | 28.44 | - | 3.64E+01 | 3.60E+01 |
| C5 | UNKN | A | 203 | 29.55 | - | 1.59E+01 | 1.61E+01 |
| C6 | UNKN | A | 203 | 29.46 | - | 1.70E+01 | 1.61E+01 |
| C7 | UNKN | A | 203 | 29.59 | - | 1.55E+01 | 1.61E+01 |
| D5 | UNKN | A | 204 | 29.28 | - | 1.95E+01 | 1.93E+01 |
| D6 | UNKN | A | 204 | 29.4 | - | 1.78E+01 | 1.93E+01 |
| D7 | UNKN | A | 204 | 29.21 | - | 2.05E+01 | 1.93E+01 |
| E5 | UNKN | A | 205 | 28.08 | - | 4.75E+01 | 5.32E+01 |
| E6 | UNKN | A | 205 | 27.92 | - | 5.35E+01 | 5.32E+01 |
| E7 | UNKN | A | 205 | 27.8 | - | 5.85E+01 | 5.32E+01 |
| F5 | UNKN | A | 206 | 27.6 | - | 6.79E+01 | 6.45E+01 |
| F6 | UNKN | A | 206 | 27.72 | - | 6.21E+01 | 6.45E+01 |
| F7 | UNKN | A | 206 | 27.69 | - | 6.35E+01 | 6.45E+01 |
| A8 | UNKN | A | 301 | 28.75 | - | 2.89E+01 | 3.00E+01 |
| A9 | UNKN | A | 301 | 28.68 | - | 3.04E+01 | 3.00E+01 |
| A10 | UNKN | A | 301 | 28.67 | - | 3.06E+01 | 3.00E+01 |
| B8 | UNKN | A | 302 | 28.92 | - | 2.54E+01 | 2.84E+01 |
| B9 | UNKN | A | 302 | 28.66 | - | 3.09E+01 | 2.84E+01 |
| B10 | UNKN | A | 302 | 28.75 | - | 2.89E+01 | 2.84E+01 |
| C8 | UNKN | A | 303 | 27.44 | - | 7.65E+01 | 8.22E+01 |
| C9 | UNKN | A | 303 | 27.24 | - | 8.88E+01 | 8.22E+01 |
| C10 | UNKN | A | 303 | 27.36 | - | 8.12E+01 | 8.22E+01 |
| D8 | UNKN | A | 304 | 28.78 | - | 2.82E+01 | 2.82E+01 |
| D9 | UNKN | A | 304 | 28.6 | - | 3.23E+01 | 2.82E+01 |
| D10 | UNKN | A | 304 | 28.99 | - | 2.42E+01 | 2.82E+01 |
| E8 | UNKN | A | 305 | 28.61 | - | 3.20E+01 | 2.70E+01 |
| E9 | UNKN | A | 305 | 29 | - | 2.40E+01 | 2.70E+01 |
| E10 | UNKN | A | 305 | 28.94 | - | 2.51E+01 | 2.70E+01 |
| F8 | UNKN | A | 306 | 28.81 | - | 2.76E+01 | 2.73E+01 |
| F9 | UNKN | A | 306 | 28.78 | - | 2.82E+01 | 2.73E+01 |
| F10 | UNKN | A | 306 | 28.89 | - | 2.60E+01 | 2.73E+01 |
| B11 | STD | A | | 28.29 | 4.00E+01 | 4.07E+01 | - |
| C11 | STD | A | | 28.14 | 4.00E+01 | 4.55E+01 | - |
| D11 | STD | A | | 28.12 | 4.00E+01 | 4.61E+01 | - |
| E11 | STD | A | | 30.33 | 1.00E+01 | 8.91E+00 | - |
| F11 | STD | A | | 30.18 | 1.00E+01 | 9.96E+00 | - |
| G11 | STD | A | | 30.37 | 1.00E+01 | 8.65E+00 | - |
| B12 | STD | A | | 31.93 | 2.50E+00 | 2.71E+00 | - |
| C12 | STD | A | | 32.36 | 2.50E+00 | 1.97E+00 | - |
| D12 | STD | A | | 32.3 | 2.50E+00 | 2.06E+00 | - |
| F12 | STD | A | | 33.76 | 6.25E-01 | 6.95E-01 | - |
| H11 | STD | A | | 33.61 | 6.25E-01 | 7.77E-01 | - |

Figure 35

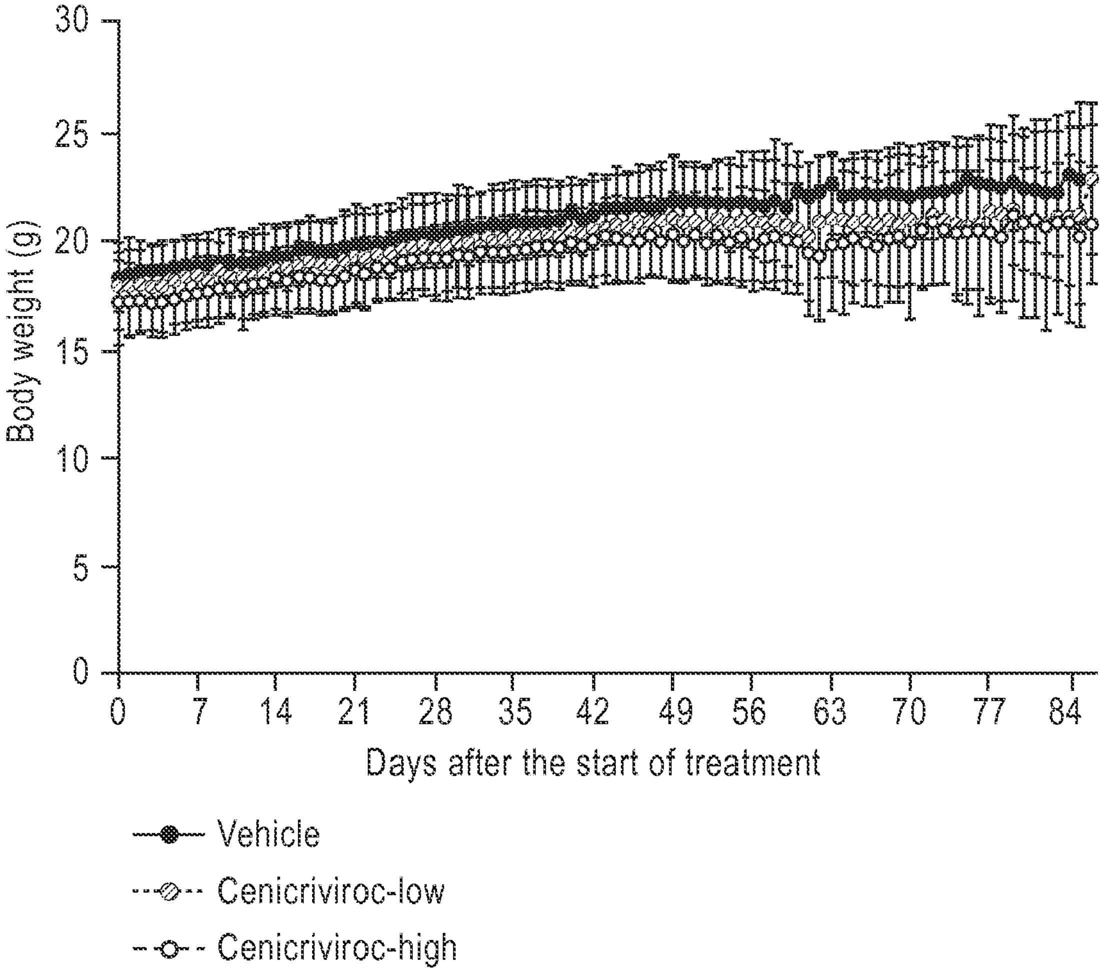
30
25
20
15
10
5
0
Body weight (g)
0
7
14
21
28
35
42
49
56
63
70
77
84
Days after the start of treatment
Vehicle
Cenicriviroc-low
Cenicriviroc-high

Figure 36

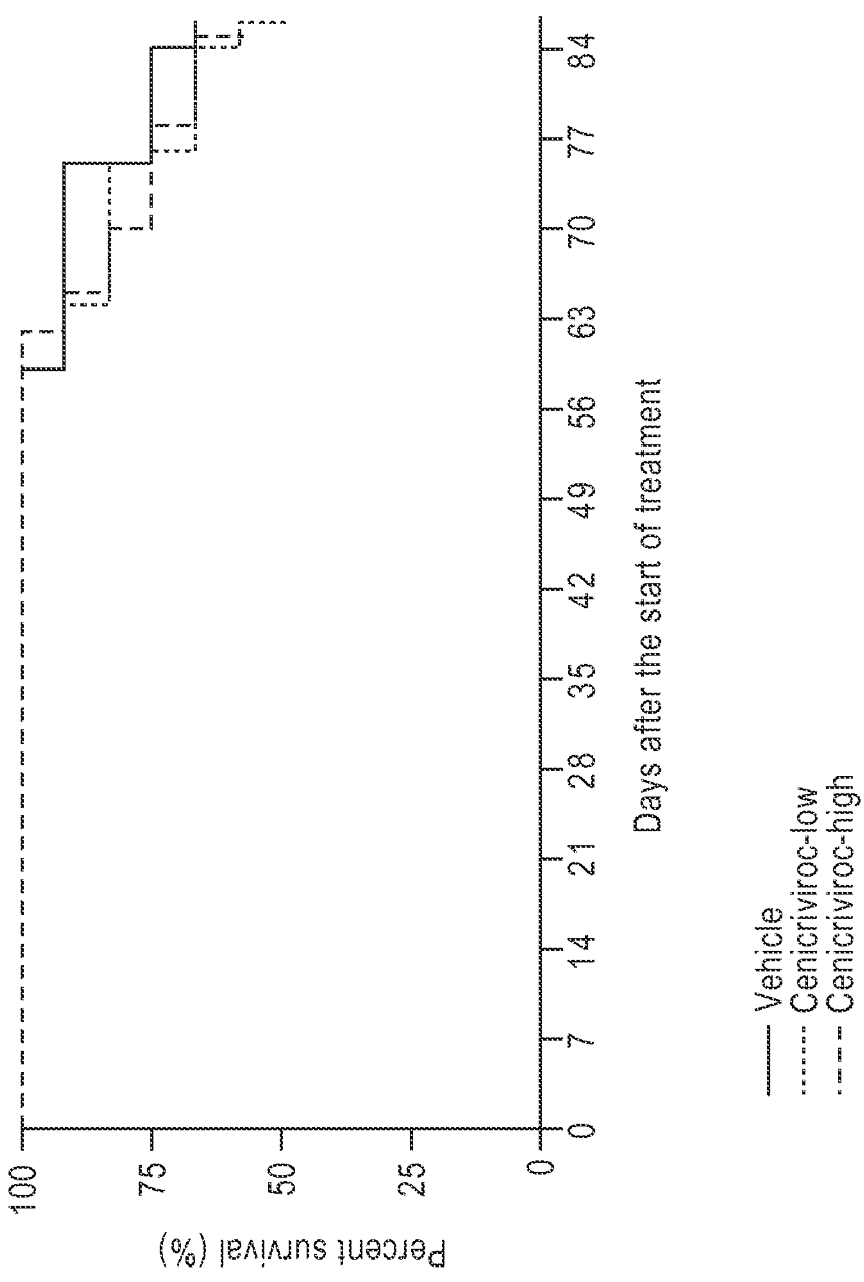
Percent survival (%)
100
75
50
25
0
0
7
14
21
28
35
42
49
56
63
70
77
84
Days after the start of treatment
Vehicle
Cenicriviroc-low
Cenicriviroc-high

Figure 37

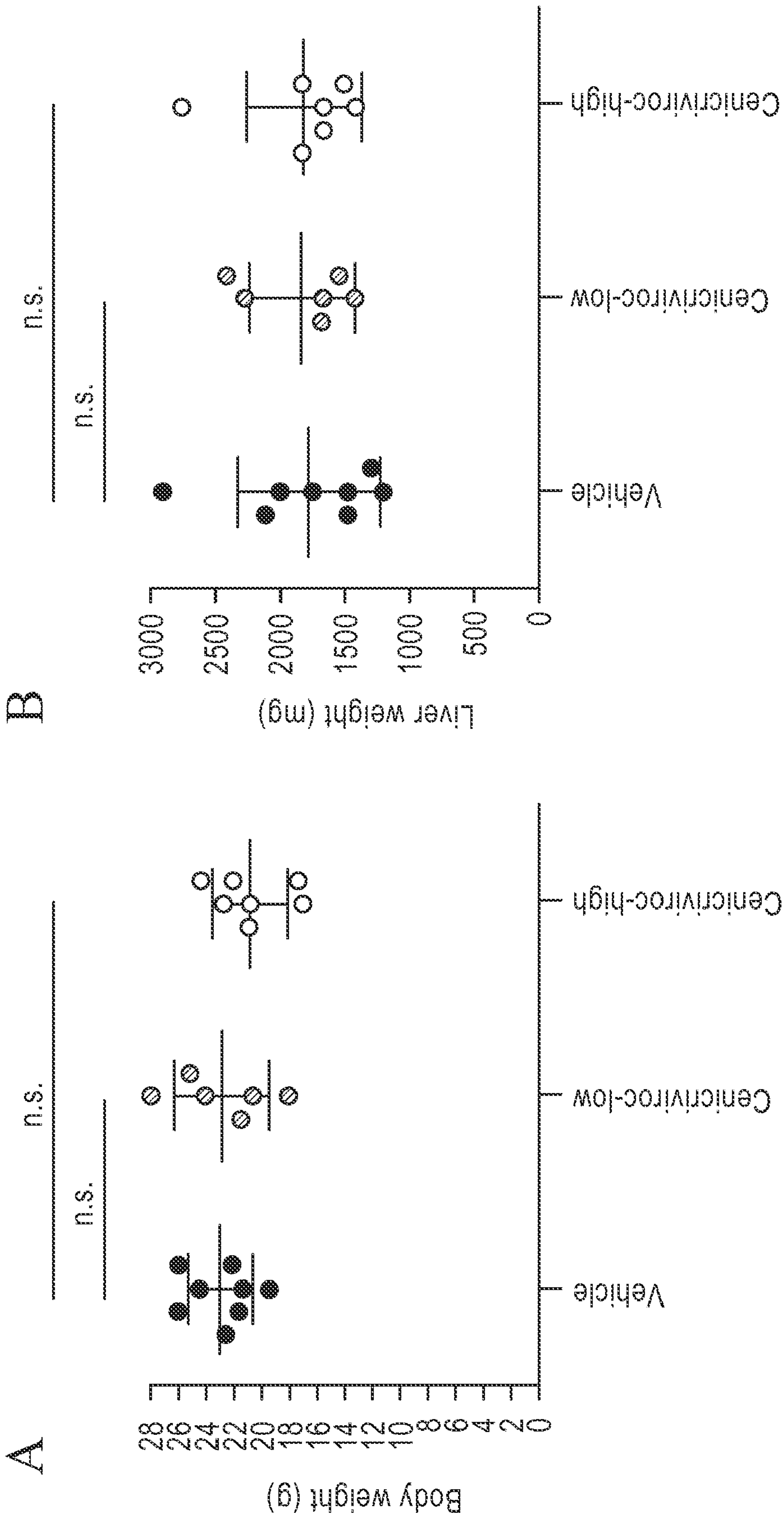
A
Body weight (g)
28
26
24
22
20
18
16
14
12
10
8
6
4
2
0
Vehicle
Cenicrivirac-low
Cenicrivirac-high
n.s.
n.s.
B
Liver weight (mg)
3000
2500
2000
1500
1000
500
0
Vehicle
Cenicrivirac-low
Cenicrivirac-high
n.s.
n.s.

Figure 37

C

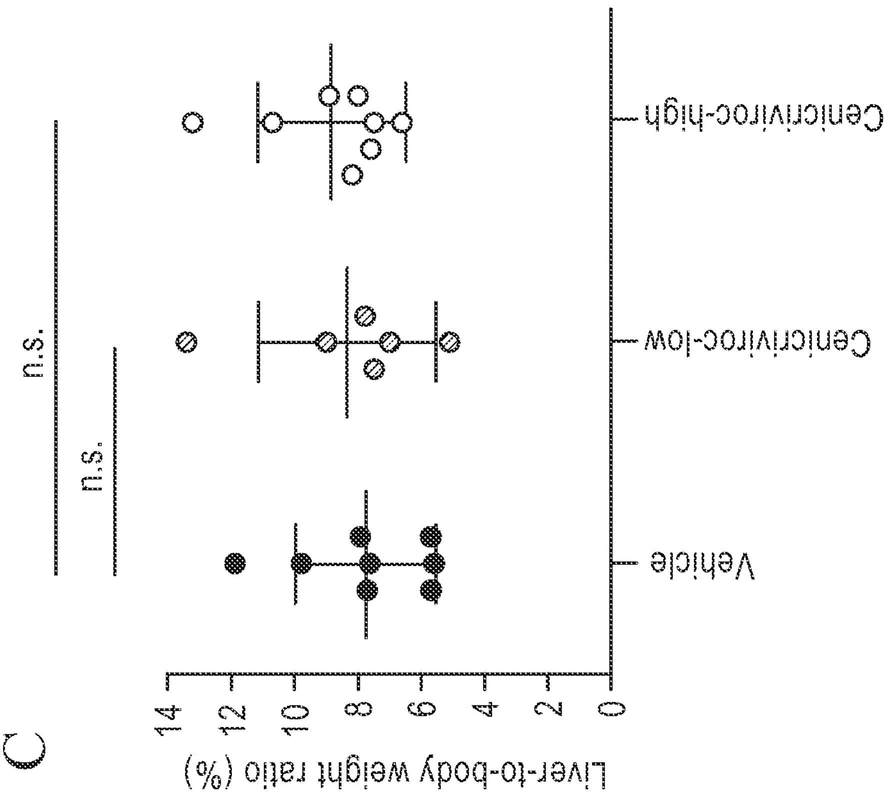
Liver-to-body weight ratio (%)
14
12
10
8
6
4
2
0
Vehicle
Cenicriviroc-low
Cenicriviroc-high
n.s.
n.s.

Figure 38A

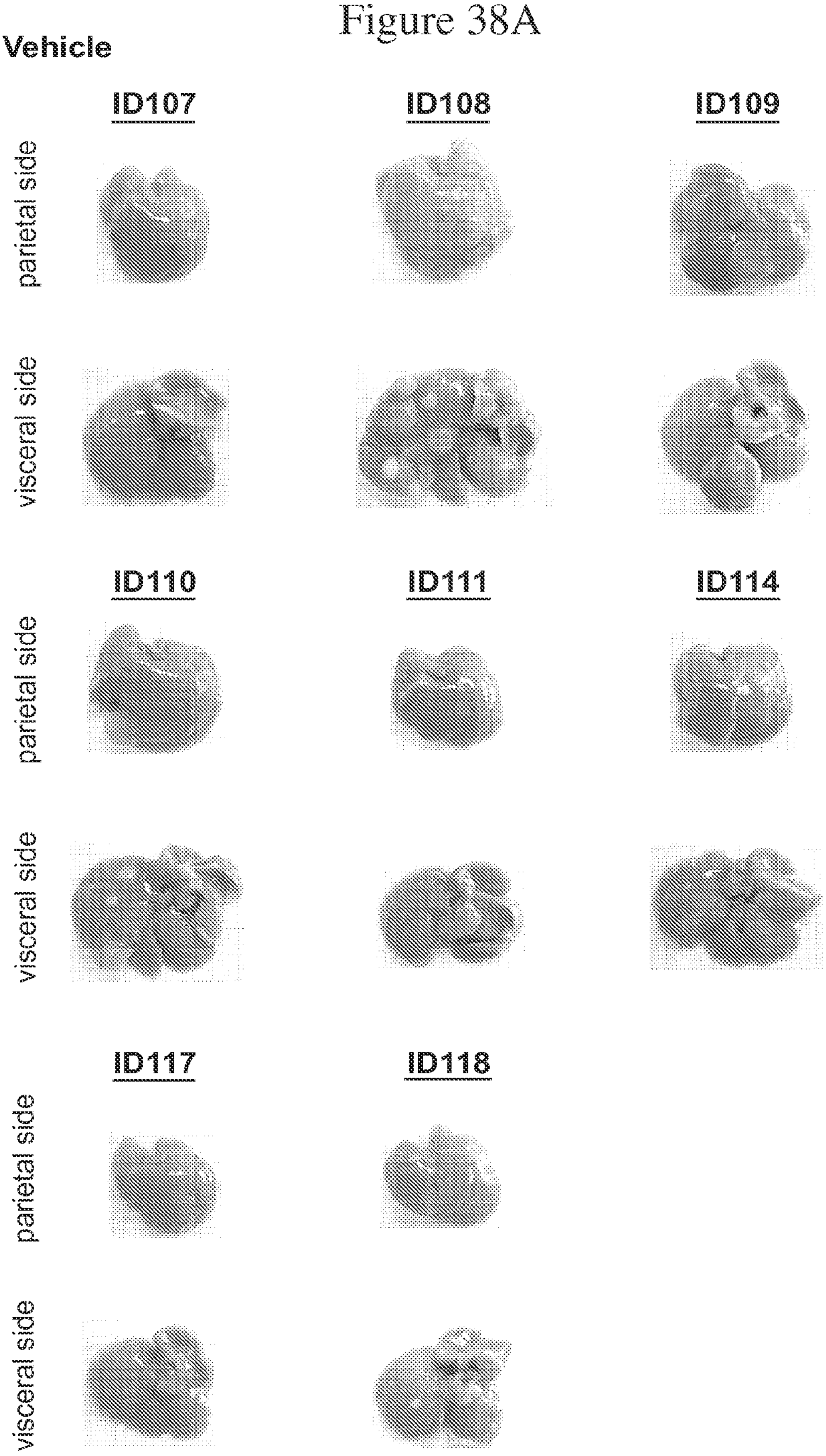
Vehicle
ID107
ID108
ID109
parietal side
visceral side
ID110
ID111
ID114
parietal side
visceral side
ID117
ID118
parietal side
visceral side

Figure 38B

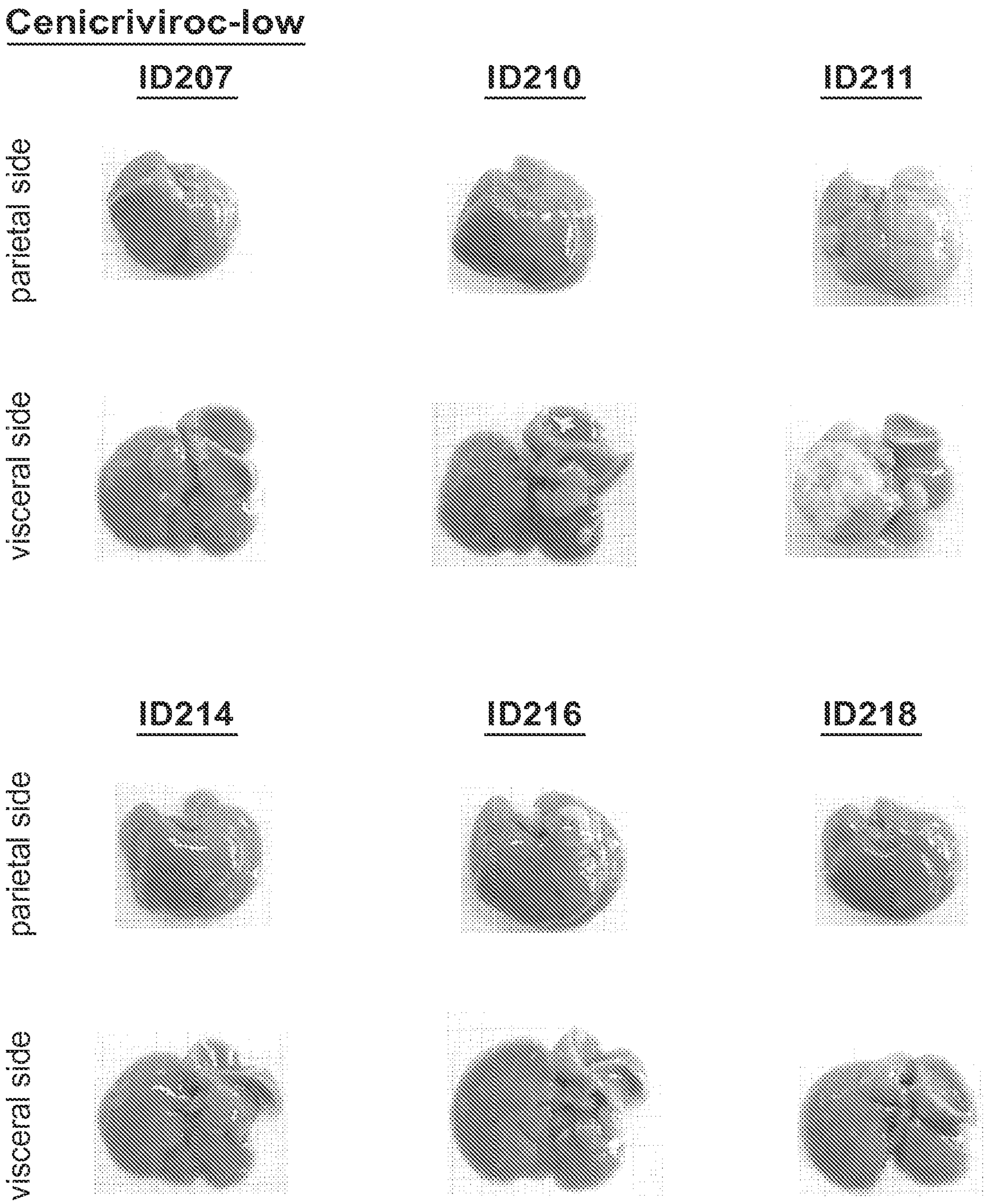
Cenicriviroc-low
ID207
ID210
ID211
parietal side
visceral side
ID214
ID216
ID218
parietal side
visceral side

Figure 38C

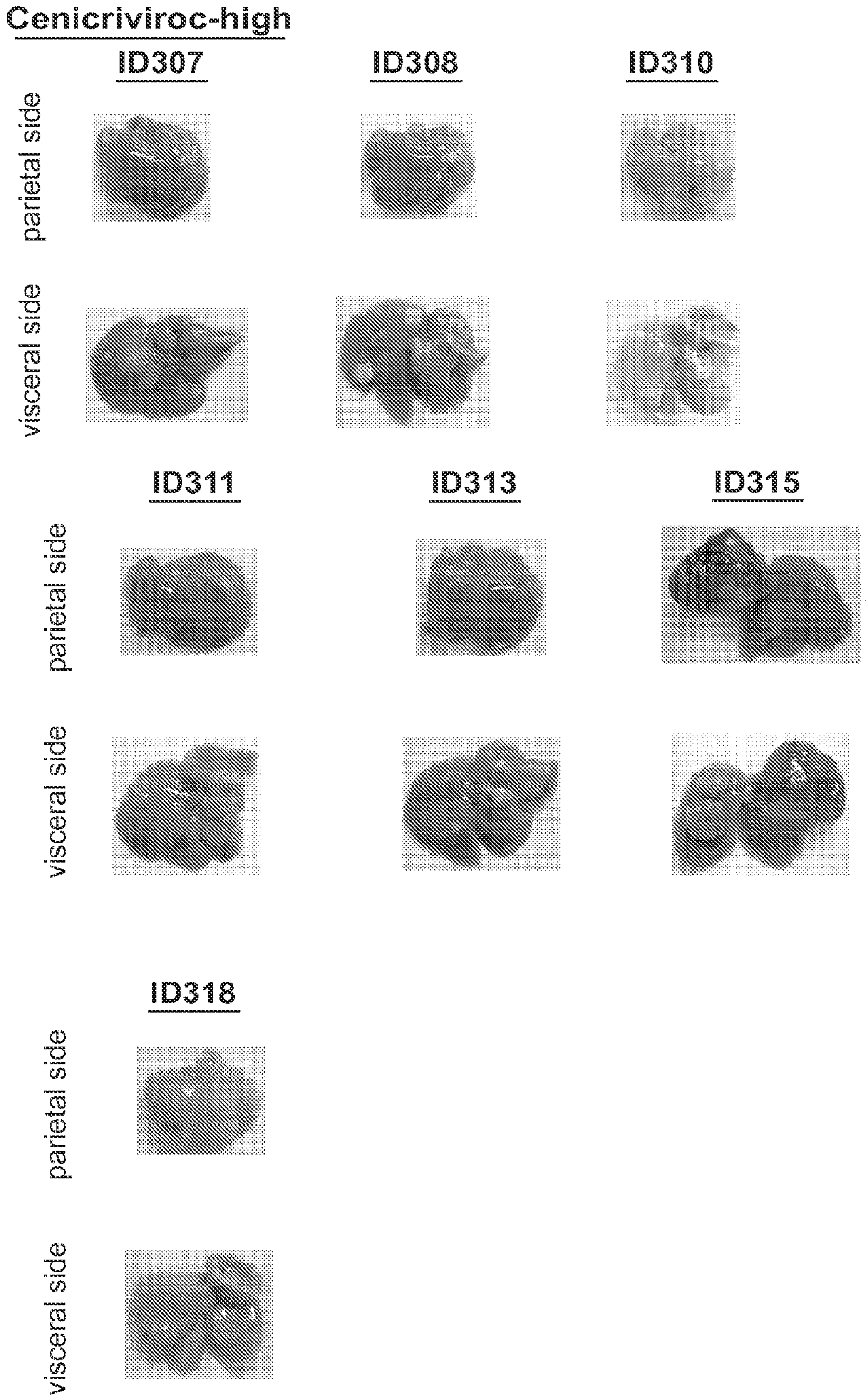
Cenicriviroc-high
ID307
ID308
ID310
parietal side
visceral side
ID311
ID313
ID315
parietal side
visceral side
ID318
parietal side
visceral side

Figure 39

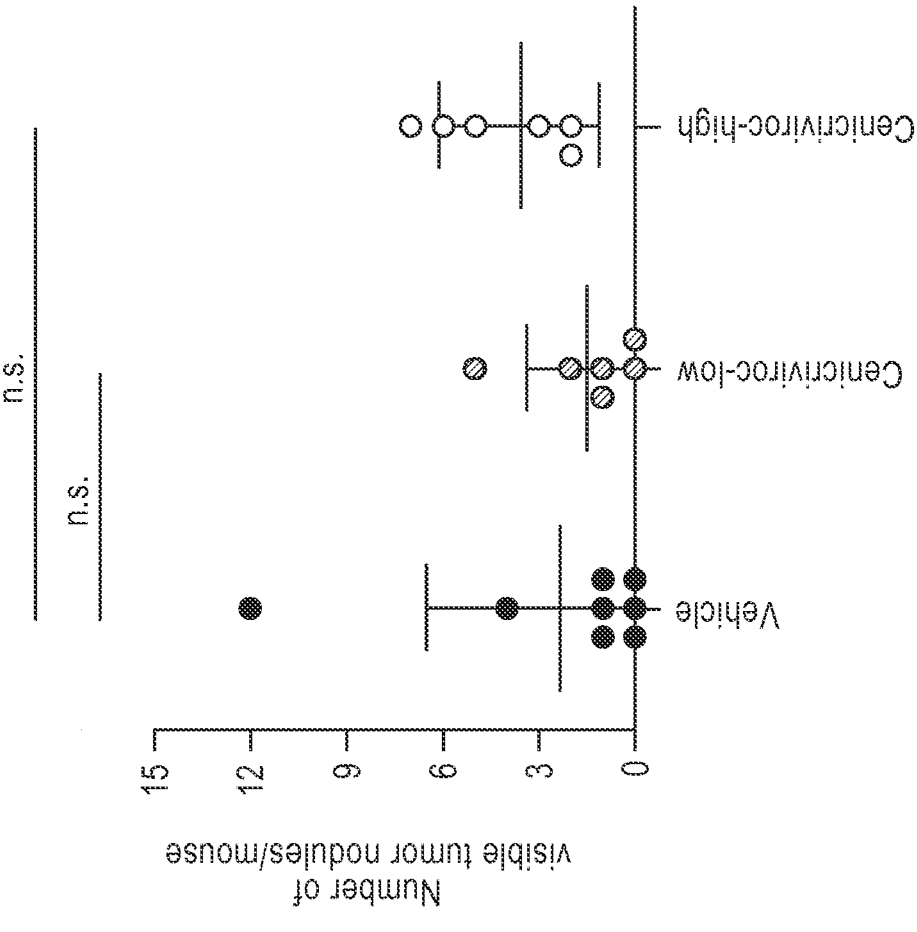
Number of visible tumor nodules/mouse
15
12
9
6
3
0
n.s.
n.s.
Vehicle
Cenicriviroc-low
Cenicriviroc-high

Figure 40

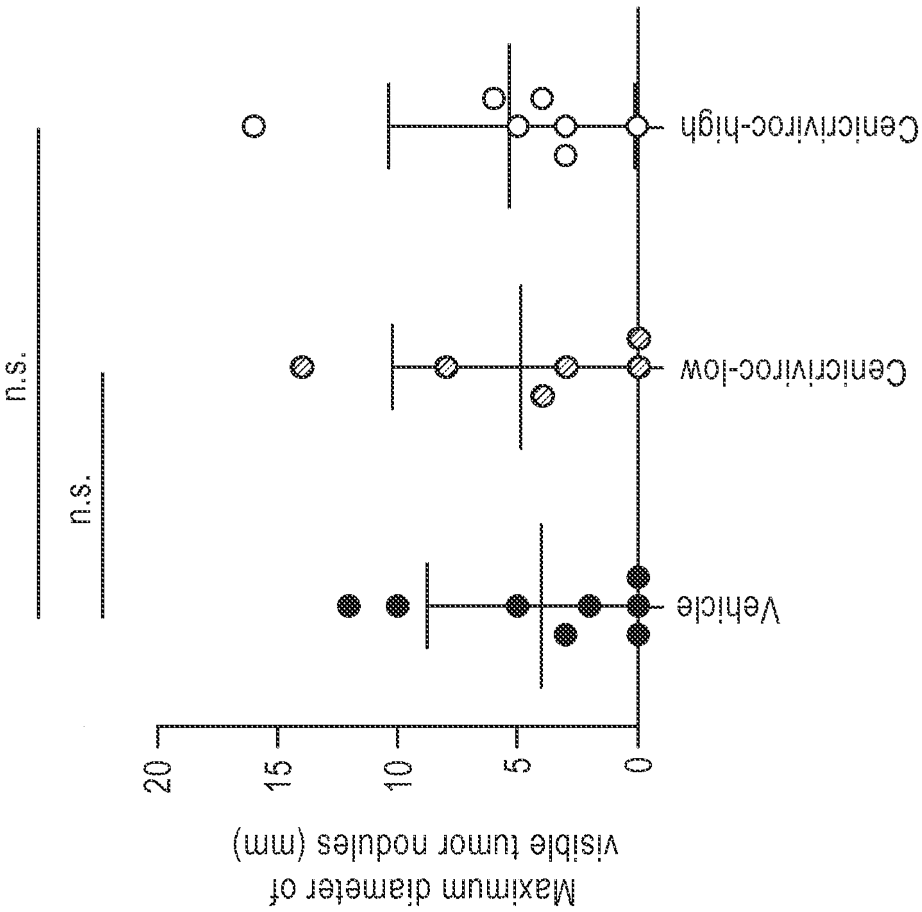
Maximum diameter of
visible tumor nodules (mm)
20
15
10
5
0
Vehicle
Cenicriviroc-low
Cenicriviroc-high
n.s.
n.s.

Figure 41

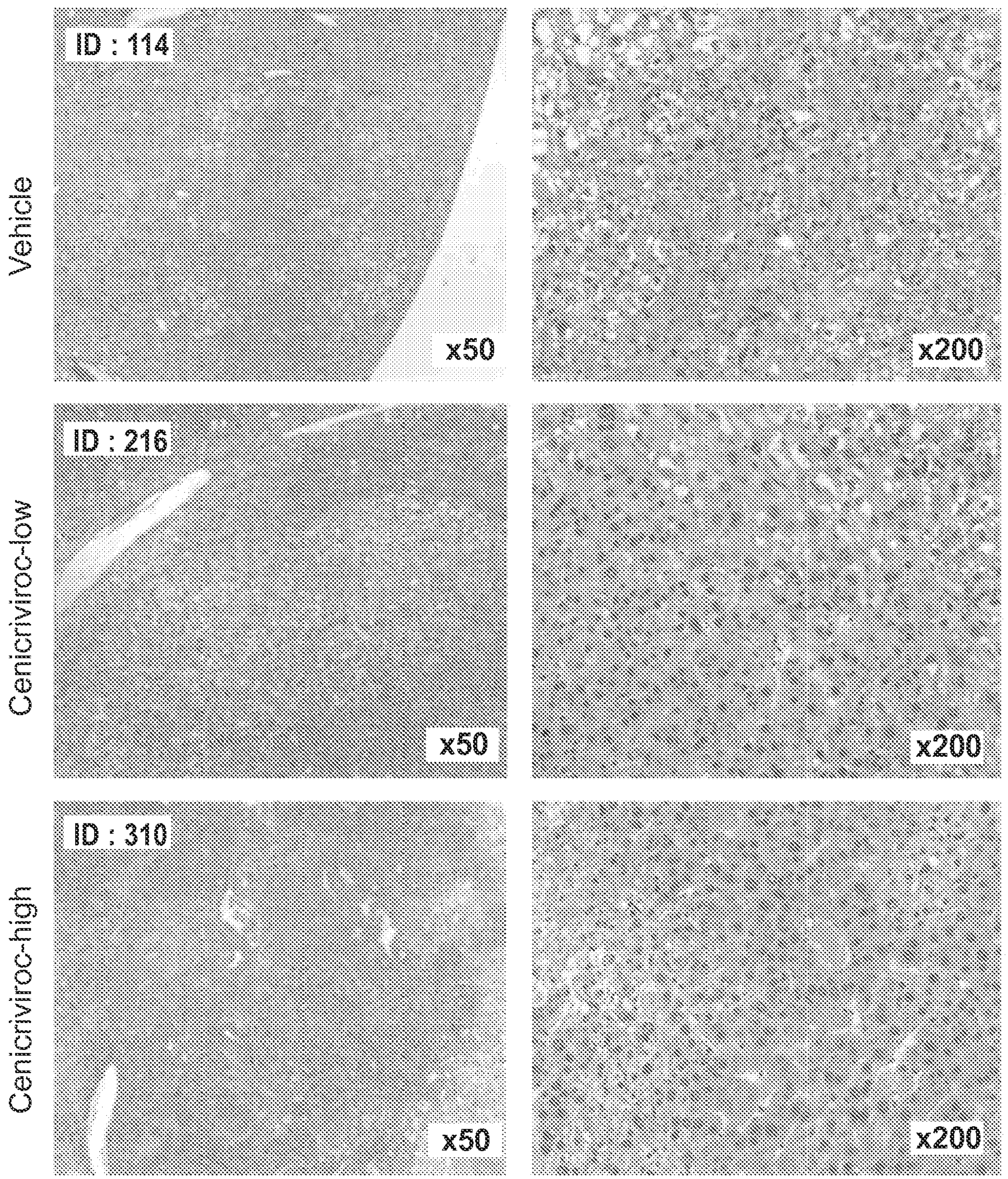
Vehicle
ID : 114
x50
x200
Cenicriviroc-low
ID : 216
x50
x200
Cenicriviroc-high
ID : 310
x50
x200

Figure 42

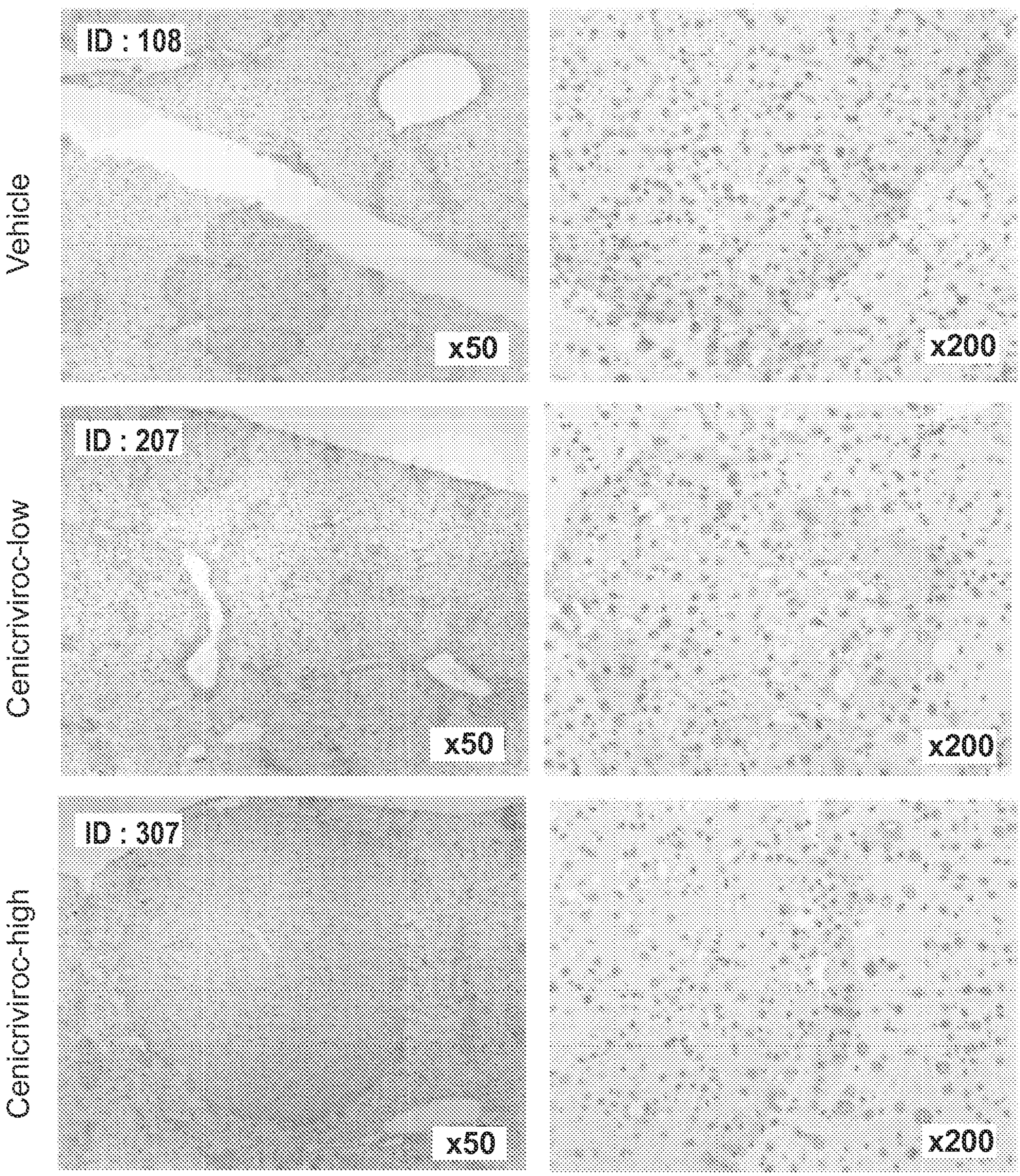
Vehicle
ID : 108
x50
x200
Cenicriviroc-low
ID : 207
x50
x200
Cenicriviroc-high
ID : 307
x50
x200

Figure 43

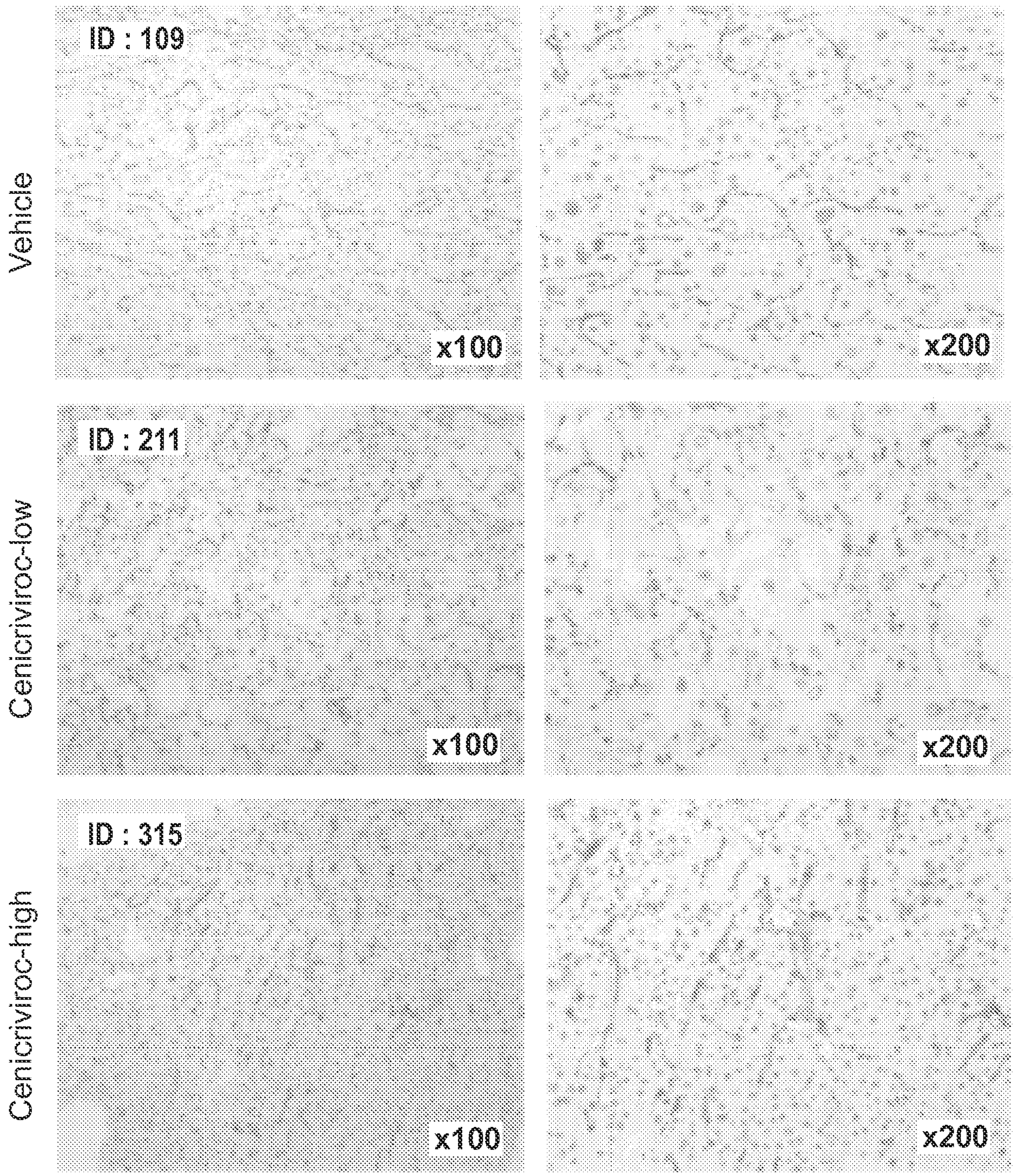
Vehicle
ID : 109
x100
x200
Cenicriviroc-low
ID : 211
x100
x200
Cenicriviroc-high
ID : 315
x100
x200

Figure 44

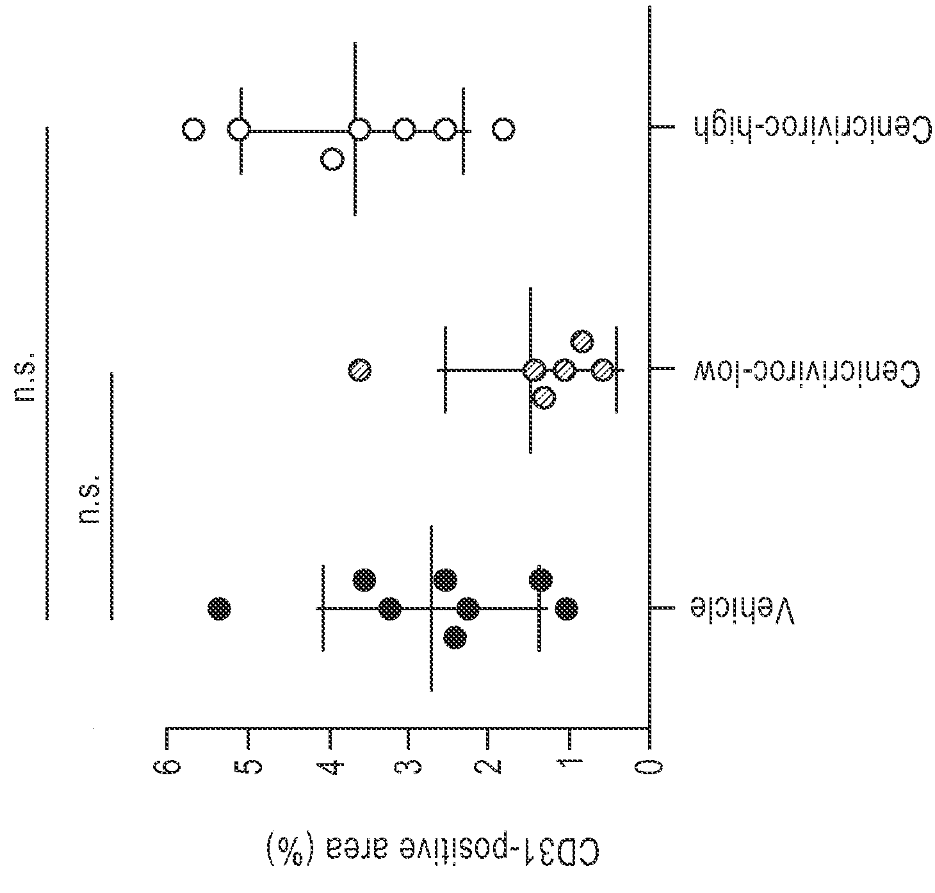
n.s.
n.s.
CD31-positive area (%)
0
1
2
3
4
5
6
Vehicle
Cenicriviroc-low
Cenicriviroc-high

# Figure 45

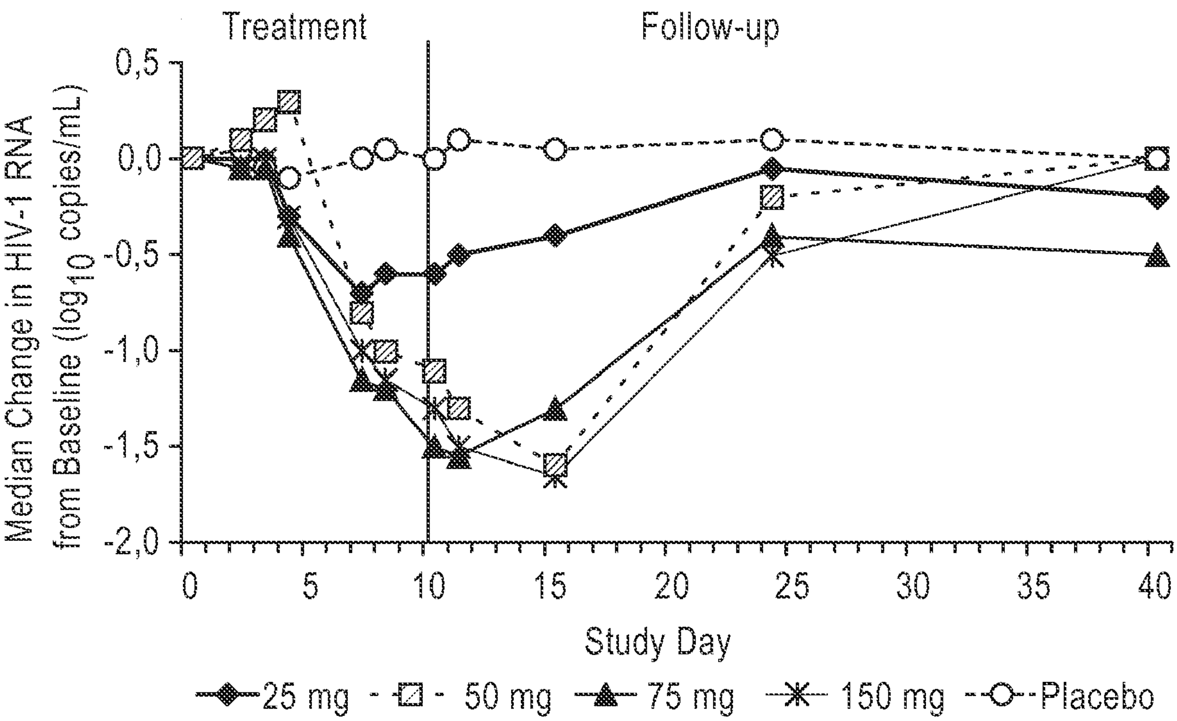
Treatment
Follow-up
Median Change in HIV-1 RNA from Baseline ($log_{10}$ copies/mL)
0,5
0,0
-0,5
-1,0
-1,5
-2,0
0
5
10
15
20
25
30
35
40
Study Day
25 mg
50 mg
75 mg
150 mg
Placebo

# Figure 46

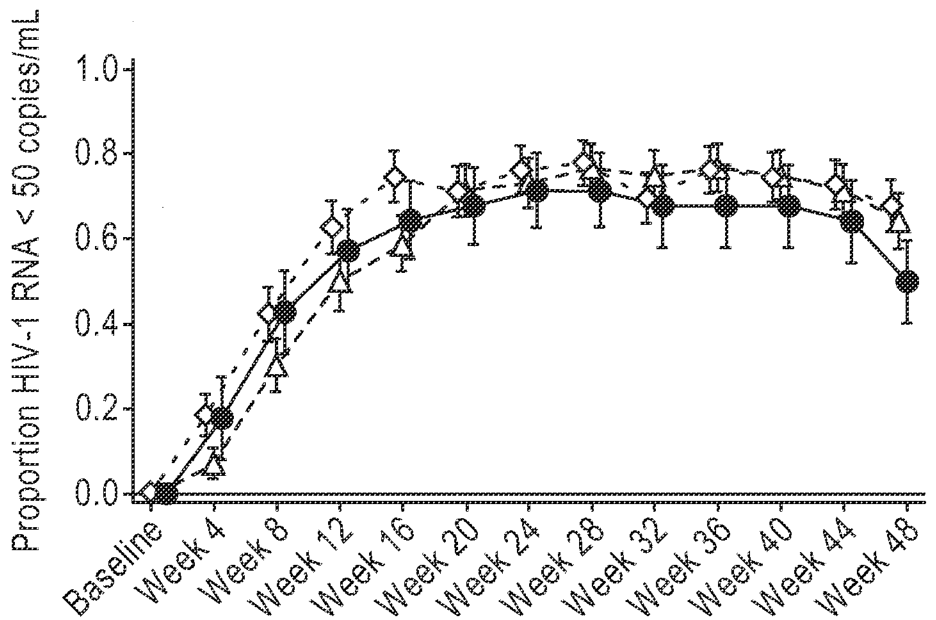


No. of subjects

| | | | | | | | | | | | | | |
|---|---|---|---|---|---|---|---|---|---|---|---|---|---|
| CVC 100 + FTC/TDF | 0/59 | 11/59 | 25/59 | 37/59 | 44/59 | 42/49 | 45/59 | 46/59 | 41/59 | 45/59 | 44/59 | 43/59 | 40/59 |
| CVC 200 + FTC/TDF | 0/56 | 4/56 | 17/56 | 28/56 | 33/56 | 40/56 | 41/56 | 43/56 | 42/56 | 43/56 | 42/56 | 40/56 | 36/56 |
| EFV + FTC/TDF | 0/28 | 5/28 | 12/28 | 16/28 | 18/28 | 19/28 | 20/28 | 20/28 | 19/28 | 19/28 | 19/28 | 18/28 | 14/28 |

Treatment group
CVC 100 mg + EFV placebo + FTC/TDF
CVC 200 mg + EFV placebo + FTC/TDF
CVC placebo + EFV 600 mg + FTC/TDF

n/N: number of subjects with HIV-1 RNA <50 copies/mL/number of subjects with data.
Source: Week 48 final CSR 652-2-202 [22].

Figure 47

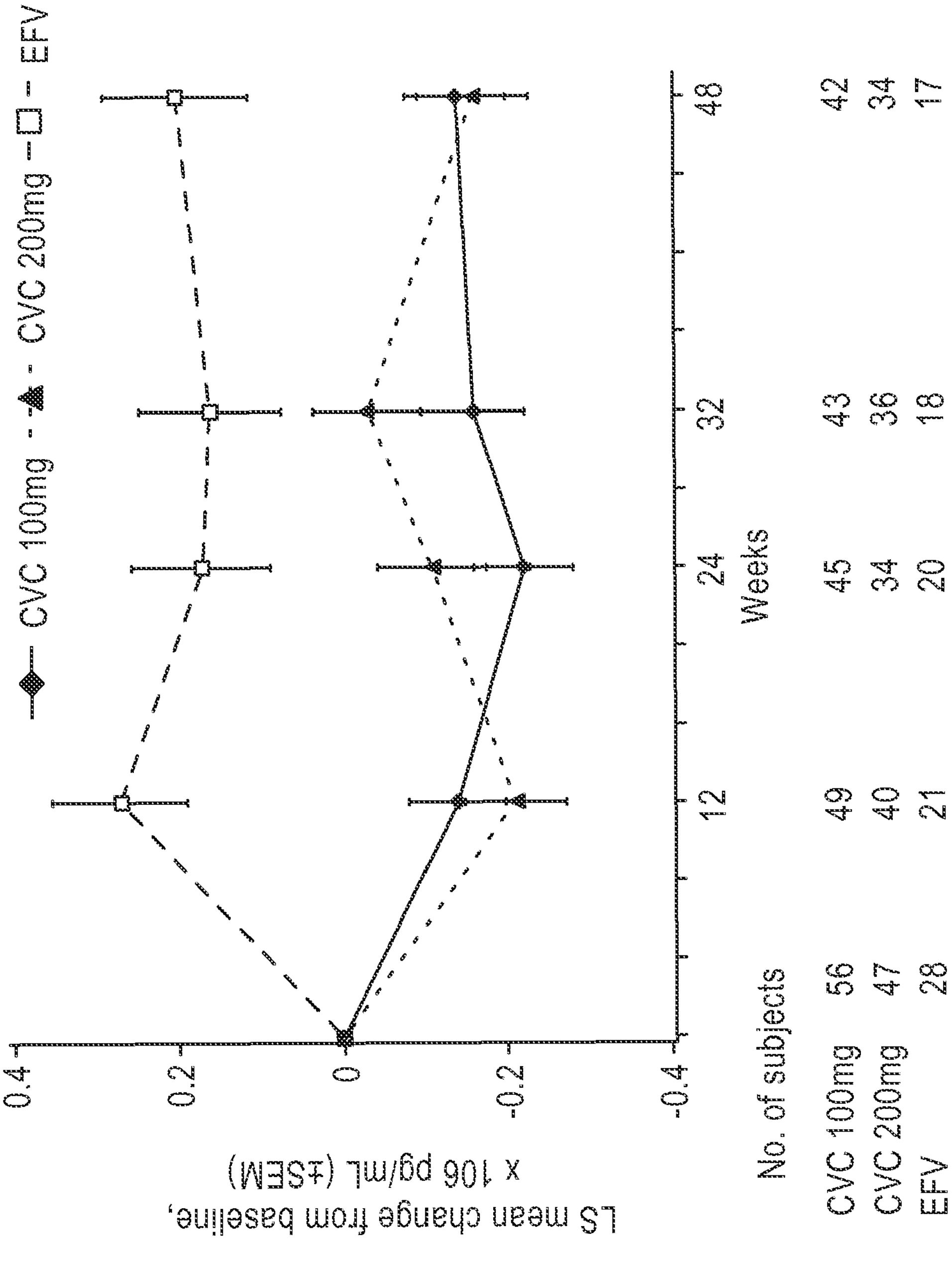
CVC 100mg
CVC 200mg
EFV
LS mean change from baseline, x 106 pg/mL (±SEM)
0.4
0.2
0
-0.2
-0.4
12
24
32
48
Weeks
No. of subjects
CVC 100mg 56 49 45 43 42
CVC 200mg 47 40 34 36 34
EFV 28 21 20 18 17

# Figure 48

APRI

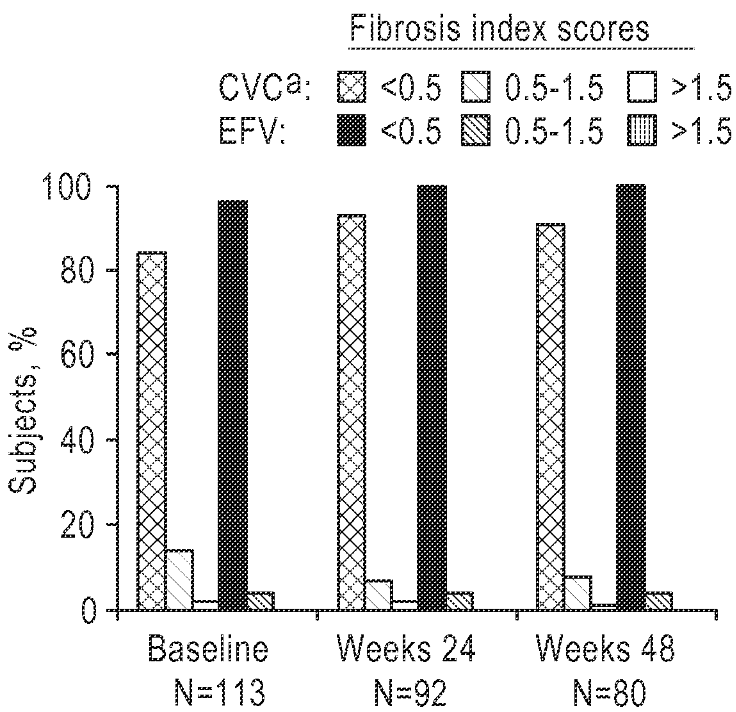


| | Decreased by one category from baseline | | |
|---|---|---|---|
| | Baseline | Week 24 | Week 48 |
| CVC | N/A | 14% | 10% |
| EFV | N/A | 5% | 6% |

FIB-4

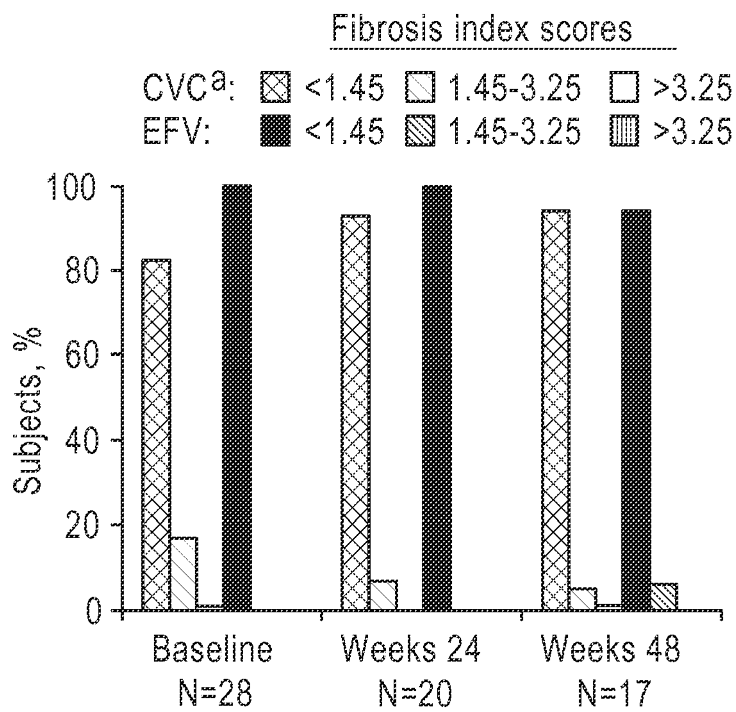


| | Decreased by one category from baseline | | |
|---|---|---|---|
| | Baseline | Week 24 | Week 48 |
| CVC | N/A | 13% | 14% |
| EFV | N/A | - | - |

Note: The proportion of subjects decreasing by one category is shown below the figures.

a CVC 100-mg and 200-mg arms pooled

# Figure 49

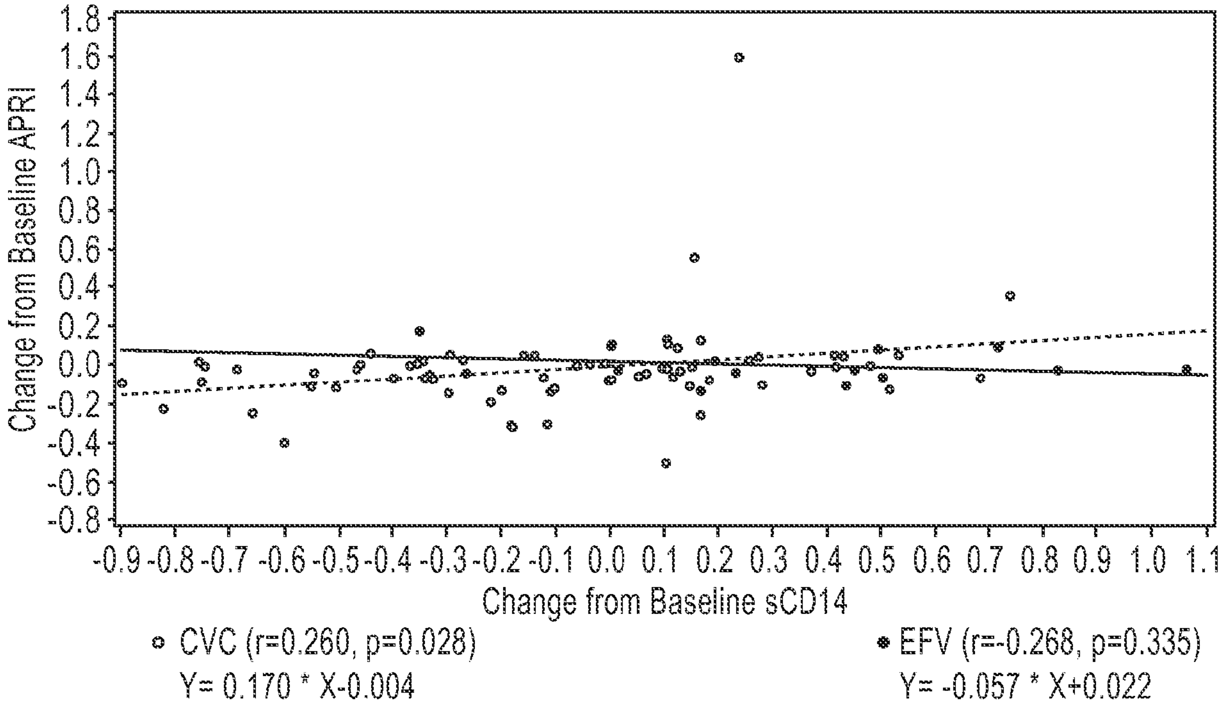


# Figure 50

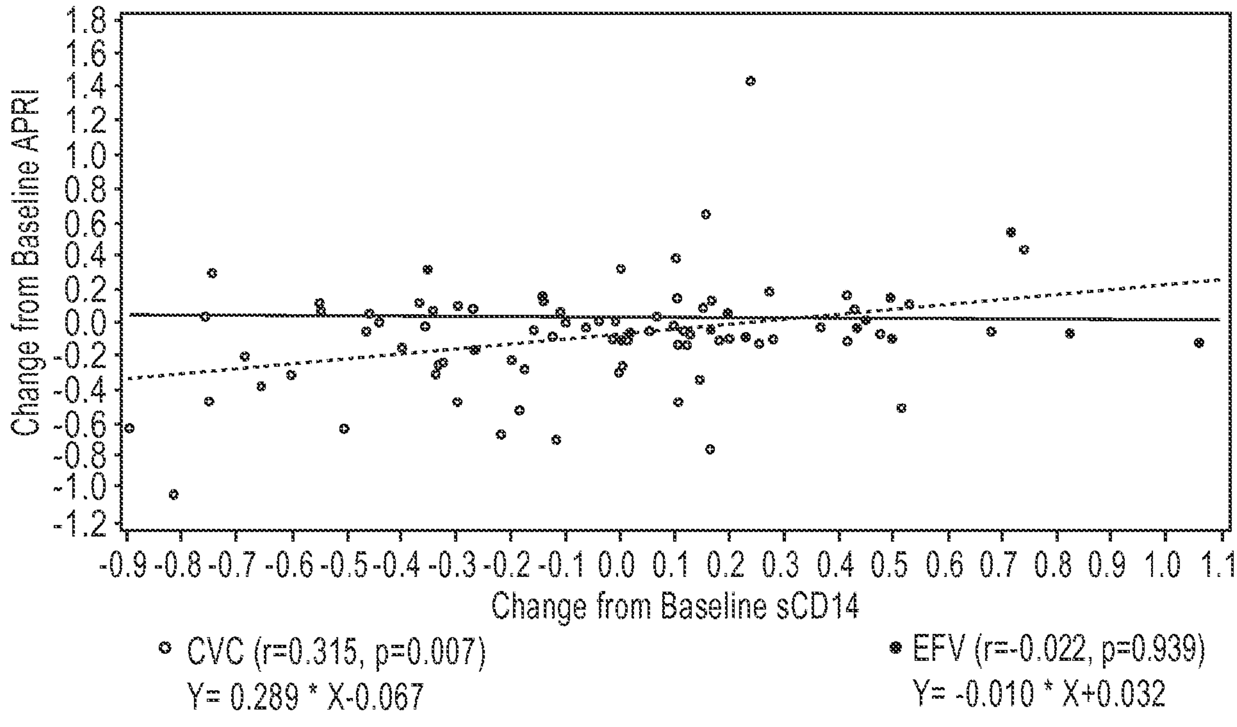

# Figure 51

Creatine Phosphokinase (IU/L)

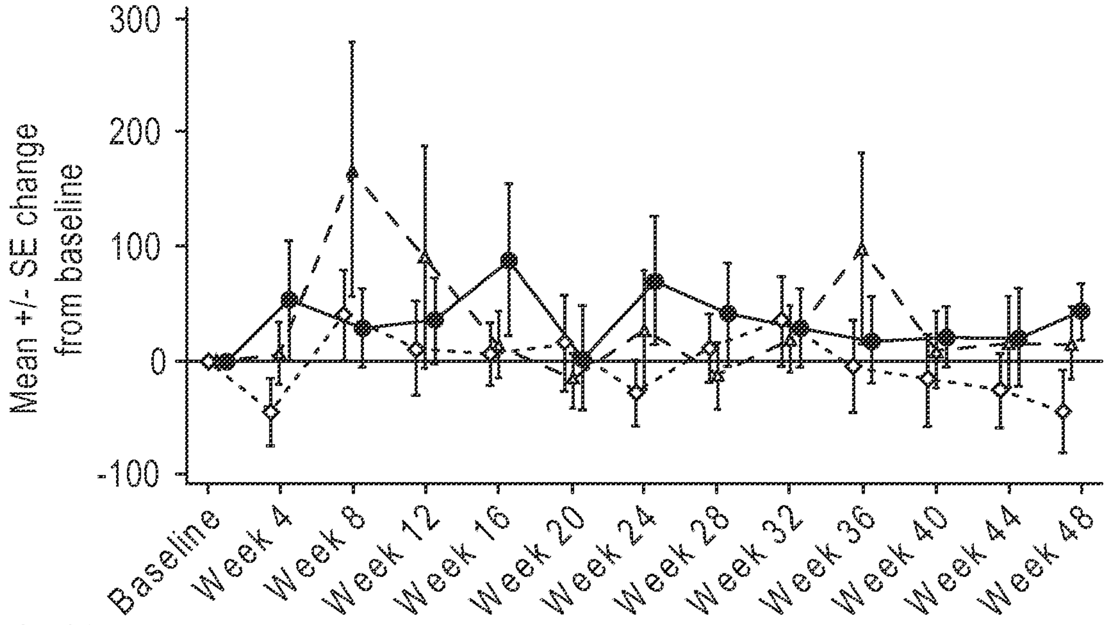


No. of subjects

| | | | | | | | | | | | | | |
|---|---|---|---|---|---|---|---|---|---|---|---|---|---|
| CVC 100 + FTC/TDF | 58 | 54 | 53 | 53 | 55 | 55 | 49 | 50 | 47 | 46 | 47 | 44 | 43 |
| CVC 200 + FTC/TDF | 57 | 54 | 53 | 53 | 51 | 50 | 46 | 46 | 45 | 43 | 42 | 42 | 41 |
| EFV + FTC/TDF | 28 | 24 | 23 | 22 | 21 | 22 | 21 | 21 | 19 | 21 | 21 | 20 | 18 |

Treatment group
CVC 100 + FTC/TDF
CVC 200 + FTC/TDF
EFV + FTC/TDF

*Note:* Values are in IU/mL.

Figure 52

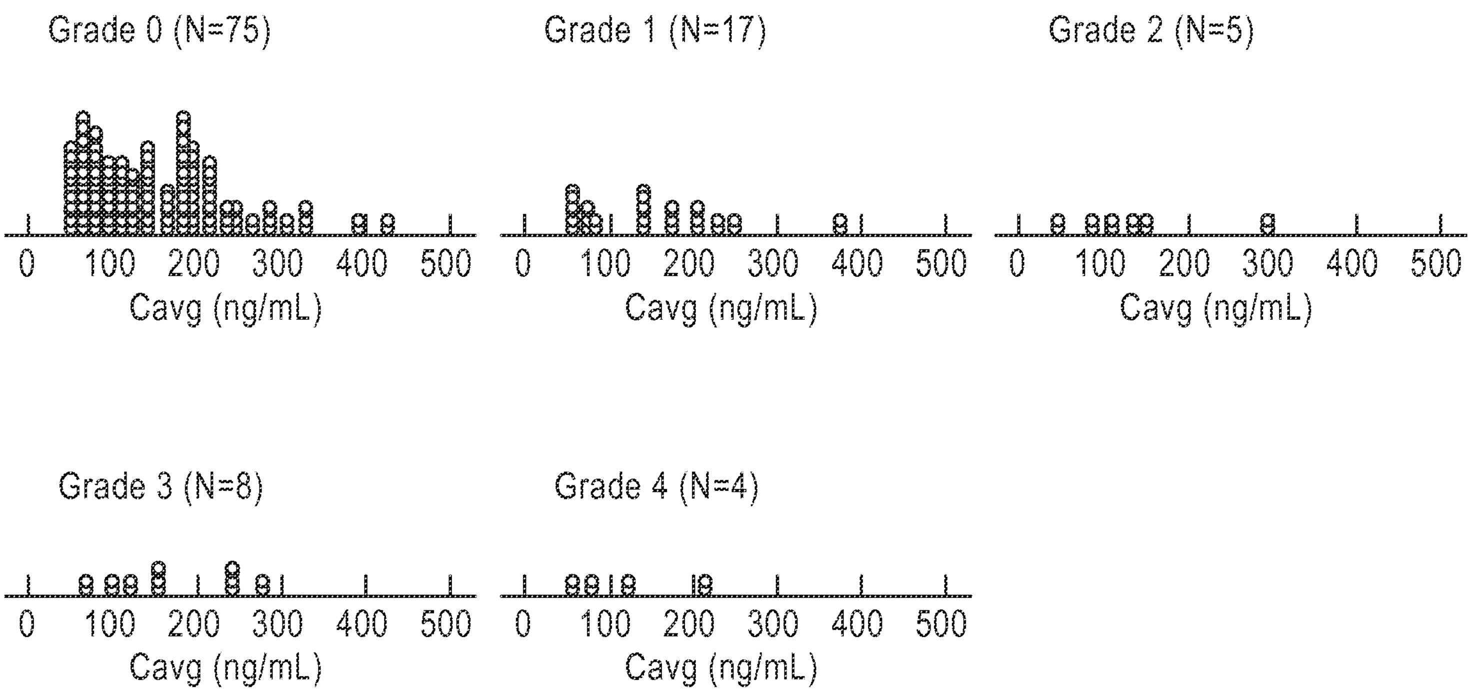
Grade 0 (N=75)
Grade 1 (N=17)
Grade 2 (N=5)
Grade 3 (N=8)
Grade 4 (N=4)
0 100 200 300 400 500
Cavg (ng/mL)

Figure 53

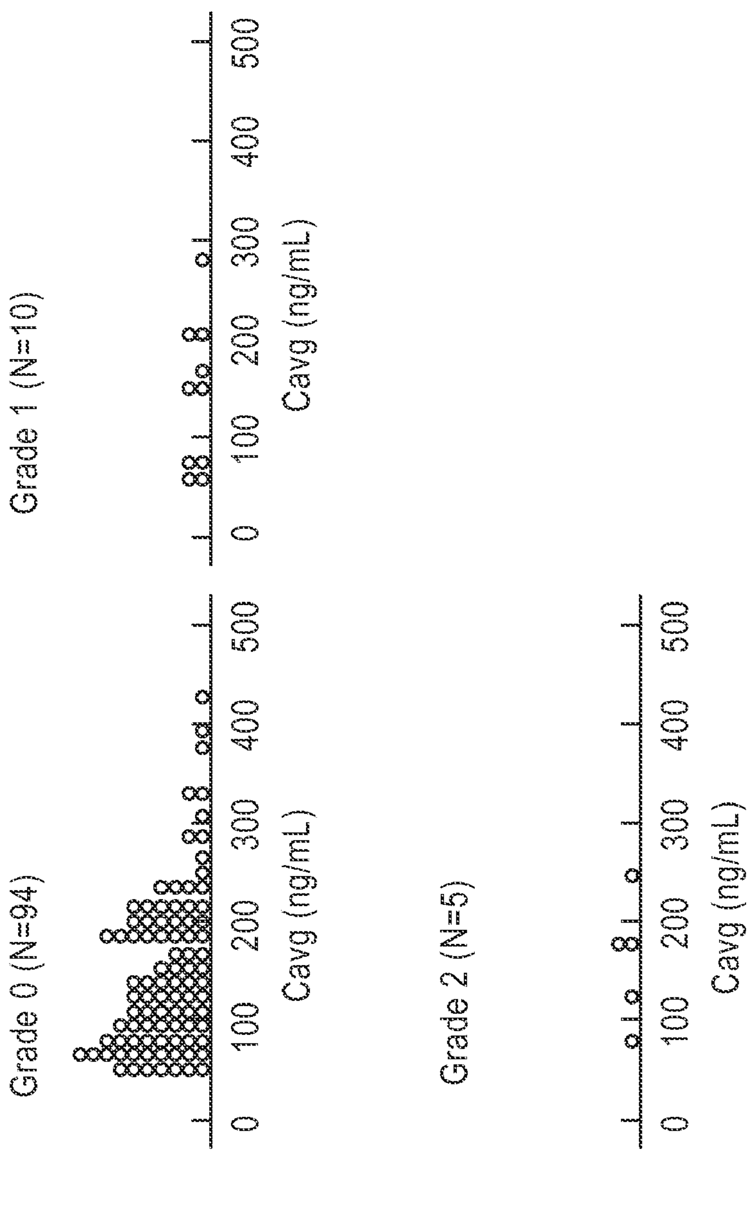
Grade 0 (N=94)
0
100
200
300
400
500
Cavg (ng/mL)
Grade 1 (N=10)
0
100
200
300
400
500
Cavg (ng/mL)
Grade 2 (N=5)
0
100
200
300
400
500
Cavg (ng/mL)

Figure 54

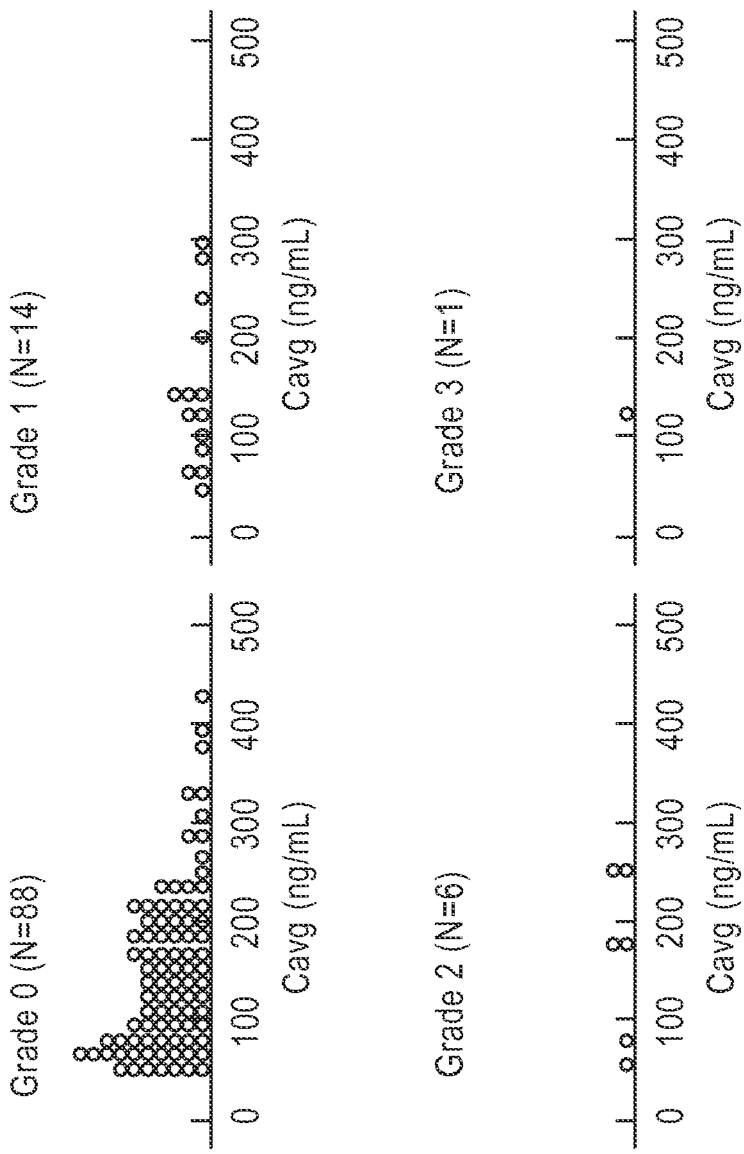

Grade 0 (N=88)
Grade 1 (N=14)
Grade 2 (N=6)
Grade 3 (N=1)
0
100
200
300
400
500
Cavg (ng/mL)

Figure 55

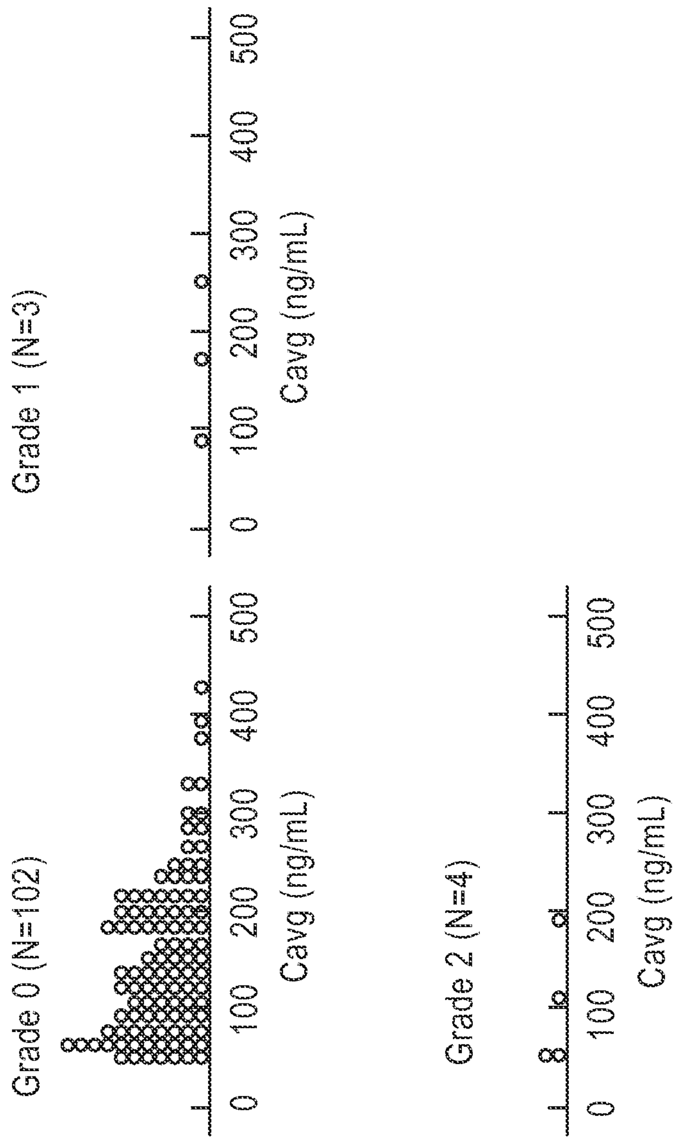
Grade 0 (N=102)
0
100
200
300
400
500
Cavg (ng/mL)
Grade 1 (N=3)
0
100
200
300
400
500
Cavg (ng/mL)
Grade 2 (N=4)
0
100
200
300
400
500
Cavg (ng/mL)

# Figure 56A

**Total Cholesterol (mg/dL) (fasting)**

Cholesterol (mg/dL)

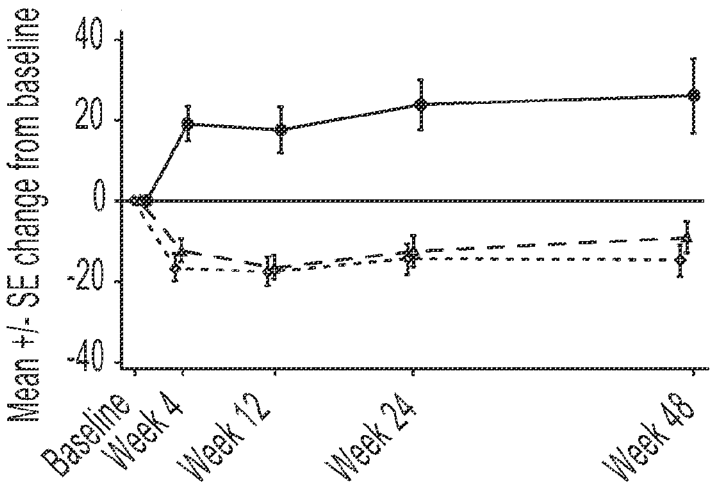


No. of subjects

| | | | | | |
|---|---|---|---|---|---|
| CVC 100 + FTC/TDF | 58 | 51 | 51 | 48 | 43 |
| CVC 200 + FTC/TDF | 56 | 50 | 51 | 45 | 38 |
| EFV + FTC/TDF | 28 | 21 | 22 | 20 | 16 |

Treatment group
CVC 100 mg + FTC/TDF
CVC 200 mg + FTC/TDF
EFV + FTC/TDF

**LDL Cholesterol (mg/dL) (fasting)**

LDL Calc (mg/dL)

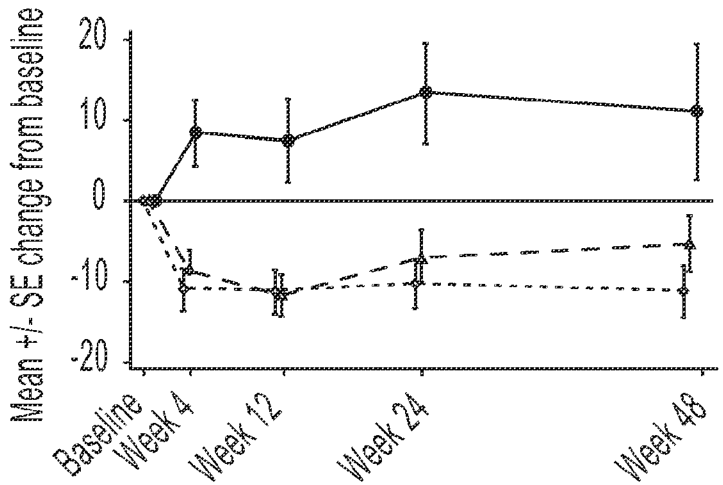


No. of subjects

| | | | | | |
|---|---|---|---|---|---|
| CVC 100 + FTC/TDF | 58 | 51 | 51 | 47 | 43 |
| CVC 200 + FTC/TDF | 54 | 48 | 49 | 43 | 35 |
| EFV + FTC/TDF | 28 | 21 | 22 | 20 | 16 |

Treatment group
CVC 100 + FTC/TDF
CVC 200 + FTC/TDF
EFV + FTC/TDF

Figure 56B

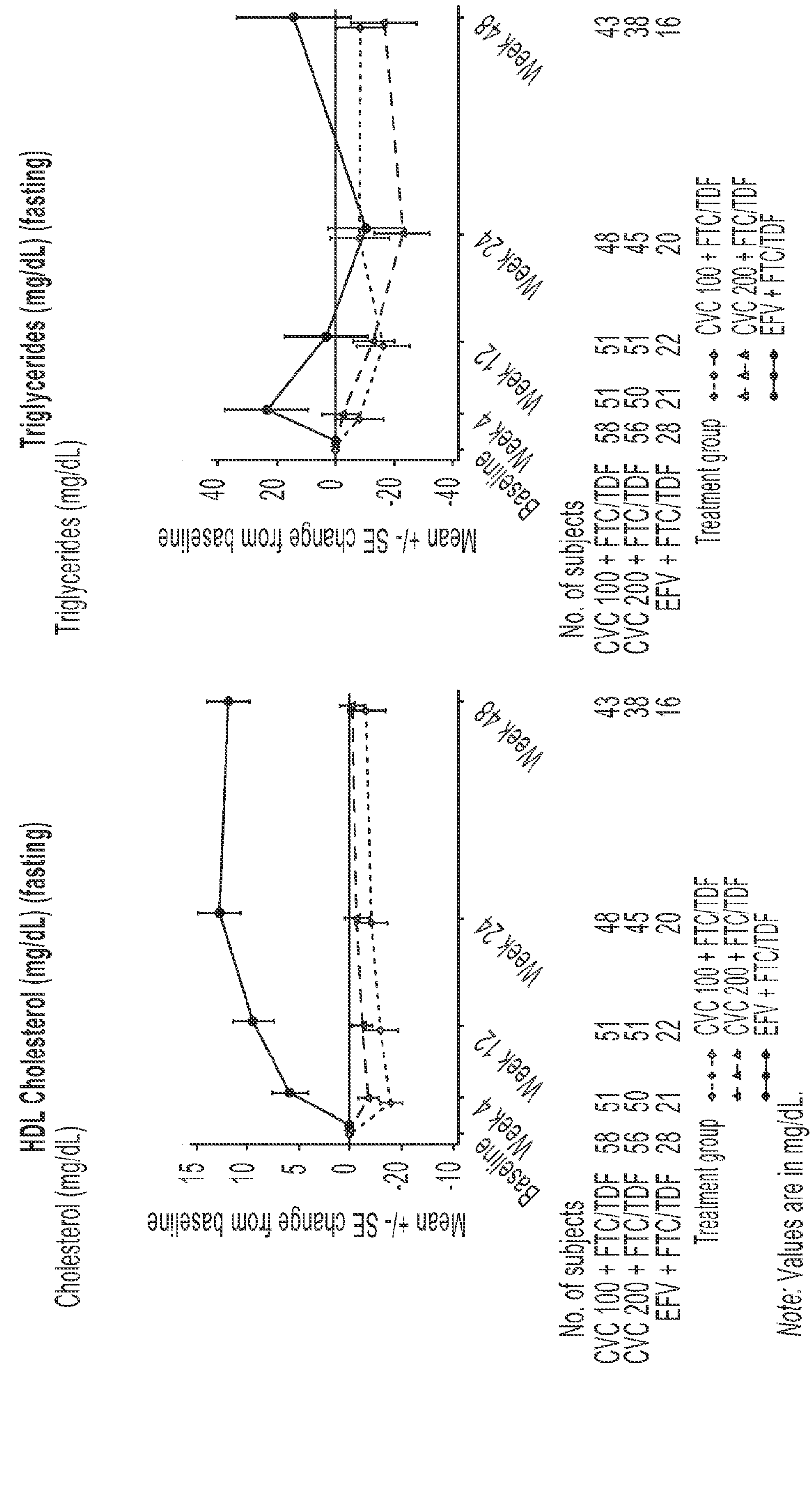

HDL Cholesterol (mg/dL) (fasting)
Cholesterol (mg/dL)
Mean +/- SE change from baseline
15
10
5
0
-20
-10
Baseline
Week 4
Week 12
Week 24
Week 48
No. of subjects
CVC 100 + FTC/TDF 58 51 51 48 43
CVC 200 + FTC/TDF 56 50 51 45 38
EFV + FTC/TDF 28 21 22 20 16
Treatment group
CVC 100 + FTC/TDF
CVC 200 + FTC/TDF
EFV + FTC/TDF
Note: Values are in mg/dL.
Triglycerides (mg/dL) (fasting)
Triglycerides (mg/dL)
Mean +/- SE change from baseline
40
20
0
-20
-40
Baseline
Week 4
Week 12
Week 24
Week 48
No. of subjects
CVC 100 + FTC/TDF 58 51 51 48 43
CVC 200 + FTC/TDF 56 50 51 45 38
EFV + FTC/TDF 28 21 22 20 16
Treatment group
CVC 100 + FTC/TDF
CVC 200 + FTC/TDF
EFV + FTC/TDF

Europäisches Patentamt
European Patent Office
Office européen des brevets

# EUROPEAN SEARCH REPORT

Application Number
EP 21 15 8407

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| Y | LEFEBVRE ET AL: "O16: Anti-fibrotic and anti-inflammatory activity of the dual CCR5 and CCR2 antagonist cenicriviroc in a mouse model of NASH", ANTIVIRAL THERAPY- AN OFFICIAL PUBLICATION OF THE INTERNATIONAL SOCIETY FOR ANTIVIRAL RESEARCH; ABSTRACTS PRESENTED AT THE 15TH INTERNATIONAL WORKSHOP ON CO-MORBIDITIES AND ADVERSE DRUG REACTIONS IN H , vol. 18, no. Suppl. 3 1 January 2013 (2013-01-01), pages A14-A15, XP009501278, ISSN: 1359-6535 Retrieved from the Internet: URL:https://www.intmedpress.com/journals/avt/abstract.cfm?id=2698&pid=88 * the whole document * ----- | 1-13 | INV. A61K31/4178 A61K31/513 A61K31/55 A61K31/4164 A61K31/167 A61P1/16 A61P13/12 |
| A | C REVIRIEGO: "Cenicriviroc Mesilate", DRUGS OF THE FUTURE, 1 January 2011 (2011-01-01), pages 511-517, XP055420530, DOI: 10.1358/dof.2011.36.7.1622066 * page 516, column 1, paragraph 1 * ----- | 1-13 | TECHNICAL FIELDS SEARCHED (IPC) |
| A | WO 2008/040548 A2 (ARES TRADING SA [CH]; BERAZA NAIARA [DE]; DREANO MICHEL [FR]; TRAUTWEI) 10 April 2008 (2008-04-10) * the whole document * ----- -/-- | 1-13 | A61K A61P |

The present search report has been drawn up for all claims

1

| Place of search | Date of completion of the search | Examiner |
|---|---|---|
| The Hague | 3 June 2021 | Collins, Sally |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches Patentamt
European Patent Office
Office européen des brevets

# EUROPEAN SEARCH REPORT

Application Number
EP 21 15 8407

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MARK M. MENNING ET AL: "Fumaric Acid Microenvironment Tablet Formulation and Process Development for Crystalline Cenicriviroc Mesylate, a BCS IV Compound", MOLECULAR PHARMACEUTICS, vol. 10, no. 11, 4 November 2013 (2013-11-04), pages 4005-4015, XP055295392, US ISSN: 1543-8384, DOI: 10.1021/mp400286s * page 4011; table 7 * ----- | 1-13 | |
| Y | LARKIN ET AL: "Lamivudine promising in chronic hepatitis B", THE LANCET, ELSEVIER, AMSTERDAM, NL, vol. 352, no. 9123, 18 July 1998 (1998-07-18), page 207, XP022108302, ISSN: 0140-6736, DOI: 10.1016/S0140-6736(05)77815-6 * the whole document * ----- | 1,2,5-8, 13 | TECHNICAL FIELDS SEARCHED (IPC) |

1

The present search report has been drawn up for all claims

| Place of search | Date of completion of the search | Examiner |
|---|---|---|
| The Hague | 3 June 2021 | Collins, Sally |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 15 8407

This annex lists the patent family members relating to the patent documents cited in the above-mentioned European search report.
The members are as contained in the European Patent Office EDP file on
The European Patent Office is in no way liable for these particulars which are merely given for the purpose of information.

03-06-2021

| Patent document cited in search report | Publication date | Patent family member(s) | Publication date |
|---|---|---|---|
| WO 2008040548 A2 | 10-04-2008 | AU 2007304439 A1 | 10-04-2008 |
| | | CA 2664413 A1 | 10-04-2008 |
| | | EP 2157975 A2 | 03-03-2010 |
| | | JP 2010505783 A | 25-02-2010 |
| | | US 2008194575 A1 | 14-08-2008 |
| | | WO 2008040548 A2 | 10-04-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20120232028
- US 61823766 B
- US 2008031942


### Non-patent literature cited in the description


- **SAIMAN Y ; FRIEDMAN SL.** *The role of chemokines in acute liver injury,* 2012, vol. 3, 213
- **ZIMMERMANN HW ; TACKE F.** Modification of chemokine pathways and immune cell infiltration as a novel therapeutic approach in liver inflammation and fibrosis. *Inflamm Allergy Drug Targets,* 2011, vol. 10, 509-536
- **SEKI E ; DE MINICIS S ; GWAK GY ; KLUWE J ; INOKUCHI S ; BURSILL CA ; LLOVET JM ; BRENNER DA ; SCHWABE RF.** CCR1 and CCR5 promote hepatic fibrosis in mice. *J Clin Invest.,* 2009, vol. 119, 1858-1870
- **SEKI E ; DE MINICIS S ; INOKUCHI S ; TAURA K ; MIYAI K ; VAN ROOIJEN N ; SCHWABE RF ; BRENNER DA.** CCR2 promotes hepatic fibrosis in mice. *Hepatology,* 2009, vol. 50, 185-197
- **MIURA K ; YANG L ; VAN ROOIJEN N ; OHNISHI H ; SEKI E.** Hepatic recruitment of macrophages promotes nonalcoholic steatohepatitis through CCR2. *Am J Physiol Gastrointest Liver Physiol.,* 2012, vol. 302, G1310-1331
- **MITCHELL C ; COUTON D ; COUTY JP ; ANSON M ; CRAIN AM ; BIZET V ; RÉNIA L ; POL S ; MALLET V ; GILGENKRANTZ H.** Dual role of CCR2 in the constitution and the resolution of liver fibrosis in mice. *Am J Pathol.,* 2009, vol. 174, 1766-1775
- **XIA Y ; ENTMAN ML ; WANG Y.** CCR2 regulates the uptake of bone marrow-derived fibroblasts in renal fibrosis. *PLoS ONE,* 2013, vol. 8 (10), e77493
- **KARLMARK KR ; WASMUTH HE ; TRAUTWEIN C ; TACKE F.** Chemokine-directed immune cell infiltration in acute and chronic liver disease. *Expert Review Gastroenterology Hepatology,* 2008, vol. 2, 233-242
- **VIELHAUER V ; ANDERS H-J ; MACK M ; CIHAK J ; STRUTZ F ; STANGASSINGER M ; LUCKOW B ; GRÖNE H-J ; SCHLÖNDORFF D.** Obstructive nephropathy in the mouse: Progressive fibrosis correlates with tubulointerstitial chemokine expression and accumulation of CC chemokine receptor 2- and 5-positive leukocytes. *J Am Soc Nephrol,* 2001, vol. 12, 1173-1187
- **SEGERER S ; MACK M ; REGELE H ; KERJASCHKI D ; SCHLÖNDORFF D.** Expression of the C-C chemokine receptor 5 in human kidney disease. *Kidney Int,* 1999, vol. 56, 52-64
- **WAI C-T ; GREENSON J ; FONTANA R ; KALBFLEISCH J ; MARRERO J ; CONJEEVARAM H ; LOK A.** A simple noninvasive index can predict both significant fibrosis and cirrhosis in patients with chronic hepatitis C. *Hepatology,* 2003, vol. 38, 518-526
- **VALLET-PICHARD A ; MALLET V ; NALPAS B ; VERKARRE V ; NALPAS A ; DHALLUIN-VENIER V ; FONTAINE H ; POL S.** FIB-4: an inexpensive and accurate marker of fibrosis in HCV infection. Comparison with liver biopsy and FibroTest. *Hepatology,* 2007, vol. 46, 32-36
- **SANDLER NG ; WAND H ; ROQUE A et al.** Plasma levels of soluble CD14 independently predict mortality in HIV infection. *JID,* 2011, vol. 203, 780-790
- **BRENCHLEY J ; PRICE DA ; SCHACKER TW ; ASHER TE ; SILVESTRI G ; RAO S ; KAZZAZ Z ; BOMSTEIN E ; LAMBOTTE O ; ALTMANN D.** Microbial translocation is a cause of systemic immune activation in chronic HIV infection. *Nature Medicine,* 2006, vol. 12, 1365-1371
- **VAJRO P ; PAOLELLA G ; FASANO A.** *Microbiota and Gut-Liver Axis: Their Influences on Obesity and Obesity-Related Liver Disease JPGN,* 2013, vol. 56, 461-468
- **ROH YS ; SEKI E.** Toll-like receptors in alcoholic liver disease, non-alcoholic steatohepatitis and carcinogenesis. *Journal of Gastroenterology and Hepatology,* 2013, vol. 28, 38-42
- **ILAN Y.** Leaky gut and the liver: A role for bacterial translocation in nonalcoholic steatohepatitis. *World J Gastroenterol,* 07 June 2012, vol. 18, 2609-2618
- **PETRASEK J ; MANDREKAR P ; SZABO G.** Toll-Like Receptors in the Pathogenesis of Alcoholic Liver Disease. *Gastroenterology Research and Practice,* 2010, 12

- **SANDLER N ; KOH C ; ROQUE A ; ECCLESTON J ; SIEGEL R ; DEMINO M ; KLEINER D ; DEEKS S ; LIANG T-J ; HELLER T.** Host Response to Translocated Microbial Products Predicts Outcomes of Patients With HBV or HCV Infection. *Gastroenterology,* 2011, vol. 141, 1220-1230
- **WIEST R ; LAWSON M ; GEUKING M.** Pathological bacterial translocation in liver cirrhosis. *J Hepatology,* 2014, vol. 60 (1), 197-209
- **SHANAB AA ; SCULLY P ; CROSBIE O ; BUCKLEY M ; O'MAHONY L ; SHANAHAN F ; GAZAREEN S ; MURPHY E ; QUIGLEY EM.** Small intestinal bacterial overgrowth in nonalcoholic steatohepatitis: association with toll-like receptor 4 expression and plasma levels of interleukin 8. *Dig Dis Sci,* 2011, vol. 56, 1524-1534
- **HENAO-MEJIA J ; ELINAV E ; JIN C ; HAO L ; MEHAL WZ ; STROWIG T ; THAISS CA ; KAU AL ; EISENBARTH SC ; JURCZAK MJ.** Inflammasome-mediated dysbiosis regulates progression of NAFLD and obesity. *Nature,* 2012, vol. 492, 179-185
- **KLIBANOV, OLGA M. ; WILLIAMS, SHANNON H. ; ILER, CAMERON A.** Cenicriviroc, an orally active CCR5 antagonist for the potential treatment of HIV infection. *Current Opinion in Investigational Drugs,* 2010, vol. 11 (8), 940-950
- **KLEINER DE et al.** Design and validation of a histological scoring system for nonalcoholic fatty liver disease. *Hepatology,* 2005, vol. 41, 1313-1321
- **FUJII H et al.** *J. Atheroscler. Thromb.,* 2009, vol. 16, 893
- **RANGWALA F et al.** *J. Pathol.,* 2011, vol. 224, 401
- **SEKI et al.** CCR1 and CCR5 promote hepatic fibrosis in mice. *J. Clin. Invest.,* 2009, vol. 119 (7), 1858-1870
- **XU et al.** Liver fibrosis: mechanisms of immune-mediated liver injury. *Cellular & Mol. Immunol.,* 2012, vol. 9, 296-301
- **KARLMARK et al.** *Expert Rev. Gastroenterol. Hepatol.,* 2008, vol. 2 (2), 233-242
- **MITCHELL C et al.** Dual role of CCR2 in the constitution and the resolution of liver fibrosis in mice. *Am J Pathol.,* 2009, vol. 174, 1766-1775
- **BERRES M-L et al.** Antagonism of the chemokine Ccl5 ameliorates experimental liver fibrosis in mice. *Journal of Clin Invest.,* November 2010, vol. 120 (11), 4129-4140
- **BENYON, RC ; MJ ARTHUR.** Extracellular matrix degradation and the role of hepatic stellate cells. *Semin Liver Dis.,* August 2001, vol. 21 (3), 373-84
- **SHETH SG ; CHOPRA S.** *Natural history and management of nonalcoholic fatty liver disease in adults,* 2014
- **CHALASANI N ; YOUNOSSI Z ; LAVINE JE ; DIEHL AM ; BRUNT EM ; CUSI K et al.** The diagnosis and management of non-alcoholic fatty liver disease: Practice Guideline by the American Association for the Study of Liver Diseases, American College of Gastroenterology, and the American Gastroenterological Association. *Hepatology,* 2012, vol. 55, 2005-23
- **MCCULLOUGH AJ.** The clinical features, diagnosis and natural history of nonalcoholic fatty liver disease. *Clin Liver Dis,* 2004, vol. 8 (3), 521-33
- **LOOMBA R ; SIRLIN CB ; SCHWIMMER JB ; LAVINE JE.** Advances in pediatric nonalcoholic fatty liver disease. *Hepatology,* 2009, vol. 50 (4), 1282-93
- **TORRES DM ; WILLIAMS CD ; HARRISON SA.** Features, diagnosis, and treatment of nonalcoholic fatty liver disease. *Clin Gastroenterol Hepatol,* 2012, vol. 10 (8), 837-58
- **SCHWENGER KJ ; ALLARD JP.** Clinical approaches to non-alcoholic fatty liver disease. *World J Gastroenterol,* 2014, vol. 20 (7), 1712-23
- **KOO SH.** Nonalcoholic fatty liver disease: molecular mechanisms for the hepatic steatosis. *Clin Mol Hepatol,* 2013, vol. 19 (3), 210-5
- **ATTAR BM ; VAN THIEL DH.** Current concepts and management approaches in nonalcoholic fatty liver disease. *ScientificWorld Journal,* 2013, vol. 2013, 481893
- **BASARANOGLU M ; BASARANOGLU G ; SENTÜRK H.** From fatty liver to fibrosis: a tale of "second hit. *World J Gastroenterol,* 2013, vol. 19 (8), 1158-65
- **COHEN JC ; HORTON JD ; HOBBS HH.** Human fatty liver disease: old questions and new insights. *Science,* 2011, vol. 332 (6037), 1519-23
- **MATTEONI CA ; YOUNOSSI ZA ; GRAMLICH T et al.** Nonalcoholic fatty liver disease: A spectrum of clinical and pathological severity. *Gastroenterology,* 1999, vol. 116 (6), 1413-1419
- World Gastroenterology Organisation Global Guidelines. *Nonalcoholic Fatty liver Disease and Nonalcoholic Steatohepatitis,* June 2012
- **AHMED MH ; ABU EO ; BYRNE CD.** Non-Alcoholic Fatty Liver Disease (NAFLD): new challenge for general practitioners and important burden for health authorities?. *Prim Care Diabetes,* 2010, vol. 4, 129-37
- **VERNON G ; BARANOVA A ; YOUNOSSI ZM.** Systematic review: the epidemiology and natural history of non-alcoholic fatty liver disease and nonalcoholic steatohepatitis in adults. *Aliment Pharmacol Ther,* 2011, vol. 34, 274-85
- **DIXON JB ; BHATHAL PS ; O'BRIEN PE.** Nonalcoholic fatty liver disease: predictors of nonalcoholic steatohepatitis and liver fibrosis in the severely obese. *Gastroenterol,* 2001, vol. 121, 91-100

- **WILLIAMS CD ; STENGER J ; ASIKE MI ; TORRES DM ; SHAW J ; CONTRERAS M et al.** Prevalence of nonalcoholic fatty liver disease and nonalcoholic steatohepatitis among a largely middle-aged population utilizing ultrasound and liver biopsy: a prospective study. *Gastroenterology,* 2011, vol. 140, 124-131
- **SCHATTENBERG JM ; SCHUPPAN D.** Nonalcoholic steatohepatitis: the therapeutic challenge of a global epidemic. *Curr Opin Lipidol,* 2011, vol. 22, 479-88
- **BAHRAMI H.** Nonalcoholic fatty liver disease in developing countries. *World J Gastroenterol,* 2005, vol. 11, 3808-3809
- **TINIAKOS DG ; VOS MB ; BRUNT EM.** Nonalcoholic fatty liver disease: pathology and pathogenesis. *Annu Rev Pathol,* 2010, vol. 5, 145-71
- **VAJRO P ; PAOLELLA G ; FASANO A.** Microbiota and gut-liver axis: their influences on obesity and obesity-related liver disease. *J Pediatr Gastroenterol Nutr,* 2013, vol. 56, 461-8
- **ROH YS ; SEKI E.** Toll-like receptors in alcoholic liver disease, non-alcoholic steatohepatitis and carcinogenesis. *J Gastroenterol Hepatol,* 2013, vol. 28 (1), 38-42
- **ILAN Y.** Leaky gut and the liver: a role for bacterial translocation in nonalcoholic steatohepatitis. *World J Gastroenterol,* 2012, vol. 18, 2609-18
- **MOSCHEN AR ; KASER S ; TILG H.** Non-alcoholic steatohepatitis: a microbiota-driven disease. *Trends Endocrinol Metab,* 2013, vol. 24, 537-45
- **SANYAL AJ ; CAMPBELL-SARGENT C ; MIRSHAHI F ; RIZZO WB ; CONTOS MJ ; STERLING RK et al.** Nonalcoholic steatohepatitis: association of insulin resistance and mitochondrial abnormalities. *Gastroenterology,* 2001, vol. 120, 1183-92
- **MCCLAIN CJ ; MOKSHAGUNDAM SP ; BARVE SS ; SONG Z ; HILL DB ; CHEN T et al.** Mechanisms of non-alcoholic steatohepatitis. *Alcohol,* 2004, vol. 34, 67-79
- **DAY CP ; SAKSENA S.** Non-alcoholic steatohepatitis: definitions and pathogenesis. *J Gastroenterol Hepatol.,* 2002, vol. 17 (3), S377-S84
- **MARRA F ; GASTALDELLI A ; SVEGLIATI BARONI G ; TELL G ; TIRIBELLI C.** Molecular basis and mechanisms of progression of non-alcoholic steatohepatitis. *Trends Mol Med.,* 2008, vol. 14, 72-81
- **CHARLTON MR ; BURNS JM ; PEDERSON RA ; WATT KD ; HEIMBACH JK ; DIERKHISING RA.** Frequency and outcomes of liver transplantation for nonalcoholic steatohepatitis in the United States. *Gastroenterol,* 2011, vol. 141, 1249-53
- **VATCHE G. AGOPIAN et al.** Liver Transplantation for Nonalcoholic Steatohepatitis. *Annals of Surgery,* 2012, vol. 256 (4), 624-633
- **MCCULLOUGH AJ.** Epidemiology of the metabolic syndrome in the USA. *J Dig Dis.,* 2011, vol. 12, 333-40

SEQUENCE LISTING

<110> TOBIRA THERAPEUTICS, INC.

<120> CENICRIVIROC FOR THE TREATMENT OF FIBROSIS

<130> N413250EP-A

<140> EP 21158407.3
<141> 2015-03-20

<150> US 61/968,829
<151> 2014-03-21

<150> US 62/024,713
<151> 2014-07-15

<150> US 62/114,304
<151> 2015-02-10

<160> 7

<170> PatentIn version 3.5

<210> 1
<211> 22
<212> DNA
<213> Artificial Sequence

<220>
<223> primer

<400> 1
aactagggaa cccactgctt aa 22

<210> 2
<211> 26
<212> DNA
<213> Artificial Sequence

<220>
<223> primer

<400> 2
tgagggatct ctagttacca gagtca 26

<210> 3
<211> 30
<212> DNA
<213> Artificial Sequence

<220>
<223> probe

<220>
<221> misc_feature
<222> (1)..(1)
<223> 5′ FAM

<400> 3

cctcaataaa gcttgccttg agtgcttcaa 30

<210> 4
<211> 19
<212> DNA
<213> Artificial Sequence

<220>
<223> primer

<400> 4
cgagtcctgc gtcgagaga 19

<210> 5
<211> 20
<212> DNA
<213> Artificial Sequence

<220>
<223> primer

<400> 5
accgggaagg aaatgaatgg 20

<210> 6
<211> 19
<212> DNA
<213> Artificial Sequence

<220>
<223> primer

<400> 6
gcaggagcgc agggttagt 19

<210> 7
<211> 23
<212> DNA
<213> Artificial Sequence

<220>
<223> probe

<220>
<221> misc_feature
<222> (1)..(1)
<223> 5'VIC

<400> 7
accggcaggc tttcctaacg gct 23